# EUROPEAN PATENT SPECIFICATION

(11) **EP 1 604 647 B1**
(45) Date of publication and mention of the grant of the patent: **07.05.2008**
(21) Application number: 05010234.2
(22) Date of filing: 11.05.2005
(51) Int. Cl.: A61K 8/896, A61Q 19/00, A61Q 1/02, A61Q 5/02, A61K 9/00, A61Q 19/10, A61Q 1/04, A61Q 5/00, A61Q 1/10, A61Q 5/06

(54) **Cosmetic composition containing polyorganosiloxane-containing epsilon-polylysine polymer, and polyhydric alcohol, and production thereof**
Kosmetische Zusammensetzung enthaltend ein Polymer aus Polyorganosiloxan und epsilon-Polylysin, und ein Polyol, und ihre Herstellung
Composition cosmétique comprenant un polymère à base de polyorganosiloxane et d' epsilon-polylysine, ainsi qu'un polyol, et sa fabrication

(30) Priority: 12.05.2004 JP 2004141778
(43) Date of publication of application: 14.12.2005
(73) Proprietor: Chisso Corporation, Osaka-shi, Osaka-fu 530-0005 (JP)
(72) Inventor: Kawasaki, Yuji, Ibi-gun Gifu 501-0521 (JP); Hori, Michimasa, Gifu-shi Gifu 500-8286 (JP); Yamamoto, Yuichi, 5-1 Goikaigan Ichiharashi Chiba 290-8551 (JP); Hiraki, Jun, Tokyo 104-8555 (JP)
(74) Representative: HOFFMANN EITLE

(56) References cited:
- EP-A- 1 477 512
- JP-A- 7 228 508
- JP-A- 2004 161 820
- JP-A- 2005 002 264

## Description

### [Technical Field]

The present invention relates to a cosmetic composition comprising a polyorganosiloxane-containing epsilon-polylysine compound or a physiologically acceptable salt thereof and polyhydric alcohol, and a method of producing such a compound.

### [Background Art]

More and more people are sensitive to chemical substances contained in cosmetics these days. To that end, a safe antibacterial preservative agent that exhibits enough antibacterial preservative effects has been desired in the cosmetic industries. It has also been desired to reduce the content of any antibacterial preservative agents used.

Epsilon-polylysine, which is a kind of antimicrobial agent to be contained in cosmetics in order to preserve the product itself or to provide antimicrobial/antibacterial effects, is safe to the skin and is stable with time after compounded in cosmetics. In addition, it exhibits good preservative and sterilization effects and is reported to be very useful as a preservative/sterilization agent for cosmetics [e.g., Japanese Patent Publication No. 05-6460 (Patent Reference 1)]. Epsilon-polylysine is an amino acid homopolymer with a significantly low volatility, so that the intended preservative/sterilization effects are less likely to be lost due to evaporation of epsilon-polylysine.

In addition, epsilon-polylysine is highly soluble to common cosmetic bases, i.e., water or some alcoholic solvents because of its good water solubility, as well as has a high affinity for emulsion-type cosmetics, in particular, for silicone such as polyorganosiloxane that is a base widely used for emulsion-type cosmetics. Thus, epsilon-polylysine can hardly form a uniform mixture with polyorganosiloxane. This means the necessity of, for example, a surfactant to be added.
[Patent Reference 1] Japanese Patent Publication No. 05-64608
[Patent Reference 2] Japanese Patent No. 1245361

### [Problems to be solved by the Invention]

Under the aforementioned circumstances, it has been desired to develop a highly preservative and antibacterial cosmetic composition that can easily be applied to both emulsion and non-emulsion type cosmetics. It has also been desired to develop a method of improving a preservative and/or antibacterial effect(s) of a cosmetic composition comprising polyorganosiloxane-containing epsilon-polylysine and thereby reducing the amount of antibacterial preservative agent to be used.

### Summary of the Invention

A first aspect of the present invention is a cosmetic composition comprising one or a combination of two or more of polyorganosiloxane-containing epsilon-polylysine compounds or a physiologically acceptable salt thereof, and polyhydric alcohol.
Furthermore, a first aspect of the present invention is a cosmetic composition comprising one or a combination of two or more of polyorganosiloxane-containing epsilon-polylysine compounds represented by the general formula (1): 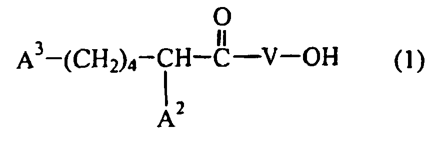 [in the general formula (1), ***V*** represents a random copolymer in which a first repeating unit represented by the general formula (5) : 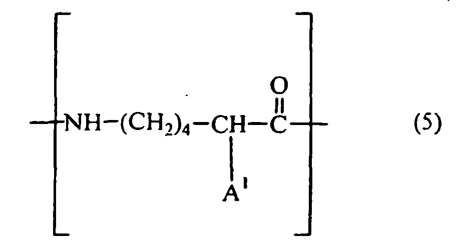 is randomly polymerized with a second repeating unit represented by the general formula (6): 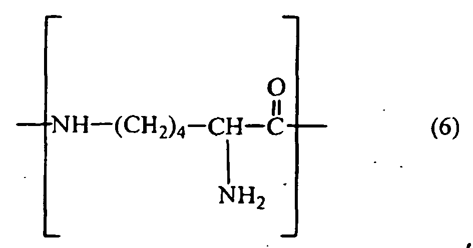 an alternating copolymer in which the first repeating unit alternates with the second repeating unit, or a block copolymer in which a block consisting only of the first repeating units is connected to another block consisting only of the second repeating units, the number of repeats ***a*** of the first repeating unit being an integer of 0 to 50, the number of repeats ***b*** of the second repeating unit being an integer of 0 to 50, ***a*** + ***b*** being equal to an integer of 1 to 50, A¹ represents a group represented by the general formula (2):

-X-Y-Z (2)

{in the general formula (2), ***X*** represents 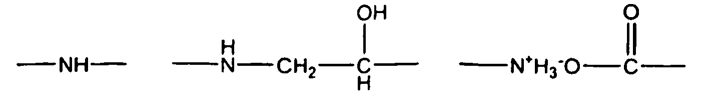 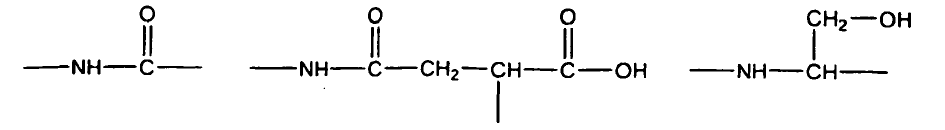 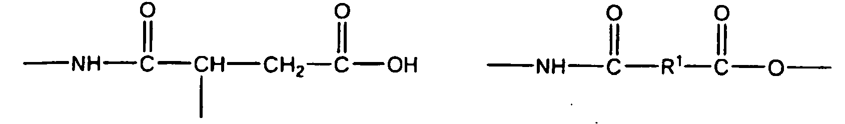 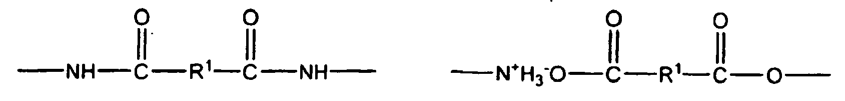 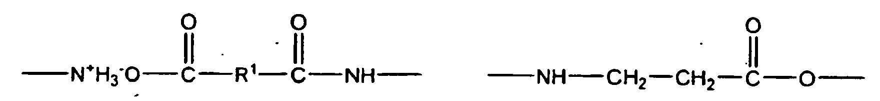 or 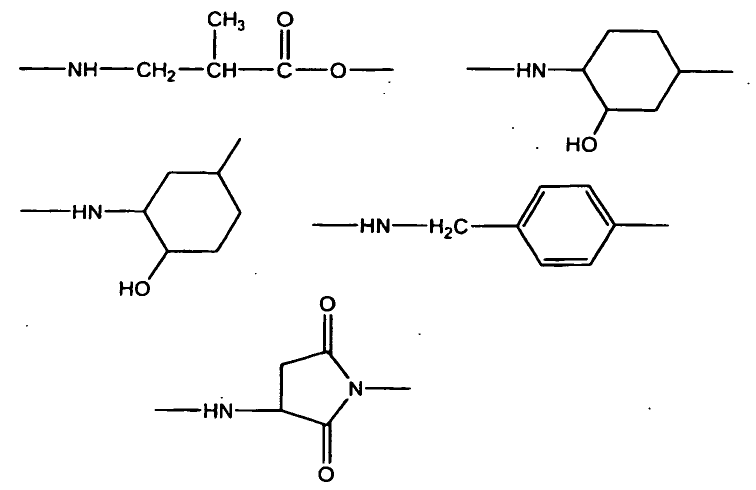 (wherein, R¹ represents a linear or branched alkylene group having 1 to 5 carbon atoms, an alkenylene group having 2 to 5 carbon atoms or an arylene group having 6 to 10 carbon atoms), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4): 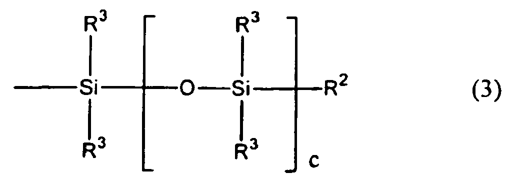 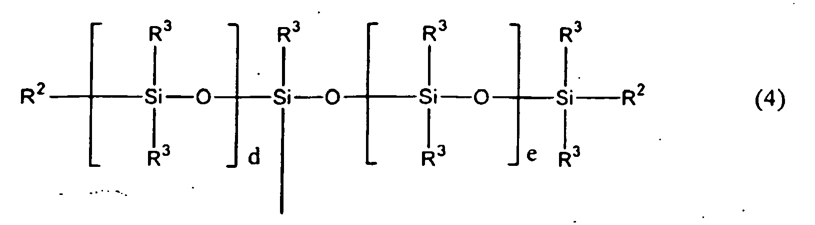 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)}, and A² and A³ are each independently an amino group or a group represented by.the aforementioned general formula (2), A² and A³ are not both amino group when ***a*** is equal to 0] or a physiologically acceptable salt thereof, and polyhydric alcohol.
It is preferable that the number of repeats ***a*** of said first repeating unit be an integer of 0 to 20, the number of repeats ***b*** of the second repeating unit be an integer of 20 to 40, ***a*** + ***b*** be 10 to 40. It is further preferable that ***a*** + ***b*** be 30 to 40.
It is particularly preferable that ***X*** in the general formula (2) be 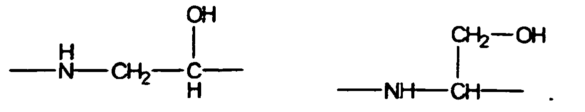 Furthermore, ***Z*** in the general formula (2), it is preferable that R² and R³ be each independently an alkyl group having 1 to 10 carbon atoms or an aryl group having 6 to 10 carbon atoms, and more preferably, be an alkyl group having 1 to 5 carbon atoms or a phenyl group. Of these, the most preferable ones are a methyl group and a n-butyl group.

In the cosmetic compositions according to the first aspect, it is preferable that polyorganosiloxane-containing epsilon-polylysine compounds or a physiologically acceptable salt thereof, in which the number of repeats ***b*** of the second repeating unit is 5 or greater, the second repeating unit being represented by the general formula (6): 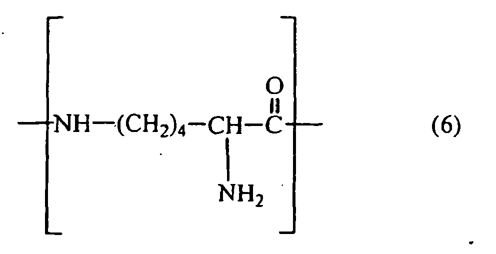 In the cosmetic compositions according to the first aspect, it is preferable that the number of repeats ***a*** of the first repeating unit and the number of repeats ***b*** of the second.repeating unit satisfy the inequality 0.1 ≦***b***/(***a*** + ***b***) ≦ 0.98, the first repeating unit being represented by the general formula (5): 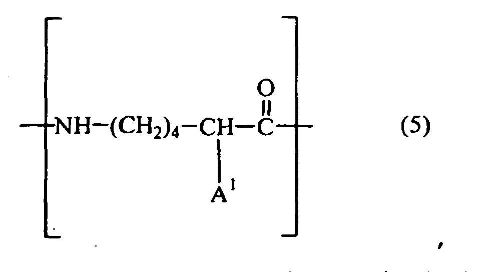 the second repeating unit being represented by the general formula (6): 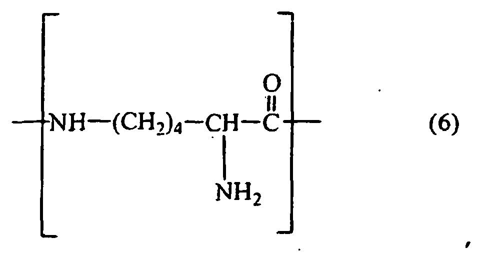 the first and second repeating units forming the polyorganosiloxane-containing epsilon-polylysine compound.

In the cosmetic compositions according to the first aspect, it is preferable that ***X*** in the group represented by the general formula (2):

-X-Y-Z (2)

forming the polyorganosiloxane-containing epsilon-polylysine compound is a group represented by: 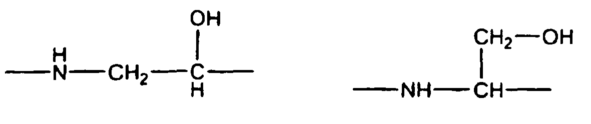 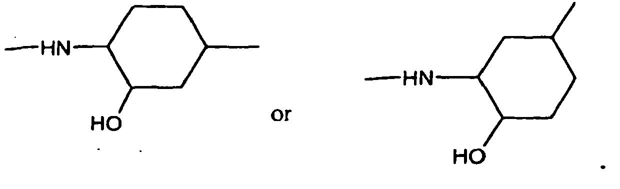 In the cosmetic compositions according to the first aspect, it is preferable that ***X*** in the group represented by the general formula (2):

-X-Y-Z (2)

forming the polyorganosiloxane-containing epsilon-polylysine compound is a group represented by: 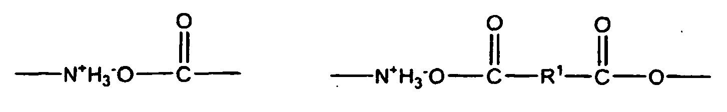 or 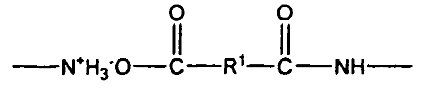 (***X*** in the general formula (2), R¹ represents a linear or branched alkylene group having 1 to 5 carbon atoms, an alkenylene group having 2 to 5 carbon atoms or an arylene group having 6 to 10 carbon atoms).
In the cosmetic compositions according to the first aspect, it is preferable that ***X*** in the group represented by the general formula (2):

-X-Y-Z (2)

forming the polyorganosiloxane-containing epsilon-polylysine compound is a group represented by: 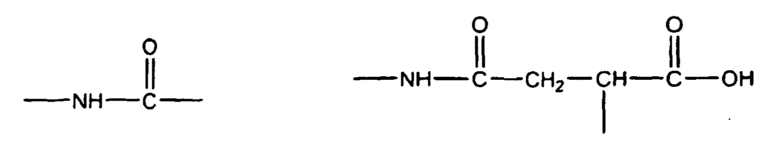 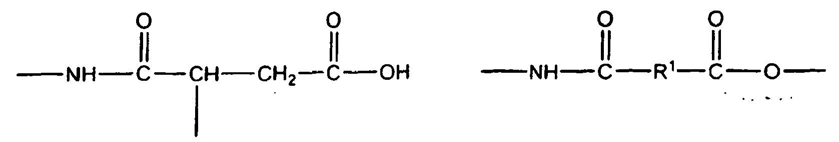 or 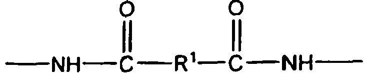 (***X*** in the general formula (2), R¹ represents a linear or branched alkylene group having 1 to 5 carbon atoms, an alkenylene group having 2 to 5 carbon atoms or an arylene group having 6 to 10 carbon atoms).
In the cosmetic compositions according to the first aspect, it is preferable that ***X*** in the group represented by the general formula (2):

-X-Y-Z (2)

forming the polyorganosiloxane-containing epsilon-polylysine compound is a group represented by: 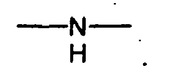 In the cosmetic compositions according to the first aspect, it is preferable that ***X*** in the group represented by the general formula (2):

-X-Y-Z (2)

forming the polyorganosiloxane-containing epsilon-polylysine compound is a group represented by: 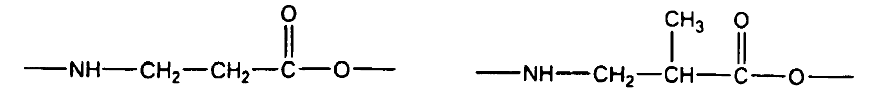 or

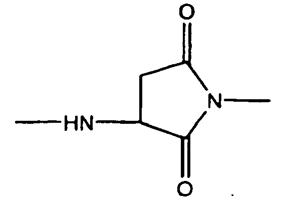

A second aspect of the present invention is a cosmetic composition comprising one or a combination of two or more of polyorganosiloxane-containing epsilon-polylysine compounds or a physiologically acceptable salt thereof, and polyhydric alcohol, the polyorganosiloxane-containing-epsilon-polylysine compound being obtained by reacting epsilon-polylysine with polyorganosiloxane.
In the cosmetic compositions according to the second aspect, it is preferable that the cosmetic composition, wherein the epsilon-polylysine is represented by the general formula (7) : 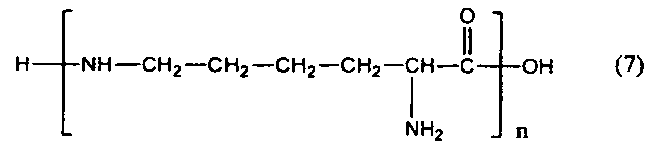 (wherein **n** is an integer of 2 to 51). It is preferable that **n** is an integer of 10 to 40, it is further preferable **n** is an integer of 30 to 40.
In the cosmetic compositions according to the second aspect, it is preferable that polyorganosiloxane has a functional group that is reactive with an amino group in the epsilon-polylysine.
In the cosmetic compositions according to the second aspect, it is preferable that he polyorganosiloxane is a polyorganosiloxane having an epoxy group, a polyorganosiloxane having a carboxylic acid or a carboxylic acid derivative, a polyorganosiloxane having a halogenated alkyl group or a polyorganosiloxane having an unsaturated group.
In the cosmetic compositions according to the second aspect, it is preferable that the polyorganosiloxane is a polyorganosiloxane represented by the general formula (8):

Q-Y-Z (8)

[in the general formula (8), ***Q*** represents 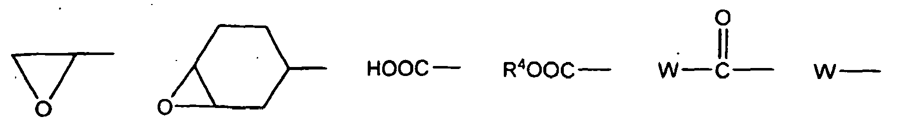 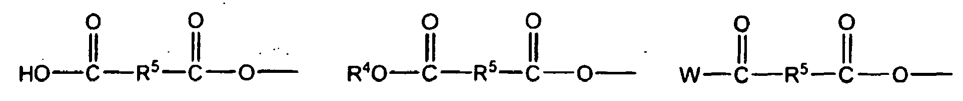 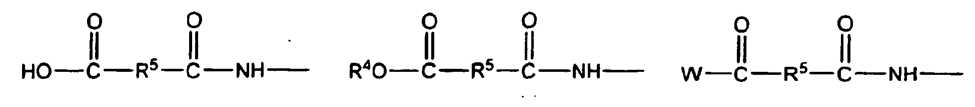 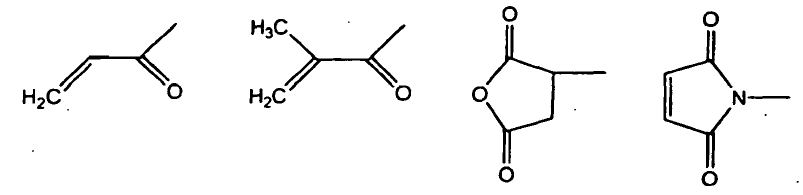 or 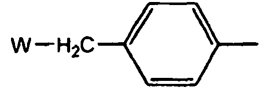 (wherein, R⁴ represents a linear or branched alkyl group having 1 to 20 carbon atoms or an aryl group having 6 to 10 carbon atoms or a trimethylsilyl group, R⁵ represents a linear or branched alkylene group having 1 to 5 carbon atoms, an alkenylene group having 2 to 5 carbon atoms or an arylene group having 6 to 10 carbon atoms, ***W*** represents chlorine, bromine or iodine), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4) : 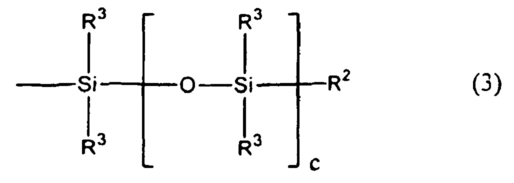 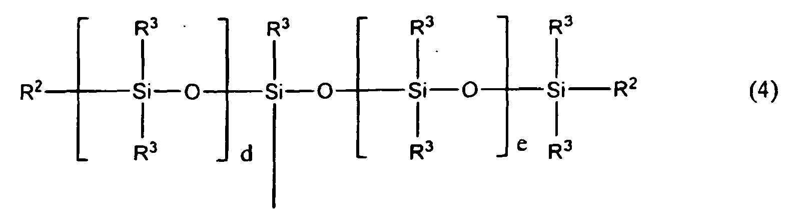 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)].
It is particularly preferable that ***Q*** in the general formula (8) be 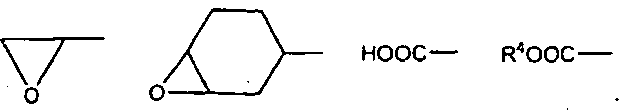 Of these, the most preferable one is: 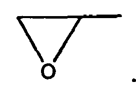 Furthermore, ***Z*** in the general formula (8), it is preferable that R² and R³ are each independently an alkyl group having 1 to 10 carbon atoms or an aryl group having 6 to 10 carbon atoms, and more preferably, are an alkyl group having 1 to 5 carbon atoms or a phenyl group. Of these, the most preferable ones are a methyl group and a n-butyl group.

A third aspect of the present invention is A method of producing a cosmetic composition comprising: mixing one or a combination of two or more of polyorganosiloxane-containing epsilon-polylysine compounds or a physiologically acceptable salt thereof, the polyorganosiloxane-containing epsilon-polylysine compound being obtained by reacting epsilon-polylysine with polyorganosiloxane, with polyhydric alcohol.
In the cosmetic compositions according to the third aspect, it is preferable that the epsilon-polylysine is represented by the general formula (7): 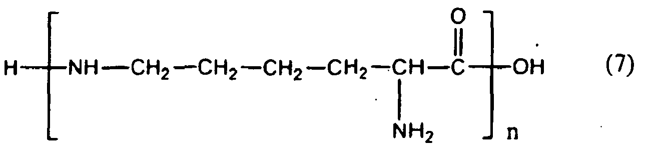 (wherein, **n** is an integer of 2 to 51).
In the cosmetic compositions according to the third aspect, it is preferable that the polyorganosiloxane has a functional group that is reactive with an amino group in the epsilon-polylysine.
In the cosmetic compositions according to the third aspect, it is preferable that the polyorganosiloxane is a polyorganosiloxane having an epoxy group, a polyorganosiloxane having a carboxylic acid or a carboxylic acid derivative, a polyorganosiloxane having a halogenated alkyl group or a polyorganosiloxane having an unsaturated group.
In the cosmetic compositions according to the third aspect, it is preferable that the polyorganosiloxane is a polyorganosiloxane represented by the general formula (8):

Q-Y-Z (8)

[in the general formula (8), ***Q*** represents 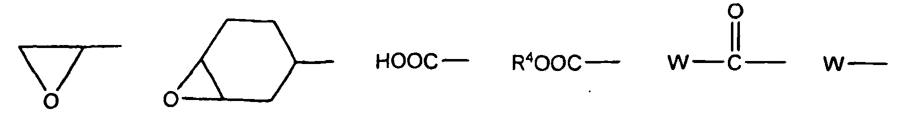 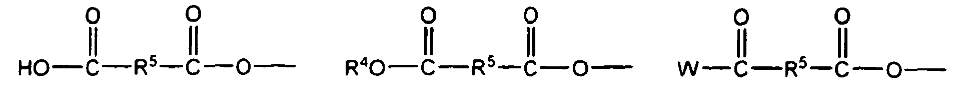 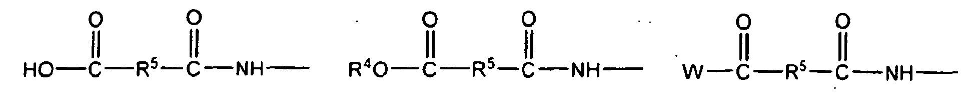 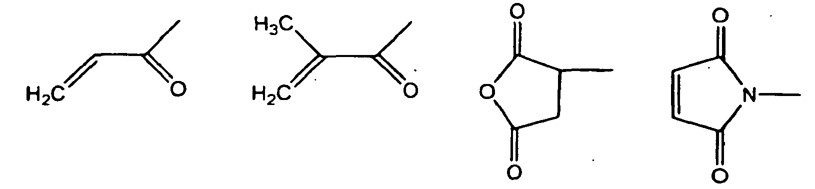 or 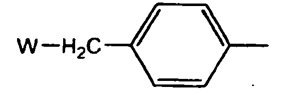 (wherein, R⁴ represents a linear or branched alkyl group having 1 to 20 carbon atoms or an aryl group having 6 to 10 carbon atoms or a trimethylsilyl group, R⁵ represents a linear or branched alkylene group having 1 to 5 carbon atoms, an alkenylene group having 2 to 5 carbon atoms or an arylene group having 6 to 10 carbon atoms, ***W*** represents chlorine, bromine or iodine), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4): 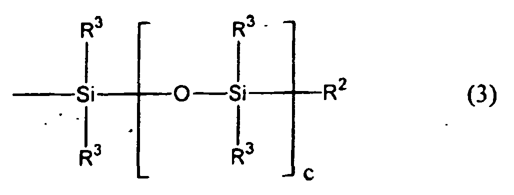 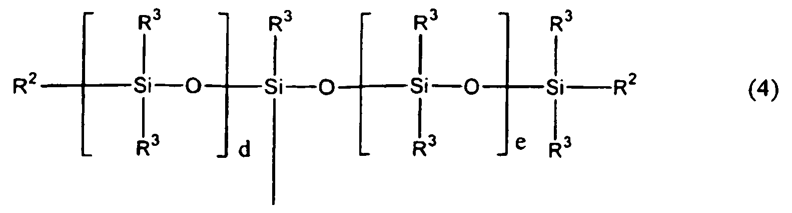 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)].
It is particularly preferable that ***Q*** in the general formula (8) be 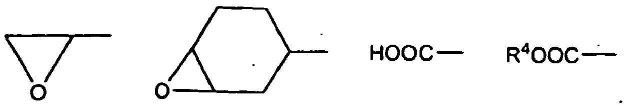 Of these, the most preferable one is: 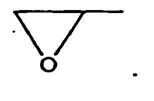 Furthermore, ***Z*** in the general formula (8), it is preferable that R² and R³ are each independently an alkyl group having 1 to 10 carbon atoms or an aryl group having 6 to 10 carbon atoms, and more preferably, are an alkyl group having 1 to 5 carbon atoms or a phenyl group. Of these, the most preferable ones are a methyl group and a n-butyl group.

In the cosmetic compositions according to the third aspect, it is preferable that the polyorganosiloxane-containing epsilon-polylysine compound is represented by the general formula (1): 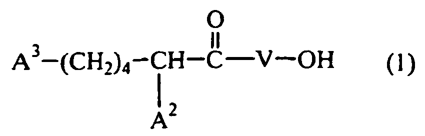 [in the general formula (1), ***V*** represents a random copolymer in which a first repeating unit represented by the general formula (5) : 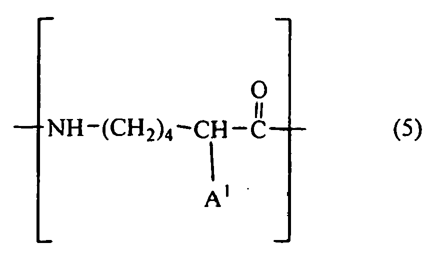 is randomly polymerized with a second repeating unit represented by the general formula (6): 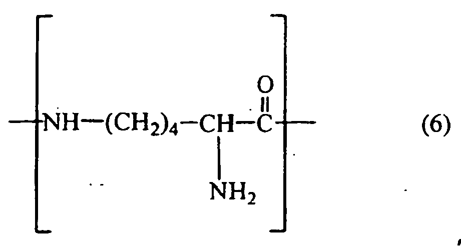 an alternating copolymer in which the first repeating unit alternates with the second repeating unit, or a block copolymer in which a block consisting only of the first repeating units is connected to another block consisting only of the second repeating units, the number of repeats ***a*** of the first repeating unit being an integer of 0 to 50, the number of repeats ***b*** of the second repeating unit being an integer of 0 to 50, ***a*** + ***b*** being equal to an integer of 1 to 50, A¹ represents a group represented by the general formula (2):

-X-Y-Z (2)

{in the general formula (2), ***X*** represents 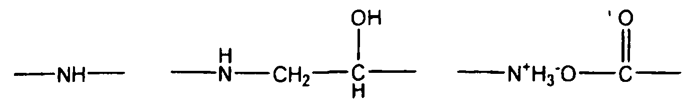 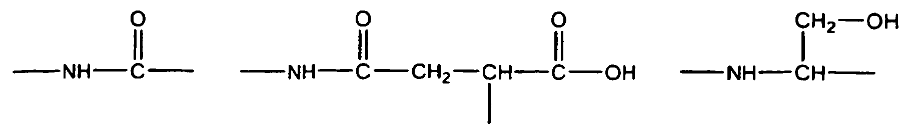 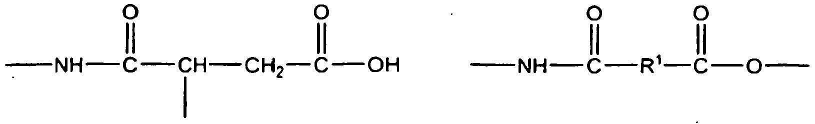 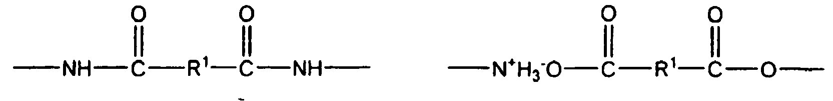 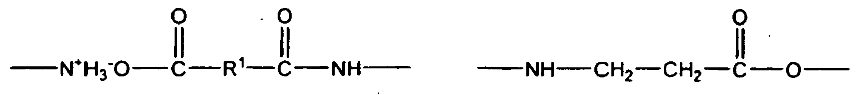 or 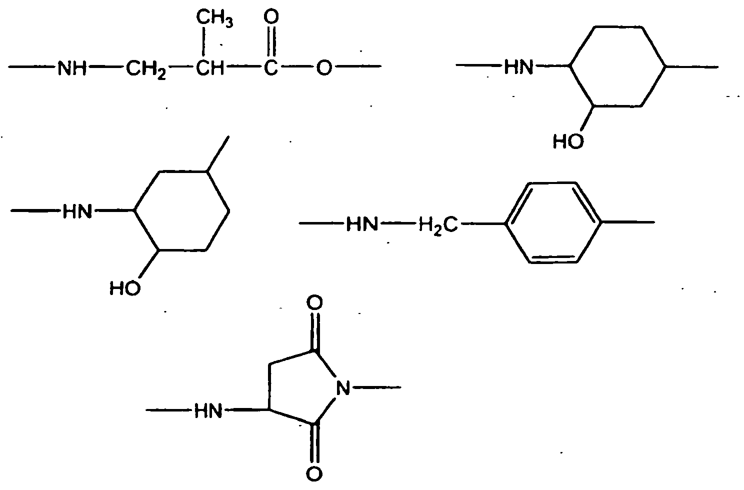 (wherein, R¹ represents a linear or branched alkylene group having 1 to 5 carbon atoms, an alkenylene group having 2 to 5 carbon atoms or an arylene group having 6 to 10 carbon atoms), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4): 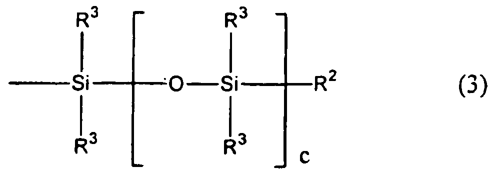 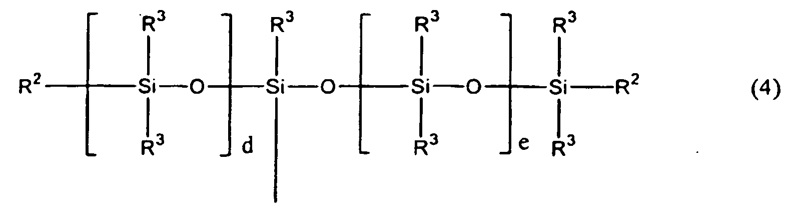 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)}, and A² and A³ are each independently an amino group or a group represented by the aforementioned general formula (2), A² and A³ are not both amino group when ***a*** is equal to 0].
It is preferable that the number of repeats ***a*** of said first repeating unit be an integer of 0 to 20, the number of repeats ***b*** of the second repeating unit be an integer of 20 to 40, ***a*** + ***b*** be 10 to 40. It is further preferable that ***a*** + ***b*** be 30 to 40.
It is particularly preferable that ***X*** in the general formula (2) be 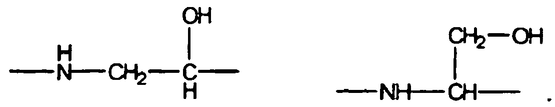 Furthermore, ***Z*** in the general formula (2), it is preferable that R² and R³ are each independently an alkyl group having 1 to 10 carbon atoms or an aryl group having 6 to 10 carbon atoms, and more preferably, are an alkyl group having 1 to 5 carbon atoms or a phenyl group. Of these, the most preferable ones are a methyl group and a n-butyl group.

In the cosmetic compositions according to the first and second aspects and the method of producing a cosmetic composition according to the third aspect of the present invention, it is preferable that the polyhydric alcohol is one or more selected from the group consisting of 1,3-butylene glycol, glycerin, propylene glycol, polyethylene glycol, dipropylene glycol, 1,2-pentanediol, 1,5-pentanediol, 1,2-hexanediol, 1,6-hexanediol, mannitol and sorbitol.
In the cosmetic compositions according to the first and second aspects and the method of producing a cosmetic composition according to the third aspect of the present invention, it is preferable that the polyhydric alcohol is one or more selected from the group consisting of ethanediol, diethylene glycol, triethylene glycol, 2-amino-2-methyl-1,3-propanediol, 1,2-propanediol, 1,3-propanediol, polypropylene glycol, 1,2-butanediol, 1,3-butanediol, 1,4-butanediol, isopentanediol, pentylene glycol, hexylene glycol, 1,3-pentanediol, 1,4-pentanediol, 1,2,3-pentanetriol, 2,3,4-pentanetriol, 1,3,4-pentanetriol, 1,3,5-pentanetriol, 1,3-hexanediol, 1,4-hexanediol, 1,5-hexanediol, 1,2,3-hexanetriol, 1,3,4-hexanetriol, 1,3,5-hexanetriol, 1,4,6-hexanetriol, erythritol, pentaerythritol, dipentaerythritol, threitol, arabitol, xylitol, ribitol, galactitol, lactitol, maltitol, inositol, panthenol, laminitol, valienamine, validamine and validatol.

In the cosmetic compositions according to the first and second aspects and the method of producing a cosmetic composition according to the third aspect of the present invention, it is preferable that the polyorganosiloxane-containing epsilon-polylysine compounds or a physiologically acceptable salt thereof is in an amount of 0.001% to 20% by weight.

In the cosmetic compositions according to the first and second aspects and the method of producing a cosmetic composition according to the third aspect of the present invention, it is preferable that the cosmetic composition is a hair treating agent. Furthermore, it is more preferable that the hair treating agent is hair dressings (e.g., hair creams, hair sprays, hair tonics, hair gels, hair lotions, hair oils, hair essences, hair waters, hair waxes, and hair mousses), shampoos, finishing rinses, hair treatments, hair setting lotions, hair dyes (e.g., hair colors, one-part hair dyes, and two-part hair dyes), perm solutions (e.g., permanent wave solutions, hair straightening solutions, and permanent wave holding agents), blood flow enhancers, scalp lotions or anti-hair loss agents.
In the cosmetic compositions according to the first and second aspects and the method of producing a cosmetic composition according to the third aspect of the present invention, it is preferable that the cosmetic composition is a skin care cosmetic. Furthermore, it is more preferable that the skin care cosmetic agent is toners, serums, whitening toners, milky lotions, whitening milky lotions, creams, whitening creams, ointments, whitening ointments, lotions, whitening lotions, oils or facial packs '
In the cosmetic compositions according to the first and second aspects and the method of producing a cosmetic composition according to the third aspect of the present invention, it is preferable that the cosmetic composition is a makeup cosmetics. Furthermore, it is more preferable that the makeup cosmetics is foundations, liquid foundations, lipsticks, lip glosses, eye shadows, powders, face powders, blushers, eye liners, mascaras or eyebrow pencils.
In the cosmetic compositions according to the first and second aspects and the method of producing a cosmetic composition according to the third aspect of the present invention, it is preferable that the cosmetic composition is a skin cleaners. Furthermore, it is more preferable that the skin cleaners is soap, cleansing creams, cleansing lotions, cleansing milks, cosmetic compositions, facial washes or body shampoos.
In the cosmetic compositions according to the first and second aspects and the method of producing a cosmetic composition according to the third aspect of the present invention, it is preferable that the cosmetic composition is a finishing cosmetics. Furthermore, it is more preferable that the finishing cosmetics is manicures. In the cosmetic compositions according to the first and second aspects and the method of producing a cosmetic composition according to the third aspect of the present invention, it is preferable that the cosmetic composition is bath agents, patches, perfumes, toothpastes, tooth washes or mouthwashes.

In this specification, the amino group indicates -NH₂ and does not include those having a substituent such as an alkylamino group, except as otherwise noted

### [Effect of the Invention]

A preferable aspect of the present invention provides a highly preservative and antibacterial cosmetic composition that can easily be applied to both emulsion and non-emulsion type cosmetics.
Another preferable aspect of the present invention allows improvement of a preservative and/or antibacterial effect (s) of a cosmetic composition comprising polyorganosiloxane-containing epsilon-polylysine and consequent reduction of the amount of antibacterial preservative agent to be used.
A still another preferable aspect of the present invention provides a highly safe antibacterial preservative agent with which any skin irritation and sensitization due to an antibacterial preservative agent are reduced.

### [Best Mode for Carrying Out the Invention]

### 1. Polyorganosiloxane-containing epsilon-polylysine

A polyorganosiloxane-containing epsilon-polylysine contained in a cosmetic composition according to the present invention is a compound represented by the aforementioned general formula (1). In addition, the present invention comprehends all possible optical isomers and racemic forms thereof with asymmetric carbon atoms, of the polyorganosiloxane-containing epsilon-polylysine represented by the aforementioned general formula (1).

A group represented by ***X*** in a group represented by the general formula (2):

―X―Y―Z (2)

which forms the polyorganosiloxane-containing
epsilon-polylysine compound having the aforementioned general formula (1) is a group represented by: 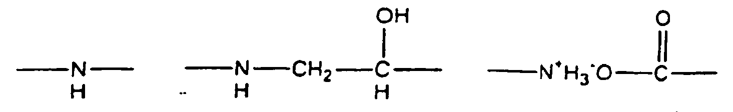 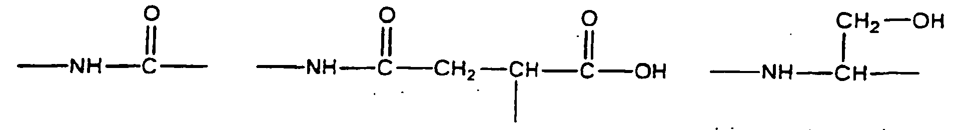 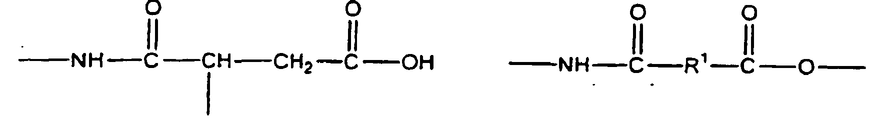 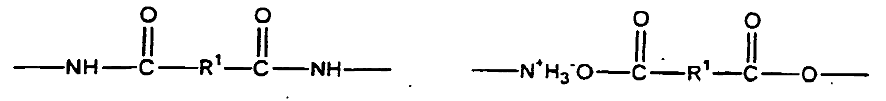 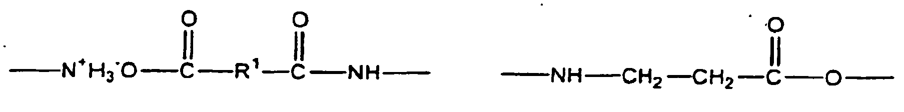 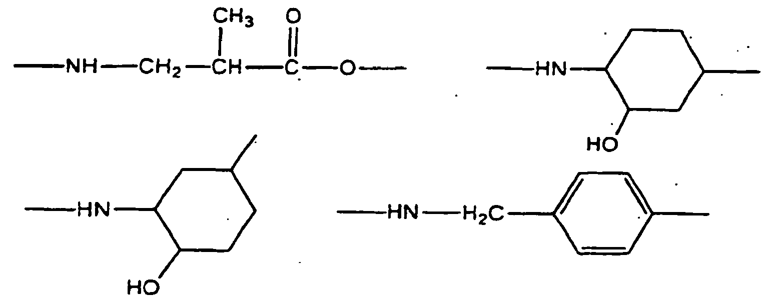 or 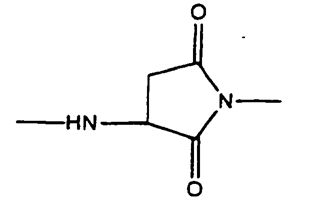 (in ***X*** in the general formula (2), R¹ represents a linear or branched alkylene group having 1 to 5 carbon atoms, an alkenylene group having 2 to 5 carbon atoms or an arylene group having 6 to 10 carbon atoms).

In the general formula (2), R¹ in ***X*** represents a linear or branched alkylene group having 1 to 5 carbon atoms, an alkenylene group having 2 to 5 carbon atoms or an arylene group having 6 to 10 carbon atoms. Examples of the alkylene group having 1 to 5 carbon atoms include a linear or blanched alkylene group such as -CH₂CH₂-, -CH₂-CH(CH₃)-, -CH₂CH(C₂H₅)-, and -CH(CH₃)-CH(CH₃)-. Examples of the alkenylene group having 2 to 5 carbon atoms include a linear or branched alkenylene group such as -CH=CH-, -CH₂-C(=CH₂)-, -CH=C(CH₃)-, and -C(CH₃) =C(CH₃)-. Examples of the arylene group having 6 to 10 carbon atoms include 1,2-phenylene, 4-methyl-1,2-phenylene, dimethyl-1,2-phenylene, and 4-ethyl-1, 2-phenylene. In R¹, more preferable are -CH₂CH₂-, -CH=CH-, -CH₂-C(=CH₂)-, -CH=C(CH₃)-, and 1,2-phenylene.

In the general formula (2), ***Y*** represents a linear or branched alkylene group having 1 to 1000 carbon atoms, of which any mutually non-adjacent methylene groups may be substituted with -O-(ether). Specific examples of ***Y*** include ethylene, trimethylene, tetramethylene, pentamethylene, hexamethylene, heptamethylene, octamethylene, nonamethylene, decamethylene, undecamethylene, dodecamethylene, tetradecamethylene, 2-methylethylene, 2-methyltrimethylene, 2-methyltetramethylene, 2-methylpentamethylene, 2-methylhexamethylene, 2-methylheptamethylene, 2-methyloctamethylene, 2-methylnonamethylene, 2-methyldecamethylene, 2-methylundecamethylene, -OCH₂CH₂CH₂-, -OCH₂ CH₂ OCH₂ CH₂ CH₂-, -CH₂ OCH₂ CH₂ CH₂-, or -(OCH₂CH₂)ₘOCH₂CH₂CH₂-, -(OCH(CH₃)CH₂)ₘOCH₂CH₂CH₂-, and -(OCH₂CH(CH₃))ₘOCH₂CH₂CH₂- (wherein m is an integer of 1 or greater). More preferably, it is a group selected from trimethylene, decamethylene, 2-methylethylene, -OCH₂CH₂CH₂-, -OCH₂CH₂OCH₂CH₂CH₂-, -CH₂OCH₂CH₂CH₂-, and -(OCH₂CH₂)ₘ OCH₂ CH₂ CH₂-

In the general formula (2), ***Z*** represents a polyorganosiloxane group having the aforementioned general formula (3) or (4). In the general formulae (3) and (4), the linear or branched alkyl group having 1 to 20 carbon atoms which is represented by R² and R³ specifically includes a methyl group, ethyl group, n-propyl group, i-propyl group, n-butyl group, i-butyl group, s-butyl group, t-butyl group, pentyl group, neopentyl group, hexyl group, heptyl group, octyl group, nonyl group, decyl group, undecyl group, dodecyl group, cyclopentyl group, cyclohexyl group, benzyl group and phenethyl group. The aryl group having 6 to 10 carbon atoms specifically includes a phenyl group, toluyl group, xylyl group, and ethylphenyl group. In the general formula (3), ***c*** is an integer of 1 to 1000. In the general formula (4), ***d*** is an integer of 1 to 1000 and ***e*** is an integer of 0 to 1000. Of these, ***c*** is preferably an integer of 1 to 200, most preferably, an integer of 1 to 100, ***d*** is preferably an integer of 1 to 200, most preferably, an integer of 1 to 100, ***e*** is preferably an integer of 0 to 200, and most preferably, an integer of 0 to 100.

Physiologically acceptable salts of the polyorganosiloxane-containing epsilon-polylysine used in the present invention may equally be applied to the present invention. Specific examples include salts with an inorganic acid, salts with an organic acid, and salts with an acidic amino acid. More specifically, examples of the salts with an inorganic acid include hydrochloride salts, hydrofluoride salts, hydrobromide salts, nitrate salts, sulfate salts, phosphate salts, perchlorate salts, and hydroiodide salts. Examples of the salts with an organic acid include formate salts, acetate salts, trifluoroacetate salts, fumarate salts, oxalate salts, tartrate salts, maleate salts, citrate salts, succinate salts, malate salts, mandelate salts, ascorbate salts, lactate salts, gluconate salts, methanesulfonate salts, toluenephosphonate salts, and benzenesulfonate salts. Examples of the salts with an acidic amino acid include aspartic acid and glutamic acid. The present invention comprehends both anhydrides and hydrates of the polyorganosiloxane-containing epsilon-polylysine used in a product according to the present invention.

### 2. Production of polyorganosiloxane-containing epsilon-polylysine

The polyorganosiloxane-containing epsilon-polylysine represented by the aforementioned general formula (1) may be obtained by reacting a polyorganosiloxane having a functional group reactive with an amino group in epsilon-polylysine with epsilon-polylysine represented by the aforementioned general formula (7). More specifically, it may be obtained by converting some amino groups in epsilon-polylysine contained in the repeating unit represented by the aforementioned general formula (7) into a polyorganosiloxane group.

The epsilon-polylysine represented by the general formula (7) may be produced either through organic synthesis or through fermentation by microorganisms. For example, it may be obtained by growing Streptomyces albulus subsp. lysinopolymerus disclosed in Japanese Patent No. 1245361 (Patent Document #2) in medium containing glucose and an iorganic salt and separating from the culture.
The epsilon-polylysine represented by the general formula (7) used in the present invention has a degree of polymerization ranging from 2 to 51 [***n*** in the general formula (7) is an integer of 2 to 51], preferably, a degree of polymerization ranging from 10 to 40 [***n*** in the general formula (7) is an integer of 10 to 40], and more preferably, a degree of polymerization ranging from 30 to 40 [***n*** in the general formula (7) is an integer of 30 to 40].

Examples of the polyorganosiloxane having a functional group reactive with an amino group in epsilon-polylysine include polyorganosiloxane having an epoxy group, polyorganosiloxane having carboxylic acid or carboxylic acid derivatives, polyorganosiloxane having a halogenated alkyl group and polyorganosiloxane having an unsaturated group.
As the polyorganosiloxane having a functional group reactive with an amino group in epsilon-polylysine, there may be a one-end modified polyorganosiloxane having a functional group in either end thereof, a both-end modified polyorganosiloxane having a functional group in both ends thereof, and a side-chain modifed polyorganosiloxane having a functional group in the side chain.

More specifically, in the present invention, it is preferable to use polyorganosiloxane represented by the general formula (8):

―Q―Y―Z (8).

In this case, ***Q*** is 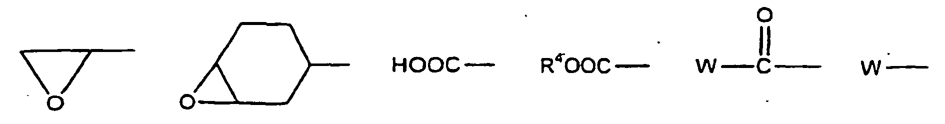 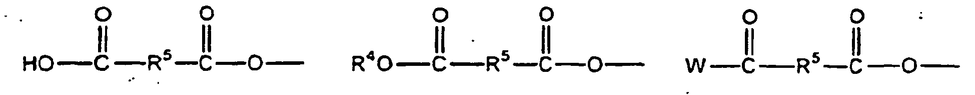 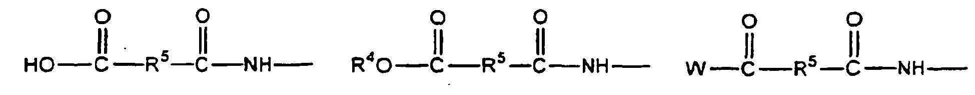 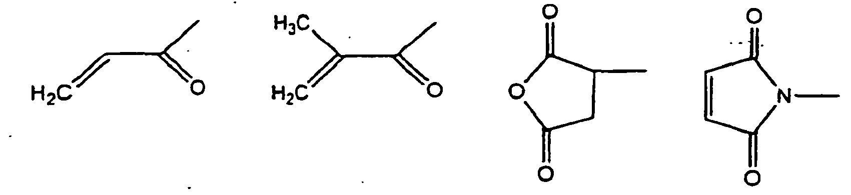 or 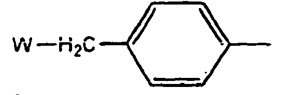 (wherein, R⁴ is a linear or branched alkyl group having 1 to 20 carbon atoms, an aryl group having 6 to 10 carbon atoms or a trimethylsilyl group, R⁵ is a linear or branched alkylene group having 1 to 5 carbon atoms, an alkenylene group having 2 to 5 carbon atoms or an arylene group having 6 to 10 carbon atoms, and ***W*** is chlorine, bromine or iodine).
R⁴ in the group represented by ***Q*** in the aforementioned general formula (8) is a linear or branched alkyl group having 1 to 20 carbon atoms, an aryl group having 6 to 10 carbon atoms or a trimethylsilyl group. Examples of the linear or branched alkyl group having 1 to 20 carbon atoms include a methyl group, ethyl group, n-propyl group, i-propyl group, n-butyl group, i-butyl group, s-butyl group, t-butyl group, pentyl group, neopentyl group, hexyl group, heptyl group, octyl group, nonyl group, decyl group, undecyl group, dodecyl group, cyclopentyl group, cyclohexyl group, benzyl group, and phenethyl group. Examples of the aryl group having 6 to 10 carbon atoms include a phenyl group, toluyl group, xylyl group and ethylphenyl group. Of these, it is particularly preferable when R⁴ is a methyl group, ethyl group, n-propyl group, i-propyl group, n-butyl group or trimethylsilyl group.
R⁵ in the group represented by ***Q*** in the aforementioned general formula (8) is similar to R¹ in ***X*** in the aforementioned general formula (2).
In the general formula (8), ***Y*** and ***Z*** are similar to ***Y*** and ***Z,*** respecticely, in the aforementioned general formula (2).

As described above, polyorganosiloxane-containing epsilon-polylysine represented by the aforementioned general formula (1) may be obtained by reacting the aforementioned the epsilon-polylysine represented by the general formula (7) with polyorganosiloxane.
The content of polyorganosiloxane in the polyorganosiloxane-containing epsilon-polylysine represented by the aforementioned general formula (1) may be controlled depending on the loading amount of polyorganoziloxane having a functional group reactive with an amino group with respect to epsilon-polylysine and the molecular weight of polyorganosiloxane.
Any solvent in which epsilon-polylysine dissolves may be used for reaction. Specific example includes, but not limited to, methanol, a mixed solvent of water and methanol, a mixed solvent of water and ethanol, a mixed solvent of water and dimethylformamide, a methanol/2-propanol mixed solvent, and a methanol/ethanol mixed solvent. The amount of the reaction solvent used is 1 to 100 times, preferably 1 to 10 times the weight of epsilon-polylysine.
The reaction temperature does not necessarily need to be a high temperature as the reaction is expected to proceed even at a room temperature. However, it is preferably 30 to 70 degrees C because a lower temperature requires a longer reaction time.

This reaction is conducted by dissolving epsilon-polylysine in a solvent and then adding dropwise polyorganosiloxane having a functional group reactive with an amino group. The time for the dropwise addition is preferably 0.01 to 2 hours. Although the reaction is expected to proceed within a short period of time because it is made between the functional groups and amino groups, the reaction time is preferably 1 to 24 hours. Since it is possible that the polyorganosiloxane having a functional group which can react with amino groups may not readily dissolve in the solvent, stirring is preferably carried out at a speed which ensures adequate mixing. After completion of the reaction, the solvent may be distilled off to obtain the silicone-modified antimicrobial polymer. In addition, it is preferable to stir the mixture at a speed to provide adequate mixing because some polyorganosiloxane having a functional group reactive with an amino group is not dissolved in the aforementioned solvent. After completion of the reaction, the solvent may be distilled off to obtain polyorganosiloxane-containing epsilon-polylysine.

### 3. Cosmetic composition comprising a polyorganosiloxane-containing epsilon-polylysine compound or a physiologically acceptable salt thereof and polyhydric alcohol

The polyhydric alcohol used in the present invention is not limited to a specific one. Instead, it may be a compound having at least two hydroxyl groups linked to a linear, branched or cyclic hydrocarbon. Specific examples of the polyhydric alcohol used in the present invention include ethanediol, diethylene glycol, triethylene glycol, polyethylene glycol, propylene glycol, dipropylene glycol, 2-amino-2-methyl-1,3-propanediol, 1,2-propanediol, 1,3-propanediol, polypropylene glycol, 1,3-butylene glycol, 1,2-butanediol, 1,3-butanediol, 1,4-butanediol, isopentanediol, pentylene glycol, hexylene glycol, 1,2-pentanediol, 1,3-pentanediol, 1,4-pentanediol, 1,5-pentanediol, 1,2,3-pentanetriol, 2,3,4-pentanetriol, 1,3,4-pentanetriol, 1,3,5-pentanetriol, 1,2-hexanediol, 1,3-hexanediol, 1,4-hexanediol, 1,5-hexanediol, 1,6-hexanediol, 1,2,3-hexanetriol, 1,3,4-hexanetriol, 1,3,5-hexanetriol, 1,4,6-hexanetriol, glycerin, erythritol, pentaerythritol, dipentaerythritol, threitol, arabitol, xylitol, ribitol, galactitol, sorbitol, mannitol, lactitol, maltitol, inositol, panthenol, laminitol, valienamine, validamine, validatol, ethyleneglycol, ethyleneglycol monoethylether, ethyleneglycol monobutylether, diethylene glycolmonomethylether, diethylene glycolmonoethylether, and 1,5-pentanediol. In particular, it is preferable to use 1,3-butylene glycol, propylene glycol, polyethylene glycol, 1,2-pentanediol, 1,2-hexanediol, or sorbitol. In the present invention, the polyhydric alcohol may equally be used in both anhydrous and hydrated forms.

The polyhydric alcohol used in the present invention may be prepared using any one of various known techniques such as organic synthesis, extraction and purification from a naturally-occurring product, or fermentation by microorganisms.
These techniques are not specifically limited by the method used for producing a cosmetic composition according to the present invention.

Any of the forms may be used for the polyhydric alcohol in the present invention, such as fine powders, microcrystals, liquids, pellets, viscous fluids, gels and the like. The most suitable form for cosmetic composition products of the present invention may be selected.

The content of polyhydric alcohol used in the present invention in the cosmetic composition is not specifically limited. However, it is arbitrarily determined within a range of 0.0001 to 80% by weight, more preferably, 0.001 to 50% by weight, from the viewpoints of material costs and the concentration range within which a desirable antibacterial preservative effect is achieved.

The cosmetic composition containing a polyorganosiloxane-containing epsilon-polylysine compound and others according to the present invention may be selected depending on a specific product formation of the cosmetic composition from fine powders, microcrystals, liquids, or pellets.
The cosmetic composition of the present invention may be formed into granules, gels, or viscous fluids by mixing it with, for example, an excipient, thickener or gelling agent.

The content of polyorganosiloxane-containing epsilon-polylysine used in the present invention in the cosmetic composition is not specifically limited. However, it is arbitrarily determined within a range of 0.0001 to 50% by weight, more preferably, 0.001 to 20% by weight, from the viewpoints of material costs and the concentration range within which a desirable antibacterial preservative effect is achieved.

The cosmetic composition available according to the present invention may be formulated in various forms such as a capsule, powder, granule, solid, liquid, gel, foam, emulsion, cream, ointment, sheet, mousse, powder dispersion, multilayer, and aerosol.
The composition according to the present invention finds applications in hair treating agents such as hair dressings (e.g., hair creams, hair sprays, hair tonics, hair gels, hair lotions, hair oils, hair essences, hair waters, hair waxes, and hair mousses), shampoos, finishing rinses, hair treatments, hair creams, hair mousses, hair setting lotions, hair colors, hair dyes (e.g., hair colors, one-part hair dyes, and two-part hair dyes), perm solutions (e.g., permanent wave solutions, hair straightening solutions, and permanent wave holding agents), blood flow enhancers, scalp lotions, and anti-hair loss agents. Another application of the composition according to the present invention includes, for example, skin care cosmetics such as toners, serums, whitening toners, milky lotions, whitening milky lotions, creams, whitening creams, ointments, whitening ointments, lotions, whitening lotions, oils, facial packs. Furthermore, still another application of the composition according to the present invention includes, for example, makeup cosmetics such as foundations, liquid foundations, lipsticks, lip glosses, eye shadows, powders, face powders, blushers, eye shadows, eye liners, mascaras, and eyebrow pencils. Another application of the composition according to the present invention includes, for example, skin cleaners such as soap, cleansing creams, cleansing lotions, cleansing milks, cosmetic compositions, facial washes, and body shampoos. Moreover, another application of the composition according to the present invention includes finishing cosmetics such as manicures. Other applications of the composition according to the present invention include, for example, cosmetic compositions in the form of bath agents, patches, perfumes, toothpastes, tooth washes, and mouthwashes.

### 4. Additives for the cosmetic composition

In addition to the essential ingredients, polyorganosiloxane-containing epsilon-polylysine and polyhydric alcohol, the cosmetic composition according to the present invention may contain the following exemplary ingredients that are arbitrarily selected and combined as additives to provide a cosmetic composition having a larger variety of functions: active ingredients such as pigmentation inhibitors, tyrosinase inhibitors, melanocyte melanogenesis inhibitors, melanogenesis promoters, humectants, cell activators/metabolic activators, antioxidants, active oxygen scavengers/radical production inhibitors, lipometabolism promoters, UV blockers/UV absorption promoters, astringents, anti-inflammatory agents/interleukin inhibitors/antiphlogistics, antiseborrheic agents, antimicrobial agents/antiviral agents, blood flow enhancers/blood vessel stimulators, anti-androgen agents, structural proteolytic enzyme (e.g., elastase, collagenase, keratin protease, serine protease, integrin degrading enzyme, involucrin degrading enzyme, filaggrin degrading enzyme, laminin degrading enzyme, fibronectin degrading enzyme, and proteoglycan degrading enzyme) inhibitors, structural protein synthesis promoters, mucopolysaccharides (e.g., hyaluronic acid, and chondroitin sulfuric acid) degrading enzyme inhibitors, mucopolysaccharides synthesis promoters, intercellular lipid production promoters/intercellular lipid condition modifiers, keratolytic agents/stratum corneum removal promoters, plasminogen-activator competitive inhibitors, Maillard reaction inhibitors, testosterone 5 alpha-reductase inhibitor/hair papilla activating agents/hair growth promoters, hair matrix cell proliferation inhibitors/hair growth inhibitors, hair swelling agents/hair protectors, and odor counteractants, and other plant-based materials, animal-based materials, microorganism-based materials, extracts and metabolites originated from naturally-occurring materials or various chemical compounds that are preferably used for preparation of cosmetic compositions.
The content in the preparation is not specifically limited but it may generally be within the concentration range of from 0.0001 to 50% by weight.

### (1) Pigmentation inhibitors

The cosmetic composition according to the present invention may contain a pigmentation inhibitor. Specific examples of the pigmentation inhibitor include p-aminobenzoic acid derivatives, salicylic acid derivatives, benzenesulfonamide derivatives, imidazole derivatives, naphthalene derivatives, hydroxyanthranilic acid or salts thereof and their derivatives, anthranilic acid derivatives, coumarin derivatives, amino acid derivatives (e.g., 2-amino-3-[1-carboxyl-2-(1H-imidazol-4-yl)ethyl)aminobutanoi c acid, 2-amino-3-[1-carboxyl-2-(1H-imidazol-4-yl)ethyllaminobutanoi c acid hydrochloride, 2-amino-3-[1-carboxyl-2-(1H-imidazol-4-yl)ethyl]aminobutanoi c acid sodium salt, and 2-amino-3-[1-carboxyl-2-(1H-imidazol-4-yl)ethyl)aminobutanoi c acid potassium salt), benzotriazole derivatives, tetrazole derivatives, imidazoline derivatives, pyrimidine derivatives, dioxane derivatives, camphor derivatives, furan derivatives, pyrone derivatives, nucleic acid derivatives, allantoin derivatives, nicotinic acid derivatives, ascorbic acid or salts thereof and their derivatives (e.g., magnesium-L-ascorbic acid phosphate, ascorbyl palmitate, ascorbyl dipalmitate, ascorbic acid hydroxyproline phosphate ester, 5-o-alpha-D-glucopyranosyl-L-ascorbic acid, L-ascorbic acid phosphate ester sodium salt, L-ascorbic acid phosphate ester potassium salt, L-ascorbic acid phosphate ester magnesium salt, L-ascorbic acid phosphate ester calcium salt, L-ascorbic acid phosphate ester aluminum salt, L-ascorbic acid sulfate ester sodium salt, L-ascorbic acid sulfate ester potassium salt, L-ascorbic acid sulfate ester magnesium salt, L-ascorbic acid sulfate ester calcium salt, L-ascorbic acid sulfate ester aluminum salt, L-ascorbic acid sodium salt, L-ascorbic acid potassium salt, L-ascorbic acid magnesium salt, L-ascorbic acid calcium salt, L-ascorbic acid aluminum salt, 6-o-alpha-D-galactopyranosyl-L-ascorbic acid, 2-o-beta-D-galactopyranosyl-L-ascorbic acid, L-ascorbic acid phosphate ester magnesium salt, L-ascorbic acid phosphate ester sodium salt, L-ascorbic acid sulfate ester sodium salt, 6-o-acylascorbic acid phosphate ester sodium salt, 6-o-acylascorbic acid phosphate ester ammonium salt, 6-o-acylascorbic acid phosphate ester isopropanolamine salt, 3-o-isopropyl-L-ascorbic acid, 6-o-alkylascorbic acid phosphate ester potassium salt, 6-o-alkylascorbic acid phosphate ester calcium salt, 6-o-alkylascorbic acid phosphate ester barium salt, 6-o-alkylascorbic acid phosphate ester ammonium salt, 6-o-alkylascorbic acid phosphate ester monoethanolamine salt, 6-o-alkylascorbic acid phosphate ester diethanolamine salt, 6-o-alkylascorbic acid phosphate ester triethanolamine salt, 6-o-alkylascorbic acid phosphate ester monoisopropanolamine salt, 6-o-alkylascorbic acid phosphate ester diisopropanolamine salt, 6-o-alkylascorbic acid phosphate ester triisopropanolamine salt, 3-o-glycosy-L-ascorbic acid, 6-o-beta-D-galactopyranosyl-L-ascorbic acid, ascorbic acid cholesterol phosphate ester, L-ascorbyl palmitate, L-ascorbyl isopalmitate, L-ascorbyl dipalmitate, L-ascorbyl diisopalmitate, L-ascorbyl stearate, L-ascorbyl isostearate, L-ascorbyl distearate, L-ascorbyl diisostearate, L-ascorbyl myristate, L-ascorbyl isomyristate, L-ascorbyl dimyristate, L-ascorbyl diisomyristate, L-ascorbyl 2-ethylhexanoate, L-ascorbyl di-2-ethylhexanoate, oleic acid-L-ascorbic acid, 2-o-alpha-D-glucosyl-L-ascorbic acid, 2-o-alpha-D-maltosyl-L-ascorbic acid, 2-o-alpha-D-maltotriosyl-L-ascorbic acid, 3-o-alpha-D-glucosyl-L-ascorbic acid, 2-o-alpha-D-maltosyl-L-ascorbic acid, 2-o-alpha-D-maltotriosyl-L-ascorbic acid, L-ascorbic acid tetraisopalmitate ester, L-ascorbic acid tetralaurate ester, L-ascorbic acid tetra-2-ethylhexanoate ester, L-ascorbic acid tetraoleate ester, 5,6-isopropylidene-L-ascorbic acid, L-ascorbic acid retinol ester, L-ascorbic acid-DL-tocopherol phosphate ester, L-3-o-ethylascorbic acid, L-ascorbic acid tristearate, L-ascorbic acid tripalmitate, L-ascorbic acid trioleate, ascorbic acid triphosphate ester, 2-o-ascorbyl cinnamate, 2-o-ascorbyl ferulate, 2-o-ascorbyl caffeate, 2-o-ascorbyl sinapate, 2-o-[6-palmitoylascorbyl]-'4'-acetoxy ferulate, DL-alpha-tocopherol-2-L-ascorbic acid phosphate diester, ascorbic acid inositol-binding derivatives, ascorbic acid phosphorus amide derivatives, ascorbic acid-arbutin binding compounds, ascorbyl-phosphoryl-cholesterol, chromanyl ascorbic acid derivatives, and ascorbic acid/sialic acid derivatives), tocopherol or salts thereof and their derivatives (e.g., alpha-tocopherol, beta-tocopherol, gamma-tocopherol, delta-tocopherol, epsilon-tocopherol, alpha-tocopheryl retinoate, aminomethylated tocopherol, hydroxymethylated tocopherol, tocopheryl phosphate ester, tocopherol acetate, tocopherol nicotinate, tocopherol succinate, tocopherol linoleate, tocopherol orotate, DL-alpha-tocopheryl glucoside, DL-alpha-tocopherylmaltoside, DL-beta-tocopheryl glucoside, DL-beta-tocopherylmaltoside, DL-gamma-tocopheryl glucoside, DL-gamma-tocopherylmaltoside, DL-delta-tocopheryl glucoside, DL-delta-tocopherylmaltoside, D-alpha-tocopheryl glucoside, D-alpha-tocopherylmaltoside, D-beta-tocopheryl glucoside, D-beta-tocopherylmaltoside, D-gamma-tocopheryl glucoside, D-gamma-tocopherylmaltoside, D-delta-tocopheryl glucoside, D-delta-tocopher-ylmaltoside, L-alpha-tocopheryl glucoside, L-alpha-tocopherylmaltoside, L-beta-tocopheryl glucoside, L-beta-tocopherylmaltoside, L-gamma-tocopheryl glucoside, L-gamma-tocopherylmaltoside, L-delta-tocopheryl glucoside, L-delta-tocopherylmaltoside, 1-(sulfoethylamino)-3-(alpha-tocopheryl-6-yloxy)propan-2-ol, 1-(carboxypropylamino)-3-(alpha-tocophoryl-6-yloxy)propan-2-ol hydrochloride, S-[3-(alpha-tocopheryl-6-yloxy)-2-hydroxypropyl]cysteine, S-[3-(alpha-tocopheryl-6-yloxy)-2-hydroxypropyl]-gamma-gluta myl cysteinyl glycine, N-[3-(alpha-tocopheryl-6-yloxy)-2-hydroxypropyl]aspartic acid, and N-[3-(alpha-tocopheryl-6-yloxy)-2-hydroxypropyl]glutamic acid), tocotrienol or salts thereof and their derivatives (e.g., alpha-tocotrienol, beta-tocotrienol, gamma-tocotrienol, delta-tocotrienol, tocotrienol acetate, tocotrienol nicotinate, tocotrienol succinate, tocotrienol linoleate, and tocotrienol orotate), kojic acid or derivatives thereof (e.g., 2-methoxymethyl-hydroxy-4H-pyran-4-one, 2-ethoxymethyl-5-hydroxy-4H-pyran-4-one, 2-benzoyloxymethyl-5-hydroxy-4H-pyran-4-one, 2-cinnamoyloxymethyl-5-hydroxy-4H-pyran-4-one, 2-phenoxymethyl-5-hydroxy-4H-pyran-4-one, kojic acid glycoside, geranyl geranyl acetone, kojic acid monobutylate, kojic acid monocaprate, kojic acid monopalmitate, kojic acid monostearate, kojic acid monocinnamate, kojic acid monobenzoate, kojic acid dibutyrate, kojic acid dipalmitate, kojic acid distearate, and kojic acid dioleate), oxybenzone, benzophenone, guaiazulene, shikonin, baicalin or salts thereof and their derivatives, baicalein or salts thereof and their derivatives, berberine or salts thereof and their derivatives, chrysin or salts thereof and their derivatives, apigenin or salts thereof and their derivatives, luteolin or salts thereof and their derivatives, acacetin or salts thereof and their derivatives, diosmetin or salts thereof and their derivatives, kaempferol or salts thereof and their derivatives, triforine or salts thereof and their derivatives, astragalin or salts thereof and their derivatives, quercetin or salts thereof and their derivatives, quercitrin or salts thereof and their derivatives, isoquercitrin or salts thereof and their derivatives, rutin or salts thereof and their derivatives, morin or salts thereof and their derivatives, myricetin or salts thereof and their derivatives, myricitrin or salts thereof and their derivatives, datiscetin or salts thereof and their derivatives, quercetagetin or salts thereof and their derivatives, isorhamnetin or salts thereof and their derivatives, pinocembrin or salts thereof and their derivatives, naringenin or salts thereof and their derivatives, hesperetin or salts thereof and their derivatives, eriodictyol or salts thereof and their derivatives, pinobanksin or salts thereof and their derivatives, aromadendrin or salts thereof and their derivatives, engeletin or salts thereof and their derivatives, taxifolin or salts thereof and their derivatives, astilbin or salts thereof and their derivatives, ampelopsin or salts thereof and their derivatives, spiraeoside, kaempferol-7-neohesperidoside, glutathione or salts thereof and their derivatives, isoflavone glycosides (e.g., 6-o-apiosylpuerarin-4'-o-glucoside, 6-o-glucosylpuerarin, 3'-hydroxypuerarin-4'-o-glucoside, and 6-o-apiosyl-3'-hydroxypuerarin), gamma-pyrone glycosides (e.g., maltol-3-o-(6'-o-apiosyl)-glucoside, and maltol-3-o-(6'-o-apiosyl)-glucoside), isononyl ferulate, ellagic acid or salts thereof and their derivatives (e.g., 5,4-dimethylellagic acid, 3,3'-dimethylellagic acid, 3,3',4-trimethylellagic acid, 3,3',4,4'-tetramethyl-5-methoxyellagic acid, 3-ethyl-4-methyl-5-hydroxyellagic acid, and amritoside), lucinol, onjisaponin, Ophiopogonis saponin, ruscogenin, sericoside, asiaticoside, hederin, senegin, benzoic acid anilides (e.g., 4-hydroxy-N-(2-hydroxyphenyl)benzoic acid amide, 4-hydroxy-N-(3-hydroxyphenyl)benzamide, 4-hydroxy-N-(4-hydroxyphenyl)benzamide, 3,5-di-t-butyl-4-hydroxy-N-(4-hydroxyphenyl)benzamide, 3,5-di-t-butyl-4-hydroxy-N-(3-hydroxyphenyl)benzamide, and 3,5-di-t-butyl-4-hydroxy-N-(2-hydroxyphenyl)benzamide), diphenylpyraline, ciproheptadine, triprolidine, dimethindene, ozagrel, isothipendyl, iproheptine, homochlorcyclizine, alimemazine, bucillamine, okitosamide, vidarabine, xanthotoxol, phenylmercuric hexachlorophene, mercuric oxide, mercurous chloride, aqueous hydrogen peroxide, zinc peroxide, placenta extracts (e.g., those derived from bovine placenta, swine placenta, equine placenta, and ovine placenta), almond (BIAN TAO) extract, *Phyllanthus emblica* extracts, *Foeniculum vulgare* leaf extract, *Atractylodes ovata* extract, *Atractylodes japonica* extract, konfuyou extract, Uncaria extract, *Uncaria gambir* extract, kakoujyuyou extract, *Glycyrrhizae radix* extract, *Gardenia jasminoides* (ZHI ZI) extract, kuranigean extract, *Sophora flavescens* extract, *Scutellaria baicalensis* (HUANG QIN) extract, *Triticum aestivum* L. (wheat) extract, *Oryza sativa* L. (rice) extract, Coriaria extract, *Woodfordia fruticosa* Sidowayah extract, sanukyu extract, sanbitoro extract, *Cassia Mimosoides* L. extract, *Bletilla striata* (BAIJI) extract, *Ligusticum chuanxiong* (CHUAN XIONG) extract, *Cassia acutifolia* extract, *Inula britannica* extract, *Lythrum anceps* extract, surigatin extract, *Angelica decursiva* (QIAN HU) extract, *Coix lachryma-jobi* L. (YI YI REN) extract, *Vitex rotundifolia L.* extract, *Vitex trifolia* (MAN JING ZI) extract, *Hamamelis* *virginiana* extract, palm extract, Parietaria extract, *Carthamus tinctorius* (HONG HUA) extract, *Morus alba* L. (SANG BAI PI) extract, *Sophora flavescens* (KU SHEN) extract, *Iris germanica* L. extract, *Iris florentina* L. extract, *Artemisia mongolia* extract, *Alnus firma* fruit extract, Hong Kong extract, *Sanguisorba officinalis* (DI YU) extract, *Daphniphyllum macropodum* extract, *Polygonum multiflorum* extract, and *Fatsia japonica* extract.

### (2) Tyrosinase inhibitor

The cosmetic composition according to the present invention may contain a tyrosinase inhibitor. Specific examples of the tyrosinase inhibitor include ascorbic acid or salts thereof and their derivatives (e.g., magnesium-L-ascorbic acid phosphate, ascorbyl palmitate, ascorbyl dipalmitate, ascorbic acid hydroxyproline phosphate ester, 5-o-alpha-D-glucopyranosyl-L-ascorbic acid, L-ascorbic acid phosphate ester sodium salt, L-ascorbic acid phosphate ester potassium salt, L-ascorbic acid phosphate ester magnesium salt, L-ascorbic acid phosphate ester calcium salt, L-ascorbic acid phosphate ester aluminum salt, L-ascorbic acid sulfate ester sodium salt, L-ascorbic acid sulfate ester potassium salt, L-ascorbic acid sulfate ester magnesium salt, L-ascorbic acid sulfate ester calcium salt, L-ascorbic acid sulfate ester aluminum salt, L-ascorbic acid sodium salt, L-ascorbic acid potassium salt, L-ascorbic acid magnesium salt, L-ascorbic acid calcium salt, L-ascorbic acid aluminum salt, 6-o-alpha-D-galactopyranosyl-L-ascorbic acid, 2-o-beta-D-galactopyranosyl-L-ascorbic acid, L-ascorbic acid phosphate ester magnesium salt, L-ascorbic acid phosphate ester sodium salt, L-ascorbic acid sulfate ester sodium salt, 6-o-acylascorbic acid phosphate ester sodium salt, 6-o-acylascorbic acid phosphate ester ammonium salt, 6-o-acylascorbic acid phosphate ester isopropanolamine salt, 3-o-isopropyl-L-ascorbic acid, 6-o-alkylascorbic acid phosphate ester potassium salt, 6-o-alkylascorbic acid phosphate ester calcium salt, 6-o-alkylascorbic acid phosphate ester barium salt, 6-o-alkylascorbic acid phosphate ester ammonium salt, 6-o-alkylascorbic acid phosphate ester monoethanolamine salt, 6-o-alkylascorbic acid phosphate ester diethanolamine salt, 6-o-alkylascorbic acid phosphate ester triethanolamine salt, 6-o-alkylascorbic acid phosphate ester monoisopropanolamine salt, 6-o-alkylascorbic acid phosphate ester diisopropanolamine salt, 6-o-alkylascorbic acid phosphate ester triisopropanolamine salt, 3-o-glycosy-L-ascorbic acid, 6-o-beta-D-galactopyranosyl-L-ascorbic acid, ascorbic acid cholesterol phosphate ester, L-ascorbyl palmitate, L-ascorbyl isopalmitate, L-ascorbyl dipalmitate, L-ascorbyl diisopalmitate, L-ascorbyl stearate, L-ascorbyl isostearate, L-ascorbyl distearate, L-ascorbyl diisostearate, L-ascorbyl myristate, L-ascorbyl isomyristate, L-ascorbyl dimyristate, L-ascorbyl diisomyristate, L-ascorbyl 2-ethylhexanoate, L-ascorbyl di-2-ethylhexanoate, oleic acid-L-ascorbic acid, 2-o-alpha-D-glucosyl-L-ascorbic acid, 2-o-alpha-D-maltosyl-L-ascorbic acid, 2-o-alpha-D-maltotriosyl-L-ascorbic acid, 3-o-alpha-D-glucosyl-L-ascorbic acid, 2-o-alpha-D-maltosyl-L-ascorbic acid, 2-o-alpha-D-maltotriosyl-L-ascorbic acid, L-ascorbic acid tetraisopalmitate ester, L-ascorbic acid tetralaurate ester, L-ascorbic acid tetra-2-ethylhexanoate ester, L-ascorbic acid tetraoleate ester, 5,6-isopropylidene-L-ascorbic acid, L-ascorbic acid retinol ester, L-ascorbic acid-DL-tocopherol phosphate ester, L-3-o-ethylascorbic acid, L-ascorbic acid tristearate, L-ascorbic acid tripalmitate, L-ascorbic acid trioleate, ascorbic acid triphosphate ester, 2-o-ascorbyl cinnamate, 2-o-ascorbyl ferulate, 2-o-ascorbyl caffeate, 2-o-ascorbyl sinapate, 2-o-[6-palmitoylascorbyl]-4'-acetoxy ferulate, DL-alpha-tocopherol-2-L-ascorbic acid phosphate diester, ascorbic acid inositol-binding derivatives, ascorbic acid phosphorus amide derivatives, ascorbic acid-arbutin binding compounds, ascorbyl-phosphoryl-cholesterol, chromanyl ascorbic acid derivatives, and ascorbic acid/sialic acid derivatives), hydroquinone or salts thereof and their derivatives (e.g., hexose glycosides such as hydroquinone alpha-D-glucose, hydroquinone beta-D-glucose, hydroquinone alpha-L-glucose, hydroquinone beta-L-glucose, hydroquinone alpha-D-galactose, hydroquinonebeta-D-galactose, hydroquinone alpha-L-galactose, and hydroquinone beta-L-galactose, pentose glycosides such as hydroquinone alpha-D-ribose, hydroquinone beta-D-ribose, hydroquinone alpha-L-ribose, hydroquinone beta-L-ribose, hydroquinone alpha-D-arabinose, hydroquinone beta-D-arabinose, hydroquinone alpha-L-arabinose, and hydroquinone beta-L-arabinose, amino sugar glycosides such as hydroquinone alpha-D-glucosamine, hydroquinone beta-D-glucosamine, hydroquinone alpha-L-glucosamine, hydroquinone beta-L-glucosamine, hydroquinone alpha-D-galactosamine, hydroquinone beta-D-galactosamine, hydroquinone alpha-L-galactosamine, and hydroquinone beta-L-galactosamine, hydroquinone glycosides such as uronic acid glycosides such as hydroquinone alpha-D-glucuronic acid, hydroquinone beta-D-glucuronic acid, hydroquinone alpha-L-glucuronic acid, hydroquinone beta-L-glucuronic acid, hydroquinone alpha-D-galacturonic acid, hydroquinone beta-D-galacturonic acid, hydroquinone alpha-L-galacturonic acid, and hydroquinone beta-L-galacturonic acid, and hydroquinone hydroxyalkylether glycosides such as hydroquinone benzyl ether, 4-beta-D-glucopyranosyloxy-1-(4-hydroxyphenoxy)butane, 5-beta-D-glucopyranosyloxy-1-(4-hydroxyphenoxy)pentane, 6-beta-D-glucopyranosyloxy-1-(4-hydroxyphenoxy)hexane, 2-beta-D-glucopyranosyloxy-1-(4-hydroxyphenoxy)propane, 2-beta-D-glucopyranosyloxy-1-(4-hydroxyphenoxy)butane, and 2-beta-D-glucopyranosyloxy-1-(4-hydroxyphenoxy)propan-3-ol), kojic acid or salts thereof and their derivatives (e.g., 2-methoxymethyl-hydroxy-4H-pyran-4-one, 2-ethoxymethyl-5-hydroxy-4H-pyran-4-one, 2-benzoyloxymethyl-5-hydroxy-4H-pyran-4-one, 2-cinnamoyloxymethyl-5-hydroxy-4H-pyran-4-one, 2-phenoxymethyl-5-hydroxy-4H-pyran-4-one, kojic acid glycoside, geranyl geranyl acetone, kojic acid monobutylate, kojic acid monocaprate, kojic acid monopalmitate, kojic acid monostearate, kojic acid monocinnamate, kojic acid monobenzoate, kojic acid dibutyrate, kojic acid dipalmitate, kojic acid distearate, and kojic acid dioleate), tocopherol or salts thereof and their derivatives (e.g., alpha-tocopherol, beta-tocopherol, gamma-tocopherol, delta-tocopherol, epsilon-tocopherol, alpha-tocopheryl retinoate, aminomethylated tocopherol, hydroxymethylated tocopherol, tocopheryl phosphate ester, tocopherol acetate, tocopherol nicotinate, tocopherol succinate, tocopherol linoleate, tocopherol orotate, DL-alpha-tocopheryl glucoside, DL-alpha-tocopherylmaltoside, DL-beta-tocopheryl glucoside, DL-beta-tocopherylmaltoside, DL-gamma-tocopheryl glucoside, DL-gamma-tocopherylmaltoside, DL-delta-tocopheryl glucoside, DL-delta-tocopherylmaltoside, D-alpha-tocopheryl glucoside, D-alpha-tocopherylmaltoside, D-beta-tocopheryl glucoside, D-beta-tocopherylmaltoside, D-gamma-tocopheryl glucoside, D-gamma-tocopherylmaltoside, D-delta-tocopheryl glucoside, D-delta-tocopherylmaltoside, L-alpha-tocopheryl glucoside, L-alpha-tocopherylmaltoside, L-beta-tocopheryl glucoside, L-beta-tocopherylmaltoside, L-gamma-tocopheryl glucoside, L-gamma-tocopherylmaltoside, L-delta-tocopheryl glucoside, L-delta-tocopherylmaltoside, L-(sulfoethylamino)-3-(alpha-tocopheryl-6-yloxy)propan-2-ol, 1-(carboxypropylamino)-3-(alpha-tocopheryl-6-yloxy)propan-2-ol hydrochloride, S-[3-(alpha-tocopheryl-6-yloxy)-2-hydroxypropyl]cysteine, S-[3-(alpha-tocopheryl-6-yloxy)-2-hydrbxypropyl]-gamma-gluta myl cysteinyl glycine, N-[3-(alpha-tocopheryl-6-yloxy)-2-hydroxypropyl]aspartic acid, and N-[3-(alpha-tocopheryl-6-yloxy)-2-hydroxypropyl]glutamic acid), tocotrienol or salts thereof and their derivatives (e.g., alpha-tocotrienol, beta-tocotrienol, gamma-tocotrienol, delta-tocotrienol, tocotrienol acetate, tocotrienol nicotinate, tocotrienol succinate, tocotrienol linoleate, and tocotrienol orotate), N-acetyl tyrosine or salts thereof and their derivatives, glutathione or salts thereof and their derivatives, ellagic acid or salts thereof and their derivatives (e.g., 3,4-dimethylellagic acid, 3,3'-dimethylellagic acid, 3,3',4-trimethylellagic acid, 3,3',4,4'-tetramethyl-5-methoxyellagic acid, 3-ethyl-4-methyl-5-hydroxyellagic acid, and amritoside), isonitrile antibiotics such as isonitrin A, isonitrin B, isonitrin C, isonitrin D, isonitrinic acid E, isonitrinic acid F, derumadein, and toricobilidein, orsellinic acid derivatives (e.g., orsellinic acid, orsellinic acid ethyl ester orcinol, p-geranyl orsellinic acid, p-geranyl orsellinic acid ethyl ester geranyl orcinol, p-farnesyl orsellinic acid, p-farnesyl orsellinic acid ethyl ester farnesyl orcinol, p-dodecanyl orsellinic acid, p-dodecanyl orsellinic acid ethyl ester dodecanyl orcinol, p-tetradecanyl orsellinic acid, p-tetradecanyl orsellinic acid ethyl ester tetradecanyl orcinol, p-hexadecanyl orsellinic acid, p-hexadecanyl orsellinic acid ethyl ester hexadecanyl orcinol, p-undecanyl orsellinic acid, p-undecanyl orsellinic acid ethyl ester undecanyl orcinol, p-tridecanyl orsellinic acid, p-tridecanyl orsellinic acid ethyl ester undecanyl orcinol, p-pentadecanyl orsellinic acid, p-pentadecanyl orsellinic acid ethyl ester pentadecanyl orcinol, ethylhexyl orsellinic acid, p-ethylhexyl orsellinic acid ethyl ester ethylhexyl orcinol, p-cyclohexylmethyl orsellinic acid, p-cyclohexylmethyl orsellinic acid ethyl ester cyclohexylmethyl orcinol, p-hydroxyethylhexyl orsellinic acid methyl ester, and p-hydroxyethylhexyl orsellinic acidhydroxyethylhexyl orcinol), umbellic acid, brefeldin, oxydesberatrol, resorcinol derivatives (4-cyclohexyl resorcinol), 3-hydroxyketone compounds (e.g., 1,5-bis(p-hydroxyphenyl)-2-hydroxypentan-4-one, 1,5-bis(o,p-dihydroxyphenyl)-2-hydroxypentan-4-one, and 1,5-bis(p-hydroxyphenyl-m-methoxyphenyl)-2-hydroxypentan-4-o ne), 1,3-diketone compounds (e.g., 1,5-bis(p-hydroxyphenyl)-2,4-pentanedione, 1,5-bis(o,p-dihydroxyphenyl)-2,4-pentanedione, and 1,5-bis(p-hydroxyphenyl-m-methoxyphenyl)-2,4-pentanedione), bishydroxybenzylamides, gamma-aminobutyric acid or derivatives thereof (e.g., N-methyl-gamma-aminobutyric acid, N-dimethyl-gamma-aminobutyric acid, and gamma-aminobutyric acid oleyl ester), hydrogen peroxide, zinc peroxide, placenta extracts, lucinol, silk extract, acacia extract, acelora extract, *Abutilon theophrasti (Semen Abutili*) extract, *Betula pendula* extract, quercus (MO SHI ZI; chestnut gall wasp) extract, chestnut extract, *Plectranthus kameba* extract, *Isodon trichocarpus* extract, *Plectranthus japonicus* (dried) extract, *Oenanthe stolonifera* extract, *Fagopyrum esculentum* extract, Durvillea extract, *Capsella bursa-pastoris* extract, *Eupatorium japonicum* (dried) extract, *Matricaria chamomilla* L. extract, *Morus alba* extract, *Gardenia jasminoides* extract, *Angelica acutiloba* extract, *Sanguisorba officinalis* extract, *Sophora flavescens* extract, *Artemisia indica* extract, *Lonicera japonica* extract, *Phellodendron amurense* extract, *Houttuynia cordata* extract, *Poria cocos* extract, *Coix lachryma-jobi* L. extract, *Lamium album var. barbatum* extract, *Humulus lupulus* extract, *Crataegus cuneata* extract, eucalyptus extract, *Achillea millefolium* extract, althaea extract, GUI PI (*Cinnamomum cassia* bark; *Cinnamomi Cortex*) extract, MAN JING ZI (*Vitex rotundifolia* fruit) extract, *Hamamelis virginiana* extract, *Morus bombycis* extract, *Platycodon grandiflorum* extract, TU SI ZI (*Cuscuta chinensis* Lam. seed) extract, HSU SUI TZU (*Euphorbia lathyris* seed) extract, SHE GAN *(Belamcanda chinensis* rhizome) extract, MA HUANG (*Ephedra sinica* stem and leaf; *Ephedrae Herba*) extract, CHUAN XIONG *(Cnidium officinale* rhizome; *Cnidii Rhizoma)* extract, DU HUO (*Aralia cordata* root and rhizome) extract, CHAI HU *(Bupleurum falcatum* root; *Bupleuri Radix)* extract, FANG FENG *(Saposhnikovia divaricata* root; *Saposhnikoviae Radix)* extract, BEI SHA SHEN (*Glehnia littoralis* root; *Glehniae Radix cum Rhizoma*) extract, HUANG QIN (*Scutellaria baicalensis* root; *Scutellariae Radix)* extract, MU DAN PI (*Paeonia suffruticosa* root; *Moutan Cortex*) extract, SHAO YAO (*Paeonia lactifolia* root; *Paeoniae Radix*) extract, *Geranium thunbergii* extract, GE GEN (*Pueraria lobata* root; *Puerariae Radix*) extract, GAN CAO (*Glycyrrhiza uralensis* root and stolon; Glycyrrhizae Radix) extract, WU BEI ZI (*Galla Rhois*) extract, *Aloe arborescens* extract, SHENG MA (*Cimicifuga simplex root; Cimicifugae Rhizoma*) extract, HONG HUA (*Carthamus tinctorius* flower; *Carthami Flos*) extract, green tea extract, red tea extract, and *Acacia catechu* extract.

### (3) Melanocyte melanogenesis inhibitor

The cosmetic composition according to the present invention may contain a melanocyte melanogenesis inhibitor. Specific examples of the melanocyte melanogenesis inhibitor include lobeline or lobeline derivatives, liquiritin derivatives (e.g., liquiritin-alpha-glucoside, and liquiritin-alpha-maltoside), phenylchroman derivatives, chromone derivatives (e.g., 2-butylchromone, 2-pentylchromone, 2-heptylchromone, 2-nonylchromone, 2-hexadecylchromone, 2-(1-ethylpentyl)chromone, 2-butyl-7-methoxychromone, 2-pentyl-7-methoxychromone, 2-heptyl-7-methoxychromone, 2-nonyl-7-methoxychromone, 2-pentadecyl-7-methoxychromone, 2-(1-ethylpentyl)-7-methoxychromone, 7-hydroxy-2-methylchromone, 7-hydroxy-2-butylchromone, 7-hydroxy-2-pentylchromone, 7-hydroxy-2-heptylchromone, 7-hydroxy-2-nonylchromone, 7-hydroxy-2-pentadecylchromone, and 7-hydroxy-2-(1-ethylpentyl)chromone), azelaic acid derivatives (e.g., azelaic acid monoalkyl ester, and azelaic acid dialkyl ester), phosphatidylglucosamine, lysophosphatidylglucosamine, phenylhydroquinone, 3-beta-D-glucopyranosyl manool, 3-beta-D-maltopyranosyl manool, substituted amino acid derivatives (e.g., DL-N-formyl-3-(1-naphthyl)alanine, DL-N-acetyl-3-(1-naphthyl)alanine, DL-N-propionyl-3-(1-naphthyl)alanine, DL-N-butyryl-3-(1-naphthyl)alanine, DL-N-isobutyryl-3-(1-naphthyl)alanine, DL-N-valeryl-3-(1-naphthyl)alanine, DL-N-isovaleryl-3-(1-naphthyl)alanine, DL-N-(2-methylvaleryl)-3-(1-naphthyl)alanine, DL-N-(3-methylvaleryl)-3-(1-naphthyl)alanine, DL-N-(4-methylvaleryl)-3-(1-naphthyl)alanine, DL-N-t-butylacetyl-3-(1-naphthyl)alanine, DL-N-pivaloyl-3-(1-naphthyl)alanine, DL-N-caproyl-3-(1-naphthyl)alanine, DL-N-(2-ethylhexanoyl)-3-(1-naphthyl)alanine, DL-N-(2-methylhexanoyl)-3-(1-naphthyl)alanine, DL-N-heptanoyl-3-(1-naphthyl)alanine, DL-N-octanoyl-3-(1-naphthyl)alanine, DL-N-(2-propylpentanoyl)-3-(1-naphthyl)alanine, DL-N-nonanoyl-3-(1-naphthyl)alanine, DL-N-decanoyl-3-(1-naphthyl)alanine, DL-N-undecanoyl-3-(1-naphthyl)alanine, DL-N-dodecanoyl-3-(1-naphthyl)alanine, DL-N-tridecanoyl-3-(1-naphthyl)alanine, DL-N-tetradecanoyl-3-(1-naphthyl)alanine, DL-N-pentadecanoyl-3-(1-naphthyl)alanine, DL-N-hexadecanoyl-3-(1-naphthyl)alanine, DL-N-heptadecanoyl-3-(1-naphthyl)alanine, DL-N-octadecanoyl-3-(1-naphthyl)alanine, DL-N-nonadecanoyl-DL-3-(1-naphthyl)alanine, DL-N-icosanoyl-3-(1-naphthyl)alanine, DL-N-acroyl-3-(1-naphthyl)alanine, DL-N-crotonyloyl-3-(1-naphthyl)alanine, DL-N-methacryloyl-3-(1-naphthyl)alanine, DL-N-vinylacetyl-3-(1-naphthyl)alanine, DL-N-cyclopropanoyl-3-(1-naphthyl)alanine, DL-N-(2-pentenoyl)-3-(1-naphthyl)alanine, DL-N-(4-pentenoyl)-3-(1-naphthyl)alanine, DL-N-(2-hexenoyl)-3-(-naphthyl)alanine, DL-N-(3-hexenoyl)-3-(1-naphthyl)alanine, DL-N-(2-methyl-3-pentenoyl)-3-(1-naphthyl)alanine, DL-N-cyclohexenoyl-3-(1-naphthyl)alanine, DL-N-(10-undecenoyl)-3-(1-naphthyl)alanine, DL-N-linoleyl-3-(1-naphthyl)alanine, DL-N-hydroxyacetyl-3-(1-naphthyl)alanine, DL-N-(6-hydroxycaproyl)-3-(1-naphthyl)alanine, DL-N-(8-hydroxyoctanoyl)-3-(1-naphthyl)alanine, DL-N-(9-hydroxynonanoyl)-3-(1-naphthyl)alanine, DL-N-(10-hydroxydecanoyl)-3-(1-naphthyl)alanine, DL-N-(11-hydroxyundecanoyl)-3-(1-naphthyl)alanine, DL-N-(12-hydroxydecanoyl)-3-(1-naphthyl)alanine, DL-N-benzoyl-3-(1-naphthyl)alanine, DL-N-(2-hydroxybenzoyl)-3-(1-naphthyl)alanine, DL-N-(3-hydroxybenzoyl)-3-(1-naphthyl)alanine, DL-N-(4-hydroxybenzoyl)-3-(1-naphthyl)alanine, DL-N-(o-toluyl)-3-(1-naphthyl)alanine, DL-N-(m-toluyl)-3-(1-naphthyl)alanine, DL-N-(p-toluyl)-3-(1-naphthyl)alanine, DL-N-(1-naphthoyl)-3-(1-naphthyl)alanine, DL-N-(2-naphthoyl)-3-(1-naphthyl)alanine, DL-N-(2-carboxybenzoyl)-3-(1-naphthyl)alanine, DL-N-(3-carboxybenzoyl)-3-(1-naphthyl)alanine, DL-N-(4-carboxybenzoyl)-3-(1-naphthyl)alanine, DL-N-(2-picolyloyl)-3-(1-naphthyl)alanine, DL-N-(3-picolyloyl)-3-(1-naphthyl)alanine, DL-N-(4-picolyloyl)-3-(1-naphthyl)alanine, DL-N-phenylacetyl-3-(1-naphthyl)alanine, DL-N-(2-phenylpropanoyl)-3-(1-naphthyl)alanine, DL-N-(3-phenylbutyryl)-3-(1-naphthyl)alanine, DL-N-(4-methylvaleryl)-3-(1-naphthyl)alanine, DL-N-valeryl-3-(1-naphthyl)alanine, DL-N-(4-methylvaleryl)-3-(1-naphthyl)alanine amide, DL-N-(4-methylvaleryl)-3-(1-naphthyl)alanine methyl ester, DL-N-(4-methylvaleryl)-3-(1-naphthyl)alanine ethyl ester, DL-N-(4-methylvaleryl)-3-(1-naphthyl)alanine propyl ester, DL-N-(4-methylvaleryl)-3-(1-naphthyl)alanine-N-butyl ester, DL-N-(4-methylvaleryl)-3-(1-naphthyl)alanine pentyl ester, DL-N-(4-methylvaleryl)-3-(1-naphthyl)alanine isopropyl ester, DL-N-(4-methylvaleryl)-3-(1-naphthyl)alanine isobutyl ester, and DL-N-(4-methylvaleryl)-3-(1-naphthyl)alanine-t-butyl ester), benzolactam derivatives, indolactam derivatives, cedrol, guaiol, 1-(4-hydroxyphenylthio)-2-propanol, beta-lactoglobulin, 2-methoxy-5-methylphenol, 5-ethyl-2-methoxyphenol, 5-N-propyl-2-methoxyphenol, 5-N-butyl-2-methoxyphenol, 5-N-hexyl-2-methoxyphenol, 5-N-heptyl-2-methoxyphenol, 5-N-decyl-2-methoxyphenol, 5-(1,1-dimethylpropyl)-2-methoxyphenol, 5-(1,1-dimethylbutyl)-2-methoxyphenol, 5-(1,1-dimethylethyl)-2-methoxyphenol, 2-methoxy-5-(1-methylpentyl)phenol, 2-methoxy-5-(1-methylhexyl)phenol, 2-methoxy-5-(3-methylhexyl)phenol, 2-methoxy-5-(6-methylheptyl)phenol, 5-(1,3-dimethylheptyl)-2-methoxyphenol, mulberrin, ferruginol, sugiol, cryptojaponol, 1,5-bis[p-hydroxyphenyl]-1,4-pentadien-3-one, 1,5-bis[o-hydroxyphenyl]-1,4-pentadien-3-one, 1,5-bis[2,4-dihydroxyphenyl]-1,4-pentadien-3-one, 1,5-bis[3-methoxy-4-hydroxyphenyl]-1,4-pentadien-3-one, haginine, agrimophol, agrimol, hydrangenol or derivatives thereof, and alkylresorcinol or derivatives thereof (e.g., 4-N-butylresorcinol), aristolone, calamenenes (e.g., calamenene, 7-hydroxycalamenene, 5-hydroxycalamenene, and 7-methoxycalamenene), trans-umbellic acid, N-alpha-benzoyl-L-arginine, N-alpha-benzoyl-L-arginine ethyl ester or N-alpha-benzoyl-L-arginine ethyl ester, 5-methyl-2(3H)-furanone, 2-buten-4-olide, 2-hydroxymethylfuran, 2,5-dimethyl-4-hydroxy-3(2H)-furanone, 2-formylfuran, 3-formylfuran, methyl alpha-furyl ketone, furfuryl acetate, 2-hydroxy-3-methyl-2-cyclopenten-1-one, 2-hydroxy-3,5-dimethyl-2-cyclopenten-1-one, 2,5-dimethyl-4-hydroxy-3(2H)-thiophenone, 2-hydroxy-3-ethyl-2-cyclopenten-1-one, tetronic acid, pentanedione, iminodibenzyls (e.g., 2,2'-iminodibenzyl, imipramine, imipramine hydrochloride, desipramine, desipramine hydrochloride, chlorimipramine, and trimipramine), dibenzocycloheptadienes (e.g., amitriptyline, amitriptyline hydrochloride, nortriptyline, and noxiptyline), tetrahydrocopalol glycoside (e.g., ketotifen fumarate, labda-8(17),13-dien-15-ol, tetrahydromanool, tetrahydrocopalol, tetrahydrocopalol glucoside, tetrahydrocopalol galactoside, tetrahydrocopalol maltoside, tetrahydrocopalol cellobioside, and tetrahydrocopalol maltotrioside), spiro ether compounds, piochelin, phenothiazine compounds, promethazine, alimemazine, alimemazine tartrate, triflupromazine, levomepromazine, chlorpromazine, cyclandelate, 4-carboxymethyloxybenzoic acid, 4-carboxymethyloxy-2-hydroxybenzoic acid, 3-(3-carboxypropyl-1-oxy)-2-hydroxybenzoic acid, 4-(3-carboxypropyl-1-oxy)benzoic acid, 4-(3-carboxypropyl-1-oxy)-2-hydroxybenzoic acid, 4-(3-carboxypropyl-1-oxy)-2-methoxybenzoic acid, 5-(3-carboxypropyl-1-oxy)-2-hydroxybenzoic acid, 4-(5-carboxypentyl-1-oxy)-2-hydroxybenzoic acid, 6-(3-carboxypropyl-1-oxy)-2-hydroxybenzoic acid, 4-(10-carboxydecane-1-oxy)-2-hydroxybenzoic acid, 4-(10-carbamoyldecane-1-oxy)-2-hydroxybenzoic acid, 4-(4-hydroxybutyl-1-oxy)benzoic acid, 4-(4-hydroxybutyl-1-oxy)-2-hydroxybenzoic acid, 4-(4-acetoxybutyl-1-oxy)benzoic acid, 4-(4-acetoxybutyl-1-oxy)-2-hydroxybenzoic acid, 4-(3-ethoxycarbonylpropyl-1-oxy)-2-hydroxybenzoic acid, 3-(2,3-dihydroxypropyl-1-oxy)-2-hydroxybenzoic acid, 4-(4-methoxybutyl-1-oxy)-2-hydroxybenzoic acid, 4-(2,3-dihydroxypropyl-1-oxy)-2-hydroxybenzoic acid, 4-carboxymethyloxy-2-hydroxybenzoic acid, 3-(3-carboxypropyl-1-oxy)-2-hydroxybenzoic acid, 4-(3-carboxypropyl-1-oxy)-2-hydroxybenzoic acid, 5-(3-carboxypropyl-1-oxy)-2-hydroxybenzoic acid, 6-(3-carboxypropyl-1-oxy)-2-hydroxybenzoic acid, 4-(5-carboxypentyl-1-oxy)-2-hydroxybenzoic acid, 4-(4-hydroxybutyl-1-oxy)-2-hydroxybenzoic acid, 4-(10-carboxydecane-1-oxy)-2-hydroxybenzoic acid, hydroxytrimethyl cyclohexanes (e.g., monosaccharide glycoside, disaccharide glycoside or trisaccharide glycoside of, for example, 2-hydroxy-4-(2,2,6-trimethyl-1-yl-cyclohexane)butane, 4-(2,2,6-trimethyl-1-yl-cyclohexane)-1-butene, 4-(2,2,6-trimethyl-1-yl-cyclohexane)-2-butene, 4-(2,2,6-trimethyl-1-yl-cyclohexane)butane, 3-methyl-3-hydroxy-5-(2,2,6-trimethyl-1-yl-cyclohexane)penta ne, 3-methyl-1-hydroxy-5-(2,2,6-trimethyl-1-yl-cyclohexane)penta ne, 3-methyl-5-(2,2,6-trimethyl-1-yl-cyclohexane)pentane, 3-methyl-1-hydroxy-5-(2,2,6-trimethyl-1-yl-cyclohexane)-3-pe ntene, 3-methyl-3-hydroxy-5-(2,2,6-trimethyl-1-yl-cyclohexane)-1-pe ntene, 3-methyl-1-hydroxy-5-(2,2,6-trimethyl-1-yl-cyclohexane)-2-pe ntene, and 2-hydroxy-4-(2,2,6-trimethyl-1-yl-cyclohexane)butane), escinol, monosaccharide glycoside, disaccharide glycoside or trisaccharide glycoside of para-hydroxycinnamic acid-4-(2,2,6-trimethyl-yl-cyclohexane)-2-butyl ester, onjisaponin, Ophiopogonis saponin, ruscogenin, sericoside, asiaticoside, hederin, senegin, 4-(2,2,6-trimethyl-1-yl-cyclohexane)-2-keto-butane, 4-(2,2,6-trimethyl-1-yl-6-cyclohexene)-2-keto-butane, 4-(2,2,6-trimethyl-1-yl-cyclohexane)-2-keto-3-butene, 4-(2,2,6-trimethyl-1-yl-6-cyclohexene)-2-keto-3-butene(beta-ionone), L-p-hydroxyphenylglycine, D-p-hydroxyphenylglycine, N-benzyloxycarbonyl-L-p-hydroxyphenylglycine, N-benzyloxycarbonyl-D-p-hydroxyphenylglycine, N-benzoyl-L-p-hydroxyphenylglycine, N-benzoyl-D-p-hydroxyphenylglycine, N-(p-methoxybenzoyl)-L-p-hydroxyphenylglycine, N-(p-methoxybenzoyl)-D-p-hydroxyphenylglycine, N-(p-hydroxybenzoyl)-L-p-hydroxyphenylglycine, N-(p-hydroxybenzoyl)-D-p-hydroxyphenylglycine, N-acetyl-L-p-hydroxyphenylglycine, N-acetyl-D-p-hydroxyphenylglycine, N-acetyl-L-p-hydroxyphenylglycine ethyl ester, N-acetyl-D-p-hydroxyphenylglycine ethyl ester, N-acetyl-L-p-hydroxyphenylglycine amide, N-acetyl-D-p-hydroxyphenylglycine amide, L-p-methoxyphenylglycine, D-p-methoxyphenylglycine, L-p-methoxyphenylglycine hydrochloride, D-p-methoxyphenylglycine hydrochloride, 4-hydroxy-3-methoxy-L-phenylglycine, 4-hydroxy-3-methoxy-D-phenylglycine, L-p-hydroxyphenylglycine ethyl amide, D-p-hydroxyphenylglycine ethyl amide, N-tert-butoxycarbonyl-L-p-hydroxyphenylglycine, N-tert-butoxycarbonyl-D-p-hydroxyphenylglycine, N-tert-butoxycarbonyl-L-p-methoxyphenylglycine, N-tert-butoxycarbonyl-D-p-methoxyphenylglycine, N-9-fluorenylmethyloxycarbonyl-L-p-methoxyphenylglycine, N-9-fluorenylmethyloxycarbonyl-D-p-methoxyphenylglycine, N-9-fluorenylmethyloxycarbonyl-L-p-methoxyphenylglycine benzyl ester hydrochloride, N-9-fluorenylmethyloxycarbonyl-D-p-methoxyphenylglycine benzyl ester hydrochloride, L-p-hydroxyphenylglycine amide, D-p-hydroxyphenylglycine amide, L-p-hydroxyphenylglycine allyl ester p-toluenesulfonate, D-p-hydroxyphenylglycine allyl ester p-toluenesulfonate, L-p-hydroxyphenylglycine benzyl ester p-toluenesulfonate, D-p-hydroxyphenylglycine benzyl ester p-toluenesulfonate, L-p-hydroxyphenylglycine ethyl ester, D-p-hydroxyphenylglycine ethylester, L-p-hydroxyphenylglycine ethyl ester hydrochloride, D-p-hydroxyphenylglycine ethyl ester hydrochloride, L-p-hydroxyphenylglycine methyl ester, and D-p-hydroxyphenylglycine methyl ester, 1,3-diallylindane-2-carboxylic acids, stachybocins, pheophorbide derivatives, eleutherin, isoeleutherin or 4-beta-hydroxyisoeleutherin, eleutherinol, spiroketal derivatives (e.g., 2-(2,4-hexadiynylidene)-1,6-dioxaspiro[4.5]deca-3-ene, 2-(2-hexynylidene)-1,6-dioxaspiro[4.4]nona-3-ene, 2-((4-methylphenyl)methylidene)-6,6-dioxaspiro[4.4]nona-3-en e, 2-(2-hexenylidene)-6,6-dioxaspiro[4.5]deca-3-ene, 2-(2-hexenylidene)-1,6-dioxaspiro[4.4]nona-3-ene, 2-hexyl-1,6-dioxaspiro[4.4]nonane, 2-(2-hexenyl)-1,6-dioxaspiro[4.4]nonane, 2-(2-hexynyl)-1,6-dioxaspiro[4.4]nonane, 2-pentyl-1,6-dioxaspiro[4.4]nonane, 1,6-dioxaspiro[4.4]nonane, 1,6-dioxaspiro[4.5]decane, 1,7-dioxaspiro[5.5]undecane, 2,3-benzo-4,4-dimethyl-1,6-dioxaspiro[4.4]nonane, 3,4-benzo-2-pentyl-1,6-dioxaspiro[4.4]nonane, 3,4-benzo-2-hexyl-1,6-dioxaspiro[4.4]nonane, 3,4-benzo-2-octyl-1,6-dioxaspiro[4.4]nonane, 2-hexyl-9,9-dimethyl-1,6-dioxaspiro[4.4]nonane, and 2-(2,4-hexadiynylidene)-1,6-dioxaspiro[4.4]nona-3-ene), malvalic acid, phosphonic acid derivatives or salts thereof, aspergillomarasmine, aminophosphonic acid derivatives or salts thereof, diphenhydramine or salts thereof, pregnenolone or derivatives thereof, lutein, farnesyl isopropanol derivatives, hexahydrofarnesyl acetone, 4-benzoylamino-2-hydroxybenzoic acid, 5-benzoylamino-2-hydroxybenzoic acid, 4-benzoylaminobenzoic acid, 4-(1-naphthoylamino)-2-hydroxybenzoic acid, 5-(1-naphthoylamino)-2-hydroxybenzoic acid, 4-(2-naphthoylamino)-2-hydroxybenzoic acid, 5-(2-naphthoylamino)-2-hydroxybenzoic acid, 4-(1-naphthoylamino)benzoic acid, 4-(2-naphthoylamino)benzoic acid, 4-phenylaminocarbonyl benzoic acid, 4-phenylaminocarbonyl-2-hydroxybenzoic acid, 5-phenylaminocarbonyl-2-hydroxybenzoic acid, 4-(1-naphthylaminocarbonyl)benzoic acid, 4-(1-naphthylaminocarbonyl)-2-hydroxybenzoic acid, 5-(1-naphthylaminocarbonyl)-2-hydroxybenzoic acid, 4-(2-naphthylaminocarbonyl)benzoic acid, 4-(2-naphthylaminocarbonyl)-2-hydroxybenzoic acid, 5-(2-naphthylaminocarboriyl)-2-hydroxybenzoic acid, hexahydrofarnesyl isopropanol derivatives, borneol-p-hydroxycinnamic acid ester glucoside, borneol-p-hydroxycinnamic acid ester maltoside, borneol-p-hydroxycinnamic acid ester maltotrioside, cinnamic acid-4-(2,2,6-trimethyl-yl-cyclohexane)-2-butyl ester derivatives, 2,4-dihydroxybenzophenone, 1-(2,4-dihydroxyphenyl)-ethanone(2',4'-dihydroxyacetophenone ), 1-(2,4-dihydroxyphenyl)-1-propanone(2',4'-dihydroxypropiophe none), 1-(2,9-dihydroxyphenyl)-1-butanone, 1-(2,4-dihydroxyphenyl)-1-pentanone, 1-(2,4-dihydroxyphenyl)-1-hexanone, 1-(2,4-dihydroxyphenyl)-1-heptanone; 1-(2,4-dihydroxyphenyl)-1-octanone, 1-(2,9-dihydroxyphenyl)-1-nonanone, 1-(2,4-dihydroxyphenyl)-1-decanone, 1-(2,4-dihydroxyphenyl)-1-undecanone, 1-(2,4-dihydroxyphenyl)-1-dodecanone, 1-(2,4-dihydroxyphenyl)-1-tetradecanone, 1-(2,9-dihydroxyphenyl)-1-hexadecanone, 1-(2,4-dihydroxyphenyl)-1-octadecanone, 1-(2-hydroxy-4-methoxyphenyl)-ethanone(2'-hydroxy-4'-methoxy acetophenone), 1-(2-hydroxy-4-methoxyphenyl)-1-propanone(2'-hydroxy-4'-meth oxypropiophenone), 1-(2-hydroxy-4-methoxyphenyl)-1-butanone, 1-(2-hydroxy-4-methoxyphenyl)-1-pentanone, 1-(2-hydroxy-4-methoxyphenyl)-1-hexanone, 1- (2-hydroxy-4-methoxyphenyl) -I-heptanone, 1-(2-hydroxy-4-methoxyphenyl)-1-octanone, 1-(2-hydroxy-4-methoxyphenyl)-1-nonanone, 1-(2-hydroxy-4-methoxyphenyl)-1-decanone, 1-(2-hydroxy-4-methoxyphenyl)-1-undecanone, 1-(2-hydroxy-4-methoxyphenyl)-1-dodecanone, 1-(2-hydroxy-4-methoxyphenyl)-1-tetradecanone, 1-(2-hydroxy-9-methoxyphenyl)-1-hexadecanone, 1-(2-hydroxy-4-methoxyphenyl)-1-octadecanone, 1-(4-hydroxy-2-methoxyphenyl)-ethanone(4'-hydroxy-2'-methoxy acetophenone), 1-(4-hydroxy-2-methoxyphenyl)-1-propanone(4'-hydroxy-2'-meth oxypropiophenone), 1-(2,4-dimethoxyphenyl)-ethanone(2',4'-dimethoxyacetophenone ), 1-(2,4-dimethoxyphenyl)-1-propanone(2',4'-dimethoxypropiophe none), lactone derivatives (e.g., 1,6-dioxaspiro[4.4]nonane-2,7-dione, 1,6-dioxaspiro[4.5]decane-2,7-dione, 4-tridecanolide, 4-dodecanolide, 4-undecanolide, 4-decanolide, 4-nonanolide, 4-octanolide, 4-heptanolide, 5-dodecanolide, 5-undecanolide, 5-decanolide, 5-nonanolide, 5-octanolide, 2-undecen-4-olide, 2-decen-4-olide, 2-nonen-4-olide, 2-hepten-4-olide, 2-undecen-5-olide, 2-decen-5-olide, 2-nonen-5-olide, 2-octen-5-olide, 4-methyl-4-dodecanolide, 4-methyl-4-undecanolide, 4-methyl-4-decanolide, 4-methyl-4-nonanolide, 4-methyl-4-heptanolide, 5-methyl-5-dodecanolide, 5-methyl-5-undecanolide, 5-methyl-5-decanolide, 5-methyl-5-nonanolide, 5-methyl-5-octanolide, 2-methoxycarbonyl-4-dodecanolide, 2-methoxycarbonyl-4-undecanolide, 2-methoxycarbonyl-4-decanolide, 2-methoxycarbonyl-4-nonanolide, 2-methoxycarbonyl-4-heptanolide, 2-methoxycarbonyl-5-undecanolide, 2-methoxycarbonyl-5-decanolide, 2-methoxycarbonyl-5-nonanolide, 2-methoxycarbonyl-5-octanolide, 2-allyl-4-undecanolide, 2-allyl-5-decanolide, 2-allyl-4-nonanolide, 2-pentyl-4-undecanolide, 2-pentyl-4-nonanolide, 2-methyl-4-undecanolide, 2-methyl-4-nonanolide, 2-(4-hydroxybutyl)-4-undecanolide, 2-(4-hydroxybutyl)-4-nonanolide, 2-(4-hydroxybutyl)-5-decanolide, 5-propyloxy-4-pentanolide, 5-allyloxy-4-pentanolide, 5-(2-hydroxyethoxy)-4-pentanolide, 8-hydroxy-4-octanolide, 6-propyloxy-5-hexanolide, 6-allyloxy-5-hexanolide, 6-(2-hydroxyethoxy)-5-hexanolide, and 9-hydroxy-5-nonanolide), echinomycin, iriflorental, iripallidal, 2'-8-C-glucosyl-7-methylaloesol coumaroyl ester, 2'-8-C-glucosyl-7-methylaloesol cinnamoyl ester, chloropyramine, *Pimpinella Anisum* extract, *Aloe arborescens* extract, *Reynoutria japonica* (HU ZHANG) extract, *Daphne pseudo-mezereum* extract, *Cassia obtusifolia* extract, JUE MING ZI (*Cassia obtusifolia* seed; *Cassiae Semen*) extract, HUANG QI (*Astragalus mongholicus* root; *Astragali Radix*) extract, *Astragalus membranaceus* extract, *Trichosanthes bracteata* (*Trichosanthes* root) extract, *Xanthium strumarium* (CHANG ER ZI) extract, *Gastrodia elata* (TIAN MA) extract, *Pyracantha fortuneana* extract, *Polygonum sachalinense* extract, WU YAO (*Lindera strychnifolia* root; *Linderae Radix*) extract, pumpkin extract, *Typha latifolia* (PU HUANG) extract, *Euphorbia kansui* (GAN SUI) extract, *Agrimonia pilosa var. japonica* (XIAN HE CAO) extract, *Lindera umbellata* extract, *Saxifraga fusca var. kikubuki* extract, sisal (*Agave sisalana*) extract, *Clematis chinensis* extract, *Clematis chinensis* extract, *Clematis chinensis* (WEI LING XIAN) extract, *Prunus lannesiana var.speciosa* extract, *Prunus sargentii* extract, *Prunus incisa* extract, *Prunus nipponica Matsumura* extract, *Prunus* x *subhirtella* extract, *Prunus lannesiana* extract, *Aster tataricus* (ZI WAN) extract, *Trachycarpus fortunei* extract, *Iris florentina L*. extract, *Clematis terniflora* (DA LIAO) extract, *Magnolia salicifolia* (XIN YI HUA) extract, *Saxifraga fortunei var. incisolobata* extract, *Oenothera tetraptera* extract, TU SI ZI *(Cuscuta chinensis* Lam. seed) extract, *Cuscuta australis* extract, *Cuscuta japonica* extract, *Artemisia absinthium L.* extract, *Achillea alpina* extract, *Dictamnus dasycarpus* (BAI XIAN PI) extract, *Anethum graveolens* extract, *Fallopia japonica var. hachidyoensis* extract, *Tribulus terrestris* extract, *Pyrrosia lingua* (SHI WEI) extract, *Typha angustifolia* L. (XIANG PU) extract*, Angelica dahurica* extract, *Buddleja Americana* L. extract, *Brickellia cabanillesy* extract, *Artemisia fukudo* extract, *Convolvulus arvensis* extract, sandalwood extract, Ganoderma lucidum (LING ZHI) extract, *Leonurus japonicus* (YI MU CAO) extract, *Salix gilgiana* extract, *Salix chaenomeloides* extract, *Salix gracilistyla* extract, *Salix integra* extract, *Salix kinuyanagi* extract, *Salix koriyanagi* extract, *Salix matsudana cv. Tortuosa* extract, *Salix Reinii* extract, *Salix sieboldiana* extract, *Toisusu urbaniana* extract, *Salix schwernii* extract, *Salix vulpina* extract, *Populus maximowiczii* extract, Myrica rubra (YANG MEI PI) extract, *Agave americana* extract, Agave *americana var. marginata* extract, *Agave americana 'Marginata'* extract, *Edgeworthia chrysantha* extract, *Enteromorpha* (green laver) extract, *Enteromorpha linza* extract, *Enteromorpha prolifera* extract, *Enteromorpha compressa* extract, *Enteromorpha intestinalis* extract, hosoeda aonori extract, *Laminaria* (sea tangle) extract, *Laminaria japonica* extract, *Laminaria ochotensis* extract, *Laminaria religiosa* extract, *Laminaria* angustata extract, *Undaria pinnatifida* extract, *Undaria undaroides* extract, *Undaria peterseniana* extract, *Hizikia fusiformis* extract, Fucus *evanescens* extract, Padina *arborescens* extract, *Padina australis* extract, kirebanoumiuchiwa extract, akabaumiuchiwa extract, *Padina crassa* extract, *Padina japonica* extract, *Padina minor* extract, etsukiumiuchiwa extract, *Eucheuma serra* extract, *Eucheuma amakusaense* extract, *Eucheuma* extract, byakushinkirinsai extract, *Chondrus ocellatus* extract, *Chondrus verrucosus* extract, *Chondrus nipponicus extract*, *Chondrus pinnulatus* extract, *Chondracanthus tenellus* extract, *Chondracanthus teedii* extract, *Chondracanthus intermedius* extract, *Dictyopteris latiuscula* extract, uraboshiyahazu extract, *Padina arborescens* extract, *Sphaerotrichia divaricata* extract, *Cymathaere* extract, *Cymathaere japonica* extract, *Sargassum hemiphyllum* extract, nagashimamoku extract, *Sargassum filicinum* extract, *Sargassum sagamianum* extract, *Sargassum nigrifolium* extract, *Sargassum piluliferum* extract, tatsukuri extract, *Sargassum patens* extract, *Sargassum thunbergii* extract, *Sargassum ringgoldianum* extract, *Sargassum confusum* extract, *Sargassum kjellmanianum* extract, *Sargassum siliquastrum* extract, *Sargassum serratifolium* extract, *Sargassum giganteifolium* extract, *Grateloupia filicina* extract, *Halymenia agardhii* extract, kuronurakusa extract, *Halymenia acuminata* extract, *Carpopeltis affinis* extract, *Gracilaria gigas* extract, *Ceratodictyon spongiosum* extract, *Lomentaria catenata* extract, himefushitsunagi extract, *Lomentaria okamurae* extract, *Laurencia intermedia* extract, *Laurencia undulata* extract, *Laurencia pinnata* extract, *Laurencia brongniartii* extract, *Odonthalia corymbifera* extract, Tila extract, Camotede azafran extract, Jamaica extract, Poleo verde extract, Navo negro extract, *Schisandra chinensis* extract, *Schisandra nigra* extract, and *Kadsura japonica* extract.

### (4) Melanogenesis promoter

The cosmetic composition according to the present invention may contain a melanogenesis promoter. Specific examples of the melanogenesis promoter include salicylic acid or salts thereof and their derivatives (e.g., salicylic acid glucoside, salicylic acid fatty acid ester, salicylic acid alcohol ether, and salicylic acid amides), salicyl alcohol or salts thereof and their derivatives, apigenin, amentoflavone, *Zanthoxylum piperitum* extract, *Aralia cordata* extract, *Angelica pubescens* extract, *Anemone flaccida* extract, kashi extract, *Circaea cordata* extract, *Ageratum conyzoides* L. extract, *Rumex japonicus* (YANG TI GEN) extract, *Gymnema sylvesta* extract, kaorou extract, gyokuyoukinka extract, *Mussaenda parviflora* extract, sabunryo extract, *Jasminum officinalis* (MO LI HUA) extract, *Alcea rosea* extract, shouraitou extract, *Arabidopsis thaliana* extract, *Convallaria keiskei* extract, *Aconitum kusnezoffii* extract, *Erythrina variegata* extract, *Eucommia* (DU ZHONG) extract, *Ruscus aculeatus* (Butcher's bloom) extract, *Ecballium elaterium* extract, *Loropetalum chinense* extract, *Sapium sebiferum* extract, *Ailanthus altissima* extract, Dianthus extract, *Polygonum aviculare* extract, *Polygonum aviculare* extract, *Corchorus olitorius* (HUANG MA) extract, *Salix subfragilis* extract, *Salix babylonica* extract, *Magnolia liliflora* extract, *Kummerowia striata* extract, tamazakifujime extract, *Wikstroemia indica* extract, *Dictyota dichotoma* extract, and shell extracts (e.g., cockle extract, *Perna viridis* extract, oyster extract, *Ostrea edulis* extract, *Patinopecten yessoensis* extract, *Ruditapes philippinarum* extract, Meretrix extract, Mactridae extract, *Nuttallia olivacea* extract, *Anadara broughtoni* extract, Haliotis extract, *Turbo cornutus* extract, *Babylonia japonica* extract).

### (5) Humectant

The cosmetic composition according to the present invention may contain a humectant. Specific examples of the humectant include gum arabic, benzoin gum, dammar gum, guaiac gum, Irish moss, karaya gum, tragacanth gum, carob gum, quince seed, agar or derivatives thereof, casein, sugars such as glucose, galactose, mannose, xylose, fructose, maltose, isomaltose, cellobiose, gentiobiose, trehalose, kojibiose, laminaribiose, nigerose, cellobiose, sanbubiose, neohesperidose, apiose, hamamelose, streptose, hydroxystreptose, dihydrostreptose, 2-methylerythrose or derivatives thereof, 2-methylerythronolactone, mycarose, cladinose, axenose, arcanose, olivomicose, chromose, evamicose, vinelose, nogalose, virenose, noviose, moenuronic acid, garosamine, sibirosamine, N-acylkansosamine, vancosamine, evanitrose, rubranitrose, tetronitrose, pyralose, alpha-octose, trioxacarcinose, aldogalose, and blastminocin or esters thereof, trehalose or derivatives thereof, D-mannosamine and derivatives thereof, isomaltooligosaccharide (panose), primeverose or derivatives thereof, dextrin, gelatin, pectin, starch, carrageenan, carboxymethyl chitin or chitosan, hydroxyalkyl (C2 to C4) chitin or chitosan with the addition of alkylene (C2 to C4) oxide such as ethylene oxide, low molecular weight chitin or chitosan, chitosan salts, sulfated chitin or chitosan, phosphated chitin or chitosan, alginic acid or salts thereof, hyaluronic acid or salts thereof, chondroitin sulfuric acid or salts thereof, beta-1,3-glucan, beta-1,4-glucan, beta-1,6-glucan, heparin, ethylcellulose, methylcellulose, carboxymethylcellulose, carboxyethylcellulose, sodium carboxyethylcellulose, hydroxyethylcellulose, hydroxypropylcellulose, nitrocellulose, crystalline cellulose, hydroxypropylmethylcellulose, polyvinyl alcohol, polyvinylmethylether, polyvinylpyrrolidone, polyvinyl methacrylate, polyacrylates, polyalkylene oxides (e.g., polyethylene oxide or polypropylene oxide) or crosslinked polymers thereof, carboxyvinyl polymers, polyethyleneimine, water-soluble polymers such as dermatan sulfate and keratan sulfate, glycerin fatty acid pyrrolidone carboxylic acid esters, glycerin fatty acid acetyl amino acid esters, pyrrolidone carboxylic acid or salts thereof, polyaspartic acid or salts thereof, polyglutamic acid or salts thereof, polylysine or salts thereof, sodium lactate, hidantoin and derivatives thereof, N-p-vinylbenzyl-D-cellobionamide, N-p-vinylbenzyl-D-lactoneamide, N-p-vinylbenzyl-D-maltonamide, N-p-vinylbenzyl-D-gluconamide, glucosyloxyethyl methacrylate, galactosyloxypropyl acrylate, mannosyloxyethyl methacrylate, glutamyl lysine, glutaurine, 1,2,4-butanetriol, carane-3,4-diol, azukisaponin (e.g., 3-o-[beta-D-glucopyranosyl-beta-D-glucuronopyranosyl]-sophor adiol, 3-o-[beta-D-glucopyranosyl-3-D-glucuronopyranosyl]-soyasapon genol B, 3-o-[beta-D-glucopyranosyl-beta-D-glucuronopyranosyl]-azukis apogenol, 3-o-[beta-D-glucopyranosyl]-28-o-[beta-D-glucopyranosyl-beta -D-glucopyranosyl]-gypsogenic acid, 3-o-[alpha-L-rhamnopyranosyl-beta-D-glucopyranosyl-beta-D-gl ucuronopyranosyl]-soyasapongenol B, and 3-o-[beta-D-glucopyranosyl-beta-D-glucuronopyranosyl]-29-o-[ beta-D-glucopyranosyl-beta-D-glucopyranosyl]-azukisapogenol), *Aucuba japonica* (Aoki) extract, *Firmiana simplex* extract, *Firmiana platanifolia* (WU TONG) extract, *Astilbe thunbergii* (CHI SHENG MA) extract, *Trifolium pratense* extract, *Thalictrum minus var. hypoleucum* extract, Aguaje (*Mauritia flexuosa*) extract, *Ipomoea purpurea* extract, *Ipomoea nil* (QIAN NIU ZI) extract, Achira (*Canna edulis*) extract, *Brassica rapa var. amplexicaulis* extract, *Gynostemma pentaphyllum* extract, *Amana edulis* (SHAN CI GU) extract, *Amaranthus* extract, *Amaranthus tricolor* extract, *Amaranthus caudatus* extract, *Amaranthus caudatus* extract, *Amaranthus cruentus* extract, *Amaranthus patulus* extract, *Amaranthus retroflexus* L. extract, *Amaranthus spinosus* extract, *Epipactis helleborine* extract, *Amaranthus hypochondriacus* extract, *Alstroemeria* spp. (alstroemeria) extract, Alkanna (*Anchusa officinalis*) extract, algarrobo (*Prosopis julifera*) extract, aloe vera extract, *Juncus effusus var. decipiens* (DENG XIN CAO) extract, *Taxus cuspidata* (ICHII) extract, strawberry extract, *Ceratonia siliqua* extract, *Draba nemorasa* (TEIREKISHI) extract, rice extract, catnip extract, *Conandron ramondioides* (Iwatabako) extract, *Selaginella tamariscina* extract, *Selaginella tamariscina* (JUAN BAI) extract, Jenipapo *(Genipa americana)* extract, *Spirodela polyrhiza* (FU PING) extract, *Althaea officinalis* L. extract, *Malva sylvestris* extract, Una de Gato (Uncaria) extract, *Flammulina velutipes* (Enokitake) extract, *Pleurotus eryngii* extract, *Pleurotus eryngii* extract, *Opuntia maxima* extract, *Inula helenium* (TU MU XIANG) extract, *Eriocaulon buergerianum* (GU JING CAO) extract, oka extract, *Cnidii monnieri* (SHE CHUANG ZI) extract, *Abelmoschus esculentus* extract, *Dioscorea tokoro* extract, *Dioscorea* extract, *Dioscorea hypoglauca* (BEI XIE) extract, *Ononis spinosa* extract, ulluco extract, *Glechoma hederacea* extract, *Glechoma hederacea* (RENSENSOU) extract, *Hydrangea macrophylla* extract, *Cascara Sagrada* extract, *Gypsophila elegans* extract, *Panax ginseng* extract, kaniwa extract, *Dianthus caryophyllus* extract, *Gerbera hybrids* extract, Camu Camu (*Myrciaria dubia*) extract, *Zantedeschia* spp. extract, *Avena sativa* extract, *Pseudocydonia sinensis* (MU GUA) extract, *Potentilla sundaica* (WEI LING CAI, FAN BAI CAI) extract, *Veronica undulata* extract, *Dianthus superbus var.longicalycinus* (SHI ZUH) extract, *Dianthus superbus var. superbus* (QU MAI, QU MAI ZI) extract, *Agave cantala* extract, *Chrysanthemum boreale* extract, *Chenopodinm quinoa willdenow* extract, *Brassica oleracea* extract, *Actinidia chinensis extract, Cucumis Sativus* extract, *Tamarix chinensis* (Seikaryu, Teiryu) extract, *Ajuga decumbens* (Kiransou) extract, *Parthenium argentatum* extract, *Asparagus cochinchinensis* (TIAN MEN DONG) extract, *Pueraria lobata* (GE GEN) extract, *Artemisia annua* (HUANG HUA HAO) extract, *Theobroma grandiflorum* extract, *Thlaspi arvense* (Sekimei, Sekimeishi) extract, *Hovenia dulcis* (ZHI JU ZI) extract, *Epiphyllum oxypetalum* (Gekkabijin) extract, *Picrorhiza kurrooa* root (HU HUANG LIAN) extract, *Cocos nucifera* extract, *Cosmos bipinnatus* extract, *Clematis apiifolia var. biternata* extract, rice oil extract, *Amorphophallus rivieri var. konjac* extract, *Crocus sativus* (YU HONG HUA, XI HONG HUA) extract, *Achras zapota* extract, *Sangre de grado* (*Cronton*) extract, *Panax* *notoginseng* (SAN QI REN SHEN) extract, *Shea (Butyrospermum parkii*) extract, *Butyrospermum parkii* extract, *Lentinula edodes* (Shiitake) extract, *Lyophyllum* extract, Hinshimeji extract, *Lyophyllum fumosum* extract, *Lyophyllum decastes* extract, *Lyophyllum connatum* extract, *Hypsizigus marmoreus* extract, *Honjimeshi Lyophyllum shimeji* extract, *Hypsizygus ulmarium* extract, *Magnolia liliflora Desr.* (XIN YI HUA) extract, XI XIN (*Asarum Heterotropoides*) extract, cactus extract, *Ophiopogon japonicus* (MAI MEN DONG) extract, *Gypsophila* extract, *Dichroa febrifuga Lour.* (CHANG SHAN) extract, *Sesbania grandiflora Pers.* extract, *Abrus precatorius* (JI GU CAO) extract, *Daphne odora* (RUI XIANG, RUI XIANG HUA, DING XIANG) extract, *Citrullus lanatus* (XI GUA) extract, *Cynanchum paniculatum* (JYOCHOUKEI) extract, *Portulaca* (MA CHI XIAN, MA CHI XIAN ZI) extract, *Dendrobium moniliforme* extract, *Dendrobium linawianum* extract, *Dendrobium nobile* extract, *Oobanasekkoku* extract, *Dendrobium okinawense* extract, *Dendrobium candidum* extract, *Dendrobium phalaenopsis* extract, *Shikakusekkoku* extract, *Dendrobium tosaense* extract, *Cedron* (*Aloysia triphylla*) extract, *Malva sylvestris var. mauritiana* extract, *Apium graveolens* extract, *Andrographis paniculata* (CHUAN XIN LIAN) extract, *Raphanus sativus* (Daikon) extract, *Eclipta prostrata* (HAN LIAN CAO) extract, *Dioscorea gracillima* (BEI XIE) extract, *Dahlia pinnata* extract, Tarwi extract, *Euphorbia lathyris* extract, *Atractylodes lancea* extract, Chanca piedra (*Phyllanthus amarus*) extract, *Tulipa* extract, *Stachys Sieboldii* extract, *Agaricus bisporus* (mushroom) extract, *Camellia japonica* extract, *Sagina japonica* (QI GU CAO) extract, *Dipsacus asper* (SENDAN) extract, *Zea mays* extract, stigmas from the female flowers of *Zea mays* (*NANBANMOU*) extract, *Equisetum hyemale* (MU ZEI) extract, *Fraxinus* (QIN PI) extract, *Eustoma grandiflorum* extract, *Canavalia gladiata* extract, *Canavalia gladiata* extract, *Zizyphus jujuba Miller var. inermis Rehder* (DA ZAO) extract, *Saussurea pulchella* (ZOKUDAN) extract, *Pholiota nameko* extract, *Boehmeria nivea* extract, *Viola odorata* extract, *Myristica fragrans* extract, *Albizia julibrissin Durazz* extract, *Albizia julibrissin Durazz* extract, *Albizia julibrissin Durazz* extract, *Albizia julibrissin Durazz* extract, *Lycoris radiata* (Nemu) extract, *Celosia argentea* extract, *Gentiana decumbens* extract, *Acer japonicum* extract, *Lupinus* extract, *Carica papaya* extract, Pajaro Bobo extract, Balata rubber extract, *Agaricacease* extract, *Agaricus arvensis* extract, *Agaricus abruptibulbus Peck* extract, *Aspidistra* elatior extract, *Pariurusu* (Christ's thorn) extract, balsamina (goya, *Momordica charantia*) extract, *Gentiana thunbergii* extract, *Sterculia* (PANG DAI HAI) extract, *Dahlia coccinea* extract, *Beta vulgaris* extract, *Thujopsis dolabrata* extract, *Helianthus annuus* extract, *Capsicum annuum var*. *grossum extract, Semiaquilegia adoxoides* (Tian kui) extract, *Agaricus blazei Murrill* extract, *Cordycips sinensis* (DONG CHONG XIA CAO) extract, *Periandra dulcis* extract, *Corylus avellana* extract, *Luffa aegyptiaca* extract, *Crassulaceae* extract, *Crassulaceae* (JING TIAN) extract, *Kochia scoparia* extract, *Kochia scoparia* extract, *Kochia scoparia* extract, *Kochia scoparia* (DI FU ZI) extract, *Impatiens balsamina* (FUNG SIN, Impatiens seeds, TOU GU CAO) extract, *Stellaria dichotoma* extract, *Sedum aizoon var*. *aizoon* (JING TIAN SAN QI) extract, *Simmondsia chinensis* extract, *Borago officinalis* extract, *Peumus boldus* extract, *Cistanche salsa* (ROU CONG RONG, *DAIGEI*) extract, *Dendrobium officinale* (TEPPI SHI FU, HUO SHAN SHI FU) extract, *Lepidium meyenii* extract, *Macadamia nut* extract, *Argyranthemum frutescens* extract, *Tropaeolum tuberosum* extract, *Tropaeolum tuberosum* extract, *Actinidia polygama* (Matatabi) extract, *Matsukasa* extract, *Poria cocos* (*FU LING*) extract, Matico (*Buddleja globosa;* Korudonshijo) extract, *Cydonia oblonga* extract, *Mullein* (*Verbascum thapsus*) extract, *Manzanilla* (Andes' chamomile) extract, *Ottelia japonica* extract, *Ottelia japonica* (RYUZETSUSO) extract, *Mentha viridis* extract, *Helichrysum bracteatum* extract, Munya extract, *Callicarpa japonica* extract, *Callicarpa japonica var*. *luxurians* (SHIJU) extract, *Millettia reticulata* (JI XUE TENG) extract, Molle (*Schinus molle*) extract, *Ilex aquifolium* extract, *Rodgersia podophylla* extract, *Polymnia sonchifolia* extract, palm extract, *Carpesium abrotanoides* (KAKUSHITZU, TENMEISEI) extract, *Aesculus hippocastanum* extract, *Chaenomeles lagenaria* extract, *Lilium* extract, *Polyporus mylittae* (LEI WAN) extract, *Citrus* *aurantifolia* extract, *Secale cereale* extract, *Allium chinense* extract, *Allium ascalonicum* (XIE BAI) extract, *Malus domestica* extract, *Gentiana triflora* extract, *Litchi chinensis* (LI ZHI, LI ZHI HE) extract, *Astragalus sinicus* extract, YI YI REN extract, Minisasanishiki extract, *Chlorella vulgaris* extract, *Chlorella pyrenoidosa* extract, *Chlorella ellipsoidea* extract, *Macrocystis pyrifera* extract, *Acanthopeltis japonica* extract, *Meristotheca papulosa* extract, *Gelidium japonicum* extract, *Amakusanori* extract, *Spirogyra sp.* extract, *Prasiola japonica* extract, *Aegagropila Linnaei* extract, *Kornmannia leptoderma* extract, *Dictyopteris divaricata* extract, *Petalonia binghamiae* extract, *Petalonia fascia* extract, *Colpomenia bullosa* extract, *Agarum clathratum* extract, *Costaria costata* extract, *Kjellmaniella gyrata* extract, *Eckloniopsis radicosa* extract, *Ecklonia stolonifera* extract, *Hedophyllum* extract, *Arthrothamnus bifidus* extract, *Alaria praelonga* extract, *Alaria crassifolia* extract, *Pelvetia wrightii* extract, *Cystoseira prolifera* extract, *Turbinaria ornata* extract, *Cystoseira hakodatensis* extract, *Myagropsis myagroides* extract, *Hiemoku* extract, *Sargassum nipponicum* extract, *Sargassum fulvellum* extract, *Coccophora langsdorfii* extract, *Bangia atropurpurea* extract, *Porphyra yezoensis* extract, *Trichogloea hequieni* extract, *Nemalion vermiculare* extract, *Scinaia japonica* extract, *Portieria hornemanni* extract, *Gloiopeltis complanata* extract, *Gloiosiphonia capillaris* extract, *Bonnemaisonia hamifera* extract, *Solieria pacifica* extract, *Solieria tenuis* extract, *Kikutosaka* extract, *Turnerella mertensiana* extract, *Hypnea charoides* extract, *Hypnea japonica* extract, *Hypnea saidana* extract, *Hypnea variabilis* extract, *Gracilaria vermiculophylla* extract, *Gracilaria chorda* extract, *Hachijotengusamodoki* extract, *Ahn feltiopsis flabelliformis* extract, *Mazzaella japonica* extract, metabolites of Bacillus subtilis (natto), Natto extract, loofah lotion, and sap from *Betula platyphylla var. japonica* or *Pinus densiflora.*

### (6) Cell activator/Metabolic activator

The cosmetic composition according to the present invention may contain a cell activator/metabolic activator. Specific examples of the cell activator/metabolic activator include vitamin A group: retinol or salts thereof and their derivatives (e.g., retinol unsaturated fatty acid esters such as retinyl linoleate, retinyl linoleate, retinyl oleate, and retinyl arachidonate), retinal or salts thereof and their derivatives, dehydroretinal or salts thereof and their derivatives, retinoic acid or salts thereof and their derivatives, retinoic acid analog compounds (e.g., 4-[[[8-(3,5-dimethylphenyl)-2-naphthalenyl)carbonyl]amino]be nzoic acid, 4-[[[8-(3-methylphenyl)-2-naphthalenyl)carbonyl]amino]benzoi c acid, 4-[[[8-(9-methylphenyl)-2-naphthalenyl]carbonyl]amino]benzoi c acid, 4-[[[8-(2-methylphenyl)-2-naphthalenyl]carbonyl]amino]benzoi c acid, 4-[[[8-(3,4-dimethylphenyl)-2-naphthalenyl]carbonyl]amino]be nzoic acid, 4-[[[8-(2,4-dimethylphenyl)-2-naphthalenyl]carbonyl]amino]be nzoic acid, 4-[[[8-(2-isopropylphenyl)-2-naphthalenyl]carbonyl]amino]ben zoic acid, 4-[[[8-(2-ethylphenyl)-2-naphthalenyl]carbonyl]amino]benzoic acid, 4-[[[8-(2-fluorophenyl)-2-naphthalenyl]carbonyl]amino]benzoi c acid, 4-[[[8-(2-methoxyphenyl)-2-naphthalenyl]carbonyl]amino]benzo ic acid, 4-[[(8-benzyl-2-naphthalenyl)carbonyl]amino]benzoic acid, and 4-[(E)-2-(8-phenyl-2-naphthalenyl)propenyl]benzoic acid), retinolic acid derivatives (e.g., 4-hydroxyphenyl retinamide), retinoid analog compounds (e.g, 4-[[(5,6-dihydro-5,5-dimethyl-8-ethyl-2-naphthalenyl)amino]c arbonyl]benzoic acid, 4-[[(5,6-dihydro-5,5,8-trimethyl-2-naphthalenyl)amino]carbon yl]benzoic acid, 4-[[(5,6-dihydro-5,5-dimethyl-8-phenyl-2-naphthalenyl)amino] carbonyl]benzoic acid, 4-[[(5,6-dihydro-5,5-dimethyl-8-ethyl-2-naphthalenyl)carbony 1]amino]benzoic acid, 4-[[(5,6-dihydro-5,5,8-trimethyl-2-naphthalenyl)-carbonyl]am ino]benzoic acid, 4-(E)-[2-(5,6-dihydro-5,5-dimethyl-8-phenyl-2-naphthalenyl)-1-propenyl]benzoic acid, 4-[[(5,6-dihydro-5,5-dimethyl-8-phenyl-2-naphthalenyl)oxy]ca rbonyl]benzoic acid, 4-[[(5,6-dihydro-5,5-dimethyl-8-phenyl-2-(naphthalenyl)carbo nyl]amino]benzoic acid, 4-[[(5,6-dihydro-5,5-dimethyl-8-phenyl-2-(naphthalenyl)carbo nyl]oxy]benzoic acid, 4-[[5,6-dihydro-5,5-dimethyl-8-(2-fluorophenyl)-naphthalenyl ]carbonyl]amino]benzoic acid, 4-[[(5,6-dihydro-5,5,6-trimethyl]-8-phenyl-2-(naphthalenyl)c arbonyl]amino]benzole acid, 4-[[(5,6-dihydro-5,5,7-trimethyl-8-phenyl-2-naphthalenyl)car bonyl]benzoic acid, 4-[[(E)-(5,6-dihydro-5,5-dimethyl-8-phenyl)-2-naphthalenyl]v inyl]benzoic acid, 4-[[[(5,6-dihydro-5,5-dimethyl-8-phenyl)-2-naphthalenyl]carb onyl]sulfamyl]benzoic acid, 4-[[[(5,6-dihydro-5,5-dimethyl-8-phenyl-2-naphthalenyl]sulfa myl]carbonyl]benzoic acid, 4-[[(5,6-dihydro-5,5-dimethyl-8-phenyl)-2-naphthalenyl]ethyl ]benzoic acid, 4-[[[(5,6-dihydro-5,5-dimethyl-8-phenyl)-2-naphthalenyl]thio carbonyl]amino]benzoic acid, 4-[[[(5,6-dihydro-5,5-dimethyl-8-phenyl)-2-naphthalenyl]carb onyl]methyl]benzoic acid, 4-[[[(5,6-dihydro-5,5-dimethyl-8-phenyl)-2-naphthalenyl]meth yl]oxy]benzoic acid, 4-[[[(5,6-dihydro-5,5-dimethyl-8-phenyl)-2-naphthenyl]oxy]me thyl]benzoic acid, 4-[[[(5,6-dihydro-5,5-dimethyl-8-(2,4-dimethylphenyl)]-2-nap hthalenyl]carbonyl]amino]benzoic acid, 4-[[[(5,6-dihydro-5,5-dimethyl-8-(4-methylphenyl)]-2-naphtha lenyl]carbonyl]amino]benzoic acid, 4-[[(5,6-dihydro-5,5-dimethyl-8-phenyl)-2-naphthalenyl]ethyl ]benzoic acid, 4-[[[(5,6-dihydro-5,5-dimethyl-8-phenyl)-2-naphthalenyl]sulf amyl]methyl]benzoic acid, 4-[[(5,6-dihydro-5,5-dimethyl-8-phenyl)-2-naphthalenyl]methy 1]amino]benzoic acid, 4-[[[(5,6-dihydro-5,5-dimethyl-8-phenyl)-2-naphthalenyl]amin o]thiocarbonyl]benzoic acid, 4-[[[(5,6-dihydro-5,5-dimethyl-8-phenyl)-2-naphthalenyl]meth yl]sulfamyl]benzoic acid, 4-[[[(5,6-dihydro-5,5-dimethyl-8-phenyl-2-naphthalenyl]amino ]methyl]benzoic acid, 4-[[[(5,6-dihydro-5,5-dimethyl-8-phenyl)-2-naphthalenyl]carb onyl]amino]-2-hydroxybenzoic acid, 4-[[[(5,6-dihydro-5,5-dimethyl-8-phenyl)-2-naphthalenyl]carb onyl]amino]-2-nitrobenzoic acid, 4-[[[(5,6-dihydro-5,5-dimethyl-8-phenyl)-2-naphthalenyl]carb onyl]amino]-2-fluorobenzoic acid, 4-[[[(5,6-dihydro-5,5-dimethyl-8-phenyl-2-naphthalenyl]carbo nyl]amino]-2-methoxybenzoic acid, 4-[[[(5,6-dihydro-5,5-dimethyl-8-(2-naphthalene)]-2-naphthal enyl]carbonyl]amino]benzoic acid, 4-[[(5,6-dihydro-8-phenyl-2-naphthalenyl)carbonyl]aminobenzo ic acid, 4-[[(5,6-dihydro-5,5-dimethyl-8-phenyl-2-naphthalenyl)carbon yl]amino]-3-fluorobenzoic acid, 4-[[(5,6-dihydro-5,5-dimethyl-8-phenyl-2-naphthalenyl)carbon yl]amino]-3-methylbenzoic acid, 4-[[(5,8,10,10a-tetrahydro-10,10-dimethyl-9-phenyl-2-anthrac enyl)carbonyl]amino]benzoic acid, 4-[[(1,1-dimethyl-3-phenyl-1H1-inden-5-yl)amino]carbonyl]ben zoic acid, 4-[(1,1-dimethyl-3-phenyl-1H-inden-5-yl-oxymethyl)benzoic acid, 4-[2-(1,1-dimethyl-3-phenyl-1H-inden-5-yl)vinyl]benzoic acid, and 4-[(1,1-dimethyl-3-phenyl-1H-inden-5-carbonyl)amino]benzoic acid), carotene or salts thereof and their derivatives (e.g., carotenoids such as alpha-carotene, beta-carotene, gamma-carotene, lycopene, cryptoxanthin, lutein, zeaxanthin, isozeaxanthin, rodoxanthin, capsanthin, and crocetin), lycopene or salts thereof and their derivatives, vitamin B group: thiamine or salts thereof and their derivatives (e.g., thiamine hydrochloride, thiamine disulfide, bisbentiamine, bisibutiamine, thiamine monophosphate disulfide, benfotiamine, cycotiamine, octotiamine, dicetiamine, fursultiamine, prosultiamine, and astaxanthin thiamine phosphate diesters), thiamine sulfate, riboflavin or salts thereof and their derivatives (e.g., flavinadenine dinucleotide, flavinmononucleotide, riboflavin phosphate diester, 1-beta-D-ribofuranosyl nicotine amide pyrophosphate diester, and 1-beta-D-ribofuranosyl nicotinic acid), pyridoxine or salts thereof and their derivatives (e.g., pyridoxine 3,4-dipalmitate, pyridoxine 3,4-dicaprylate, and pyridoxine sulfate diesters), pyridoxal or salts thereof and their derivatives, pyridoxiamine or salts thereof and their derivatives, cyanocobalamin or salts thereof and their derivatives, cobalamins (e.g., methylcobalamin, adenosylcobalamin, hydroxocobalamin, and aquacobalamin), folic acid or salts thereof and their derivatives, nicotinic acid or salts thereof and their derivatives, pantothenic acid or salts thereof and their derivatives, biotin or salts thereof and their derivatives, choline or salts thereof and their derivatives, inositol or salts thereof and their derivatives, vitamin C group: ascorbic acid or salts thereof and their derivatives , vitamin D group: ergocalciferol or salts thereof and their derivatives, cholecalciferol and salts thereof and their derivatives (e.g., 1alpha-hydroxy-22-[(1-hydroxy-1-methyl)-2-cyclopenten-4-yl-o xy]-23,24,25,26,27-pentanolcholecalciferol, [1R,4R]-1alpha-hydroxy-22-[(1-hydroxy-1-methyl)-2-cyclopente n-4-yl-oxy]-23,24,25,26,27-pentanolcholecalciferol, [1R,4S]-1alpha-hydroxy-22-[(1-hydroxy-1-methyl)-2-cyclopente n-4-yl-oxy]-23,24,25,26,27-pentanolcholecalciferol, [1S,4R]-1alpha-hydroxy-22-[(1-hydroxy-1-methyl)-2-cyclopente n-4-yl-oxy]-23,24,25,26,27-pentanolcholecalciferol, and [1S,4S]-lalpha-hydroxy-22-[(1-hydroxy-1-methyl)-2-cyclopente n-4-yl-oxy]-23,24,25,26,27-pentanolcholecalciferol), dihydrotachysterol or salts thereof and their derivatives, vitamin E group: tocopherol or salts thereof and their derivatives, tocotrienol or salts thereof and their derivatives, ubiquinone or salts thereof and their derivatives, vitamin K group: phytonadione or salts thereof and their derivatives, menaquinone or salts thereof and their derivatives, menadione or salts thereof and their derivatives, menadiol or salts thereof and their derivatives, vitamin F group: linoleic acid or salts thereof and their derivatives, linolenic acid or salts thereof and their derivatives, and arachidonic acid or salts thereof and their derivatives, carnitine or salts thereof and their derivatives, ferulic acid or salts thereof and their derivatives, gamma-oryzanol or salts thereof and their derivatives, orotic acid or salts thereof and their derivatives, vitamin P group: rutin or salts thereof and their derivatives, eriocitrin or salts thereof and their derivatives, hesperidin or salts thereof and their derivatives, vitamin L group: anthranilic acid or salts thereof and their derivatives, adenylthiomethyl pentose or salts thereof and their derivatives, vitamin U group: methylmethionine sulfonium chloride or derivatives thereof, valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, and histidine or derivatives thereof (e.g., N-octyloxycarbonyl-beta-alanyl-L-histidine, N-dodecyloxycarbonyl-beta-alanyl-L-histidine, N-(12-amino-1-oxododecyl)-L-histidine, N-2-ethylhexyl oxycarbonyl-beta-alanyl-L-histidine hydrochloride, N-hexadecyloxycarbonyl-beta-alanyl-L-histidine, N-octylaminocarbonyl-beta-alanyl-L-histidine, N-dodecylaminocarbonyl-beta-alanyl-L-histidine, N-dodecylsulfonyl-beta-alanyl-L-histidine, and N-dodecylamino-oxalyl-beta-alanyl-L-histidine), sulfates, phosphates, nitrates, and citrates thereof, or amino acids including amino acid derivatives such as pyrrolidonecarboxylic acid, alpha-hydroxy acids such as glycolic acid, citric acid, malic acid, tartaric acid, lactic acid and succinic acid, 2-hydroxycarboxylic acids (e.g., methyllactic acid, 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecanoic acid, alpha-hydroxylauric acid, alpha-hydroxymyristic acid, alpha-hydroxypalmitic acid, alpha-hydroxystearic acid, alpha-hydroxyarachidonic acid, cerebronic acid, alpha-hydroxynervonic acid, mandelic acid, benzilic acid, phenyllactic acid, atrolactic acid, 2-(4'-hydroxyphenyl)-2-hydroxyethanoic acid, 2-(4'-chlorophenyl)-2-hydroxyethanoic acid, 2-(3'-hydroxy-4'-methoxyphenyl)-2-hydroxyethanoic acid, 2-(4'-hydroxy-3'-methoxyphenyl)-2-hydroxyethanoic acid, 3-(2'-hydroxyphenyl)-2-hydroxypropanoic acid, 3-(4'-hydroxyphenyl)-2-hydroxypropanoic acid, 2-(3',4'-dihydroxyphenyl)-2-hydroxyethanoic acid, glyceric acid, erythrohic acid, ribonic acid, arabinonic acid, xylonic acid, lyxonic acid, allonic acid, altronic acid, gluconic acid, mannonic acid, gulonic acid, idonic acid, galactonic acid, talonic acid, glucoheptonic acid, galactoheptonic acid, tartronic acid, and mucic acid), polyhydroxycarboxylic acids or hydroxypolycarboxylic acids (e.g., gluconolactone, galactonolactone, glucuronolactone, galacturonolactone, gulonolactone, ribonolactone, saccharic acid lactone, pantoyllactone, glucoheptonolactone, mannonolactone, and galactoheptonolactone), 2-keto acids (e.g., glyoxylic acid, 2-ketoethanoic acid methyl, benzoylformic acid, methyl benzoylformate, ethyl benzoylformate, phenylpyruvic acid, methyl phenylpyruvate, ethyl phenylpyruvate, 2-ketobutanoic acid, 2-ketopentanoic acid, 2-ketohexanoic acid, 2-ketoheptanoic acid, 2-ketooctanoic acid, 2-ketododecanoic acid, and 2-ketooctanoic acid methyl), quinic acid, isocitric acid, tropic acid, trethocanic acid, 3-chlorolactic acid, cerebronic acid, citramalic acid, agaricic acid, aleuritic acid, pantoic acid, lactobionic acid, hexulosonic acid, photosensitizer 301, hinokitiol, pantothenic acid or derivatives thereof, allantoin, pentadecanoic acid glyceride, linolenic acid or derivatives thereof, eicosapentaenoic acid or derivatives thereof, docosahexaenoic acid or derivatives thereof, estradiol, ethenyl estradiol, antiarol or glycosides thereof, lyoniresinol or glycosides thereof, rhododendrol or glycosides thereof, platyphylonol or glycosides thereof, lactone compounds (e.g., D-glucurono-6,3-lactone, alpha-D-glucoheptonic-gamma-lactone, delta-glucuronolactone, alpha, beta-glucooctonic-gamma-lactone, L-gulonic-gamma-lactone, gamma-D-galactonolactone, D-saccharic-1,4-lactone, D-saccharic-3,6-lactone, D-ribonic-gamma-lactone, alpha-butyrolactone, gamma-butyrolactone, alpha-octanoic lactone, gamma-octanoic lactone, nonanoic lactone, gamma-valerolactone, D-mannoic-delta-lactone, D-xyloic-delta-lactone, D-arabinoic-delta-lactone, stearoyl-delta-gluconolactone, DL-pantolactone, and palmitoyl-DL-pantolactone), 3-hydroxy-3,4-dicarboxy-1,4-butanolid or derivatives thereof, 6-benzylaminopurine or derivatives thereof, 1,4-diazadicyclooctane, 2,5-dimethylfuran, 2-methylfuran, 2,5-diphenylfuran, 1,3-diphenylisobenzofuran, rutin, tectorigenin 7-xylosylglucoside, captopril, alacepril, lisinopril, enalapril, delapril, benazepril, cilazapril, imidapril, quinapril, trandolapril, perindopril, temocapril, losartan, endoserine, galactomannan saccharide polymers, mucin, trimethylglycine, proteoglycan, *Lactobacillus* extract, *Lactobacillus* culture extract, *Lactobacillus* fermented milk extract, *Bifidobacterium* extract, *Bifidobacterium* culture extract, *Bifidobacterium* fermented milk extract, placenta extract, LING ZHI extract, spleen extract, thymus extract, yeast extract, yeast culture extract, yeast fermentation extract, filamentous fungi extract, filamentous fungi culture extract, *Basidiomycetes* extract, *Basidiomycetes* culture extract, bacteria cell disruption extract, bacteria culture extract, cultured human dermis cell disruption extract, *Rehmannia glutinosa* (DI HUANG) extract, *Foeniculum vulgare* (HUI XIANG) extract, *Acanthopanax senticosus* (Ezoukogi) extract, *Hordeum vulgare* (Ohmugi) extract, *Echinops latifolius Tausch* extract, *Hypericum perforatum L. var. angustifolium* extract, *Hypericum perforatum* extract, *Petroselinum crispum* extract, *Apium graveolens var. secalinum* extract, *Rehmannia glutinosa Libosch*. *var. hueichingensis* extract, *Anthemis nobilis* extract, *Garcinia cambogia* extract, *Artemisia apiacea* (QING HAO) extract, *Epipactis thunbergii* extract, *Quillaja saponaria* extract, *Cephalanthera falcata* extract, *Cephalanthera erecta* extract, *Calendula officinalis* extract, *Neottia nidus-avis (Neottia nidus-avis var. nidus-avis)* extract, *Cephalanthera longibracteata* extract, *Pterocarya rhoifolia* (walnuts) extract, *Filipendula multijuga* extract, *Adenophora stricta* (SHA SHEN) extract, *Prunus salicina* extract, *Juglans regia* extract, *Equisetum arvense* extract, *Ananas comosus* extract, *Orchis aristata* extract, *Belamcanda chinensis* (SHE GAN) extract, *Fagus crenata* extract, *Physalis alkekengi var*. *franchetii* (TOROKON) extract, *Grifola frondosa* (Maiake) extract, *Lythrum anceps* (QIAN QU CAI) extract, *Hibiscus syriacus* (Mukuge) extract, *Sapindus mukurossi* (ENMEIPI) extract, soybean sprout extract, *Eucalyptus* extract, *Saxifraga stolonifera* (HU ER CAO) extract, *Rubus suavissimus* (TIAN CHA) extract, *Lactuca sativa* extract, *Aloe arborescens* extract, *HUANG QIN* extract, tonka beans extract, *Gentiana amarella* extract, *Arctium lappa* extract, *Lithospermum erythrorhizon* extract, *Daucus Carota* extract, *Hamamelis virginiana* extract, *Humulus lupulus* extract, *Coix lachryma-Jobi L.* extract, *Lamium album var. barbatum* extract, *Swertia japonica* extract, *Angelica acutiloba* extract, *Calendula officinalis* extract, *Hydrangea serrata Ser. var. thunbergii* extract, *Hypericum erectum* extract, *Cucumis sativus* extract, *Thymus vulgaris* extract, *Rosmarinus* *officinalis* extract, *Petroselium crispum* extract, *Pachydictyon coriaceum* extract, *Dilophus okamurae* extract, *Gatsugarakonbu* extract, *Laminaria longipedalis* extract, *Laminaria diabolica* extract, *Laminaria yezoensis* extract, *Laminaria longissima* extract, *Laminaria yendoana* extract, *Oochijimikonbu* extract, *Galaxaura fastigiata Decaisne* extract, *Galaxaura falcata Kjellman* extract, *Helminthocladia australis* extract, *Helminthocladia yendoana* extract, *Kagekinori* extract, *Cirrulicarpus gmelini* extract, *Gracilaria bursa-pastoris* extract, and *Gracilaria textorii* extract.

### (7) Antioxidant

The cosmetic composition according to the present invention may contain an antioxidant. Specific examples of the antioxidant include ascorbic acid or salts thereof and their derivatives (e.g., magnesium-L-ascorbic acid phosphate, ascorbyl palmitate, ascorbyl dipalmitate, ascorbic acid hydroxyproline phosphate ester, 5-o-alpha-D-glucopyranosyl-L-ascorbic acid, L-ascorbic acid phosphate ester sodium salt, L-ascorbic acid phosphate ester potassium salt, L-ascorbic acid phosphate ester magnesium salt, L-ascorbic acid phosphate ester calcium salt, L-ascorbic acid phosphate ester aluminum salt, L-ascorbic acid sulfate ester sodium salt, L-ascorbic acid sulfate ester potassium salt, L-ascorbic acid sulfate ester magnesium salt, L-ascorbic acid sulfate ester calcium salt, L-ascorbic acid sulfate ester aluminum salt, L-ascorbic acid sodium salt, L-ascorbic acid potassium salt, L-ascorbic acid magnesium salt, L-ascorbic acid calcium salt, L-ascorbic acid aluminum salt, 6-o-alpha-D-galactopyranosyl-L-ascorbic acid, 2-o-beta-D-galactopyranosyl-L-ascorbic acid, L-ascorbic acid phosphate ester magnesium salt, L-ascorbic acid phosphate ester sodium salt, L-ascorbic acid sulfate ester sodium salt, 6-o-acylascorbic acid phosphate ester sodium salt, 6-o-acylascorbic acid phosphate ester ammonium salt, 6-o-acylascorbic acid phosphate ester isopropanolamine salt, 3-o-isopropyl-L-ascorbic acid, 6-o-alkylascorbic acid phosphate ester potassium salt, 6-o-alkylascorbic acid phosphate ester calcium salt, 6-o-alkylascorbic acid phosphate ester barium salt, 6-o-alkylascorbic acid phosphate ester ammonium salt, 6-o-alkylascorbic acid phosphate ester monoethanolamine salt, 6-o-alkylascorbic acid phosphate ester diethanolamine salt, 6-o-alkylascorbic acid phosphate ester triethanolamine salt, 6-o-alkylascorbic acid phosphate ester monoisopropanolamine salt, 6-o-alkylascorbic acid phosphate ester diisopropanolamine salt, 6-o-alkylascorbic acid phosphate ester triisopropanolamine salt, 3-o-glycosy-L-ascorbic acid, 6-o-beta-D-galactopyranosyl-L-ascorbic acid, ascorbic acid cholesterol phosphate ester, L-ascorbyl palmitate, L-ascorbyl isopalmitate, L-ascorbyl dipalmitate, L-ascorbyl diisopalmitate, L-ascorbyl stearate, L-ascorbyl isostearate, L-ascorbyl distearate, L-ascorbyl diisostearate, L-ascorbyl myristate, L-ascorbyl isomyristate, L-ascorbyl dimyristate, L-ascorbyl diisomyristate, L-ascorbyl 2-ethylhexanoate, L-ascorbyl di-2-ethylhexanoate, oleic acid-L-ascorbic acid, 2-o-alpha-D-glucosyl-L-ascorbic acid, 2-o-alpha-D-maltosyl-L-ascorbic acid, 2-o-alpha-D-maltotriosyl-L-ascorbic acid, 3-o-alpha-D-glucosyl-L-ascorbic acid, 2-o-alpha-D-maltosyl-L-ascorbic acid, 2-o-alpha-D-maltotriosyl-L-ascorbic acid, L-ascorbic acid tetraisopalmitate ester, L-ascorbic acid tetralaurate ester, L-ascorbic acid tetra-2-ethylhexanoate ester, L-ascorbic acid tetraoleate ester, 5,6-isopropylidene-L-ascorbic acid, L-ascorbic acid retinol ester, L-ascorbic acid-DL-tocopherol phosphate ester, L-3-o-ethylascorbic acid, L-ascorbic acid tristearate, L-ascorbic acid tripalmitate, L-ascorbic acid trioleate, ascorbic acid triphosphate ester, 2-o-ascorbyl cinnamate, 2-o-ascorbyl ferulate, 2-o-ascorbyl caffeate, 2-o-ascorbyl sinapate, 2-o-[6-palmitoylascorbyl]-4'-acetoxy ferulate, DL-alpha-tocopherol-2-L-ascorbic acid phosphate diester, ascorbic acid inositol-binding derivatives, ascorbic acid phosphorus amide derivatives, ascorbic acid-arbutin binding compounds, ascorbyl-phosphoryl-cholesterol, chromanyl ascorbic acid derivatives, and ascorbic acid/sialic acid derivatives), stearic acid ester, tocopherol or salts thereof and their derivatives (e.g., alpha-tocopherol, beta-tocopherol, gamma-tocopherol, delta-tocopherol, epsilon-tocopherol, alpha-tocopheryl retinoate, aminomethylated tocopherol, hydroxymethylated tocopherol, tocopheryl phosphate ester, tocopherol acetate, tocopherol nicotinate, tocopherol succinate, tocopherol linoleate, tocopherol orotate, DL-alpha-tocopheryl glucoside, DL-alpha-tocopherylmaltoside, DL-beta-tocopheryl glucoside, DL-beta-tocopherylmaltoside, DL-gamma-tocopheryl glucoside, DL-gamma-tocopherylmaltoside, DL-delta-tocopheryl glucoside, DL-delta-tocopherylmaltoside, D-alpha-tocopheryl glucoside, D-alpha-tocopherylmaltoside, D-beta-tocopheryl glucoside, D-beta-tocopherylmaltoside, D-gamma-tocopheryl glucoside, D-gamma-tocopherylmaltoside, D-delta-tocopheryl glucoside, D-delta-tocopherylmaltoside, L-alpha-tocopheryl glucoside, L-alpha-tocopherylmaltoside, L-beta-tocopheryl glucoside, L-beta-tocopherylmaltoside, L-gamma-tocopheryl glucoside, L-gamma-tocopherylmaltoside, L-delta-tocopheryl glucoside, L-delta-tocopherylmaltoside, 1-(sulfoethylamino)-3-(alpha-tocopheryl-6-yloxy)propan-2-ol, 1-(carboxypropylamino)-3-(alpha-tocopheryl-6-yloxy)propan-2-ol hydrochloride, S-[3-(alpha-tocopheryl-6-yloxy)-2-hydroxypropyl]cysteine, S-[3-(alpha-tocopheryl-6-yloxy)-2-hydroxypropyl]-gamma-gluta myl cysteinyl glycine, N-[3-(alpha-tocopheryl-6-yloxy)-2-hydroxypropyl]aspartic acid, and N-[3-(alpha-tocopheryl-6-yloxy)-2-hydroxypropyl]glutamic acid), tocotrienol or salts thereof and their derivatives (e.g., alpha-tocotrienol, beta-tocotrienol, gamma-tocotrienol, delta-tocotrienol, tocotrienol acetate, tocotrienol nicotinate, tocotrienol succinate, tocotrienol linoleate, tocotrienol orotate), dihydropyridine derivatives (e.g., methyl-3-phenyl-2-propenyl 1,4-dihydro-2,6-dimethyl-4-(3-nitrophenyl)pyridine-3,5-dicar boxylate and salts thereof), benzochroman derivatives, norujihidoroguasereten acid, butylhydroxytoluene (BHT), butylhydroxyanisole (BHA), hydroxytyrosol, parahydroxyanisole, coenzyme Qn (n = 7 to 10), pyrroloquinolinequinone, propyl gallate, sesamol, sesamoline, gossypol, maritimein, sulfuretin, xanthene-2,7-diols, caffeoylquinic acids, propolis, carotenoids (e.g., alpha-carotene, beta-carotene, gamma-carotene, lycopene, lutein, violaxanthin, spirilloxanthin, spheroidene, and astaxanthin), phlorotannin, *Akebia quinata* (MU TONG) extract, *Hydrangea serrata Ser. var. thunbergii* (Amacha) extract, WU LONG CHA extract, *Psoralea corylifolia L.* extract, *Cymbidium kanran* extract, *Fortunella japonica* extract, *Rhus chinensis* extract, *Geranium thunbergii* (LAO GUAN CAO) extract, *Sesamum indicum* extract, sesami cultured cell extractt, *Scrophulariaceae* (XUAN SHEN) extract, rice bran extract, *Punica granatum* extract, *Zanthoxylum piperitum* (SHAN JIAO) extract, *Pyrus communis L.* extract, *Rheum palmatum* (DA HUANG) extract, *Lycopersicon esculentum* extract, *Nandina domestica* seed (NAN TIAN seed) extract, *Cirsium japonicum* (DA JI) extract, *Rosa multiflora* (EIJITSU) extract, *Capsicum annuum cvs.* extract, *Pistacia vera* extract, *Eriobotrya japonica* extract, *Areca catechu* (OHOBUKUPI, BING LANG ZI) extract, *Petasites japonicus* extract, *Chaenomeles speciosa* (MU GUA) extract, *Inula britannica L. ssp*. *linariaefolia (Turcz.) Kitam* extract, *Ephedra sinica* (MA HUANG) extract, *Mimosa pudica* extract, *Ocimum basilicum L.* extract, *Berchemia lineata* extract, *Polygonum hydropiper* extract, *Watafujiutsugi* (MI MENG HUA) extract, *Chlamydomonas pertusa* extract, *Akayukimo* extract, *Padina crassa* extract, *Akabaumiuchiwa* extract, *Hypericum erectum* seed extract, *Hamamelis virginiana* extract, *Syzygium aromaticum* extract, *Melissa officinalis* extract, *Plectranthus japonicus* extract, *Betulaceae betula* extract, *Salvia* extract, *Rosmarinus officinalis* extract, *Nandina domestica* seed extract, *Ginkgo biloba* extract, green tea extract, and *Syzygium aromaticum* extract.

### (8) Active oxygen scavenger/Radical scavenger

The cosmetic composition according to the present invention may contain an active oxygen scavenger/radical scavenger. Specific examples of the active oxygen scavenger/radical scavenger include superoxide dismutase, catalase, glutathione peroxidase, bilirubin, quercetin, quercitrin, catechin, catechin derivatives, rutin or derivatives thereof, gallic acid or salts thereof and their derivatives, curcumin or salts thereof and their derivatives, transferrin, ceruloplasmin, coenzyme Qn (n = 7 to 10), uric acid, bilirubin, metallothionein, stilbenegalloyl glycosides (e.g., 3,5,4'-trihydroxystilbene-4'-o-beta-D-(6"-galloyl)glucopyran oside, and 3,5,4'-trihydroxystilbene-4'-o-beta-D-(2"-galloyl)glucopyran oside), chlorogenic acid phospholipid ester, chlorogenic acid sphingosine ester and derivatives thereof, chlorogenic acid glycolipid ester, chlorogenic acid sugar ester, chlorogenic acid sterol ester, thiazole derivatives or salts thereof (e.g., 2-(3,4-diethoxyphenyl)-4-(3-carboxy-9-hydroxyphenyl)thiazole, 2-(3,4-diethoxyphenyl)-4-[3-carboxy-4-hydroxy-5-(2-methyl-2-propenyl)phenyl]thiazole and 2-(3,4-diethoxyphenyl)-4-(3-carboxy-4-hydroxy-5-methylphenyl )thiazole, 2-(3,4-diethoxyphenyl)-4-(3-carboxy-5-methoxyphenyl)thiazole ), hydroxymatairesinol, allohydroxymatairesinol, hydantoin derivatives, *Haematoxylum campechianum L*. (logwood) extract, *Mallotus japonicus* (Akamegashiwa) extract, *Akaminoakane* extract*, Rubia tinctorum* extract, *Rubia tinctorum* (QIAN CAO GEN) extract, *Hydrangea (Ajisai)* extract, *Quercus variabills* extract, *Ficus carica* extract, *Ginkgo biloba* (YIN GUO) extract, *Asparagus officinalis var. altilis* extract, *Prunus mume* (WU MEI) extract, *Quercus stenophylla* extract, *Artctostaphylos uva-ursi* extract, estragon extract, *Sophora japonica* (HUAI HUA, HUAI HUA MI) extract, *Quercus dentata* (KOKUJU, KOKUYOU) extract, *Inula salicina var*. *asiatica* extract, *Kaba* extract, Betulaceae extract, *Quercus acutissima* (*BOKUSOU*) extract, *Trachelospermum asiaticum* extract, *Citrus kinokuni* (*Citrus leiocarpa, Citrus tachibana, Citrus tangerina, Citrus leiocarpa, Citrus leiocarpa, Citrus reticulata*) extract, santara extract, coffee extract, *Symphytum* *peregrinum* (Hireharisou) extract, *Sasa veitchii* (Sasa) extract, *Perilla frutescens* extract, *Perilla frutescens var. crispa* extract, *Perilla frutescens var. acuta* extract, *Perilla frutescens var. discolor* extract, *Paeonia lactifolia* (SHAO YAO) extract, *Quercus serrata* extract, *Quercus crispula* extract, *Lagerstroemia indica* extract, *Lycii radicis cortex* extract, *Quercus myrsinaefolia* extract, *Trifolium repens* (clover) extract, *Armoracia rusticana* extract, red tea extract, green tea extract, *Farfugium japonicum* extract, *Rubus suavissimus* leaf (TIAN CHA) extract, *Lindera strychnifolia* extract, *Rosa laevigata* (JIN YING ZI) extract, *Arachis hypogaea L.* (peanut) extract, *Carnegiea gigantea* extract, *Cinnamomum cassia* extract, *Cinnamomum zeylanicum* extract, *Cinnamomum japonicum* (GUI PI) extract, *Cinnamomum cassia* (GUI ZHI) extract, *Campsis grandiflora* (LING XIAO HUA) extract, *Nelumbo nucifera* (LIAN) extract, *Brassica campestris* extract, Hadakamugi extract, *Anemarrhena asphodeloides* (ZHI MU) extract, *Rosa rugosa* (MEI GUI HUA) extract, *Rosa* (Bara) extract, Barbasco extract, *Spinacia oleracea L.* extract, *Fagus crenata* extract, *Fagus grandifolia* extract, *Fagus japonica* extract, *Fagus sylvatica* extract, *Calystegia japonica* extract, *Atractylodes lancea* (CANG ZHU) extract, *Paeonia suffruticosa* (MU DAN) extract, *Origanum majorana* (Hanahakka) extract, *Cucumis melo* extract, *Oenothera stricta Ledeb*. *ex Link* extract, *Spiraea thunbergii* extract, *Orchids* extract, *Momordica grosvenori* extract, *Aspalathus linearis* extract, *Iso flosmaris* extract, *Plocamium telfairiae* extract, *Plocamium leptophyllum* extract, *Betula grossa* extract, *Ocimum basilicum* extract, *Saussurea lappa* (MU XIANG) extract, *Geranium robertianum* extract, and *Cymbopogon citratus* extract.

### (9) Lipometabolism promoter

The cosmetic composition according to the present invention may contain a lipometabolism promoter. Specific examples of the lipometabolism promoter include phthalazine derivatives (e.g., 4-ethyl-1-(beta-hydroxyethylamino)phthalazine, 4-N-propyl-1-(beta-hydroxyethylamino)phthalazine, 4-N-butyl-1-(beta-hydroxyethylamino)phthalazine, and 4-N-butyl-1-(beta-hydroxypropylamino)phthalazine), xanthin derivatives (e.g., caffeine, theophylline, theobromine, xanthin, aminophylline, choline theophylline, diprophylline, proxyphylline and oxtriphylline), *Cocculus trilobus* (MU FANG JI) extract, *Cirsium japonicum* extract, *Cephalonoplos segetum* extract, *Cirsium borealinipponense* extract, *Cirsium maritimum* extract, *Cirsium japonicum* extract, *Rhaponticum uniflorum* extract, *Theobroma cacao* extract, *Gentianella alborosea* extract, *Sinomenium acutum* (FAN JI) extract, *Curcuma zedoaria* (E ZHU) extract, *Fumaria officinalis* extract, *Platycodon grandiflorum* (JIE GENG, JIE GENG GEN) extract, *Hedera thombea* extract, *Piper nigrum* (Koshou) extract, *Cola acuminata* extract, *Alisma plantago-aquatica L. var*. *orientale* (ZE XIE) extract, *Citrus grandis* extract, *Cornus officinalis* (SHAN ZHU YU) extract, *Menispermaceae* extract, *Sinomenium acutum* extract, *Dendropolyporus umbellatus* (ZHU LING) extract, *Centella aiatica* extract, *Gelidium elegans* extract, *Gentiana scabra* extract, *Gentiana scabra* (LONG DAN) extract, *Elsholtzia ciliata* extract, *Musa acuminata* extract, Puer tea (PU ER CHA) extract, prum extract, and *Ledebouriella seseloides* (FANG FENG) extract.

### (10) UV blocker/UV absorption promoter

The cosmetic composition according to the present invention may contain a UV blocker/UV absorption promoter. Specific examples of the UV blocker/UV absorption promoter include benzophenone derivatives (e.g., 2-hydroxy-4-methoxybenzophenone, 2-hydroxy-4-methoxybenzophenone-5-sulfonic acid, sodium 2-hydroxy-4-methoxybenzophenone-5-sulfonate, dihydroxydimethoxybenzophenone, sodium dihydroxydimethoxybenzophenone sulfonate, 2,4-dihydroxybenzophenone, and tetrahydroxybenzophenone), 1,2-dihydroxy-4-(2-hydroxyethyl)benzene derivatives (e.g., 1-(2-(9-(3,4-dihydroxycinnamoyl)-3-rhamnosyl)glucosyl)ethyl-3-hydroxy-4-methoxybenzene, 1-(2-(6-(3,4-dihydroxycinnamoyl)-3-rhamnosyl)glucosyl)ethyl-3-hydroxy-4-methoxybenzene, 1-(2-(2-acetyl-9-(3,9-dihydroxycinnamoyl)-3-rhamnosyl)glucos yl)ethyl-3,4-dihydroxybenzene, 1-(2-(2-acetyl-6-(3,4-dihydroxycinnamoyl)-3-rhamnosyl)glucos yl)ethyl-3,4-dihydroxybenzene, 1-(2-(4-(3,4-dihydroxycinnamoyl)-3-rhamnosyl)glucosyl)ethyl-3,4-dihydroxybenzene, 1-(2-(6-(9-hydroxy-3-methoxycinnamoyl)-3-rhamnosyl)glucosyl) ethyl-3,4-dihydroxybenzene, 1-(2-(4-(3,4-dihydroxycinnamoyl)-3-rhamnosyl)glucosyl)-1-met hoxyethyl-3,4-dihydroxybenzene, 1-(2-(4-(3,4-dihydroxycinnamoyl)-3-rhamnosyl)glucosyl)-1-hyd roxyethyl-3,4-dihydroxybenzene, 1-(2-(6-(3,9-dihydroxycinnamoyl)-3-xylosyl)glucosyl)ethyl-3, 4-dihydroxybenzene, 1-(2-(3-allosyl-6-(3-hydroxy-4-methoxycinnamoyl))glucosyl)et hyl-3,4-dihydroxybenzene, 1-(2-(3-glucosyl-6-(3-hydroxy-4-methoxycinnamoyl))glucosyl)e thyl-3-hydroxy-4-methoxybenzene, 1-(2-(3-allosyl-6-(3-hydroxy-4-methoxycinnamoyl)glucosyl)eth yl-3-hydroxy-4-methoxybenzene, 1-(2-(4-(3,9-dihydroxycinnamoyl)glucosyl)ethyl)-3,4-dihydrox ybenzene, 1-(2-(3-(3, 4-dihydroxycinnamoyl)glucosyl)ethyl)-3,4-dihydroxybenzene, 1-(2-(2-(3,4-dihydroxycinnamoyl)glucosyl)ethyl)-3,4-dihydrox ybenzene, and 1-(2-(6-(3,4-dihydroxycinnamoyl)glucosyl)ethyl)-3,4-dihydrox ybenzene), paraaminobenzoic acid derivatives (e.g., paraaminobenzoic acid, ethyl paraaminobenzoate, glyceryl paraaminobenzoate, amyl paradimethylaminobenzoate, and octyl paradimethylaminobenzoate), methoxycinnamic acid derivatives (e.g., ethyl paramethoxycinnamate, isopropyl paramethoxycinnamate, octyl paramethoxycinnamate, 2-ethoxyethyl paramethoxycinnamate, sodium paramethoxycinnamate, potassium paramethoxycinnamate, and mono-2-ethylhexanoic acid glyceryl diparamethoxycinnamate), anthranilic acid derivatives (e.g., methyl anthranilate), urocanic acid derivatives (e.g., urocanic acid, and ethyl urocanate), coumarin derivatives, amino acid-based compounds, benzotriazole derivatives, tetrazole derivatives, imidazoline derivatives, pyrimidine derivatives, dioxane derivatives, camphor derivatives, furan derivatives, pyrone derivatives, nucleic acid derivatives, allantoin derivatives, nicotinic acid derivatives, pyridoxine derivatives, pyridoxal derivatives, pyridoxiamine derivatives, amethoflavone, neosakuranin, 6-dehydrokawain, triazine derivatives (e.g., 4,4'4''-(1,3,5-triazine-2,4,6-triyltriimino)trisbenzoic acid tris-2-ethylhexyl), phosphatidylchromanol derivatives (e.g., 1,2-dilauroylglycero-3-phospho-2'-hydroxymethyl-2',5',7',8'-tetramethyl-6'-hydroxychroman, 1,2-dimyristoylglycero-3-phospho-2'-hydroxymethyl-2',5',7',8 '-tetramethyl-6'-hydroxychroman, 1,2-dipalmitoylglycero-3-phospho-2'-hydroxymethyl-2',5',7',8 '-tetramethyl-6'-hydroxychroman, 1,2-distearoylglycero-3-phospho-2'-hydroxymethyl-2',5',7',8' -tetramethyl-6'-hydroxychroman, 1,2-diarachidonylglycero-3-phospho-2'-hydroxymethyl-2',5',7' ,8'-tetramethyl-6'-hydroxychroman, 1-myristoyl-2-palmitoylglycero-3-phospho-2'-hydroxymethyl-2' ,5',7',8'-tetramethyl-6'-hydroxychroman, 1-myristoyl-2-stearoylglycero-3-phospho-2'-hydroxymethyl-2', 5',7',8'-tetramethyl-6'-hydroxychroman, 1-palmitoyl-2-myristoylglycero-3-phospho-2'-hydroxymethyl-2' ,5',7',8'-tetramethyl-6'-hydroxychroman, 1-palmitoyl-2-stearoylglycero-3-phospho-2'-hydroxymethyl-2', 5',7',8'-tetramethyl-6'-hydroxychroman, 1-stearoyl-2-myristoylglycero-3-phospho-2'-hydroxymethyl-2', 5',7',8'-tetramethyl-6'-hvdroxychroman, and 1-stearoyl-2-palmitoylglycero-3-phospho-2'-hydroxymethyl-2', 5',7',8'-tetramethyl-6'-hydroxychroman), zinc-L-carnosine complex, umbelliferone, esculin, benzyl cinnamate, cinoxate, oxybenzone, dioxybenzone, octabenzone, sulisobenzone, benzoresorcinol, arbutin, guaiazulene, shikonin, baicalin, baicalein, berberine, neoheliopan, chlorophylls, xanthophylls, Escalol, zinc oxide, talc, Kaolin, benzoin (Ansokkou) extract, *Trimeresurus flavoviridis* extract, *Cladophora japonica* extract, *Cladophora sakai* extract, *Cladophora glomerata* extract, *Tremella* extract, *Nostoc commune* extract, FA CAI extract, *Spirulina* extract, *Trichodesmium* extract, Funorinoushike extract, *Gelidium divaricatum* extract, *Gelidium pussillum* extract, *Gelidium pacificum* extract, and *Porphyridium cruentum* extract.

### (11) Astringent

The cosmetic composition according to the present invention may contain an astringent. Specific examples of the astringent include succinic acid, allantoin, zinc chloride, zinc sulfate, zinc oxide, calamine, zinc p-phenolsulfonate, aluminum potassium sulfate, resorcin, ferric chloride, tannins (e.g., tannic acid, hamamelitannin, Acer tannin, gallotannin such as tetragalloylglucose, pentagalloylglucose, hexagalloylglucose, heptagalloylglucose, octagalloylglucose, nonagalloylglucose, decagalloylglucose, undecagalloylglucose, and dodecagalloylglucose, and ellagitannin such as tellimagrandin I, tellimagrandin II, casuarictin, pedunculagin, geraniin, isoterchebin, granatin A, granatin B, chebulic acid, chebulagic acid, casuarinin, nupharin, procyanidin B-2, theasinensin A, and theasinensin B),red grapes extract, *Phaseolus angularis* (CHI XIAO DOU) extract, *Althaea* extract, *Urticaceae* extract, *Cascarilla* extract, *Ceiba pentandra* extract, *Paullinia cupana* extract, *Rubus idaeus* extract, *Fragaria x ananassa Duch* extract, *Rubus phoenicolasius* extract, *Rubus parvifolius* extract, *Rubus palmatus* extract, *Rubus idaeus* extract, *Aloe arborescens* extract, *Crataegus cuneata* extract, *Melissa officinalis L.* extract, *Vaccinium vitis-idaea L.* (YUE JU) extract, Cola vera extract, *Saccharum officinarum* extract, *Magnoliae cortex* extract, *Lonicera japonica* (Kinginka, REN DONG) extract, *Equisetum arvense L.* (*MONKEI*) extract, *Larix occidentalis* extract, *Sambucus nigra* (elder) extract, *Taraxacum* spp. (PU GONG YING) extract, *Sentariumu* extract, *Taraxacum albidum* extract, *Taraxacum mongolicum* extract, *Vigna umbellata* extract, *Galeola* *altissima* extract, *Juglans regia var. orientis* extract, *tormentilla* extract, *Rosa multiflora* extract, *Casuarina equisetifolia* extract, *Alchemilla vulgaris ssp. japonica* extract, *Ricinus communis L.* seed extract, *Vitis* spp. extract, *Prunus domestica* extract, *Veronica spicata* extract, visnaga extract, *Vaccinium myrtillus* extract, *Juglans mandshurica Maxim. var. mandshurica* extract, *Menyanthes trifoliata* extract, *Commiphora molmol* extract, *Melissa officinalis* extract, *Hamamelis japonica var.obtusata* extract, *Populus maximowiczii* extract, *Latania* (Krameria) extract, *Citrus limonum extract,* and *Hinashige* extract.

### (12) Anti-inflammatory agent/interleukin inhibitor/histamine release inhibitor

The cosmetic composition according to the present invention may contain an anti-inflammatory agent/interleukin inhibitor/histamine release inhibitor. Specific examples of the anti-inflammatory agent/interleukin inhibitor/histamine release inhibitor include quinolinone derivatives, dibenzoxepin derivatives, thiotropocin, phthalimide derivatives, flurbiprofen, felbinac, bufexamac, suprofen, 1,4-diphenylpropylpiperazine derivatives, calyxin compounds, chromanol glycoside(2-(alpha-D-glucopyranosyl)methyl-2,5,7,8-tetrameth ylchroman-6-ol), ichthammol, indometacin, kaolin, diphenhydramine hydrochloride, d-camphor, DL-camphor, salicylic acid, sodium salicylate, methyl salicylate, acetylsalicylic acid, hydrocortisone, guaiazulene, chamazulene, chlorpheniramine maleate, diphenhydramine hydrochloride, clemastine fumarate, ciproheptadine hydrochloride, promethazine hydrochloride, piperazine derivatives, alpha-D-phenylglycoside derivatives, glycyrrhizic acid or salts thereof and their derivatives (e.g., alpha-glycyrrhizic acid, beta-glycyrrhizic acid, methyl alpha-glycyrrhizate ester, methyl beta-glycyrrhizate ester, trisodium alpha-glycyrrhizate, monopotassium alpha-glycyrrhizate, dipotassium alpha-glycyrrhizate, monoammonium alpha-glycyrrhizate, trisodium beta-glycyrrhizate, monopotassium beta-glycyrrhizate, dipotassium beta-glycyrrhizate, and monoammonium beta-glycyrrhizate), glycyrrhetinic acid or salts thereof and their derivatives (e.g., alpha-glycyrrhetinic acid, beta-glycyrrhetinic acid, stearyl alpha-glycyrrhetinate, stearyl beta-glycyrrhetinate, pyridoxine alpha-glycyrrhetinate, pyridoxine beta-glycyrrhetinbeta-glycyrrhetinate, glycerin alpha-glycyrrhetinate, glycerin beta-glycyrrhetinate, and disodium 3-succinyloxyglycyrrhetinate), mefenamic acid, phenylbutazone, ibuprofen, ketoprofen, allantoin, calcium pantothenate, panthenol or salts thereof and their derivatives such as panthenyl ethyl ether, epsilon-aminocaproic acid, diclofenac sodium, tranexamic acid or derivatives thereof (e.g., trans-4-benzyloxycarbonylaminomethyl cyclohexane carboxylic acid, trans-4-p-nitrobenzyloxycarbonylaminomethyl cyclohexane carboxylic acid, trans-4-p-chlorobenzyloxycarbonylaminomethyl cyclohexane carboxylic acid, trans-4-p-bromobenzyloxycarbonylaminomethyl cyclohexane carboxylic acid, trans-4-m-chlorobenzyloxycarbonylaminomethyl cyclohexane carboxylic acid, trans-4-p-methoxybenzyloxycarbonylaminomethyl cyclohexane carboxylic acid, trans-4-p-methylbenzyloxycarbonylaminomethyl cyclohexane carboxylic acid, sodium trans-4-benzyloxycarbonylaminomethyl cyclohexane carboxylate, ammonium trans-4-p-nitrobenzyloxycarbonylaminomethyl cyclohexane carboxylate, potassium trans-4-p-chlorobenzyloxycarbonylaminomethyl cyclohexane carboxylate, potassium trans-4-p-bromobenzyloxycarbonylaminomethyl cyclohexane carboxylate, calcium trans-4-m-chlorobenzyloxycarbonylaminomethyl cyclohexane carboxylate, magnesium trans-4-p-methoxybenzyloxycarbonylaminomethyl cyclohexane carboxylate, monoethanol amine trans-4-p-methylbenzyloxycarbonylaminomethyl cyclohexane carboxylate, trans-4-t-butyloxycarbonylaminomethyl cyclohexane carboxylic acid, trans-4-(2-phenylisopropyloxycarbonylaminomethyl)cyclohexane carboxylic acid, trans-4-(p-biphenylisopropyloxycarbonylaminomethyl)cyclohexa ne carboxylic acid, trans-4-(3,5-dimethoxyphenylisopropyloxycarbonylaminomethyl) cyclohexane carboxylic acid, trans-4-(p-methylphenylisopropyloxycarbonylaminomethyl)cyclo hexane carboxylic acid, sodium trans-4-t-butyloxycarbonylaminomethyl cyclohexane carboxylate, potassium trans-4-(2-phenylisopropyloxycarbonylaminomethyl)cyclohexane carboxylate, calcium trans-4-(p-biphenylisopropyloxycarbonylaminomethyl)cyclohexa ne carboxylate, magnesium trans-4-(3,5-dimethoxyphenylisopropyloxycarbonylaminomethyl) cyclohexane carboxylate, monoethanol amine trans-4-(p-methylphenylisopropyloxycarbonylaminomethyl)cyclo hexane carboxylaet, trans-4-(9-fluorenylmethyloxycarbonylaminomethyl)cyclohexane carboxylic acid, trans-4-methylsulfonylethyloxycarbonylaminomethyl cyclohexane carboxylic acid, sodium trans-4-(9-fluorenylmethyloxycarbonylaminomethyl)cyclohexane carboxylate, potassium trans-4-methylsulfonylethyloxycarbonylaminomethyl cyclohexane carboxylate, trans-4-(pyridine-4'-methyloxycarbonylaminomethyl)cyclohexan e carboxylic acid, trans-4-(2,2,2-trichloroethyloxycarbonylaminomethyl)cyclohex ane carboxylic acid, potassium trans-4-(2-trimethylsilylethoxycarbonylaminomethyl)cyclohexa ne carboxylate, sodium trans-4-(pyridine-4'-methyloxycarbonylaminomethyl)cyclohexan e carboxylate, potassium trans-4-(2,2,2-trichloroethyloxycarbonylaminomethyl)cyclohex ane carboxylate, and calcium trans-4-(2-trimethylsilylethoxycarbonylaminomethyl)cyclohexa ne carboxylate), sulfatide, astaxanthin fatty acid di ester (e.g., astaxanthin dilauric acid ester, astaxanthin dimyristic acid ester, astaxanthin dipentadecanoic acid ester, astaxanthin dipalmitate ester, astaxanthin dipalmitoleic acid ester, astaxanthin diheptadecanoic acid ester, astaxanthin dielaidic acid ester, astaxanthin diricinoleic acid ester, astaxanthin petroselic acid ester, astaxanthin vaccenic acid ester, astaxanthin eleostearic acid ester, astaxanthin punicic acid ester, astaxanthin licaneic acid ester, astaxanthin parinaric acid ester, astaxanthin gadolic acid ester, astaxanthin 5-eicosenic acid ester, astaxanthin 5-dococenoic acid ester, astaxanthin cetoleic acid ester, astaxanthin erucic acid ester, astaxanthin 5,13-docosadienoic acid ester, astaxanthin selacholeic acid ester, astaxanthin decenoic acid ester, astaxanthin stiling acid ester, astaxanthin dodecenoic acid ester, astaxanthin dioleic acid ester, astaxanthin distearic acid ester, astaxanthin dieicosapentaenoic acid ester, astaxanthin didocosahexaenoic acid ester, astaxanthin dilinoleic acid ester, astaxanthin dilinolenic acid ester, and astaxanthin diarachidonic acid ester), astaxanthin diglycerophosphoric acid esters (e.g., astaxanthin diglycerophosphoric acid ester, astaxanthin glycerophosphoric acid palmitic acid, astaxanthin glycerophosphatidylcholine palmitic acid, astaxanthin glycerophosphatidylcholine DHA, astaxanthin glycerophosphatidylinositol palmitic acid, astaxanthin glycerophosphatidylinositol DHA, astaxanthin glycerophosphatidylinositol linoleic acid, and astaxanthin glycerophosphatidylcholine linoleic acid), steviol glycoside,benzimidazole derivatives (e.g., 1-(2-ethoxyethyl)-2-[1-(2-(4-(1-(4,4-dimethyl-2-oxazolin-2-y 1)-1-methylethyl)phenyl)ethyl)piperidin-4-yl]-1H-benzimidazo le, 2-[4-(2-(4-(1-(2-ethoxyethyl)benzimidazole-2-yl)piperidin-1-yl)ethyl)phenyl]-2-methylpropanoic acid, ethyl 2-[4-(2-(4-(1-(2-ethoxyethyl)benzimidazole-2-yl)piperidin-1-yl)ethyl)phenyl]-2-methyl propanate, 1-(2-ethoxyethyl)-2-[1-(2-(9-(1,1-dimethyl-2-hydroxyethyl)ph enyl)ethyl)piperidin-4-yl]-1H-benzimidazole, 1-(2-hydroxyethyl)-2-[1-(2-(4-(1-(4,4-dimethyl-oxazolin-2-yl)-1-methylethyl)phenyl)ethyl)piperidin-4-yl]-1H-benzimidazol e, and 2-[4-(2-(4-(1-(2-hydroxyethyl)benzoimidazole-2-yl)piperidin-1-yl)ethyl)phenyl]-2-methylpropanoic acid), alanine derivatives or salts thereof(N-[3-isopentyl-4-methyl-2-[4-(3-trifluoromethylbenza mide)benzoylimino]-3H-thiazoline-5-carbonyl]-L-alanine, N-[3-isopentyl-4-methyl-2-[4-(3-trifluoromethylbenzamide)ben zoylimino]-3H-thiazoline-5-carbonyl]-2-methylalanine, and N-[3-isopentyl-4-methyl-2-[4-(3-trifluoromethylbenzamide)ben zoylimino]-3H-thiazoline-5-carbonyl]-N-methyl-L-alanine), thiazoline derivatives or salts thereof (e.g., 3-isopentyl-4-methyl-2-[4-(3-trifluoromethylbenzamide)benzoy limino]-3H-thiazoline-5-carboxylic acid, and 4-isobutyl-3-methyl-2-[4-(3-trifluoromethylbenzamide)benzoyl imino]-3H-thiazoline-5-carboxylic acid), chlorpheniramine maleate, diphenhydramine hydrochloride, diphenylpyraline hydrochloride, carbinoxamine maleate, hydrocortisone butyrate, sulfonated acidic mucopolysaccharides or salts thereof, sulfonated dextran or salts thereof, fluoromethylhistidine or hydrochlorides thereof, histidine methyl ester or hydrochlorides thereof, GAN CAO (*Glycyrrhiza uralensis* root and stolon; *Glycyrrhizae Radix*) extract, ZI AO (*Lithospermum erythrorhizon* root; *Lithospermi Radix*) extract, EIJITSU (*Rosa multiflora* fruit; *Rosae Multiflorae Fructus*) extract, propolis, LANYOU (*Polygonum tinctorium* leaf) extract, ER CHA (paste made from *Acacia catechu*) extract, *Persea americana* extract, YU ZHU (*Polygonatum odoratum var*. *pluriflorum* root and stolon) extract, *Fritillaria verticillata Willd. var*. *thunbergii* extract, BEI MU (the bulb or scale leaf of *Fritillariae bulbus*) extract, JITANTOU (*Elephantopus scaber*) extract, *Polygonum bistorta* extract, YU JIN (*Curcuma longa* root) extract, *Echinacea* (*Echinacea angustifolia*) extract, HUANG LIAN (*Coptis chinensis* rhizome) extract, XUAN FU (flower of *Inula britannica ssp. japonica*) extract, *Hypericum erectum* (Otogirisou) extract, *Juglans mandshurica var*.*sachalinensis* extract, orange extract, Cassia, *Tetrapanax papyrifer* (TONG CAO) extract, *Agastache rugosa* extract, *Artemisia capillaris* (YIN CHEN HAO) extract, *Nasturtium officinale* extract, *Smilax glabra* (TU FU LING, SHAN GUI LAI) extract, *Gentiana amarella* extract, *Dendrobium* nobile extract, GAO BEN or TANG GAO BEN *(Ligusticum sinense* rhizome) extract, KOURYOUKYOU (*Alpinia officinarum* rhizome) extract, *Rubus chingii* (FU PEN ZI) extract, *Cremastra appendiculata* extract, *Prunus pseudocerasus* extract, *Salvia* (sage) extract, *Digitalis purpurea* extract, *Tilia japonica* extract, *Shorea robusta* extract, *Ficus religiosa* extract, SHA REN or SHUKUSHA *(Amomum* fruit) extract, *Coix lacryma-jobi* extract, NAN TIAN ZHU (*Nandina domestica Thunb. var*. *leucocarpa*) extract, *Hedera helix extract, Filipendula ulmaria* extract, *Achillea millefolium* (milfoil) extract, *Polygala senega var*. *latifolia* extract, *Echinops grijisii* extract, GOU JI (*Cibotium barometz* rhizome) extract, *Thymus vulgaris* (Hyakurikou) extract, *Stephania cepharantha* extract, *Prunus persica* extract, *Imperata cylindrica var*. *koenigii* extract, DONG GUA ZI *(Benincasa hispida* seed) extract, SHI YAO (*Houttuynia cordata*) extract, *Petroselinum crispum* extract, *Tribulus terrestris* fruit (BAI JI LI, CI JI LI) extract, *Cyperus rotundus* (XIANG FU ZI) extract, *Trapa japonica* (poppy seed) extract, *Althaea officinalis* extract, *Piper kadsura* (NANTOU) extract, Phaseolus radiatus (LU DOU) extract, *sandalwood* extract, butchers' bloom extract, pansy extract, *Chrysanthemum indicum* (KUYOKU) extract, *Arisaema heterophyllum* (TIAN NAN XING) extract, ZHI SHI (*Citrus aurantium* fruit) extract, *Richadella dulcifica* extract, *Aphananthe aspera* extract, *Prunus persica* (TAO) extract, *Centaurea cyanus* extract, *Myrica gale var. tomentosa* extract, *Viscum album var*. *coloratum* (HU JI SHENG) extract, *Ardisia japonica* (ZI JIN NIU) extract, *Artemisia montana* extract, *Artemisia indica* (AI YE) extract, *Rosmarinus officinalis* (Mannenrou) extract, *Polygala tenuifolia* extract, *Chaetomorpha spiralis* extract, *Chaetomorpha moniligera* extract, Mizojyuzumo extract, *Desmarestia ligulata* extract, *Myelophycus simplex* extract, *Sargassum horneri* extract, *Sargassum micracanthum* extract, *Neodilsea yendoana* extract, Marubaakaba extract, *Ahnfeltiopsis paradoxa* extract, *Palmaria palmata* extract, *Hibiscus tiliaceus* extract, *Atropa belladonna* extract, *Magnolia obovata* extract, *Ulmus davidiana* (YUHI, YUHAKUHI, YUBA) extract, *Epipactis helleborine* extract, *Neottia nidus-avis var. mandshurica* extract, *Cephalanthera longibracteata Blume* extract, *Orchis aristata* (including variants and original species) extract, *Valeriana fauriei Briq* extract, *Oenothera tetraptera* extract, *Ligusticum chuanxiong* extract, XING REN (*Prunus armeniaca* pit) extract, *Lonicera japonica* extract, Dang Gui (*Angelica acutiloba* root) extract, *Foeniculum vulgare* extract, and *Mentha piperita* extract.

### (13) Antiseborrheic agent

The cosmetic composition according to the present invention may contain an antiseborrheic agent. Specific examples of the antiseborrheic agent include chroman derivatives, pyridoxine or salts thereof and their derivatives, pyridoxal or salts thereof and their derivatives, pyridoxiamine or salts thereof and their derivatives, sulfur, *Cannabis sativa* (HUO MA REN) extract, *Lamium album var. barbatum* (ZOKUDAN) extract, *Nasturtium officinale* (cresson) extract, *Valeriana fauriei* (KISSOUKON) extract, Clematis extract, Kumasebasekisu, *Saponaria officinalis* extract, *Pterocarpus* extract, *Aesculus hippocastanum* extract, *Glycin max* (Daidzu) extract, Calendula officinalis (marigold) extract, *Dioscorea opposita* extract, *Dioscorea japonica* extract, *Tropaeolum majus* extract, *Tussilago farfara* (KUAN DONG HUA, KUAN DONG YE) extract, *Cayratia japonica* extract, giant kelp extract, *Camellia japonica 'Hijirimen'* extract, *Gelidium elegans* extract, and *Acrosorium yendoi* extract.

### (14) Antimicrobial agent

The cosmetic composition according to the present invention may contain an antimicrobial agent. Specific examples of the antimicrobial agent include acrinol, sulfur, gluconic acidcalcium, chlorhexidine gluconate, sulfamin, mercurochrome, lactoferrin or hydrolysates thereof, alkyldiaminoethylglycine chloride solution, triclosan, sodium hypochlorite, chloramine T, calcium hypochlorite, iodine compounds, iodoform, 1-alkyl carbapenem compounds, N-substituted azepane derivatives or salts thereof, sorbic acid or salts thereof, propionic acid or salts thereof, salicylic acid or salts thereof, dehydroacetic acid, parahydroxybenzoic acid esters, 2-keto-3-deoxyoctonic acid fatty acid esters, deoxyinositol derivatives (e.g., quercitol), unsaturated inositol derivatives (e.g., conduritol), inositol dimethyl ether, methyl inositol, inositol methyl ether, inosamine, deoxyinosadiamine, shikimic acid, quinic acid, undecylenic acid, thiamine laurylsulfate, thiamine laurylnitrate, phenol, cresol, p-chlorophenol, p-chloro-m-xylenol, p-chloro-m-cresol, thymol, phenethyl alcohol, o-phenylphenol, irgasan CH3565, halocarban, hexachlorophene, chlorhexidine, ethanol, methanol, isopropyl alcohol, benzyl alcohol, propylene glycol, 2-phenoxyethanol, 1,2-pentanediol, zinc pyrithione, chlorobutanol, isopropylmethylphenol, elemol, vetiverol, patchouli alcohol, nonionic surfactants (e.g., polyoxyethylene lauryl ether, polyoxyethylene nonylphenyl ether, and polyoxyethylene octylphenyl ether), amphoteric surfactants, anionic surfactants (e.g., sodium lauryl sulfate, and lauroylsarcosine potassium), cationic surfactants (cetyl trimethyl ammonium bromide, benzalkonium chloride, benzethonium chloride, methylrosaniline chloride), formaldehyde, hexamine, brilliant green, malachite green, crystal violet, Germall, photosensitizer 101, photosensitizer 201, photosensitizer 401, N-long-chain acyl basic amino acid derivatives and acid addition salts thereof, zinc oxide, hinokitiol, propolis, KU SHEN (*Sophora flavescens* root) extract, *Ferula assafoetida* (Agi) extract, *Thujopsis dolabrata* extract, *Alpinia* zerumbet extract, *Alpinia katsumadai* (SOUZUKU) extract, *Amaranthus lividus* (*Amaranthus viridis*) extract, *Amaranthus retroflexus* extract, *Amaranthus spinosus* extract, *Iresine* extract, *Amaranthus tricolor* extract, *Hosonagabiyu* extract, *Plectranthus japonicus* extract, *Lonicera* *gracilipes var. glabra* extract, *Asiasarum sieboldii* extract, *Asarum europaeum* extract, *Ulmus laciniata* (Retsubanire) extract, *Patrinia scabiosifolia* (BAI JIANG) extract, *Uncaria rhynchophylla* (TIAO TENG GOU) extract, *Zanthoxylum simulans* (SHU JIAO) extract, *Matricaria chamomilla* extract, *Cinchona succirubra* extract, *Cinchona pubescens* extract, *Phellodendron amurense* (HUANG BAI) extract, *Carum carvi* extract, *Piper betle* extract, *Sasa* veitchii extract, *Vaccinium macrocarpon* extract, *Citrus paradisi* extract, *Celosia cristata* (JI GUAN HUA, JI GUAN ZI) extract, Laurus *nobilis* (Gekkeiju) extract, *Rosa chinensis* (YUE JI HUA) extract, *Copaifera officinalis* extract, *Prunus jamasakura* extract, *Prunus pendula f. ascendens extract, Prunus maximowiczii* extract, *Prunus x yedoensis* extract, *Prunus* verecunda extract, *Prunus x kanzakura* extract, *Beta vulgaris* extract, *Cimicifuga simplex* (SHENG MA) extract, *Quisqualic acid* (SHI JUN ZI) extract, *Betulaceae betula* extract, *Cinnamomum zeylanicum* extract, Rumex acetosa extract, *Taraxacum officinale* extract, *Juniperus communis* (*Juniperus rigida*) extract, *Mentha* piperita extract, *Melissa officinalis* extract, CAO GUO *(Amomum tsao-ko*) extract, *Allium cepa* extract, *Cichorium intybus* extract, *Ulmus macrocarpa* (BUTEI) extract, *Tetragonia tetragonioides* (BANKYOU) extract, TU GEN (*Cephaelis ipecacuanha* root) extract, *Allium tuberosum* (JIU ZI) extract, *Allium fistulosum* extract, *Prunus davidiana* extract, patchouli extract, *Lycoris radiata* (SHI SUAN, Manjushage) extract, *Chamaecyparis obtusa* extract, *Pimenta dioica* extract, *Pulsatilla chinensis* (XING SE CAO) extract, *Rubus spp.* extract, *Humulus lupulus* extract, *Digenea simplex* (HAI REN CAO) extract, *Magnolia sprengeri* extract, *Lobelia chinensis* (BAN BIAN LIAN) extract, *Cryptotaenia japonica* extract, *Lithospermum erythrorhizon* (ZI AO) extract, *Phyllostachys pubescens extract,* Phytolacca (SHANG LU) extract, *Yucca* extract, *Angelica dahurica* extract, *Lavandula officinalis* extract, *Forsythia suspensa* (LIAN QIAO) extract, *Forsythia viridissima* extract, *Chondrus giganteus* extract, Irish moss (*Chondrus crispus*) extract, Akamomijinori extract, *Ulva pertusa* extract, *Monostroma nitidum* extract, *Monostroma latissimum* extract, *Monostroma grevillei* extract, *Chordariales Ishigeaceae* extract, *Ishige okamurae* extract, *Eisenia bicyclis* extract, *Delisea japonica* extract, *Grateloupia elliptica* extract, *Grateloupia divaricata* extract, *Grateloupia imbricata* extract, *Rhodomela larix* extract, *Batrachospermum gelatinosum* extract, *Batrachospermum sirodotii* extract, *Batrachospermum gallaei* extract, and *Alpinia oxyphylla* (YI ZHI) extract.

### (15) Blood flow enhancer/blood vessel stimulator

The cosmetic composition according to the present invention may contain a blood flow enhancer/blood vessel stimulator. Specific examples of the blood flow enhancer/blood vessel stimulator include tocopherol or salts thereof and their derivatives, tocotrienol or salts thereof and their derivatives, cepharanthine, carpronium chloride, eugenol derivatives (e.g., acetyl eugenol, methyl eugenol, methyl isoeugenol, ethyl eugenol, ethyl isoeugenol, and eugenol salicylate), minoxidil, capsicum tincture, nonylic vanillylamide, cantharis tincture, ginger tincture, mentha oil, L-menthol, camphor, nicotinic acidbenzyl, cinnarizine, tolazoline, acetyl choline, verapamil, ichthammol, alpha-borneol, cyclandelate, nonylic vanillylamide, capsaicin, zingerone, *Swertia japonica* extract, garlic extract, carrot extract, *Aloe arborescens* extract, *Gentiana amarella* extract, *Angelica acutiloba* extract, *Angelica keiskei* (Ashitaba) extract, *Arnica montana* extract, *Scirpus fluviatilis* (SAN LENG) extract, *Carum carvi* extract, *Citrus unshiu* (CHEN PI) extract, *Corylus heterophylla* (ZHEN ZI) extract, *Diospyros kaki* extract, SHI DI (Kaki calyx) extract, *Cinchonae cortex* extract, *Cymbidium pumilum* extract, *Verbena officinalis* (MA BIAN CAO) extract, *Papaver rhoeas* extract, *Nuphar japonicum* (KOUHONE) extract, *Arctium lappa* (NIU BANG, NIU BANG ZI) extract, *Lobelia sessilifolia* extract, *Forsythia viridissima* extract, *Zingiber officinale Rosc* (SHENG JIANG) extract, *Acorus calamus var. angustatus* (SHI CHANG PU, JIU JIE CHANG PU) extract, *Crataegus monogyna* extract, *Swertia japonica* (TOUYAKU) extract, (*Citrus aurantium* (TOUHI) extract, DAN SHEN (*Salvia miltiorrhiza* root) extract, *Thymus vulgaris* extract, SAN QI REN SHEN (*Panax japonicus* radix) extract, *Polygonum multiflorum* (HE SHOU WU) extract, *Codonopsis lanceolata* (SHIYOUJIN) extract, *Capsicum annuum* (BANSHOU) extract, DANG GUI (*Angelica acutiloba* root) extract, *Ligustrum lucidum* (NU ZHEN ZI) extract, *Citrus natsudaidai* extract, *Sambucus sieboldiana* (Niwatoko) extract, *Corylus heterophylla* (ZHEN ZI) extract, *Scopolia japonica* (Scopolia rhizoma) extract, *Mentha arvensis var. piperascens* (BO HE, BO HE YE) extract, BEI SHA SHEN (*Glehnia littoralis* root) extract, *Echinops setifer* extract, *Daphne genkwa* (Choujizakura, YUAN HUA) extract, black currant extract, *Tilia miqueliana* (Bodaijyu) extract, *Melilotus* sp. extract, *Citrus junos* extract, *Chimonanthus praecox* (Roubai) extract, *Ostericum sieboldii* extract, *Hibiscus sabdariffa* extract, and *Rosa canina* extract.

### (16) Anti-androgen agent

The cosmetic composition according to the present invention may contain an anti-androgen agent. Specific examples of the anti-androgen agent include follicular hormone (e.g., estrone, estradiol, and ethynylestradiol), isoflavone, oxendolone, 4',5,7-trihydroxy-8-prenylflavanone, 4',5,7-trihydroxy-8-prenylflavone, 3,3',4',5,7-pentahydroxy-8-prenylflavone, nicorandil, and cyclosporin.

(17) Structural proteolytic enzyme (matrix metalloproteinase such as elastase, collagenase, keratin protease, serine protease, integrin degrading enzyme, involucrin degrading enzyme, filaggrin degrading enzyme, laminin degrading enzyme, fibronectin degrading enzyme, and proteoglycan degrading enzyme) inhibitor/structural proteolytic enzyme expression inihibitor

The cosmetic composition according to the present invention may contain a structural proteolytic enzyme (e.g., a matrix metalloproteinase such as elastase, collagenase, keratin protease, serine protease, integrin degrading enzyme, involucrin degrading enzyme, filaggrin degrading enzyme, laminin degrading enzyme, fibronectin degrading enzyme, proteoglycan degrading enzyme) inhibitor/structural proteolytic enzyme expression inihibitor. Specific examples of the structural proteolytic enzyme) inhibitor/structural proteolytic enzyme expression inihibitor include adenine derivatives (e.g., butanetriol-9-adenine and phosphate adducts thereof, propanediol-9-adenine and phosphate adducts thereof, and pentanetriol-9-adenine and phosphate adducts thereof), carbostyril derivatives or salts thereof, dicarboxylic acids (e.g., glutaric acid, adipic acid, pimelic acid, suberic acid, azelaic acid, sebacic acid, 1, 9-nonamethylene dicarboxylic acid, and 1,10-decamethylene dicarboxylic acid), rosmarinic acid, ursolic acid, oleanolic acid, hydroxamic acid derivatives, esculetin derivatives, anthocyanidins, nordihydroguaiaretic acid, 20-carboxy-16-hydroxy-21-nor-5 alpha-7,9(11)-lanostadiene-3,24-dione, ubiquinone, plastquinone, juglone, shikonin, quinizarin, alizarin, abietin, levopimaric acid, betulin, alpha-amyrin, catechin compounds (e.g., catechin, epigallocatechin, epigallocatechin gallate, epicatechin, epicatechin gallate, and catechin rhamnopyranoside catechin), diisopropyl fluorophosphate, dimethyl (2R,6R)-2,6-dihydroxy-4-(tert-butyldimethylsilyloxy)-(3Z)-he pten-1,7-dicarboxylate, N,N'-bis-[(1S,2R)-2-hydroxyindan-1-yl]-(2R,6R)-2,6-dibenzylo xy-4-hydroxyheptane dicarboxylic acid-1,7-diamide, dimethyl (2R,6R)-2,6-dihydroxy-4-(tert-butyldimethylsilyloxy)heptane-1,7-dicarboxylate, dimethyl (2R,6R)-2,6-dibenzyloxy-4-(tert-butyldimethylsilyloxy)heptan e-1,7-dicarboxylate, (2R,6R)-2,6-dibenzyloxy-4-(tert-butyldimethylsilyloxy)heptan e-1,7-dicarboxylic acid, N,N'-bis[(1S,2R)-2-hydroxyindan-1-yl]-(2R,6R)-2,6-dibenzylox y-4-(tert-butyldimethylsilyl)oxyheptane-1,7-dicarboxylic acid diamide, N,N'-bis[(1S,2R)-2-hydroxyindan-1-yl]-(2R,6R)-2,6-dibenzylox y-4-hydroxyheptane-1,7-dicarboxylic acid diamide, N,N'-bis[(1S,2R)-2-hydroxyindan-1-yl]-(2R,6R)-2,4,6-trihydro xyheptane-1,7-dicarboxylic acid diamide, N,N'-bis[(1S,2R)-2-hydroxyindan-1-yl]-(2S,6S)-2,6-dibenzylox y-4-hydroxyheptane-1,7-dicarboxylic acid diamide, dimethyl (2S,6S)-2,6-dihydroxy-4-(tert-butyldimethylsilyloxy)-(3Z)-he pten-1,7-dicarboxylate, dimethyl (2S,6S)-2,6-dihydroxy-4-(tert-butyldimethylsilyloxy)heptane-1,7-dicarboxylate, dimethyl (2S,6S)-2,6-dibenzyloxy-4-(tert-butyldimethylsilyloxy)heptan e-1,7-dicarboxylate, (2S,6S)-2,6-dibenzyloxy-4-(tert-butyldimethylsilyloxy)heptan e-1,7-dicarboxylic acid, N,N'-bis[(1S,2R)-2-hydroxyindan-1-yl]-(2S,6S)-2,6-dibenzylox y-4-(tert-butyldimethylsilyl)oxyheptane-1,7-dicarboxylic acid diamide, N,N'-bis[(1S,2R)-2-hydroxyindan-1-yl]-(2R,6R)-2,6-dibenzylox y-4-hydroxyheptane-1,7-dicarboxylic acid diamide, 3-[[4-(4-fluorophenoxy)-benzenesulfonyl]-(1-hydroxycarbamoyl cyclopentyl)-amino]-propionic acid, 4-[4-(4-fluorophenoxy)-benzenesulfonylamino]-tetrahydropyran -4-carboxylic acid hydroxyamide, 4-[4-(4-chlorophenoxy)-benzenesulfonylmethyl]-tetrahydropyra n-4-carboxylic acid hydroxyamide, 3-[[4-(4-fluorophenoxy)-benzenesulfonyl]-(1-hydroxycarbamoyl cyclobutyl)-amino]-propionic acid, 4-(4'-chlorobiphenyl-4-yl)-2-[2-(1,3-dioxo-1,3-dihydroisoind ol-2-yl)-ethyl]-4-oxobutyric acid, 1-[4-(4-fluorobenzyloxy)-benzenesulfonyl]-2-hydroxycarbamoyl piperidin-3-yl)-carbamic acid isopropyl ester, 2-[4-(4-fluorophenoxy)-benzenesulfonylamino]-N-hydroxy-2-met hylpropionamide, 3-[4-(4-fluorophenoxy)-benzenesulfonyl]-2,N-dihydroxypropion amide, 3-(4-phenoxybenzenesulfonyl)-7-oxabicyclo[2.2.1]heptane-2-ca rboxylic acidhydroxyamide, (4-benzylbenzyl)-[2-(2,2-dimethyl-1-methylcarbamoyl-propylca rbamoyl)-4-(4'-fluorobiphenyl-4-yl)-butyl]-phosphinic acid, 2-amino-3-[4-(4-fluorophenoxy)-benzenesulfonyl]-N-hydroxypro pionamide, N-hydroxy-2-(4-phenylpiperidine-1-sulfonyl)-acetamide, *Thymus quinquecostatus* extract, *Prunella vulgaris var. lilacina Prunella* (XIA KU CAO) extract, *Lagerstroemia speciosa* (Banaba) extract, *Crataegus pinnatifida* extract, *Crataegus cuneata* (SHAN ZHA ZI) extract, *Magnolia officinalis* (HOU PO) extract, *Psidium guajava* extract, *Schizandrae Fructus* (WU WEI ZI) extract, *Plantaginis Herba* extract, *Rosa centifolia* extract, *Hypericum perforatum* extract, *Stellaria media* (Hakobe) extract, *Achyranthes fauriei* (NIU XI) extract, *Magnolia obovata* (HOU PO, PO) extract, *Vitis labrusca* extract, *Vitis vinifera* extract, *Vitis coignetiae* extract, *Citrus reticulata* extract, *Bupleurum falcatum root* (CHAI HU) extract; *HUANG QIN (Scutellaria baicalensis root; Scutellariae Radix)* extract, *Hypericum erectum* extract, *Sophora flavescens* extract, *Morus alba* extract, *Cinnamomum cassia* extract, *Geranium thunbergii* extract, *Symphytum peregrinum* extract, *Salvia splendens* extract, *Sambucus nigra* extract, *Tilia miqueliana* extract, *Paeonia suffruticosa* extract, *Epipactis papillosa* extract, *Neottia nidus-avis var. mandshurica* extract, *Cephalanthera longibracteata* extract, *Orchis aristata* (including variants and original species) extract, *Agave americana var. marginata* extract, *Agave sisalana* extract, and *Adenophora tripylla var. japonica* extract.

### (18) Structural protein synthesis promoter

The cosmetic composition according to the present invention may contain a structural protein synthesis promoter. Specific examples of the structural protein synthesis promoter include ethanolamine derivatives, pentoxifylline, serine derivatives, geraniol, crocetin, methyl 4-(2-ethylhexyloxy)-2-hydroxybenzoate, methyl 2-hydroxy-4-(3,5,5-trimethylhexyloxy)benzoate, methyl 4-cyclohexylmethoxy-2-hydroxybenzoate, methyl 4-(2-cyclohexylethoxy)-2-hydroxybenzoae, methyl 4-(3,7-dimethyl-6-octenyloxy)-2-hydroxybenzoate, ethyl 3-(2-ethylhexyl oxy)-5-hydroxybenzoate, methyl 5-(2-ethylhexyloxy)-2-hydroxybenzoate, methyl 2-hydroxy-5-(3,5,5-trimethylhexyloxy)benzoate, methyl 5-(2-cyclohexylethoxy)-2-hydroxybenzoate, methyl 4-N-hexyloxy-2-hydroxybenzate, methyl 2-hydroxy-4-N-octyloxybenzoate, methyl 4-N-decyloxy-2-hydroxybenzoate, methyl 5-N-hexyloxy-2-hydroxybenzoate, 4-(2-ethylhexyl oxy)-2-hydroxybenzoic acid, 2-hydroxy-4-(2,5,5-trimethylhexyloxy)benzoic acid, 4-cyclohexylmethoxy-2-hydroxybenzoic acid, 4-(2-cyclohexylethoxy)-2-hydroxybenzoic acid, 4-(3,7-dimethyl-6-octenyloxy)-2-hydroxybenzoic acid, 3-(2-ethylhexyl oxy)-5-hydroxybenzoic acid, 5-(2-ethylhexyl oxy)-2-hydroxybenzoic acid, 5-(2-ethylhexyloxy)-2-hydroxybenzoic acid, 2-hydroxy-5-(3,5,5-trimethylhexyloxy)benzoic acid, 5-(2-cyclohexylethoxy)-2-hydroxybenzoic acid, 4-N-hexyloxy-2-hydroxybenzoic acid, 5-N-hexyloxy-2-hydroxybenzoic acid, 2-hydroxy-4-N-octyloxybenzoic acid, 4-N-decyloxy-2-hydroxybenzoic acid, N-(2-hydroxyethyl)-4-(2-ethylhexyl oxy)-2-hydroxybenzamide, N-ethyl-4-(2-ethylhexyloxy)-2-hydroxybenzamide, 2-acetoxy-4-cyclohexylmethoxybenzoic acid, sodium 4-(2-ethylhexyloxy)-2-hydroxybenzoate, methyl 4-[(2E)-3,7-dimethyl-2,6-octadienyloxy]-2-hydroxybenzoate, ethyl 4-[(2E)-3,7-dimethyl-2,6-octadienyloxy]-2-hydroxybenzoate, ethyl 5-[(2E)-3,7dimethyl-2,6-octadienyloxy]-2-hydroxybenzoate, ethyl 3-[(2E)-3,7-dimethyl-2,6-octadienyloxy]-2-hydroxybenzoiate, ethyl 3-[(2E)-3,7-dimethyl-2,6-octadienyloxy]-5-hydroxybenzoate, ethyl 4-[(2E)-3,7-dimethyl-2,6-octadienyloxy]-3-methoxybenzoate, methyl 4-[(2E,6E)-3,7,11-trimethyl-2,6,10-dodecatrienyloxy]-2-hydro xybenzoate, (2E)-3,7-dimethyl-2,6-octadienyl 4-[(2E)-3,7-dimethyl-2,6-octadienyloxy]-2-hydroxybenzoate, 4-[(2E)-3,7-dimethyl-2,6-octadienyloxy]-2-hydroxybenzoic acid, 5-[(2E)-3,7-dimethyl-2,6-octadienyloxy]-2-hydroxybenzoic acid, 3-[(2E)-3,7-dimethyl-2,6-octadienyloxy]-2-hydroxybenzoic acid, 3-[(2E)-3,7-dimethyl-2,6-octadienyloxy]-5-hydroxybenzoic acid, 2-hydroxy-4-[(2E,6E)-3,7,11-trimethyl-2,6,10-dodecatrienylox y]benzoic acid, 4-[(2E)-3,7-dimethyl-2,6-octadienyloxy]-3-methoxybenzoic acid, 2-acetoxy-4-[(2E)-3,7-dimethyl-2,6-octadienyloxy]benzoic acid, N-(2-hydroxyethyl)-4-[(2E)-3,7-dimethyl-2,6-octadienyloxy]-2 -hydroxybenzamide, 4-[(2E)-3,7-dimethyl-2,6-octadienylamino]-2-hydroxybenzoic acid, dimethyl-2,6-octadienyloxybenzoic acid, N-(2-hydroxyethyl)-4-[(2E)-3,7-dimethyl-2,6-octadienyloxy]-2 -hydroxybenzamide, 4-[(2E)-3,7-dimethyl-2,6-octadienylamino]-2-hydroxybenzoic acid, 3-dodecyloxybenzoic acid, 3-(12-hydroxydodecyloxy)benzoic acid, 4-dodecyloxybenzoic acid, 4-(12-hydroxydodecyloxy)benzoic acid, 3-(12-hydroxyoctadecyloxy)benzoic acid, 4-(12-hydroxyoctadecyloxy)benzoic acid, 3-(11-hydroxyundecyloxy)benzoic acid, 4-(11-hydroxyundecyloxy)benzoic acid, 3-[(2E)-3,7-dimethyl-2,6-octadienyloxy]benzoic acid, 4-[(2E)-3,7-dimethyl-2,6-octadienyloxy]benzoic acid, 3-[(2E,6E)-3,7,11-trimethyl-2,6,10-dodecatrienyloxy]benzoic acid, 4-[(2E,6E)-3,7,11-trimethyl-2,6,10-dodecatrienyloxy]benzoic acid, 4-[3,7-dimethyl-9-(2,6,6-trimethyl-1-cyclohexen-1-yl)-2,4,6, 8-nonatetraenyloxy]benzoic acid, 4-[3,4-dihydro-2,5,7,8-tetramethyl-2-(4,8,12-trimethyltridec yl)-2H-1-benzopyrane-6-oxy]benzoic acid, 4-[(2E,6E)-3,7,11-trimethyl-2,6,10-dodecatrienyloxy]benzamid e, 4-[(2E)-3,7-dimethyl-2,6-octadienyloxy]benzamide, 4-(2-methyl-2-butenyloxy)benzamide, 4-(2-ethylhexyloxy)benzamide, 4-dodecyloxybenzamide, 4-(12-hydroxydodecyloxy)benzamide, 4-(12-hydroxyoctadecyloxy)benzamide, 4-(11-hydroxyundecyloxy)benzamide, 4-(10-hydroxydecyloxy)benzamide, 4-isostearyloxybenzamide, N-(2-hydroxyethyl)-4-[(2E,6E)-3,7,11-trimethyl-2,6,10-dodeca trienyloxy]benzamide, N,N-dimethyl-4-[(2E,6E)-3,7,11-trimethyl-2,6,10-dodecatrieny loxy]benzamide, N,N-di[(2E)-3,7-dimethyl-2,6-octadienyl]-4-aminobenzamide, 4-[N'-methoxycarbonyl-N-[(2E)-3,7-dimethyl-2,6-octadienylami no] benzamide, 4-[N-acetyl-N-[(2E)-3,7-dimethyl-2,6-octadienyl]]aminobenzam ide, N-(2-hydroxyethyl)-4-[(2E,6E)-3,7,11-trimethyl-2,6,10-dodeca trienyloxy]-2-hydroxybenzamide, N,N-diethyl-4-[(2E,6E)-3,7,11-trimethyl-2,6,10-dodecatrienyl oxy]-2-hydroxybenzamide, *Pisum sativum* extract, *Callophyllis* extract, *Mastocarpus mamillosus* extract, *Hosoibonori* extract, *Nobonori* extract, *Ceramium kondoi* extract, *Ceramium tenerrimum* extract, *Ceramium paniculatum* extract, *Ceramium japonicum* extract, *Champia expansa* extract, *Polysiphonia morrowii* extract, *Futoigusa* extract, *Cuphea micropetala* extract, *Chondria dasyphylla* extract, *Motsuyurena* extract, *Beniyanagikori* extract, *Chondria expansa* extract, *Chondria lancifolia* extract, *Eutrema japonica* extract, and *Intsia* extract.

### (19) Mucopolysaccharides (e.g., hyaluronic acid, and chondroitin sulfuric acid) degrading enzyme inhibitor

The cosmetic composition according to the present invention may contain mucopolysaccharides (e.g., hyaluronic acid, and chondroitin sulfuric acid) degrading enzyme inhibitor. Specific examples of the mucopolysaccharides (e.g., hyaluronic acid, and chondroitin sulfuric acid) degrading enzyme inhibitor include anacardic acid or derivatives thereof (e.g., 6-pentadecatrienyl salicylic acid methylether, 6-pentadecatrienyl salicylic acid ethylether, 6-pentadecatrienyl salicylic acid propylether, 6-pentadecatrienyl salicylic acid butylether, 6-pentadecatrienyl salicyl alcohol methylether, 6-pentadecatrienyl salicyl alcohol ethylether, 6-pentadecatrienyl salicyl alcohol propylether, 6-pentadecatrienyl salicyl alcohol butylether, 6-pentadecatrienyl salicylaldehyde methylether, 6-pentadecatrienyl salicylaldehyde ethylether, 6-pentadecatrienyl salicylaldehyde propylether, 6-pentadecatrienyl salicylaldehyde butylether, 2-methylether-6-pentadecatrienyl cinnamic acid, 2-ethylether-6-pentadecatrienyl cinnamic acid, 2-propylether-6-pentadecatrienyl cinnamic acid, 2-butylether-6-pentadecatrienyl cinnamic acid, 2-methylether-6-pentadecatrienyl cinnamic alcohol, 2-ethylether-6-pentadecatrienyl cinnamic alcohol, 2-propylether-6-pentadecatrienyl cinnamic alcohol, and 2-butylether-6-pentadecatrienyl ]cinnamic alcohol), isoprenylated benzophenone derivatives (e.g., garcinol, isogarcinol , xantothymol, isoxantothymol, and gucchiferron), *Epimedium grandiflorum var*. *thunbergianum* extract, *Epimedium grandiflorum Morr. var. Grandiflorum* (YIN YANG HUO) extract, *Polygonatum falcatum* extract, *Polygonatum sibiricum* (HUANG JING) extract, *Origanum rotundifolium* extract, *Dryopteridaceae* extract, *Polygala tenuifolia* extract, *Prunus jamasakura* extract, *Pinellia ternata Breit* (BAN XIA) extract, ZHI SHI (*Citrus aurantium* fruit) extract, *Lycium chinense* (GOU Qi, *GOU QI ZI, GOU QI YE, DI GU PI*) extract, *Evodia rutaecarpa* (WU ZHU YU) extract, *Polygala senega* extract, *Melia azedarach var*. *subtripinnata* extract, *Tamarindus indica* extract, *Artemisia dracunculus* extract, *Rheum coreanum* (DA HUANG) extract, *Phyllostachys nigra* *var. henonis* (ZHU RU) extract, *Bellis perennis* (*daisy*) extract, *Terminalia chebura* extract, *Berchemia lineata* extract, *Hibiscus syriacus* extract, *Laurus nobilis* extract, *Cyrtomium fortunei* extract, *Ulva pertusa* extract, *Macrocystis integrifolia* extract, Neoshitis luetokeana extract, *Ptilophora subcostata* extract, *Pterocladiella capillacea* extract, *Pterocladia capillacea* extract, *Yatabella* extract, *Gelidiella acerosa* extract, *Chondrus yendoi* extract, *Chondrus armatus* extract, *Caulerpa okamurae* extract, *Bryopsis plumosa* extract, *Codium fragile* extract, *Codium subtubulosum* extract, *Codium cylindricum* extract, *Codium latum* extract, *Ceratophyllum demersum* extract, *Cladosiphon okamuranus* extract, *Nemacystus decipiens* extract, *Colpomenia sinuosa* extract, *Ecklonia cava* extract, *Gloiopeltis furcata* extract, *Gloiopel tis tenax* extract, grape leaf extract, *Zanthoxylum piperitum* extract, and *Astragalus sinicus* extract.

### (20) Mucopolysaccharides synthesis promoter

The cosmetic composition according to the present invention may contain mucopolysaccharides synthesis promoter. Specific examples of the mucopolysaccharides synthesis promoter include stilbene derivatives or salts thereof, mollugin or salts thereof and their derivatives, N-acetylglucosamine, *Linum usitatissimum* extract, *Broussonetia papyrifera* (CHOJITSU) extract, *Broussonetia kazinoki* extract, *Origanum majorana L*. extract, *Artocarpus altilis* extract, Hirakotoji extract, *Chondrus elatus* extract, *Acrosorium flabellatum* extract, *Acrosorium venulosum* extract, *Acrosorium polyneurum* extract, *Ulva reticulata* extract, *Ulva arasakii* extract, *Callophyllis japonica* extract, *Callophyllis crispata* extract, *Callophyllis palmata* extract, *Callophyllis adhaerens* extract, *Callophyllis* adnata extract, *Callophyllis cristata* extract, Etsukinotosakamodoki extract, Nankaitosakamodoki extract, and *Callophyllis rhynchocarpa* extract.

### (21) Intercellular lipid production promoter/intercellular lipid condition modifier

The cosmetic composition according to the present invention may contain an intercellular lipid production promoter/intercellular lipid condition modifier. Specific examples of the intercellular lipid production promoter/intercellular lipid condition modifier include phospholipids (e.g., phosphatidylethanol, phosphatidylcholine, phosphatidyltriethanolamine, phosphatidylserine, phosphatidic acid, phosphatidylglycerin, phosphatidylinositol, diacylphosphatidylcholine, diacylphosphatidylethanolamine, diacylphosphatidylinositol, diacylphosphatidylserine, 1-cysteinyl phosphatidic acid, 2-cysteinyl phosphatidic acid, 1-glutathionyl phosphatidic acid, 2-glutathionyl phosphatidic acid, 1-azelaoyl phosphatidic acid, 2-azelaoyl phosphatidic acid, 1-hydroxyacylphosphatidic acid, 2-hydroxyacylphosphatidic acid, ceramide, glucosylceramide, galactosylceramide, cerebroside, phosphatidylglucosylacylglycerin, N-oleoylsphingosine, N-(12-hydroxyoctadecanoyl)sphingosine, N-(16-hydroxyhexadecanoyl)sphingosine, N-salicyloyl phytosphingosine, sphingomyelin, soybean lecithin, and egg yolk lecithin), sterins (cholesterol, dihydrocholesterol, cholesteryl stearate, cholesteryl hydroxystearate, cholesteryl macadamiate, beta-sitosterol, stigmasterol, campesterol, ergosterol, 5-dihydroergosterol, phytosterol, 25-hydroxycholesterol, 26-hydroxycholesterol, 19-hydroxycholesterol, 22-ketocholesteroloxime, 6-ketocholesterol and 7-ketocholesterol or derivatives thereof), N-acetylneuraminic acid (sialic acid), N-glucosol neuraminic acid, gangliosides (e.g., gangliotriaose, gangliotetraose, globotetraose, neolactotetraose, neolactohexaose, and neolactooctaose), oligosulfated hyaluronic acid, hydroxytamoxifen compounds, glycoglycerolipids, pentoxifylline, 3-deazaadenosine, carboxamide derivatives, inositol polyamines, sialic acid sialylic acid, triterpenic acid derivatives, lactose, lactosamine derivatives, sulfated chitin derivatives, albumin, *Panax ginseng* extract, *Panax japonicus* extract, *Lilium lancifolium* extract, *Lilium japonicum* extract, *Lilium brownii* *var. colchesteri* extract, and *Papaver rhoca* extract.

### (22) Keratolytic agent/stratum corneum removal promoter

The cosmetic composition according to the present invention may contain a keratolytic agent/stratum corneum removal promoter. Specific examples of the keratolytic agent/stratum corneum removal promoter include tropolone and derivatives thereof, resorcin, lactic acid, urea, salicylic acid, guanidine, ethanolamine, *Trichosanthes kirilowii var. japonica* (KAROKON) extract, *Trigonella foenum-graecum* extract, *Lablab purpureus* (BIAN DOU) extract, *Lens esculenta* extract, *Cicer arietinum* extract, and *Vigna radiata* extract.

### (23) Plasminogen-activator competitive inhibitor

The cosmetic composition according to the present invention may contain a plasminogen-activator competitive inhibitor. Specific examples of the plasminogen-activator competitive inhibitor include *Arnica unalaschkensis var. tschonoskyi* extract, *Humulus japonicus* extract, *Humulus lupulus var. cordifolius* extract, and *Rubus idaeus* extract.

### (24) Maillard reaction inhibitor

The cosmetic composition according to the present invention may contain a Maillard reaction inhibitor. Specific examples of the Maillard reaction inhibitor include aminoguanidine, flavanones (e.g., naringin, naringenin, liquiritin, liquiritigenin, digallic acid, luteoic acid, ellagic acid, chlorogenic acid, glucogallin, tetralin, hamamelitannin, gallotannin or gallic acid tannin, tannic acid, geraniin, gallic acid, galloylgallic acid, ellagitannin, hexagalloylglucose, heptagalloylglucose, tetragalloylglucose, trigalloylglucose, pentagalloylglucose, digalloyl quinic acid, and trigalloyl quinic acid), 2-hydroxyphenylalkylamine derivatives or salts thereof, phenylpropenoic acid derivatives (e.g., 3-[2,3-bis(methoxymethoxy)phenyl]propenoic acid, 7-(4-hydroxy-3-methoxyphenyl)hepta-2,4,6-trienoic acid, 3-(3,5-dimethoxy-4-hydroxyphenyl)propenohydrazide, and N'-isopropylidene-3-(2-methoxyphenyl)propenohydrazide), citric acid or salts thereof, *Acorus gramineus* (SHI CHANG GEN) extract, *Bletilla striata* extract, *Mallotus japonicus* extract, *Diospyros kaki* (including SHI DI) extract, *Morus alba* extract, *Hypericum perforatum* extract, *Centella asiatica* extract, *Galega* extract, *Eriodictyon californicum* extract, *Rosa canina* extract, *Thujopsis dolabrata* extract, *Reynoutria japonica* extract, *Pyrola japonica* extract, *Celosia cristata L.* extract, *Betulaceae Betula* extract, *Crataegus monogyna* extract, *Achillea millefolium* extract, *Ilex latifolia* extract, *Houttuynia cordata* extract, tormentilla extract, *Ophiopogon japonicus* extract, *Thujopsis dolabrata* extract, *Vitis spp.* extract, *Polygonum aviculare* extract, *Sapindus mukurossi* extract, *Chaenomeles sinensis* extract, *Chaenomeles lagenaria* extract, and *Anthemis nobilis* extract.

### (25) Testosterone 5 alpha-reductase inhibitor/hair papilla activating agent/hair growth promoter

The cosmetic composition according to the present invention may contain testosterone 5 alpha-reductase inhibitor/hair papilla activating agent/hair growth promoter. Specific examples of the testosterone 5 alpha-reductase inhibitor/hair papilla activating agent/hair growth promoter include gamma-amino-beta-hydroxybutyric acid esters (e.g., gamma-amino-beta-hydroxybutyric acid methyl ester, gamma-amino-beta-hydroxybutyric acid ethyl ester, gamma-amino-beta-hydroxybutyric acid propyl ester, gamma-amino-beta-hydroxybutyric acid butyl ester, gamma-amino-beta-hydroxybutyric acid ethylhexyl ester, gamma-amino-beta-hydroxybutyric acid hexadecyl ester, gamma-amino-beta-hydroxybutyric acid lauryl ester, gamma-amino-beta-hydroxybutyric acid stearyl ester, gamma-amino-beta-hydroxybutyric acid oleyl ester, gamma-amino-beta-hydroxybutyric acid benzyl ester, gamma-amino-beta-hydroxybutyric acid phenyl ester, gamma-amino-beta-hydroxybutyric acid ethylglycol ester, gamma-amino-beta-hydroxybutyric acid sorbitol ester, gamma-amino-beta-hydroxybutyric acid polyoxyethylene glycol ester, and gamma-amino-beta-hydroxybutyric acid glycerin ester), amineoxides (e.g., oleyldimethylamine oxide, stearyldimethylamine oxide, palmityldimethylamine oxide, myristyldimethylamine oxide, lauryldimethylamine oxide, dimethyllaurylethoxyamine oxide, dihydroxyethyllaurylamine oxide, and coco-dimethylamine oxide), alkylbetaines (e.g., coco-fatty acid amide propylbetaine, coco-betaine, betaine lauryldimethyl aminoacetate, lauric acid amide propylbetaine, laurylhydroxysulfobetaine, and 2-alkyl-N-carboxymethyl-N-hydroxyethyl imidazolynium betaine), pyrimidine-N-oxide derivatives (e.g., 2-amino-4-methyl-6-piperidinopyrimidine-3-oxide, 2-amino-4-methyl-6-(1-pyrrolidinyl)pyrimidine-3-oxide, 2-amino-4-methyl-6-morpholinopyrimidine-3-oxide, 2-amino-4-methyl-6-[1-(4-methylpiperazinyl)]pyrimidine-3-oxi de, 2-amino-4-(1-hexahydroazepinyl)-6-methylpyrimidine-1-oxide, 2-amino-4-dimethylamino-6-methylpyrimidine-1-oxide, 2-amino-4-allylamino-6-methylpyrimidine-1-oxide, 2-amino-4-benzylamino-6-methylpyrimidine-1-oxide, 2-amino-4,5-methyl-6-piperidinopyrimidine-3-oxide, 2-amino-4-ethyl-6-morpholinopyrimidine-3-oxide, 2-amino-4-methyl-5-nitro-6-piperidinopyrimidine-3-oxide, 2,5-diamino-4-methyl-6-piperidinopyrimidine-3-oxide, 2-amino-4-methyl-5,6-bis(1-pyrrolidinyl)pyrimidine-3-oxide, 2-amino-4-methyl-5-piperidino-6-(1-pyrrolidinyl)pyrimidine-3 -oxide, 2-methyl-4-amino-6-piperidinopyrimidine-3-oxide, 2-methyl-4-amino-5-bromo-6-(1-pyrrolidinyl)pyrimidine-3-oxid e, 2-methyl-4-amino-5-nitro-6-piperidinopyrimidine-3-oxide, 2-methyl-4,5-diamino-6-piperidinopyrimidine-3-oxide, 2-methyl-4-amino-5,6-bis(1-pyrrolidinyl)pyrimidine-3-oxide, 2-amino-4-methyl-6-piperidinopyrimidine-3-oxide monohydrochloride, 2-acetylamino-4-methyl-6-piperidinopyrimidine-3-oxide, 2,4-diamino-6-phenoxypyrimidine-3-oxide, 2,4-diamino-6-(2,4-dichlorophenoxy)pyrimidine-3-oxide, 2,4-diamino-6-(2,4,6-trichlorophenoxy)pyrimidine-3-oxide, 2,4-diamino-5-nitroso-6-(2,4-dichlorophenoxy)pyrimidine-3-ox ide, 2,4-diamino-5-nitro-6-(2,4,6-trichlorophenoxy)pyrimidine-3-o xide, 2,4-diamino-5-nitro-6-(2,4-dichlorophenoxy)pyrimidine-3-oxid e, 2,4,5-triamino-6-(2,4-dichlorophenoxy)pyrimidine-3-oxide, and 2,4-diamino-5-bromo-6-(2,4-dichlorophenoxy)pyrimidine-3-oxid e), p-menthane-3,8-diol, monoglyceryl-D-glucoside monotridecanoate, 1-o-N-pentadecylglycero-D-glucoside, glyceride sulfates such as monopentadecanoic acid glyceride sulfuric acid ester salt, monopentadecyl glycelyl ether sulfuric acid ester salt, 1-o-hexadecyl-2-o-methylglycerin, 1-o-octadecyl-2-o-methylglycerin, 1-o-oleyl-2-o-methylglycerin, acetylcarnitine or salts thereof, geranyl geranyl acetone, hydroxamic acid derivatives or salts thereof, zingerone glycoside, benzeneoxyacetic acid derivatives(e.g., [5-[2-[1-phenyl-1-(3-pyridyl)methylideneaminooxy]ethyl]-7,8-dihydronaphthalen-1-yloxy]acetic acid), isorhamnetin-3-robinobioside, xanthone derivatives, proanthocyanidins (e.g., grapeseed extract proanthocyanidin, proanthocyanidin isolated from apple, proanthocyanidin isolated from pine, purified procyanidin oligomer, procyanidin B-1, procyanidin B-2, procyanidin B-3, and procyanidin C-1), *Quercus glauca* extract, *Angelica archangelica* extract, *Prunus armeniaca* (XING REN) extract, *Prunus armeniaca* extract, *Pyrola japonica* (Ichiyakusou) extract, *Cytisus-scoparius* extract, *Plantago asiatica* (CHE QIAN ZI, CHE QIAN CAO) extract, *Alnus sieboldiana* extract, *Olea europaea* extract, *Veronica persica* extract, *Polygonatum sibiricum* extract, *Trichosanthes cucumeroides* extract, *Trichosanthes kirilowi Maximowicz* (WANG GUA) extract, *Chrysanthemum morifolium* extract, *Chrysanthemum indicum* extract, *Chrysanthemum zawadskii* extract, *Catalpa ovata* extract, *Aleurites moluccana* extract, *Cinnamomum camphora* extract, Cubeb extract, *Rhamnus japonica var*. *decipiens* extract, *Schizonepeta tenuifolia* (JING JIE, JING JIE SUI) extract, *Osmanthus fragrans var. aurantiacus* extract, *Antirrhinum majus* extract, JU PI (citrus peel) extract, *Camellia x hiemalis* extract, *Coriandrum sativum* extract, *Magnolia kobus* extract, *Magnolia liliflora* (XIN YI) extract, *Kadsura japonica* extract, *Kadsura japonica* extract, *Schisandra nigra* extract, Koronbo extract, *Marsdenia condurango* extract, *Camellia sasanqua* extract, *Ipomoea batatas Lam* extract, *Zizyphus jujuba* (SUAN ZAO REN) extract, *Sophora subprostrata* (SHAN DOU GEN) extract, *Solanum tuberosum L.* extract, *Blechnum niponicum* extract, *Gardenia jasminoides (Gardeniae Fructus)* extract, *Coriandrum sativum* extract, *Stevia rebaudiana* extract, *Euphorbia pekinensis* (DA JI) extract, *Ilex latifolia* (DA YE DONG QING, YI YE CHA, KU DING CHA) extract, *Aralia elata* extract, *Picrasma quassioides* (Nigaki) extract, *Rhus javanica* (WU BEI ZI) extract, *Drynaria fortunei* (GU SHI BU) extract, *Magnolia denudata* extract, *Iris florentina L.* extract, *Dryopteris nipponensis Koidz.* extract, *Gnaphalium affine* (SHU QU CAO) extract, *Codonopsis pilosula* (DANG SHEN) extract, *Garcinia mangostana Linn* extract, *Zingiber mioga* extract, *Melaleuca alternifolia* extract, *Alnus firma* (Yasha) extract, *Alnus pendula* extract, *Alnus sieboldiana* extract, *Leonurus heterophyllus* extract, *Alnus hirsuta var. sibirica* (Yamahannoki) extract, *Euphoria longana* (LONG YAN ROU) extract, LU GEN (*Phragmites communis*, *Phragmitis Rhizoma*) extract, logwood extract, *Lantana camara* extract.

### (26) Hair matrix cell proliferation inhibitor/hair growth inhibitor

The cosmetic composition according to the present invention may contain a hair matrix cell proliferation inhibitor/hair growth inhibitor. Specific examples of the hair matrix cell proliferation inhibitor/hair growth inhibitor include phthalazinones, benzoxazinones, phosphonic acid derivatives, cyproterone, 5-alpha-androstene-3alpha, 17beta-diol, medroxyprogesterone, norethisterone, mestanolone, *Iris tectorum* (Ichihatsu) extract, HE SHOU WU (*Polygonum multiflorum* radix) extract, Kantou extract, *Cremastra appendiculata* extract, *Gracilaria bursa-pastoris* extract, CANG ZHU (*Atractylodes lancea* rhizome) extract, *Genista tinctoria* extract, FU PING CAO (*Spirodela polyrrhiza* leaf) extract, myrrh (*Commiphora myrrha*) extract, *Champia parvula* extract, and *Sargassum siliquastrum* extract.

### (27) Hair swelling agent/hair protector

The cosmetic composition according to the present invention may contain a hair swelling agent/hair protector. Specific examples of the hair swelling agent/hair protector include ethanolamine, urea, guanidine, silicones, blueberry (*Vaccinium corymbosum*) extract, and mango extract.

### (28) Odor counteractant

The cosmetic composition according to the present invention may contain an odor counteractant. Specific examples of the odor counteractant include *Anethum graveolens* extract, Elemi (*Canarium luzonicum*) extract, vanilla beans extract, and *Pinus thunbergii* extract.

### 5. Ingredients used as additives

Specific examples of ingredients derived from a plant source, which are used as additives for the cosmetic composition according to the present invention, include plant extracts such as *Sabia japonica* (QING FENG TENG) extract, *Gastrodia elata Blume f. viridis* extract, *Ribes sativum* extract, *Ulmus parvifolia* (ROUYUPI) extract, aguai guasu extract, abiu extract, abiurana-abiu extract, yellow sapote extract, *Cananga odorata* extract, *Orchis graminifolia* extract, uva tea extract, *Epipactis papillosa* extract, unboku extract, elderberry (*Sambucus nigra*) extract, *Agave filifera cv. Compacta* extract, *Agave victoriae-reginae* extract, *Carnegiea gigantea* extract, *Calocarpum sapota* extract, *Orchis fauriei* extract, *Lygodium japonicum* (HAI JIN SHA) extract, karaya extract, garyu extract, *Coriolus versicolor* extract, candelilla (*Euphorbia antisyphilitica*) extract, *Canna generalis* extract, *Satureja* *montana* extract, *Cereus peruvianus* extract, cabebu extract, *Quillaja saponaria* extract, *Opuntia leucotricha* extract, *Op untia tuna* extract, guapeba vermelha extract, *Cephalanthera shizuoi* extract, gooseberry extract, Curitiba extract, *Curculigo latifolia* extract, *Ilex rotunda* (KYUHITSUOU) extract, *Sorghum vulgare seed* extract, zapote amarillo (*Pouteria campechiana*) extract, salmonberry (*Rubus spectabilis*) extract, SHA LAN CI TOU (*Echinops turczaninovii*) extract, *Hylocereus* and *Selenicereus* extract, *Epimedium sempervirensextract, Cymbidium goeringii* extract, *Elettaria cardamomum* extract, shirobanatsuta extract, *Cinchona succirubra* extract, swimberry extract, *Chrysophyllum cainito* extract, *Cymbidium ensifolium* extract, *Pinus sylvestris* extract, *Vaccinium myrtillus* extract, *Capparis spinosa* extract, *Phoradendron serotinum* extract, senninsaboten extract, senboku extract, zougechu extract, *Chimonanthus praecox 'Concolor'* extract, *Dioscorea cirrhosa* extract, dark sweet cherry extract, *Opuntia vulgaris* extract, chokonosutei extract, *Artemisia argyi* extract, *Neottia kiusiana* extract, *Galeola septentrionalis* extract, *Commelina communis* (ousekisou) extract, dewberry extract, *Uncaria rhynchophylla* extract, *Astragalus gummifer* extract, *Gleditschia triacanthos* extract, *Dioscoreophyllum cumminsii* extract, *Brassica campestris* extract, *Orchis joo-iokiana* extract, pao doce extract, hakukayumatou extract, huckleberry extract, batata (Caiapo; *Ipomoea batatas*) extract, *Epipactis papillosa var. sayekiana* extract, balata extract, *Cymbidium x nishiuchianum* extract, pecan nut extract, hinachiyodori extract, *Neottia asiatica* extract, *Pfaffia paniculata* extract, branjen extract, Furcellaria fastigiata extract, blonde psyllium (*Plantago ovata*) extract, *Cymbidium dayanum* extract, henequen extract, berry extract, persea extract, peruvian bark (*Cinchona succirubra*) extract, *Pereskia grandifolia* extract, *Rubus procumbens* extract, *Cymbidium sinense* extract, *Pouteria sapota* extract, *Pouteria lucuma* extract, *Echinacea angustifolia* extract, *Vaccinium myrtillus* extract, massaranduba extract, massaranduba do ceará extract, massala de boi extract, matta oglio extract, *Morus alba* extract, green sapote extract, purple maize extract, *Opuntia megacantha* extract, *Pereskia aculeata* extract, Chinese rhubarb (*Rheum palmatum*) extract, Morello cherry extract, yakawamurasakiimo extract, *Monarda fistulosa* extract, *Echinops ritro* extract, red currant extract, red pitaya (*Hylocereus costaricensis*) extract, *Cymbopogon citratus* extract, *Copernicia cerifera Mart.* extract, and loganberry extract.

Specific examples of ingredients derived from a plant source, which are used as additives for the cosmetic composition according to the present invention, include Chlorophyceae extracts susch as *Dunaliella* sp. extract, *Pandorina morum* extract, *Volvox aureus* extract, *Volvox* sp. extract, *Palmella* extract, *Tetraspora* extract, *Spirogyra* sp. and *Mougeotia* sp. extract, *Draparnaldia* extract, *Ulothrix zonata* extract, *Fortiella* extract, *Valonia macrophysa* extract, *Valonia aegagropila* extract, *Boergesenia forbesii* extract, *Caulerpa racemosa var*.*laetevirens* extract, *Caulerpa brachypus* extract, *Caulerpa scalpelliformis var.intermedia* extract, *Acetablaria ryukyuensis* extract, *Closterium* extract, *Coleochaete extract, Cosmarium* extract, *Dictyosphaeria cavernosa* extract, *Oedogonium* extract, *Pediastrum* extract, *Trentepohlia aurea* extract, *Zygnema* extract, and *Vaucheria* extract; Cyanophyceae extracts such as *Aphanothece sacrum* extract, *Microcystis* extract, and *Oscillatoria* extract; Phaeophyceae extracts such as *Pilayella littoralis* extract, *Ectocarpus* sp. extract, *Botrytella* sp. extract, *Ralfsia fungiformis* extract, *Sphacelaria tribuloides* extract, *Halopteris filicina* extract, *Cutleria cylindrica* extract, *Cutleria multifida* extract, *Cutleria adspersa* extract, *Dictyota* sp. extract, *Spatoglossum pacificum* extract, *Stypopodium zonale* extract, *Elachista taeniaeformis* extract, *Haloth rix ambigua* extract, *Leathesia difformis* extract, *Saundersella simplex* extract, *Chordaria flagelliformis* extract, *Eudesme virescens* extract, *Tinocladia crassa* extract, *Papenfussiella kuromo* extract, *Acrothrix pacifica* extract, *Carpomitra costata* extract, *Sporochnus radiciformis* extract, *Nereia intricata* extract, *Desmarestia tabacoides* extract, *Akkesiphycus lubricum* extract, *Punctaria latifolia* extract, *Asperococcus* sp. extract, *Coilodesme japonica* extract, *Colpomenia sinuosa* extract, *Hydroclathrus clathratus* extract, *Chnoospora implexa* extract, *Stictyosiphon soriferus* extract, *Striaria attenuata* extract, *Scytosiphon lomentaria* extract, *Dictyosiphon foeniculaceus* extract, *Chorda filum* extract, *Ecklonia kurome* extract, *Thallasiophyllum clathrus* extract, *Macrocystis pyrifera* extract, and Buruukimo zoku buruukimo extract; Rhodophyceae extracts such as *Porphyra pseudolinearis* extract, *Porphyra dentata* extract, *Porphyra* sp. extract, *Porphyra variegata* extract, *Porphyra amplissima* extract, *Rhodochorton howei* extract, *Liagora caenomyce* extract, *Lia gora japonica* extract, *Liagora* sp. extract, *Nemalion multifidum* extract, *Dermonema pulvinatum* extract, *Dermonema frappieri* extract, *Scinaia okamurae* extract, *Actinotrichia fra gilis* extract, *Ptilonia okadae* extract, *Gelidium subfastigiatum* extract, *Gelidium tenue* extract, *Gelidium* sp. extract, *Gelidium linoides* extract, *Dudresnaya japonica* extract, *Dudresnaya minima* extract, *Hyalosiphonia caespitosa* extract, *Pikea yoshizakii* extract, *Dumontia contorta* extract, *Dumontia simplex* extract, *Masudaphycus irregulare* extract, *Constantinea subulifera* extract, *Rhodopeltis borealis* extract, *Contarinia okamurae* extract, *Peyssonnelia caulifera* extract, *Peyssonn elia japonica* extract, *Amphiroa anceps* extract, *Corallina officinalis* extract, *Grateloupia ramosissima* extract, *Grateloupia livida* extract, *Grateloupia okamurae* extract, *Grateloupia carnosa* extract, *Grateloupia turuturu* extract, *Grateloupia lanceolata* extract, *Grateloupia kurogii* extract, *Prionitis patens* extract, *Polyopes polyideoides* extract, *Prionitis* sp. extract, *Prionitis angusta* extract, *Polyopes prolifera* extract, *Prionitis crispata* extract, *Prionitis divaricata* extract, *Prionitis articulata* extract, *Prionitis cornea* extract, *Prionitis elata* extract, *Prionitis ramosissima* extract, *Cryptonemia schmitziana* extract, *Schimmelmannia plumosa* extract, *Tichocarpus crinitus* extract, *Callophyllis okamurae* extract, *Kallymenia sessilis* extract, *Kallymenia sagamiana* extract, *Kallymenia callophylloides* extract, *Schmitzia japonica* extract, *Tsengia nakamurae* extract, *Tsengia* *lancifolia* extract, *Platoma izunosimensis* extract, *Schizymenia dubyi* extract, *Halarachnion latissimum* extract, *Sebdenia yamadae* extract, *Catenella caespitosa* extract, *Phacelocarpus japonicus* extract, *Caulacanthus ustulatus* extract, *Sarcodia ceylanica* extract, *Gracilaria incurvata* extract, *Gracilaria rhodocaudata* extract, *Gracilaria srilankia* extract, *Gracilaria sublittoralis* extract, *Gracilaria vieillardii* extract, *Gracilaria edulis* extract, *Gracilaria eucheumoides* extract, *Gracilaria lemaneiformis* extract, *Gracilaria punctata* extract, *Gracilaria arcuata* extract, *Gracilaria blodgettii* extract, *Gracilaria coronopifolia* extract, *Gracilaria cuneifolia* extract, *Gracilaria salicornia* extract, *Tylotus lichenoides* extract, *Ahnfeltia plicata* extract, *Ahnfeltia concinna* extract, *Stenogramma interrupta* extract, *Chondrus yendoi* extract, *Gloiocladia japonica* extract, *Fauchea spinulosa* extract, *Fauchea stipitata* extract, *Chrysymenia wrightii* extract, *Chrysymenia okamurae* extract, *Coelarathrum opuntia* extract, *Coela rathrum boergesenii* extract, *Botryocladia leptopoda* extract, *Cryptarachne polyglandulosa* extract, *Rhodymenia intricata* extract, *Sparlingia pertusa* extract, *Halichrysis micans* extract, *Halosaccion yendoi* extract, *Champia bifida* extract, *Champia expansa* extract, *Campylaephora hypnaeoides* extract, *Campylaephora crassa* extract, *Herpochondria elegans* extract, *Reinboldiella schmitziana* extract, *Marionella* extract, *Congregatocarpus* extract, *Neoholmesia* extract, *Sorella repens* extract, *Polyneura japonica* extract, *Neohypophyllum middendorfii* extract, *Myriogramme polyneura* extract, *Hideophyllum yezoense* extract, *Acrosorium venulosum* extract, *Acrosorium flabellatum* extract, *Acrosorium polyneurum* extract, *Acrosorium yendoi* extract, *Hymenena tenuis* extract, *Martensia fragilis* extract, *Caloglossa continua* extract, *Dasya sessilis* extract, *Heterosiphonia japonica* extract, *Heterosiphonia pulchra* extract, *Rhodoptilum plumosum* extract, *Digenea simplex* extract, *Pterosiphonia pinnulata* extract, *Kintarosiphonia fibrillosa* extract, *Symphyocladia marchantioides* extract, *Symphyocladia latiuscula* extract, *Symphyocladia linearis* extract, *Herposiphonia fissidentoides* extract, *Herposiphonia* *subdisticha* extract, *Melanamansia glomerata* extract, *Melanamansia japonica* extract, *Melanamansia mitsuii* extract, *Enantiocladia okamurae* extract, *Lenormandiopsis lorenzii* extract, *Neurymenia fraxinifolia* extract, *Cyanidium caldarium* extract, *Nemalionopsis tortuosa* extract, *Thorea okadae* extract; Charophyceae extracts such as *Chara* extract, *Lamprothamnium* extract, *Nitellopsis obtusa* extract, *Lychnothamnus* extract, *Nitella flexilis* extract, *Nitella* extract, *Tolypella* extract; and Chrysophyceae extracts such as *Chromulina* sp. extract.

Specific examples of ingredients derived from an animal source, which are used as additives for the cosmetic composition according to the present invention, include cockscomb extract, placenta extract derived from bovine placenta, swine placenta or human placenta, extract derived from swine or bovine stomach, duodenum, intestine, or spleen or degradation products thereof, extract derived from bovine or swine brain tissue, collagen derivatives such as water-soluble collagen, acylated collagen, collagen hydrolysate, elastin, elastin hydrolysate, water-soluble elastin derivatives, keratin and degradation products thereof or their derivatives, silk protein and degradation products thereof or their derivatives, swine or bovine blood protein degradation products (globin peptide), bovine or swine hemoglobin degradation products (e.g., hemin, hematin, heme, protoheme, and heme iron), cow's milk, casein and degradation products thereof or their derivatives, nonfat dry milk and degradation products thereof or their derivatives, lactoferrin or degradation products thereof, hen's egg ingredients, fish meat degradation products, and nucleic acid-related substances (e.g., ribonucleic acid, and deoxyribonucleic acid).

Specific examples of ingredients derived from a microorganism source, which are used as additives for the cosmetic composition according to the present invention, include yeast metabolites, yeast extracts, bacteria metabolites, bacteria extract, metabolites of mold or mushroom, actinomycete metabolite, extractfrom mold or mushroom, actinomycete extract, Bacillus natto metabolite, Bacillus natto extract, rice fermented extract, rice bran (red bran, white bran) fermented extract, euglena extract or degradation products thereof or their water-soluble derivatives, lactic fermented raw milk or nonfat dry milk product, and trehalose or derivatives thereof.

Materials derived from a plant or animal or microorganism source may comprise any sites, cells, tissues, organs, and metabolites derived from a transgenic lines or cell fusion products. In addition, tissue-derived cultured cells (e.g., cultured cells derived from animals, such as fibroblasts, Langerhans cells, macrophages, epidermal cells, and liver cells), undifferentiated cells, cells under differentiation processes, and metabolites thereof, which may be obtained by cultivation of sites, cells, tissues, organs, may be used.

Specific examples of naturally-occurring ingredients which may be used as additives include sea water such as deep water, e.g., sea water salt, dried sea water, inorganic salts obtained from the Dead Sea or Atlantic Ocean or Pacific Ocean (e.g., sodium chloride, magnesium chloride, and potassium chloride), sea mud or fango, such as sea mud or fango from various countries and regions, e.g., Italian fango, German fango, Eifel fango, and Freiburg fango (ingredients: silicon dioxide, titanium dioxide, aluminum oxide, iron oxide, manganese oxide, sodium oxide, potassium oxide, magnesium oxide, calcium oxide, strontium oxide, sodium, potassium, magnesium, calcium, chromium, iron, copper, nickel, zinc, lead, manganese, arsenic, water), Shotoku Ishi (a special stone containing 22 kinds of minerals produced in northern area of Japan), natural smectite, bentonite, and hectorite.

Materials derived from a plant or animal or microorganism source and other extracts derived from naturally-occurring materials, which are used as additives, may be subjected to standard procedures (for example, an appropriate combination of grinding, milling, washing, extraction, degradation, fermentation or metabolic shifting by microorganisms, fractionation, purification, compression, filtration, drying, pulverization, granulation, dissolution, sterilization, pH adjustment, deodorization, and bleaching) depending on the type and formation of the product to which they are added, and then selected from any of the resulting materials.

Extraction solvents may be selected in view of the intended use and type of the product and/or a subsequent procedure to be performed. In general, however, it is preferable that the extraction solvent be selected from one or a mixed solvent of two or more of water, lower alcohol or aqueous lower alcohol such as methanol, ethanol, propylalcohol, isopropylalcohol, butanol, and isobutanol, polyhydric alcohol or aqueous polyhydric alcohol such as propylene glycol, 1,3-butylene glycol, 1,2-butylene glycol, 1,4-butylene glycol, 1,5-pentanediol, 1,2-pentanediol, 1,3-pentanediol, 1,4-pentanediol, 1,3,5-pentanetriol, 1,2-hexanediol, 1,5-hexanediol, 1,6-hexanediol, pentylene glycol, hexylene glycol, glycerin, and polyethylene glycol (molecular weight 100 to 100,000), various organic solvents such as acetone, ethyl acetate, diethylether, dimethylether, ethylmethylether, dioxane, acetonitrile, xylene, benzene, chloroform, carbon tetrachloride, phenol, and toluene, acids (e.g., hydrochloric acid, sulfric acid, nitric acid, phosphoric acid, formic acid, and acetic acid) and alkalines (e.g., sodium hydroxide, potassium hydroxide, calcium hydroxide, and ammonia) of which normality has been appropriately adjusted. Depending on the specific usage, it should be noted that water alone may be used when the use of a solvent is not recommended or ethanol, which can easily be removed after extraction may be usd alone or as a mixed solvent with water. Alternatively, it may be an extraction of squeezed material.

Exact details of the extraction procedure may depend on the temperature of the solvent and the weight ratio of the solvent relative to the raw material. Exact details of the time for extraction may depend on the type of the raw material and solvent used.
The temperature of the solvent may be in the range of -4 degrees C to 100 degrees C. A temperature around 10 to 40 degrees C would be preferable from the viewpoint of the stability of the ingredients contained in a raw material. The weight ratio of the solvent to the raw material may be in the range of 4:1 to 1:100 (raw material:solvent), and a weight ratio of 1:1 to 1:10 is particularly preferable.

Examples of the degradation mainly include acid degradation, alkaline degradation, enzymatic degradation, and high temperature/high pressure degradation. Acid degradation preferably involves use of an inorganic or organic acid such as hydrochloric acid, sulfric acid, nitric acid, phosphoric acid, acetic acid, formic acid, oxalic acid, hydrogen bromide, perchloric acid, and periodic acid. Alkaline degradation preferably involves use of, for example, sodium hydroxide, potassium hydroxide, calcium hydroxide, ammonium hydroxide, barium hydroxide, sodium carbonate, ammonium carbonate, calcium carbonate, magnesium hydroxide, and sodium silicate. In acid or alkaline degradation, exact details of the concentration, reaction time, and reaction temperature may be determined depending on the raw material in question. Enzymatic degradation preferably involves use of an enzyme capable of degrading proteins, polysaccharides, lipids or complexes thereof which have an important role or function associated with the structure of cells or tissues. Examples of the enzyme may include proteolytic enzymes (proteases) such as aminopeptidase, dipeptidase, dipeptidylpeptidase, tripeptidylpeptidase, carboxypeptidase, serine protease, trypsin, chymotrypsin, cysteine protease, thiol protease, papain, aspartic endopeptidase, metalloendopeptidase, bromelain, thermolysin, pronase, pepsin, rennin, pancreatin, chymopapain, ficin, collagenase, and elastase, polysaccharolytic enzymes such as amylase, Taka-amylase, cellulase, hemicellulase, pectinase, polygalacturonase, dextranase, and pullanase, cell wall degrading enzymes such as hen egg white lysozyme, human lysozyme, papaya lysozyme, turnip lysozyme, barley lysozyme, zymolyase, lysozyme chloride, glucanase,and chitinase. In the enzymatic degradation, there is no limitation on the concentration, reaction time, reaction temperature and pH of the solution, which may preferably have values suitable for enzyme.

Fermentation or metabolic shifting by microorganisms may be achieved by inoculating a substrate with at least one kind of microorganisms and growing them on the substrate. Inoculation of the substrate with the microorganisms may be made by direct injection of them into the substrate or alternatively, they may be adhered on a carrier such as alginic acid, polyvinyl, and gelatin, and inoculated as fine beads made of the microorganisms in question and the carrier. Furthermore, they may be fixed on the surface of a bioreactor. Any kind of microorganisms may be used for the fermentation or metabolic shifting by microorganisms herein. It is, in general, defined as microorganisms other than pathogenic microorganisms having strong toxicity towards living organisms. Examples of the microorganism used include the following: those belonging to yeast such as *Aciculoconidium* sp., *Actonia* sp., *Aessosporon* sp., *Ambrosiozyma* sp., *Anthomyces* sp., *Apiotrichum* sp., *Arthroascus* sp., *Arxula* sp., *Ashbia* sp., *Ashbya* sp., *Asporomyces* sp., *Atelosaccharomyces* sp., *Azymoprocandida* sp., *Babjevia* sp., *Ballistosporomyces* sp., *Bensingtonia* sp., *Blastobotrys* sp., *Blastodendrion* sp., *Blastoderma* sp., *Blastoschizomyces* sp., *Botryoascus* sp., *Botryozyma* sp., *Brettanomyces* sp. (e.g., *Brettanomyces bruxellensis,* and *Brettanomyces anomalus*), *Bullera* sp., *Bulleromyces* sp., *Candida* sp. (e.g., *Candida albicans, Candida amylolenta, Candida anomala, Candida boidinii, Candida entomaea, Candida etchellsii, Candida famata, Candida fermentati, Candida guilliermondii, Candida halophila, Candida intermedia, Candida krusei*, *Candida lactosa, Candida lipolytica*, *Candida mogii, Candida parapsilosis, Candida sake, Candida tropicalis, Candida versatilis,* and *Candida vulgaris*)*, Castellania* sp., *Chlamydozyma* sp., *Chromotorula* sp., *Citeromyces* sp., *Cladosporium* sp., *Clavispora* sp., *Crebrothecium* sp., *Cryptococcus* sp., *Debaryomyces* sp. (e.g., *Debaryomyces delbrueckii, Debaryomyces halotolerans,* and *Debaryomyces hansenii*)*, Debaryozyma* sp., *Dekkera* sp., *Dekkeromyces* sp., *Dematium* sp., *Dipodascus* sp., *Eeniella* sp., *Endomycessp., Endomycopsissp., Eremascussp., Eremothecium* sp., *Eutorulopsis* sp., *Fabospora* sp., *Fellomyces* sp., *Filobasidium* sp., *Galactomyces* sp., *Geotrichoides* sp., *Geotrichum* sp., *Guilliermondella* sp., *Hanseniaspora* sp., *Hansenula* sp. (e.g., *Hansenula anomala, Hansenula kluyveri*, *Hensenula miso, Hansenula polymorpha*, and *Hansenula wickerhamii) , Hypomyces* sp., *Issatchenkia* sp., *Kloeckera* sp. (e.g., *Kloeckera brevis, Kloeckera fluorescens,* and *Kloeckera japonica) , Kloeckeraspora* sp., *Kluyveromyces* sp. (e.g., *Kluyveromyces bulgaricus, Kluyveromyces marxianus,* and *Kluyveromyces thermotolerans), Kockovaella* sp., *Kurtzmanomyces* sp., *Leucosporidium* sp., *Lipomyces* sp., *Metschnikowia* sp., *Microanthomyces* sp., *Monilia* sp., *Monospora* sp., *Monosporella* sp., *Mrakia* sp., *Myceloblastanon* sp., *Mycocandida* sp., *Mycoderma* sp., *Mycotorula* sp., *Mycotoruloides* sp., *Myxozyma* sp., *Nadsonia* sp., *Nectaromyces* sp., *Nematospora* sp., *Octosporomyces* sp., *Ogataea* sp., *Oidium* sp., *Oospora* sp., *Oosporidium* sp., *Pachysolen* sp., *Parasaccharomyces* sp., *Paratorulopsis* sp., *Parendomyces* sp., *Petasospora* sp., *Pichia* sp. (e.g., *Pichia amylophila, Pichia farinosa, Pichia guilliermondii, Pichia membranifaciens,* and *Pichiamogii*), *Pityrosporum* sp., *Procandida* sp., *Procandida* sp., *Proteomyces* sp., *Pseudomonilia* sp., *Pseudosaccharomyces* sp., *Pseudozyma* sp., *Rhodomyces* sp., *Rhodosporidium* sp., *Rhodotorula* sp., *Saccharomyces* sp. (e.g., *Saccharomyces aceti, Saccharomyces cerasi, Saccharomyces cerevisiae, Saccharomyces exiguus, Saccharomyces unisporus,* and *Saccharomyces fibuligera*)*, Saccharomycodes* sp., *Saccharomycopsis* sp., *Saturnispora* sp., *Schizoblastosporion* sp., *Schizosaccharomyces* sp., *Schwanniomyces* sp., *Selenotila* sp., *Selenozyma* sp., *Smithiozyma* sp., *Sporidiobolus* sp., *Sporobolomyces* sp., *Sporothrix* sp., *Stephanoascus* sp., *Sterigmatomyces* sp., *Sympodiomyces* sp., *Syringospora* sp., *Tetrapisispora* sp., *Torula* sp., *Torulaspora* sp., *Torulopsis* sp., *Trichosporon* sp., *Udeniomyces* sp., *Waltomyces* sp., *Willia* sp., *Williopsis* sp., *Wingea* sp., *Xanthophyllomyces* sp., *Yamadazyma* sp., *Zendera* sp., *Zygofabospora* sp., *Zygopichia* sp., *Zygosaccharomyces* sp., and *Zymodebaryomyces* sp., ; those belonging to bacteria such as *Acetobacter* sp. (e.g., *Acetobacter aceti*), *Achromoba cter* sp., *Acidianus* sp., *Acidobacterium* sp., *Acidithiobacillus* sp., *Acrocarpospora* sp., *Actinoalloteichus* sp., *Actinocorallia* sp., *Actinokineospora* sp., *Actinomadura* sp., *Actinoplanes* sp., *Actinopolyspora* sp., *Actinosynnema* sp., *Aerococcus* sp., *Aeromicrobium* sp., *Agrobacterium* sp., *Agromyces* sp., *Ahrensia* sp., *Alcaligenes* sp., *Alicyclobacillus* sp., *Alloiococcus* sp., *Alteromonas* sp., *Amorphosporan gium* sp., *Ampullariella* sp., *Amycolata* sp., *Amycolatopsis* sp., *Aquaspirillum* sp., *Arcanobacterium* sp., *Arthrobacter* sp., *Aureobacterium* sp., *Azotobacter* sp., *Bacillus* sp. (e.g., *Bacillus brevis,* and *Bacillus subtilis*)*, Bacteroides* sp., *Beneckea* sp., *Bifidobacterium* sp. (e.g., *Bifidobacterium bifidum, Bifidobacterium longum, Bifidobacterium breve,* and *Bifidobacterium infantis*), *Brachybacterium* sp., *Brevibacillus* sp., *Brevibacterium* sp., *Brevundimonas* sp., *Burkholderia* sp., *Carnobacterium* sp., *Catellatospora* sp., *Cellulomonas* sp., *Chainia* sp., *Chromobacterium* sp., *Chryseobacterium* sp., *Citrobacter* sp., *Clavibacter* sp., *Corynebacterium* sp., *Couchioplanes* sp., *Cryptosporangium* sp., *Curtobacterium* sp., *Dactylosporangium* sp., *Deinococcus* sp., *Delftia* sp., *Demetria* sp., *Dermacoccus* sp., *Dermatophilus* sp., *Elytrosporangium* sp., *Enterobacter* sp., *Erwinia* sp., *Escherichia* sp., *Eubacterium* sp. , *Excellospora* sp., *Exiguobacterium* sp., *Faenia* sp., *Flammeovirga* sp., *Flavobacterium* sp., *Flexibacter* sp., *Geodermatophilus* sp., *Globicatella* sp., *Gluconacetobacter sp., Gluconoacetobacter* sp., *Glycomyces* sp., *Gordona* sp., *Gordonia* sp., *Halobacterium* sp., *Halococcus* sp., *Herbidospora* sp., *Hydrogenophilus* sp., *Hyphomicrobium* sp., *Hyphomonas* sp., *Intrasporangium* sp., *Janibacter* sp., *Jonesia* sp., *Kibdelosporangium* sp. , *Kineococcus* sp., *Kineosporia* sp., *Kitasatoa* sp., *Kitasatospora* sp., *Kitasatosporia* sp., *Klebsiella* sp., *Kocuria* sp., *Kurthia* sp., *Lactobacillus* sp. (e.g., *Lactobacillus rimae, Lactobacillus divergens, Lactobacillus carnis, Lactobacillus piscicola, Lactobacillus acidophilus, Lactobacillus amylophilus, Lactobacillus animalis, Lactobacillus brevis, Lactobacillus casei, Lactobacillus curvatus, Lactobacillus bulgaricus, Lactobacillus delbrueckii, Lactobacillus fermentum, Lactobacillus fructivorans, Lactobacillus fructosus, Lactobacillus helveticus, Lactobacillus hilgardii, Lactobacillus homohiochii, Lactobacillus kefiri, Lactobacillus malefermentans, Lactobacillus murinus*, *Lactobacillus paracasei, Lactobacillus paracasei subsp. tolerans, Lactobacillus parakefiri, Lactobacillus pentosus, Lactobacillus plantarum, Lactobacillus reuteri, Lactobacillus rhamnosus, Lactobacillus sakei, Lactobacillus confusus, Lactobacillus viridescens, Lactobacillus johnsonii, Lactobacillus viscosus, Lactobacillus ferm entatae, Lactobacillus acidophil-aerogenes, Lactobacillus leichmannii, Lactobacillus gasseri, Lactobacillus bifidus, Lactobacillus jugurt, Lactobacillus caucasicus, Lactobacillus arabinosus, Lactobacillus kunkeei, Lactobacillus nagelii, Lactobacillus fornicalis, Lactobacillus pentoaceticus, Lactobacillus xylosus,* and *Lactobacillus minutus*)*, Leuconostoc* sp. (e.g., *Leuconostoc lactis, Leuconostoc dextranicum, Leuconostoc mesenteroides*, *Leuconostoc oenos, Leuconostoc paramesenteroides, Leuconostoc cremoris,* and *Leuconostoc citrovorum), Listonella* sp., *Lucibacterium* sp., *Luteococcus* sp., *Magnetospirillum* sp., *Marinilabilia* sp., *Marinospirillum* sp. *Mesorhizobium* sp., *Metallosphaera* sp., *Methylobacterium* sp., *Microbispora* sp., *Micrococcus* sp., *Microellobosporia* sp., *Micromonospora* sp., *Mycobacterium* sp., *Mycoplasma* sp., *Nocardia* sp., *Nocardioides* sp., *Nonomuraea* sp., *Nonomuria* sp., *Oceanospirillum* sp., *Ochrobactrum* sp., *Oerskovia* sp., *Oligella* sp., *Paenibacillus* sp., *Pediococcus* sp. (e.g., *Pediococcus cerevisiae, Pediococcus pentosaceus,* and *Pediococcus urinae-equi*), *Pedobacter* sp., *Peptococcus* sp., *Peptostreptococcus* sp., *Pilimelia* sp., *Pimelobacter* sp., *Planobispora* sp., *Planococcus* sp., *Planomonospora* sp., *Prevotella* sp., *Propionibacterium* sp., *Proteus* sp., *Protomonas* sp., *Pseudomonas* sp., *Pseudonocardia* sp., *Rahnella* sp., *Rarobacter* sp., *Rathayibacter* sp., *Rhizobium* sp., *Rhizomonas* sp., *Rhodobacter* sp., *Rhodococcus* sp., *Rhodopseudomonas* sp., *Rhodospirillum* sp., Rothia sp., *Rubrobacter* sp., *Ruegeria* sp., *Sa ccharomonospora* sp., *Saccharothrix* sp., *Serratia* sp., *Sinorhizobium* sp., *Sphingobacterium* sp., *Sphingomonas* sp., *Sporolactobacillus* sp., *Stenotrophomonas* sp., *Streptoalloteichus* sp., *Streptococcus* sp. (e.g., *Streptococcus durans*, *Streptococcus faecalis, Streptococcus faecium, Streptococcus bovis, Streptococcus equinus, Streptococcus mutans, Streptococcus salivarius, Streptococcus thermophilus, Streptococcus agalactiae, Streptococcus mitis, Streptococcus pyogenes, Streptococcus pneumoniae, Streptococcus lactis, Streptococcus dysgalactiae, Streptococcus sanguis, Streptococcus acidominimus*, *Streptococcus avium, Streptococcus uberis, Streptococcus cremoris,* and *Streptococcus diacetilactis*)*, Streptomyces* sp., *Streptosporangium* sp., *Streptoverticillium* sp., *Terrabacter* sp., *Thermoactinomyces* sp*., Thermobifida* sp., *Thermobispora* sp., *Thermocrispum* sp., *Th ermomonospora* sp., *Thermoplasma* sp., *Thiobacillus* sp., *Thiomonas* sp., *Thiosphaera* sp., *Weissella* sp., *Xanthobacter* sp., *Xanthomonas* sp., *Zymomonas* sp.; and those belonging to Ascomycotina, Basidiomycotina, and Deuteromycotina (imperfect fungi) other than yeast such as *Allomyces* sp., *Amoebidium* sp., *Amorphotheca* sp., *Arthroderma* sp., *Ascoidea* sp., *Ascobolus* sp., *Ascodesmis* sp., *Aspergillus* sp., *Aureobasidium* sp., *Botryosphaeria* sp., *Botryotinia* sp., *Brachybasidium* sp., *Byssochlamys* sp., *Capnodium* sp., *Ceratocystis* sp., *Ceratomyces* sp., *Chaetomium* sp., *Chrysella* sp., *Chytridium* sp., *Claviceps* sp., *Cochliobolus* sp., *Coemansia* sp., *Coleosporium* sp., *Coniochaetidium* sp., *Cordyceps* sp., *Cronartium* sp., *Cyttaria* sp., *Dothidea* sp., *Endogone* sp., *Entomophthora* sp., *Emericella* sp., *Eupenicillium* sp., *Eurotium* sp., *Exobasidium* sp., *Gibberella* sp., *Glomus* sp., *Graphiola* sp., *Gymnoascus* sp., *Harpella* sp., *Helicomyces* sp., *Helvella* sp., *Hemicarpenteles* sp., *Hyphochytrium* sp., *Hypocrea* sp., *Laboulbenia* sp., *Labyrinthula* sp., *Leptosphaeria* sp., *Leptosphaerulina* sp., *Lophodermium* sp., *Melanotaenium* sp., *Microascus* sp., *Microstroma* sp., *Medeolaria* sp., *Melampsora* sp., *Melamsporella* sp., *Morchella* sp., *Monascus* sp., *Monilinia* sp., *Mycospharella* sp., *Nannizzia* sp., *Nectria* sp., *Neolecta* sp., *Neurospora* sp., *Nodulosphaeria* sp., *Olpidium* sp., *Peziza* sp., *Penicillium* sp., *Perenospora* sp., *Pestalotiopsis* sp., *Phomopsis* sp., *Phragmidiella* sp., *Pneumocystis* sp., *Preussia* sp., *Pleospora* sp., *Puccinia* sp., *Pythium* sp., *Ravenelia* sp., *Rickia* sp., *Rhinocladiella* sp., *Rhizidiomyces* sp., *Rhizoctonia* sp., *Sclerocleista* sp., *Saprolegnia* sp., *Sclerotinia* sp., *Sclerotum* sp., *Septobasidium* sp., *Sordaria* sp., *Sporidiobolus* sp., *Stigmatomyces* sp., *Sydowiella* sp., *Talaromyces* sp., *Taphrina* sp., *Thraustochytrium* sp., *Tolyposporium* sp., *Trichoglossum* sp., *Trichoma,* sp., *Ustilago* sp., *Verticillium* sp., and *Xylaria* sp.

In the fermentation or metabolic shifting by microorganisms, various compounds may be added to the raw material in order to modulate or activate proliferation/metabolism of microorganisms or induce a unique biosynthesis/degradation pathway in addition to the ingredients derived from plant, animal or other naturally-occurring sources. Examples of the compounds that can be added include carbon sources such as carbohydrates, e.g., glucose, fructose, galactose, sucrose, maltose, mannose, lactose, glycerin, and starch or hydrocarbons, e.g., ethane, methane, propane, and butane, and fatty acids, e.g., formic acid, acetic acid, propionic acid, lauric acid, palmitic acid, oleic acid, linoleic acid, and linolenic acid, nitrogen sources such as ammonium salts, e.g., ammonium sulfate, ammonium chloride, and ammonium phosphate, urea, uric acid or amino acid, vitamins required for various different microorganisms, compounds containing potassium, calcium, magnesium, sodium, sulfur, phosphor, and chlorine, furthermore, iron, copper, zinc, cobalt, nickel, boron, manganese, molybdenum, tin, selenium, silicon, arsenic, vanadium, chromium, and fluorine. The optimum temperature, amount of supplied oxygen, pH or pressure which affect the proliferation and metabolism of the various different microorganisms may be determined depending on the distinguishing properties of the microorganisms in question. For example, the temperature may be determined in a range of 10 to 50 degrees C and pH may be determined in a range of 1 to 14.

Fractionation or purification may be achieved by using any one of well-known techniques including, other than solvent extraction, for example, fractionation by liquid chromatography (e.g., ion-exchange chromatography, ion-exclusion chromatography, affinity chromatography, gel filtration chromatography, size exclusion chromatography, hydrophilic adsorption chromatography, hydrophobic adsorption chromatography, and ligand exchange chromatography) dialysis through a semipermeable membrane, crystallization or recrystallization.of components, filtration using a filter, membrane filter, ultrafilter, activated carbon or filter aid, centrifugation or fractional precipitation which is a modified version of it, and density gradient separation such as equilibrium density gradient precipitation.

### 6. Other compounds used as additives for the cosmetic composition according to the present invention

Specific examples of other compounds which are used as additives for the cosmetic composition according to the present invention include inorganic pigments such as anhydrous silicic acid, magnesium silicate, talc, kaolin, bentonite, hectorite, natural or synthetic smectite, stevensite, mica, mica-titanium, bismuth oxychloride, zirconium oxide, magnesium oxide, zinc oxide, titanium oxide, calcium carbonate, magnesium carbonate, yellow iron oxide, red iron oxide, black iron oxide, ultramarine blue, chromium oxide, chromium hydroxide, carbon black, and calamine; inorganic reducing agent such as aqueous hydrogen peroxide, sodium persulfate, ammonium persulfate, sodium perborate, urea peroxide, sodium percarbonate, sodium tripolyphosphate peroxide, sodium bromate, potassium bromate, sodium pyrophosphate peroxide, sodium orthophosphate peroxide, sodium silicate to which hydrogen peroxide is added, sodium sulfate to which hydrogen peroxide is added, sodium chloride to which hydrogen peroxide is added, beta-tyrosinase solution, mushroom extract, strontium sulfate, sodium sulfide, barium sulfide, and calcium sulfide; oxidizing reducing agent such as thioglycolic acids or salts thereof (calcium thioglycolate, sodium thioglycolate, lithium thioglycolate, magnesium thioglycolate, and strontium thioglycolate); dyes such as 5-amino-orthocresol, 2-amino-4-nitrophenol, 2-amino-5-nitrophenol, 1-amino-4-methylaminoanthraquinone, 3,3'-iminodiphenol, 2,4-diaminophenoxyethanol hydrochloride, 2,4-diaminophenol hydrochloride, toluene-2,5-diamine hydrochloride, nitroparaphenylenediamine hydrochloride, para-phenylenediamine hydrochloride, N-phenylparaphenylenediamine hydrochloride, meta-phenylenediamine hydrochloride, ortho-aminophenol, N-phenylparaphenylenediamine acetate, 1,4-diaminoanthraquinone, 2,6-diaminopyridine, 1,5-dihydroxynaphthalene, toluene-2,5-diamine, toluene-3,4-diamine, nitroparaphenylenediamine, para-aminophenol, para-nitroorthophenylenediamine, para-phenylenediamine, para-methylaminophenol, picramic acid, sodium picramate, N,N'-bis(4-aminophenyl)-2,5-diamino-1,4-quinonediimine, 5-(2-hydroxyethylamino)-2-methylphenol, N-phenylparaphenylenediamine, meta-aminophenol, meta-phenylenediamine, 5-amino-orthocresol sulfate, 2-amino-5-nitrophenol sulfate, ortho-aminophenol sulfate, ortho-chloroparaphenylenediamine sulfate, 4,4'-diaminodiphenylamine sulfate, 2,4-diaminophenol sulfate, toluene-2,5-diamine sulfate, nitroparaphenylenediamine sulfate, para-aminophenol sulfate, para-nitroorthophenylenediamine sulfate, para-nitromethaphenylenediamine sulfate, para-phenylenediamine sulfate, para-methylaminophenol sulfate, meta-aminophenol sulfate, meta-phenylenediamine sulfate, catechol, diphenylamine, alpha-naphthol, hydroquinone, phloroglucine, sodium 2-hydroxy-5-nitro-2',4'-diaminoazobenzene-5'-sulfonate, and hematein; fragrances including natural animal essences such as musk, civet, castoreum, and ambergris, botanical fragrances such as anise essential oil, angelica essential oil, ylang ylang essential oil, iris essential oil, fennel essential oil, orange essential oil, cananga essential oil, caraway essential oil, cardamom essential oil, guaiacwood essential oil, cumin essential oil, Lindera essential oil, cinnamon essential oil, geranium essential oil, copaiba balsam essential oil, coriander essential oil, perilla essential oil, cedarwood essential oil, citronella essential oil, jasmine essential oil, palmarosa sofia essential oil, cedar essential oil, spearmint essential oil, Western mint essential oil, star anis essential oil, tuberose essential oil, clove essential oil, Neroli essential oil, wintergreen essential oil, tolu balsam essential oil, patchouli essential oil, rose essential oil, palmarosa essential oil, Chamaecyparis obtusa essential oil, Hiba essential oil, sandalwood essential oil, petitgrain essential oil, bay essential oil, vetivert essential oil, bergamot essential oil, Peru balsam essential oil, bois de rose essential oil, ho camphor essential oil, mandarin essential oil, eucalyptus essential oil, lime essential oil, lavender essential oil, linaloe essential oil, lemongrass essential oil, lemon essential oil, rosemary essential oil, and Japanese mint essential oil, and other synthetic fragrances; pigments and coloring agents such as 5-amino-o-cresol, o-aminophenol, m-aminophenol, p-aminophenol, 2,6-diaminopyridine, 5-(2-hydroxylethylamino)-2-methylphenol, N,N-bis(beta-hydroxyl)-p-phenylenediamine sulfate salts, N,N-bis(2-hydroxyethyl)-p-phenylenediamine, p-nitro-o-phenylenediamine, p-phenylenediamine, m-phenylenediamine, N-phenyl-p-phenylenediamine, 2-hydroxy-5-nitro-2',4'-diaminoazobenzenesodium sulfate, toluene-2,5-diamine, 2-(2'-hydroxyethylamino)-5-aminotoluene, N,N-bis(beta-hydroxyl)-p-phenylenediamine, N,N-bis(2-hydroxyethyl)-p-phenylenediamine sulfate salts, 5-amino-o-cresol sulfate salts, p-aminophenol sulfate salts, o-chloro-p-phenylenediamine sulfate salts, 2-(2'-hydroxyethylamino)-5-aminotoluene sulfate salts, 4,4'-diaminodiphenylamine sulfate salts, p-methylaminophenol sulfate salts, p-phenylenediamine sulfate salts, m-phenylenediamine sulfate salts, toluene-2,5-diamine sulfate salts, 2,4-diaminophenoxyethanol hydrochloride, toluene-2,5-diamine hydrochloride, m-phenylenediamine hydrochloride, 2,4-diaminophenol hydrochloride, 3,3'-iminodiphenol, p-phenylenediamine hydrochloride, N-phenyl-p-phenylenediamine hydrochloride, N-phenyl-p-phenylenediamine acetate salts, 1,5-dihydroxynaphthalene, toluene-3,4-diamine, p-methylaminophenol, N,N'-bis(4-aminophenyl)-2,5-diamino-1,4-quinonediimine, o-aminophenol sulfate salts, 2,4-diaminophenol sulfate salts, m-aminophenol sulfate salts, 2-amino-4-nitrophenol, 2-amino-5-nitrophenol, 1-amino-4-methylaminoanthraquinone, nitro-p-phenylenediamine hydrochloride, 1,4-diaminoanthraquinone, nitro-p-phenylenediamine, picramic acid, sodium picramate, 2-amino-5-nitrophenol sulfate salts, resorcinol, nitro-p-phenylenediamine sulfate salts, p-nitro-o-phenylenediamine sulfate salts, p-nitro-m-phenylenediamine sulfate salts, Red No. 2 (Acid Red 27), Red No. 3 (Acid Red 51), Red No. 102 (Acid Red 18), Red No. 104-(1) (Acid Red 92), Red No. 105-(1) (Acid Red 94), Red No. 106 (Acid Red 52), Red No. 201 (Pig. Red 57-1), Red No. 227 (Acid Red 33), Red No. 230-(1) (Acid Red 87), Red No. 230-(2) (Acid Red 87), Red No. 231 (Acid Red 92), Red No. 232 (Acid Red 94), Red No. 401 (Acid Violet 9), Red No. 502 (Food Red 6), Red No. 503 (Acid Red 26), Red No. 504 (Food Red 1), Red No. 506 (Acid Red 88), Yellow No. 4 (Acid Yellow 23), Yellow No. 5 (Food Yellow 3), Yellow No. 202-(1) (Acid Yellow 73), Yellow No. 202-(2) (Acid Yellow 73), Yellow No. 203 (Acid Yellow 3), Yellow No. 402 (Acid Yellow 40), Yellow No. 403-(1) (Acid Yellow 1), Yellow No. 406 (Acid Yellow 36), Yellow No. 407 (Acid Yellow 11), Orange No. 205 (Acid Orange 7), Orange No. 207 (Acid Red 95), Orange No. 402 (Acid Orange 20), Green No. 3 (Food Green), Green No. 204 (Solv. Green 7), Green No. 205 (Acid Green 5), Green No. 401 (Acid Green 1), Green No. 402 (Acid Green 3), Brown No. 201 (Acid Orange 24), Violet No. 401 (Acid Violet No. 43), Blue No. 1 (Food Blue 2), Blue No. 2 (Acid Blue 74), Blue No. 202 (Acid Blue 5), Blue No. 203 (Acid Blue 5), Blue No. 205 (Acid Blue 9), Black No. 401 (Acid Black 1), red cabbage color, monascus color, Catechol tannin, madder color, annatto color, Sepia color, turmeric oleoresin color, imperial yellow (from the flowers of the *Sophora japonica*)*,* krill color color, Japanese persimmon color, caramel, gold, silver, Gardenia jasminoides color, corn color, onion color, tamarind color, spirulina color, cherry color, laver color, hibiscus color, grape juice color, grape skin color, marigold color, purple sweet potato color, Dioscorea alata color, lac color, and rutin; fats and oils such as avocado oil, almond oil, fennel oil, perilla oil, olive oil, orange oil, Orange roughy oil, sesame oil, cocoa butter, chamomile oil, carrot oil, cucumber oil, beef tallow fatty acid, kukui nut oil, safflower oil, shea butter, liquid shea butter, soya bean oil, camellia oil, corn oil, rape seed oil, persic oil, castor oil, cotton seed oil, peanut oil, turtle oil, mink oil, egg yolk oil, palm oil, palm kernel oil, Japan wax, coconut oil, beef tallow, and pig tallow or hydrogenated versions of these oils and fats (hardened oils/fats) ; waxes such as beeswax, carnauba wax, spermaceti wax, lanolin, lanolin oil, lanolin anhydrous, hard lanolin, candelilla wax, montan wax, shellac wax, rice wax, squalene, squalane, and pristane; mineral oils such as liquid paraffin, petrolatum, paraffin, ozokerite, ceresin, and microcrystalline wax; fatty acids including natural fatty acids such as lauric acid, myristic acid, palmitic acid, stearic acid, behenic acid, oleic acid, linoleic acid, linolenic acid, docosahexaenoic acid, eicosapentaenoic acid, 12-hydroxystearic acid, undecylenic acid, tall oil, and lanolin fatty acid and synthetic fatty acids such as isononanoic acid, caproic acid, 2-ethylbutanoic acid, isopentanoic acid, 2-methylpentanoic acid, 2-ethylhexanoic acid, and isopentanoic acid; alcohols including natural alcohols such as ethanol, isopropanol, laurylalcohol, cetyl alcohol, stearyl alcohol, oleyl alcohol, lanolin alcohol, cholesterol, phytosterol, and phenoxyethanol, and synthetic alcohols such as 2-hexyldecanol, isostearyl alcohol, and 2-octyldodecanol; esters such as isopropyl myristate, isopropyl palmitate, butyl stearate, hexyl laurate, myristyl myristate, oleyl oleate, decyl oleate, octyldodecyl myristate, hexyldecyl dimethyloctanoate, diisobutyl adipate, 2-hexyldecyl adipate, diheptylundecyl adipate, N-alkylglycol monoisostearate, isocetyl isostearate, trimethylolpropane triisostearate, ethylene glycol diethylhexanoate, neopentylglycol diethylhexanoate, cetyl 2-ethylhexanoate, trimethylolpropane triethylhexanoate, pentaerythritol tetraethylhexanoate, cetyl octanoate, octyldodecyl gum, cetyl lactate, myristyl lactate, diethyl phthalate, dibutyl phthalate, lanolin acetate, ethylene glycol monostearate, propylene glycol monostearate, and propylene glycol dioleate; metal soaps such as aluminum stearate, magnesium stearate, zinc stearate, calcium stearate, zinc palmitate, magnesium myristate, zinc laurate, and zinc undecylenate;
anionic surfactants (e.g., alkylcarboxylic acid salts, alkylsulfonic acid salts, alkylsulfate ester salts, and alkylphosphate ester salts), alkylsulfate ester salts (e.g., sodium lauryl sulfate, potassium lauryl sulfate, triethanolamine lauryl sulfate, ammonium lauryl sulfate, sodium cetyl sulfate, and sodium stearyl sulfate), primary alcohol sulfate ester salts, alkyl ether sulfate ester salts (e.g., triethanolamine polyoxyethylene lauryl sulfate, and sodium polyoxyethylene lauryl sulfate), alkyl and alkylallyl ether sulfate ester salts (e.g., N-acylmethylalanine salts, N-acylglutaminate salts, N-acylmethyltaurine salts, N-acylisethionate salts, N-acylglycine salts, alkylphosphate salts, alkylethercarboxylate salts, alkylsulfonate salts, alkylsulfosuccinate salts, sodium polyoxyethylene lauryl ether sulfate, triethanolamine polyoxyethylene lauryl ether sulfate, ammonium polyoxyethylene lauryl ether sulfate, sodium polyoxyethylene alkyl ether sulfate, triethanolamine polyoxyethylene alkyl ether sulfate, diethanolamine polyoxyethylene alkyl ether sulfate, and ammonium polyoxyethylene alkyl ether sulfate); anionic surfactants including fatty acid soaps such as higher fatty acid alkylolamide sulfate ester salts, higher fatty acid ester sulfate ester salts (e.g., sodium hardened coconut oil fatty acid glycerin sulfate), polyoxyethylene alkyl ether carboxylic acid, polyoxyethylene alkylallyl ether carboxylate salts, sulfated oils such as sulfated caster oil, mono- or dialkyl or alkenyl phosphate ester salts, polyoxyethylene mono- or dialkyl or alkenyl phosphate ester salts (e.g., sodium monolauryl phosphate, sodium isostearyl phosphate, arginine 2-hexyldecyl phosphate, potassium 2-heptylundecyl phosphate, sodium polyoxyethylene (4 to 10) lauryl ether phosphate, sodium monocetyl phosphate, arginine monomyristyl phosphate, sodium monooleyl phosphate, potassium polyoxyethylene (2 to 10) lauryl ether phosphate, and sodium dioctyl phosphate), phosphate ester salts of polyoxyethylene alkyl ether (e.g., polyoxyethylene lauryl ether phosphate, polyoxyethylene oleyl ether phosphate, polyoxyethylene cetyl ether phosphate, polyoxyethylene stearyl ether phosphate, polyoxyethylene alkyl ether phosphate, and polyoxyethylene alkylphenyl ether phosphate), sulfonate salts of higher fatty acid amide or sulfonate salts of higher fatty acid ester (e.g., alpha-olefin sulfonate salts, N-myristoyl-N-methyltaurine sodium, coconut oil fatty acid methyltaurine sodium, and lauroyl methyltaurine sodium), alkylbenzene sulfonic acid salts (e.g., dodecylbenzene sulfonic acid, dodecylbenzene sulfonic acid monoethanolamine, dodecylbenzene sulfonic acid diethanolamine, dodecylbenzene sulfonic acid triethanolamine, and sodium dodecylbenzene sulfonate), sulfosuccinate salts (e.g., sodium diethylhexyl sulfosuccinate, sodium sulfosuccinate, disodium lauryl sulfosuccinate, disodium polyoxyethylene sulfosuccinate, disodium polyoxyethylene lauryl sulfosuccinate, disodium polyoxyethylene lauroyl ethanolamide ester sulfosuccinate, disodium undecylenoylamide ethylsulfosuccinate, sodium monolauroyl monoethanolamide polyoxyethylene sulfosuccinate, and sodium laurylpolypropylene glycol sulfosuccinate), N-acyl sarcosine salts (e.g., lauroyl sarcosine sodium, and palmitoyl sarcosine sodium), condensation products of a higher fatty acid and an amino acid (e.g., N-acyl glutamate salts, sodium N-stearoyl glutamate, potassium N-palmitoylglutamate, sodium N-lauroyl-L-glutamate, disodium N-stearoyl-L-glutamate, sodium N-myristoyl-L-glutamate, myristoyl methyltaurine sodium, palmitoyl methyltaurine sodium, stearoyl methyltaurine sodium, N-palmitoyl-beta-alanine arginine, N-palmitoyl asparaginate ditriethanol amine), oleate salts, stearate salts, laurate salts, and palmitate salts (examples of salts of the anionic surfactant include alkaline metal salts such as sodium salts, potassium salts, organic ammonium salts such as monoethanol amine salts, diethanol amine salts, triethanol amine salts, L-lysine salts, and L-arginine salst, and ammonium salts) ; cationic surfactants such as quaternary ammonium salts (e.g., alkyl trimethyl ammonium chloride, cetyl ammonium chloride, cetyl ammonium bromide, lauryl ammonium chloride, lauryl ammonium bromide, stearyl ammonium chloride, stearyl ammonium bromide, cetyl dimethyl ammonium chloride, cetyl dimethyl ammonium bromide, lauryl dimethyl ammonium chloride, stearyl dimethyl ammonium chloride, lauryl trimethyl ammonium chloride, lauryl trimethyl ammonium bromide, stearyl dimethyl cetyl ditallow dimethyl ammonium chloride, dicetyl ammonium chloride, dicetyl ammonium bromide, dilauryl ammonium chloride, dilauryl ammonium bromide, distearyl ammonium chloride, distearyl ammonium bromide, dicetyl methyl ammonium chloride, dicetyl methyl ammonium bromide, dilauryl methyl ammonium chloride, dilauryl methyl ammonium bromide, distearyl methyl ammonium chloride, distearyl methyl ammonium bromide, cetyl trimethyl ammonium chloride, cetyl trimethyl ammonium bromide, stearyl trimethyl ammonium chloride, stearyl trimethyl ammonium bromide, lauryl trimethyl ammonium bromide, dialkyl dimethyl ammonium chloride, dicetyl dimethyl ammonium chloride, distearyl dimethyl ammonium chloride, dicocoyl dimethyl ammonium chloride, myristyl dimethyl benzyl ammonium chloride, stearyl dimethyl benzyl ammonium chloride, lanolin fatty acid aminopropyl ethyldimethylammonium ethyl sulfate, cetyl trimethyl ammonium chloride, stearyl trimethyl ammonium chloride, behenyl trimethyl ammonium chloride, behenyl dimethylhydroxyethyl ammonium chloride, stearyl dimethylbenzyl ammonium chloride, distearyl dimethyl ammonium chloride, distearoylethyl hydroxyethylmonium methosulfate, dicocoylethyl hydroxyethylmonium methosulfate, dipalmitoylethyl hydroxyethylmonium methosulfate, and cetyltriethyl ammoniummethyl sulfate, alkyl trimethyl ammonium chloride, alkyl dimethylbenzyl ammonium chloride, dialkyl dimethyl ammonium chloride, alkyl trimethyl ammonium bromide, alkyl pentaethoxy ammonium chloride, ditallow dimethyl ammonium chloride, ditallow dimethyl ammonium methyl sulfate, ditallow dipropyl ammonium phosphate, ditallow dimethyl ammonium nitrate, di(coconutalkyl) dimethyl ammonium chloride, di(coconutalkyl) dimethyl ammonium bromide, coconut ammonium chloride, stearamidopropyl PG-dimonium chloride phosphate, stearamidopropyl ethyldimonium ethosulfate, stearamidopropyl dimethyl(myristyl acetate) ammonium chloride, stearamidopropyl dimethylcetearyl ammonium tosylate, stearamidopropyl dimethyl ammonium chloride, stearamidopropyl dimethyl ammonium lactate, lanolin fatty acid aminopropyl ethyldimethylammonium ethyl sulfate (N(N'-Lanolin fatty acid amide propyl) N-ethyl-N,N-dimethyl ammonium), cationated cellulose, acyl diethylaminoethylamide, acyl dimethylaminopropylamide, polyoxyethylene fatty acid amide, myristyl dimethylamine oxide, betaine N-lauryldimethyl aminoacetate, betaine N-myristyldimethyl aminoacetate, and betaine N-stearyldimethyl aminoacetate), bis-fatty acid ester salts (e.g., hydroxypropyl-bis-lauric acid amidopropyl-N,N-dimethyl ammonium chloride, hydroxypropyl-bis-myristic acid amidopropyl-N,N-dimethyl ammonium chloride, hydroxypropyl-bis-palmitic acid amidopropyl-N,N-dimethyl ammonium chloride, hydroxypropyl-bis-stearic acid amidopropyl-N,N-dimethyl ammonium chloride, hydroxypropyl-bis-behenic acid amidopropyl-N,N-dimethyl ammonium chloride, hydroxypropyl-bis-oleic acid amidopropyl-N,N-dimethyl ammonium chloride, hydroxypropyl-bis-isostearic acid amidopropyl-N,N-dimethyl ammonium chloride, hydroxypropyl-bis-coconut fatty acid amidopropyl-N,N-dimethyl ammonium chloride, hydroxypropyl-bis-palm fatty acid amidopropyl-N,N-dimethyl ammonium chloride, hydroxypropyl-bis-tallow fatty acid amidopropyl-N,N-dimethyl ammonium chloride, hydroxypropyl-bis-lauric acid amidoethyl-N,N-diethyl ammonium chloride, hydroxypropyl-bis-myristic acid amidoethyl-N, N-diethyl ammonium chloride, hydroxypropyl-bis-palmitic acid amidoethyl-N,N-diethyl ammonium chloride, hydroxypropyl-bis-stearic acid amidoethyl-N,N-diethyl ammonium chloride, hydroxypropyl-bis-behenic acid amidoethyl-N,N-diethyl ammonium chloride, hydroxypropyl-bis-oleic acid amidoethyl ammonium chloride, hydroxypropyl-bis-isostearic acid amidoethyl-N,N-diethyl ammonium chloride, hydroxypropyl-bis-coconut fatty acid amidoethyl-N,N-diethyl ammonium chloride, hydroxypropyl-bis-palm fatty acid amidoethyl-N,N-diethyl ammonium chloride, hydroxypropyl-bis-tallow fatty acid amidoethyl-N,N-diethyl ammonium chloride, hydroxypropyl-bis-lauric acid amidopropyl-N,N-diethyl ammonium chloride, hydroxypropyl-bis-myristic acid amidopropyl-N,N-diethyl ammonium chloride, hydroxypropyl-bis-palmitic acid amidopropyl-N,N-diethyl ammonium chloride, hydroxypropyl-bis-stearic acid amidopropyl-N,N-diethyl ammonium chloride, hydroxypropyl-bis-behenic acid amidopropyl-N,N-diethyl ammonium chloride, hydroxypropyl-bis-oleic acid amidopropyl-N,N-diethyl ammonium chloride, hydroxypropyl-bis-isostearic acid amidopropyl-N,N-diethyl ammonium chloride, hydroxypropyl-bis-coconut fatty acid amidopropyl-N,N-diethyl ammonium chloride, hydroxypropyl-bis-palm fatty acid amidopropyl-N,N-diethyl ammonium chloride, and hydroxypropyl-bis-tallow fatty acid amidopropyl-N,N-diethyl ammonium chloride), stearamidopropyl PG-dimonium chloride phosphate, behenamidopropyl PG dimonium chloride, stearamidopropyl ethyldimonium ethosulfate, stearamidopropyl dimethyl(myristyl acetate) ammonium chloride, stearamidopropyl dimethylcetearyl ammonium tosylate, stearamidopropyl dimethyl ammonium chloride, and stearamidopropyl dimethyl ammonium lactate salts; nonionic surfactants such as glycerin fatty acid esters (e.g., glyceryl monoundecylen, glyceryl diundecylenate, glyceryl monomyristate, glyceryl dimyristate, glyceryl monopalmitate, glyceryl dipalmitate, glyceryl monostear, glyceryl distear, glyceryl monoole, monocotton oil fatty acid glycerin, glycerin monoerucate, glycerin sesquioleate, glycerin monostearate, glycerin alpha, alpha'-oleate pyroglutamate, and glycerin monostearate malate), polyglycerin fatty acid esters (e.g., diglyceryl monomyristate, diglyceryl dimyristate, diglyceryl trimyristate, diglyceryl monopalmitate, diglyceryl dipalmitate, diglyceryl tripalmitate, diglyceryl monostearate, diglyceryl distearate, diglyceryl tristearate, diglyceryl monooleate, diglyceryl dioleate, diglyceryl trioleate, tetraglyceryl monopalmitate, tetraglyceryl dipalmitate, tetraglyceryl tripalmitate, tetraglyceryl tetrapalmitate, tetraglyceryl pentapalmitate, tetraglyceryl monostearate, tetraglyceryl distearate, tetraglyceryl tristearate, tetraglyceryl tetrastearate, tetraglyceryl pentastearate, tetraglyceryl monooleate, tetraglyceryl dioleate, tetraglyceryl trioleate, tetraglyceryl tetraoleate, tetraglyceryl pentaoleate, hexaglyceryl monopalmitate, hexaglyceryl dipalmitate, hexaglyceryl tripalmitate, hexaglyceryl tetrapalmitate, hexaglyceryl pentapalmitate, hexaglyceryl monostearate, hexaglyceryl distearate, hexaglyceryl tristearate, hexaglyceryl tetrastearate, hexaglyceryl pentastearate, hexaglyceryl monooleate, hexaglyceryl dioleate, hexaglyceryl trioleate, hexaglyceryl tetraoleate, hexaglyceryl pentaoleate, decaglyceryl monopalmitate, decaglyceryl dipalmitate, decaglyceryl tripalmitate, decaglyceryl tetrapalmitate, decaglyceryl pentapalmitate, decaglyceryl hexapalmitate, decaglyceryl heptapalmitate, decaglyceryl octapalmitate, decaglyceryl nonapalmitate, decaglyceryl decapalmitate, decaglyceryl monostearate, decaglyceryl distearate, decaglyceryl tristearate, decaglyceryl tetrastearate, decaglyceryl pentastearate, decaglyceryl hexastearate, decaglyceryl heptastearate, decaglyceryl octastearate, decaglyceryl nonastearate, decaglyceryl decastearate, decaglyceryl monooleate, decaglyceryl dioleate, decaglyceryl trioleate, decaglyceryl tetraoleate, decaglyceryl pentaoleate, decaglyceryl hexaoleate, decaglyceryl heptaoleate, decaglyceryl octaoleate, decaglyceryl nonaoleate, decaglyceryl decaoleate, decaglyceryl monohydroxystearate, decaglyceryl dihydroxystearate, decaglyceryl trihydroxystearate, decaglyceryl tetrahydroxystearate, decaglyceryl pentahydroxystearate, decaglyceryl hexahydroxystearate, decaglyceryl heptahydroxystearate, decaglyceryl octahydroxystearate, decaglyceryl nonahydroxystearate, decaglyceryl decahydroxystearate, hexaglyceryl polyricinoleate, decaglyceryl polyricinoleate, polyoxyethylene glycerin monostearate, polyoxyethylene glycerin distearate, polyoxyethylene glycerin monooleate, and polyoxyethylene glycerin monomyristate), polyoxyethylene glycerin fatty acid esters (e.g., polyoxyethylene glycerin monostearate, polyoxyethylene glycerin monoisostearate, and polyoxyethylene glycerin triisostearate), sorbitan fatty acid esters (e.g., sorbitan monolaurate, sorbitan monooleate, sorbitan sesquioleate, sorbitan trioleate,sorbitan monopalmitate, sorbitan monostearate, sorbitan distearate, sorbitan dioleate, sorbitan monopalmitate, sorbitan tristearate, sorbitan trioleate, diglycerin sorbitan penta-2-ethylhexylate, diglycerin sorbitan tetra-2-ethylhexylate, polyoxyethylene sorbitan monooleate, polyoxyethylene sorbitan monostearate, polyoxyethylene sorbitan monopalmitate, polyoxyethylene sorbitan monolaurate, polyoxyethylene sorbitan trioleate, and polyoxyethylene sorbitan pentaoleate), propylene glycol fatty acid esters (e.g., propylene glycol monooctanoate, propylene glycol monodecanoate, and propylene glycol monostearate), sucrose fatty acid esters (e.g., sucrose monolaurate esters, sucrose dilaurate esters, sucrose monopalmitate esters, sucrose dipalmitate esters, sucrose monostearate esters, and sucrose distearate esters), dextrin fatty acid esters (e.g., dextrin palmitate), coconut oil fatty acid alkanolamide, polyoxyethylene caster oil, polyoxyethylene hydrogenated caster oil, polyoxyethylene hydrogenated caster oil monoisostearate, polyoxyethylene hydrogenated caster oil triisostearate, polyoxyethylenehydrogenated caster oil monopyroglutamate monoisostearate diester, polyoxyethylene hydrogenated caster oil maleate, polyoxyethylene alkylether (e.g., alkylpolyglucoside, polyoxyethylene cetyl ethers, polyoxyethylene stearyl ethers, polyoxyethylene behenyl ethers, polyoxyethylene oleyl ethers, polyoxyethylene lauryl ethers, polyoxyethylene-2-octyldodecyl ethers, and polyoxyethylene cholestanol ethers), polyoxyethylene alkyl phenyl ethers (e.g., polyoxyethylene nonyl phenyl ethers, and polyoxyethylene octyl phenyl ethers), polyoxyethylene fatty acid esters (e.g., polyethylene glycol monooleate, polyethylene glycol monostearate, and polyethylene glycol monolaurate), lanolin derivatives (e.g., polyoxyethylene lanolin, polyoxyethylene lanolin alcohol, and polyoxyethylene sorbitol lanolin), POE/POP-alkyl ethers (e.g., POE/POP-cetyl ethers, POE/POP-2-decyltetradecyl ethers, POE/POP-monobutyl ethers, POE/POP-hydrated lanolin, POE/POP-glycerin ethers, and Tetronic), and alkanolamides (e.g., coconut oil fatty acid diethanolamides, lauric acid monoethanolamides, and fatty acid isopropanolamides); amphoteric surfactants such as betaine amphoteric surfactants (e.g., 2-alkyl-N-carboxymethyl-N-hydroxyethyl imidazolinium betaine, lauryl dimethylaminoacetic acid betaine, undecylcarboxymethoxyethyl carboxymethyl imidazolinium betaine sodium, undecylhydroxyethyl imidazolinium betaine sodium, undecyl-N-hydroxyethyl-N-carboxymethyl imidazolinium betaine, alkyldiaminoethyl glycine hydrochloride solutions, stearyl dihydroxyethyl betaine, stearyl dimethylaminoacetic acid betaine, stearyl dimethyl betaine sodium solutions, coconut oil alkyl-N-carboxyethyl-N-hydroxyethyl imidazolinium betaine sodium, coconut oil alkyl betaine, coconut oil alkyl dimethylaminoacetic acid betaine, coconut oil fatty acid amidopropyl betaine, coconut oil fatty acid-N-carboxymethoxyethyl-N-carboxyethyl imidazolinium betaine sodium, N-lauroyl-N'-carboxymethyl-N'-hydroxyethyl ethylene diamine sodium, N-cocoyl-N'-carboxymethyl-N'-hydroxyethyl ethylene diamine sodium, lauryl dimethylaminoacetic acid hydroxysulfobetaine, coconut oil alkyl dimethylaminoacetic acid hydroxysulfobetaine, lauryl aminopropionic acid triethanol amine, beta-lauryl aminopropionic acid sodium, lauric acid amidopropyl dimethylaminoacetic acid betaine, lauric acid amidopropyl dimethylaminoacetic acid hydroxysulfobetaine, coconut oil fatty acid amidopropyl dimethylaminoacetic acid hydroxysulfobetaine, palm oil fatty acid amidopropyl dimethylaminoacetic acid hydroxysulfobetaine, coconut oil fatty acid amidopropyl dimethylaminoacetic acid betaine, palm oil fatty acid amidopropyl dimethylaminoacetic acid betaine, lauryl-N-carboxymethoxyethyl-N-carboxymethyl imidazolinium disodium dodecanoyl sarcosine, lauryl diaminoethyl glycine sodium, lauric acid amidopropyl betaine solution, lauryl sulfobetaine, lauryl hydroxysulfobetaine, 2-heptadecyl-N-carboxymethyl-N-hydroxyethyl imidazolinium betaine, cocodimethyl carboxymethyl betaine, lauryl dimethyl carboxymethyl betaine, lauryl dimethyl alpha-carboxyethyl betaine, cetyl dimethylcarboxymethyl betaine, cetyl dimethyl betaine, lauryl bis-(2-hydroxyethyl)carboxymethyl betaine, stearyl bis-(2-hydroxypropyl)carboxymethyl betaine, oleyl dimethyl gamma-carboxypropyl betaine, lauryl bis-(2-hydroxypropyl) alpha-carboxyethyl betaine, cocodimethyl sulfopropyl betaine, stearyldimethyl sulfopropylbetaine, lauryl dimethyl sulfoethyl betaine, lauryl bis-(2-hydroxyethyl)sulfopropyl betaine, LEBON 2000 (Sanyo Chemical Industries, Ltd.), Cocamidopropyl betaine, Cocamidopropyl hydroxy sultaine, imidazolinium amphoteric surfactants (e.g., bis(stearyl-N-hydroxyethyl imidazoline)chloroacetic acid complexes, N-alkyl-N-carboxymethyl-N-hydroxyethyl imidazolinium betaine, N-alkyl-N-carboxymethyl-N-hydroxyethyl ethylene diamine/lauryl sulfate and salts thereof, coconut oil alkyl-N-carboxyethoxyethyl-N-carboxyethyl imidazolinium disodium hydroxide, coconut oil alkyl-N-carboxymethoxyethyl-N-carboxyethyl imidazolinium disodium hydroxide, coconut oil alkyl-N-carboxymethoxyethyl-N-carboxyethyl imidazolinium disodium lauryl sulfate, 2-undecyl-N,N,N-(hydroxyethylcarboxymethyl)-2-imidazoline sodium, 2-cocoyl-2-imidazolinium hydroxide-1-carboxyethyloxydisodium salts, OVAZOLIN 662 (TOHO Chemical Industry Co.), SWANOL AM-101 (Nikko Chemicals Co., Ltd.), and ANON GLM (NOF Corporation)), aminopropionic acid amphoteric surfactants (e.g., alkylaminopropionate and alkylaminodipropionate such as dodecylaminopropionate sodium), dodecylaminopropane sulfonate sodium, N-higher alkylaspartic acids or salts thereof, coamidopropyl polyethylene glycol dimonium chloride phosphate, cocamidopropyl hydroxysultain, lauroyl sarcosinate sodium, alkyl amine oxide (e.g., decyl amine oxide, cocoamine oxide, myristoyl amine oxide, palmitoyl amine oxide, alkyl dimethyl amine oxide, and alkyl amidopropyl amine oxide); natural surfactants (e.g., lecithin, sodium caseinate, sphingolipids, saponin, monosaccharide polyhydric alcohol fatty acid esters or disaccharide polyhydric alcohol fatty acid esters such as mannosyl polyhydric alcohol fatty acid esters, glucosyl polyhydric alcohol fatty acid esters, and galactosyl polyhydric alcohol fatty acid esters, and oligosaccharide polyhydric alcohol fatty acid esters); amino acid surfactants (e.g., reaction products of fatty acid chloride and an amino acid in a broad sense, such as glycine, sarcosine, beta-alanine, N-methyl-beta-alanine, taurine, and N-methyltaurine, for example, capric acid chloride, lauric acid chloride, myristic acid chloride, palmitic acid chloride, stearic acid chloride, oleic acid chloride, behenic acid chloride, coconut oil fatty acid chloride); silicone surfactants (e.g., polyoxyalkylene-modified organopolysiloxane such as SILICONE KF-6011 (manufactured by Shin-Etsu Chemical Co., Ltd.), KF-6012 (manufactured by Shin-Etsu Chemical Co., Ltd.), KF-6013 (manufactured by Shin-Etsu Chemical Co., Ltd.), KF-6015 (manufactured by Shin-Etsu Chemical Co., Ltd.), KF-6016 (manufactured by Shin-Etsu Chemical Co., Ltd.), KF-6017 (manufactured by Shin-Etsu Chemical Co., Ltd.), X-22-4991 (manufactured by Shin-Etsu Chemical Co. , Ltd.), NUC SILICONE L7002 (manufactured by Nippon Unicar Company Limited), SH-3772C (manufactured by Dow Corning Toray Silicone Co.,Ltd.), and SH-3775C (manufactured by Dow Corning Toray Silicone Co.,Ltd.), and long-chain alkyl-containing polyoxyalkylene-modified organopolysiloxane such as ABIL EM-90 (manufactured by Goldschmidt GmBH), ABIL B9806 (manufactured by Goldschmidt GmBH), and SILICONE KF-6026 (manufactured by Shin-Etsu Chemical Co., Ltd.)); silicone compounds such as polyether-modified silicone (e.g., polyoxyethylene/methylpolysiloxane copolymers, and poly(oxyethylene/oxypropylene)methylpolysiloxane copolymers), oxazoline-modified organopolysiloxane, alkyl-modified organopolysiloxane, styryl ketone silicone derivatives, dimethyl polysiloxane, methylphenylpolysiloxane, epoxy-modified silicone, fluorine-modified silicone, alcohol-modified silicone, alkyl-modified silicone, alkoxy-modified silicone, amino-modified silicone, ammonium-modified polymer silicone, organopolysiloxane, methylphenylpolysiloxane, polysiloxane, poly(N-acylalkyleneimine)-modified silicone, dimethylpolysiloxane, nitrogen-containing acryl-silicone graft copolymers, decamethylcyclopentasiloxane, fluorine-substituted alkyl-modified silicone, organopolysiloxanyl silylalkyl-modified silicone, tricyclic diterpene carboxylic acid-modified silicone, trifluoroalkyl-modified silicone, divinylpolydimethylsiloxane, dihydrogeno polydimethylsiloxane, dihydrogeno polydimethylsiloxane divinylpolydimethyl siloxane polymers, trimethylsiloxysilicate, polyoxypropylene/methylpolysiloxane copolymers, ethylpolysiloxane, ethylmethylpolysiloxane, ethylphenylpolysiloxane, octamethylcyclotetrasiloxane, diorganopolysiloxane, octamethylcyclotetrasiloxane, tetramethyltetraphenyltetracyclosiloxane, polyether-modified polysiloxane, amino-modified polysiloxane, epoxy-modified polysiloxane, fluorine-modified polysiloxane, alcohol-modified polysiloxane, alkyl-modified polysiloxane, dodecamethylcyclohexasiloxane, lauryl methicone copolyol, aminoethylaminopropylsiloxane/dimethylsiloxane copolymers, and eugenol-modified silicone; and hair treatment agents such as selenium disulfide, alkyl isoquinolinium bromide solutions, zinc pyrithione, biphenamine, thianthrene, quinine hydrochloride, and strong ammonia solutions.

One or more of the following additives may also be used in the present invention: hormones, sequestrants, pH adjusting agents, chelating agents, antiseptic/antifungal agents, agents capable of generating a cool sensation, stabilizers, emulsifiers, animal and plant proteins and degradation products thereof, animal and plant polysaccharides and degradation products thereof, animal and plant glycoproteins and degradation products thereof, antiphlogistic/antiallergic agents, wound healing agents, foam boosters, thickeners, enzymes, purified waters (e.g., electronic water, restructuring of the water clusters from usual groups down to the smaller number of molecules per water cluster), deodorants/deodorization agents.

The present invention is further described below in conjunction with some examples thereof. However, it should be noted that the present invention is not limited thereto.

### [Examples]

### [Synthesis Example 1]

A 100-mL three-necked flask equipped with a magnetic stirrer, a condenser tube, and a thermometer was charged with 10.0 g of epsilon-polylysine (number average molecular weight: 4,090) and 30 g of methanol. The mixture was stirred at room temperature to dissolve the epsilon-polylysine. Then, the temperature was increased to 50 degrees C, and 2.1 g (8.05 x 10⁻³ mol) of (3-glycidoxypropyl)-pentamethyldisiloxane represented by the general formula (9) was added dropwise for 5 minutes. The reaction was continued for 3 hours while the temperature was kept at 50 degrees C. After 3 hours, the reaction mixture was cooled and 10.0 g of ethanol was added thereto. Subsequently, volatile components in the reaction mixture were removed on an evaporator under reduced pressure to yield 11.8 g of pentamethylsiloxane-containing epsilon-polylysine as a slightly yellow solid.

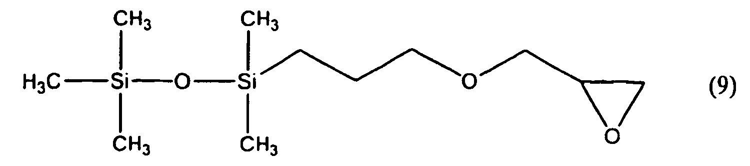

### [Synthesis Example 2]

A 100-mL three-necked flask equipped with a magnetic stirrer, a condenser tube, and a thermometer was charged with 10.0 g of epsilon-polylysine (number average molecular weight: 4,090) and 30.0 g of methanol. The mixture was stirred at room temperature to dissolve the epsilon-polylysine. Then, 20.0 g of 2-propanol was added, the temperature was increased to 70 degrees C, and 2.9 g of polydimethylsiloxane (number average molecular weight: 1,000) represented by the general formula (10) having an epoxy group at one end was added dropwise for 5 minutes. The reaction was continued for 3 hours while the temperature was kept at 70 degrees C. After the reaction mixture was cooled to room temperature, volatile components in the reaction mixture were removed on an evaporator under reduced pressure to yield 12.6g of polydimethylsiloxane-containing epsilon-polylysine as a slightly yellow solid. This compound had a residual amino group content of 97% and silicone/epsilon - polylysine = 23/77 (weight ratio).

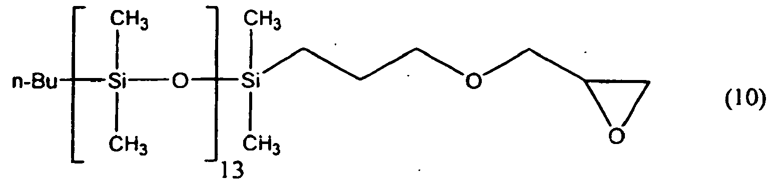

### [Synthesis Example 3]

A 100-mL three-necked flask equipped with a magnetic stirrer, a condenser tube, and a thermometer was charged with 5.0 g of epsilon-polylysine (number average molecular weight = 4,090) and 30.0 g of methanol. The mixture was stirred at room temperature to dissolve the epsilon-polylysine. The temperature was increased to 50 degrees C, and then 5.0 g (33.8 x 10⁻³ mol) of (3-glycidoxypropyl)-pentamethyldisiloxane represented by the aforementioned general formula (9) was added dropwise for 10 minutes. The reaction was continued for 3 hours while the temperature was kept at 50 degrees C. After 3 hours, the reaction mixture was cooled and 10.0 g of ethanol was added thereto. Subsequently, volatile components in the reaction mixture were removed on an evaporator under reduced pressure to yield 10.0 g of yellow syrupy compound, pentamethylsiloxane-containing epsilon-polylysine. This polymer had a residual amino group content of 50% and silicone/epsilon - polylysine = 50/50 (weight ratio).

### [Synthesis Example 4]

A 100-mL three-necked flask equipped with a magnetic stirrer, a condenser tube, and a thermometer was charged with 5.0 g of epsilon-polylysine (number average molecular weight = 4,090) and 20.0 g of methanol. The mixture was stirred at room temperature to dissolve the epsilon-polylysine. Then, 20.0 g of 2-propanol was added. After the temperature was increased to 70 degrees C, 5.0 g of polydimethylsiloxane (number average molecular weight: 1,000) having an epoxy group at one end, which was similar to the one used in the aforementioned general formula (10), was added dropwise for 5 minutes. The reaction was continued for 3 hours while the temperature was kept at 50 degrees C. After the reaction mixture was cooled to room temperature, volatile components in the reaction mixture were removed on an evaporator under reduced pressure to yield 9.9 g of slightly yellow solid, polydimethylsiloxane-containing epsilon-polylysine. This polymer had a residual amino group content of 91% and silicone/epsilon - polylysine = 50/50 (weight ratio).

### [Synthesis Example 5]

A 100-mL three-necked flask equipped with a magnetic stirrer, a condenser tube, and a thermometer was charged with 5.00 g of epsilon-polylysine (number average molecular weight = 4,090) and 20.0 g of methanol. The mixture was stirred at room temperature to dissolve the epsilon-polylysine. Then, 20.0 g of 2-propanol was added, the temperature was increased to 50 degrees C, and 6.12 g of polydimethylsiloxane (number average molecular weight: 5,000) represented by the general formula (11) having an epoxy group at one end was added dropwise for 5 minutes. The reaction was continued for 3 hours while the temperature was kept at 70 degrees C. After the reaction mixture was cooled to room temperature, volatile components in the reaction mixture were removed on an evaporator under reduced pressure to yield 11.1 g of slightly yellow solid, polydimethylsiloxane-containing epsilon-polylysine. This polymer had a residual amino group content of 97% and silicone/epsilon - polylysine = 55/45 (weight ratio).

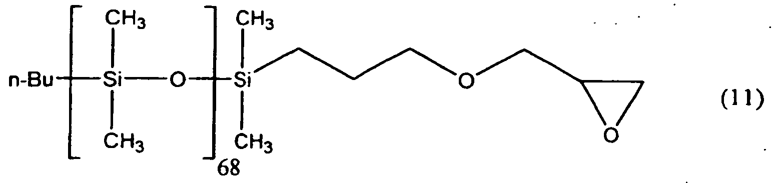

### [Synthesis Example 6]

A 100-mL three-necked flask equipped with a magnetic stirrer, a condenser tube, and a thermometer was charged with 2.00 g of epsilon-polylysine (number average molecular weight = 4,090) and 20.0 g of methanol. The mixture was stirred at room temperature to dissolve the epsilon-polylysine. Then, 20.0 g of 2-propanol was added, the temperature was increased to 50 degrees C, and 9.14 g of polydimethylsiloxane (number average molecular weight: 5,000) represented by the aforementioned general formula (11) having an epoxy group at one end was added dropwise for 5 minutes. The reaction was continued for 3 hours while the temperature was kept at 70 degrees C. After the reaction mixture was cooled to room temperature, volatile components in the reaction mixture were removed on an evaporator under reduced pressure to yield 11.0 g of slightly yellow liquid, polydimethylsiloxane-containing epsilon-polylysine. This polymer had a residual amino group content of 91% and silicone/epsilon - polylysine = 80/20 (weight ratio).

### [Synthesis Example 7]

A 100-mL three-necked flask equipped with a magnetic stirrer, a condenser tube, and a thermometer was charged with 3.0 g of epsilon-polylysine (number average molecular weight = 4,090) and 20.0 g of methanol. The mixture was stirred at room temperature to dissolve the epsilon-polylysine. After the temperature was increased to 50 degrees C, 10.0 g of polydimethylsiloxane (number average molecular weight: 1,000) having an epoxy group at one end, which was similar to the one used in the aforementioned general formula (10), was added dropwise for 10 minutes. The reaction was continued for 3 hours while the temperature was kept at 50 degrees C. After 3 hours, the reaction mixture was cooled and 10.0 g of ethanol was added thereto. Subsequently, volatile components in the reaction mixture were removed on an evaporator under reduced pressure to yield 12.6 g of yellow-white highly viscous liquid, polydimethylsiloxane-containing epsilon-polylysine.

### [Synthesis Example 8]

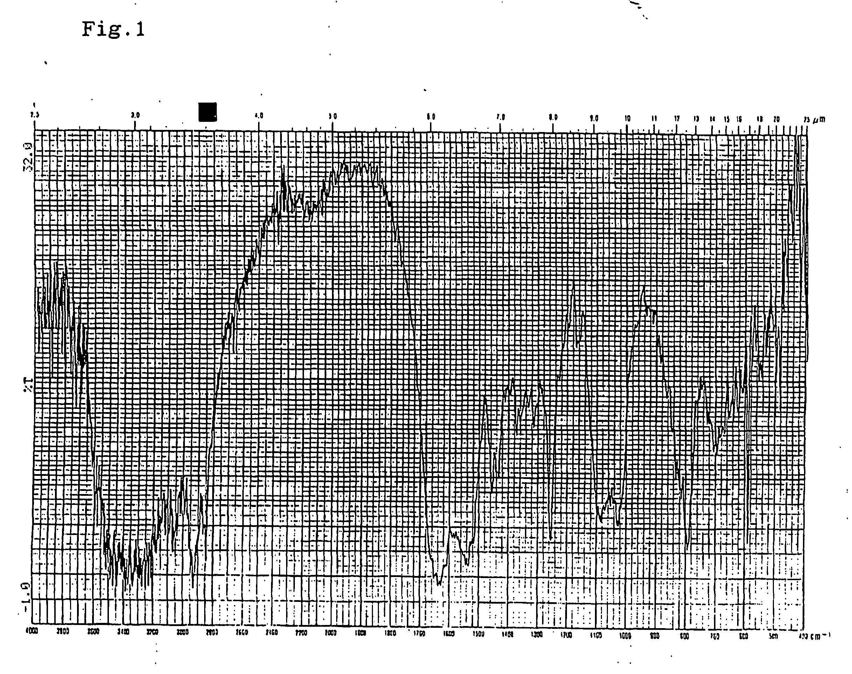

A 100-mL three-necked flask equipped with a magnetic stirrer, a condenser tube, and a thermometer was charged with 10.0 g of epsilon-polylysine (number average molecular weight = 4, 090) and 30. 0 g of methanol. The mixture was stirred at room temperature to dissolve the epsilon-polylysine. After the temperature was increased to 30 degrees C, 3.1 g of polydimethylsiloxane (number average molecular weight: 1,000) represented by the general formula (12) having a carboxylic acid group at one end was added dropwise for 5 minutes. The reaction was continued for 1 hour while the temperature was kept at 30 degrees C. After 1 hour, the reaction mixture was cooled and 10.0 g of ethanol was added thereto. Subsequently, volatile components in the reaction mixture were removed on an evaporator under reduced pressure to yield 11.6 g of slightly yellow solid. An infrared absorption spectrum of the compound showed absorption bands due to epsilon-polylysine and polydimethylsiloxane, respectively (Fig. 1). The compound in question, which is insoluble to water, was washed with water to remove any un-reacted epsilon-polylysine. It was dissolved in hexane and treated with 0.1 N hydrochloric acid solution. As a result, epsilon-polylysine was obtained in the aqueous phase. This indicates that the compound obtained in this reaction was an ammonium salt of epsilon-polylysine and a compound having the general formula (8), i.e., polydimethylsiloxane-containing epsilon-polylysine.

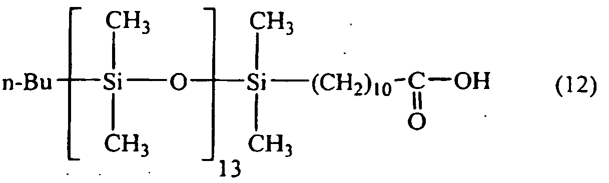

### [Example 1]

The compound obtained in the Synthesis Example 1 was diluted with methanol to prepare a 10.0-wt.% methanol solution. Then, purified water containing 1,000 ppm 1,3-butylene glycol was used to prepare a solution of the compound obtained in the Synthesis Example 1 at a concentrations of 6.25 ppm, 12.5 ppm, 25 ppm, 50 ppm, 100 ppm, 200 ppm, 400 ppm, and 800 ppm.

### [Example 2]

The compound obtained in the Synthesis Example 2 was diluted with methanol to prepare a 10.0-wt.% methanol solution. Then, purified water containing 1,000 ppm glycerin was used to prepare a solution of the compound obtained in the Synthesis Example 2 at a concentration of 6.25 ppm, 12.5 ppm, 25 ppm, 50 ppm, 100 ppm, 200 ppm, 400 ppm, and 800 ppm.

### [Comparative Example 1]

The compound obtained in the Synthesis Example 1 was diluted with methanol to prepare a 10.0-wt.% methanol solution. Then, purified water was used as Comparative Example 1 to prepare a solution of the compound obtained in the Synthesis Example 1 at a concentration of 6.25 ppm, 12.5 ppm, 25 ppm, 50 ppm, 100 ppm, 200 ppm, 400 ppm, and 800 ppm.

### [Comparative Example 2]

The compound obtained in the Synthesis Example 2 was diluted with methanol to prepare a 10.0-wt.% methanol solution. Then, purified water was used as Comparative Example 2 to prepare a solution of the compound obtained in the Synthesis Example 2 at a concentration of 6.25 ppm, 12.5 ppm, 25 ppm, 50 ppm, 100 ppm, 200 ppm, 400 ppm, and 800 ppm.

### (Antibacterial Preservative Effect Assay)

An assay was made for an antibacterial preservative effect of the preparations obtained in the Examples 1 and 2 and the Comparative Examples 1 and 2 against *Escherichia coli* IFO3972. The culture medium used for this assay was a normal bouillon medium (hereinafter, "NB medium"), a 4. 5-mL aliquot of which was dispensed in a test tube. The tube was sealed with an aluminum closure and sterilized along with its content in an autoclave at 121 degrees C, 1.1 kPa for 15 minutes. The resulting NB medium was used for the assay. The preparations obtained in the Examples 1 and 2 and the Comparative Examples 1 and 2 were added at 0.5 mL to the total amount of the sterilized NB medium, which was stirred thoroughly for uniform dispersion. Then, a 0.1-mL suspension of E. coli, which had been precultured so that each medium initially contains 1 x 10⁵ cells/mL, was inoculated and cultured in an incubator at 36 degrees C for 48 hours while shaking. After completion of the incubation, the growth of the bacteria was visually determined and the results were recorded according to the turbidity of the medium associated with the growth of the bacteria as: "+" for lines with turbid medium, and "-" for lines with non-turbid medium. These results were summarized in Table 1 below.

**[Table 1]**

| Concentration of the polyorganosiloxane -containing epsilon-polylysine (ppm) | 0 | 6.25 | 12.5 | 25 | 50 | 100 | 200 | 400 | 800 |
|---|---|---|---|---|---|---|---|---|---|
| Example 1 | + | - | - | - | - | - | - | - | - |
| Example 2 | + | + | - | - | - | - | - | - | - |
| Comparative Example 1 | + | + | - | - | - | - | - | - | - |
| Comparative Example 2 | + | + | + | + | + | - | - | - | - |

The Examples 1 and 2 and Comparative Examples 1 and 2 in the Table 1 indicate that the lines having polyorganosiloxane-containing the epsilon-polylysine and the polyhydric alcohol at a significantly lower content exhibited a high antibacterial preservative effect against E. coli as compared with those having only the polyorganosiloxane-containing epsilon-polylysine. Therefore, a combination of the polyorganosiloxane-containing epsilon-polylysine and the polyhydric alcohol can reduce the concentration of the polyorganosiloxane-containing epsilon-polylysine in a cosmetic composition required for achieving a desired antibacterial preservative effect.

### (Production Examples of Cosmetic Compositions)

Different cosmetic compositions were produced according to the present invention. Some examples are given below, but the present invention is not limited to these examples.

| (Production Example 1) Milky lotion | wt.% |
|---|---|
| polyether-modified organopolysiloxane | 5.0 |
| diglyceryl monostearate | 1.5 |
| decamethylcyclotetrasiloxane | 20.0 |
| | |
| dimethylpolysiloxane | 10.0 |
| liquid paraffin | 10.0 |
| | |
| fragrance | 0.1 |
| compound in Synthesis Example 1 | 0.1 |
| 1,3-butylene glycol | 15.0 |
| | |
| sodium chloride | 0.2 |
| purified water | balance |

| (Production Example 2) Serum | wt.% |
|---|---|
| sorbitan sesquiisostearate | 3.0 |
| decamethylcyclopentasiloxane | 10.0 |
| | |
| isononyl isomyristate | 8.0 |
| perfluoropolyether | 0.5 |
| fragrance | 0.1 |
| compound in Synthesis Example 2 | 0.05 |
| glycerin | 3.0 |
| propylene glycol | 10.0 |
| | |
| ascorbic acid 2-glucoside | 3.0 |
| sodium citrate | 0.5 |
| purified water | balance |

| (Production Example 3) Cream | wt.% |
|---|---|
| petrolatum | 8.0 |
| N-(3-hexadecyloxy-2-hydroxypropyl)-N-2-hydrox yethylhexadecanamide | 2.0 |
| | |
| squalene | 20.0 |
| | |
| cetyl alcohol | 5.0 |
| glycerin monostearate | 2.0 |
| polyoxyethylene (20) sorbitan monolaurate | 2.0 |
| arginine | 0.1 |
| ceramide 3 | 0.5 |
| sodium hydrogen phosphate | 0.85 |
| compound in Synthesis Example 1 | 0.2 |
| glycerin | 5.0 |
| 1,3-butylene glycol | 5.0 |
| dimethylpolysiloxane | 3.0 |
| fragrance | 0.1 |
| purified water | balance |

| (Production Example 4) Toner | wt.% |
|---|---|
| sorbitol | 2.0 |
| 1,3-butylene glycol | 4.0 |
| compound in Synthesis Example 2 | 1.0 |
| polyoxyethylene (25) oleylether | 2.0 |
| carboxyvinyl polymer | 0.2 |
| ethanol | 15.0 |
| | |
| purified water | balance |

| (Production Example 5) Foundation | wt.% |
|---|---|
| decamethylcyclopentasiloxane | 45.0 |
| | |
| dimethylpolysiloxane | 5.0 |
| dimethyldistearyl ammonium hectorite | 4.0 |
| hydrophobic treated titanium oxide | 10.0 |
| | |
| hydrophobic treated talc | 6.0 |
| hydrophobic treated mica | 6.0 |
| hydrophobic treated red iron oxide | 1.6 |
| hydrophobic treated yellow iron oxide | 0.7 |
| hydrophobic treated black iron oxide | 0.2 |
| compound in Synthesis Example 1 | 0.1 |
| dipropylene glycol | 5.0 |
| 2-amino-2-methyl-1,3-propanediol | 0.5 |
| fragrance | 0.1 |
| purified water | balance |

| (Production Example 6) Liquid foundation | wt.% |
|---|---|
| decamethylcyclopentasiloxane | 16.0 |
| | |
| dimethylpolysiloxane | 8.0 |
| 12-hydroxystearic acid | 1.0 |
| | |
| fluorine-modified silicone | 5.0 |
| spherical silicone resin powder | 3.0 |
| fluorine compound-treated titanium oxide fine powder | 8.0 |
| fluorine compound-treated mica-titanium | 1.0 |
| fluorine compound-treated titanium oxide | 5.0 |
| fluorine compound-treated red iron oxide | 0.9 |
| fluorine compound-treated yellow iron oxide | 2.0 |
| fluorine compound-treated black iron oxide | 1.0 |
| | |
| ethanol | 15.0 |
| compound in Synthesis Example 2 | 0.5 |
| 1,6-hexanediol | 2.0 |
| glycerin | 3.0 |
| magnesium sulfate | 1.0 |
| fragrance | 0.05 |
| purified water | balance |

| (Production Example 7) Lipstick | wt.% |
|---|---|
| candelilla wax | 5.0 |
| ceresin wax | 15.0 |
| | |
| beeswax | 3.0 |
| decamethylcyclopentasiloxane | 15.0 |
| | |
| propylene glycol dicaprate | 10.0 |
| | |
| polyglyceryl diisostearate | 8.0 |
| sorbitan sesquioleate | 1.0 |
| polyether-modified organopolysiloxane | 2.0 |
| compound in Synthesis Example 1 | 0.05 |
| 1,3-butylene glycol | 1.0 |
| Red No. 201 (Pig. Red 57-1) | 1.5 |
| | |
| Yellow No. 401 (Pig. Yellow 1) | 1.0 |
| | |
| hydrophobic treated titanium oxide | 1.0 |
| hydrophobic treated mica-titanium | 5.0 |
| fragrance | 1.0 |
| cetyl isooctanoate | balance |

| (Production Example 8) Eye shadow | wt.% |
|---|---|
| decamethylsiloxane | 15.0 |
| | |
| dimethylpolysiloxane | 10.0 |
| | |
| polyethylene glycol (10) lauryl ether | 0.5 |
| silicone-treated chromium oxide | 6.5 |
| silicone-treated ultramarine | 4.0 |
| silicone-treated titanium-coated mica | 6.0 |
| sodium chloride | 2.0 |
| propylene glycol | 8.0 |
| compound in Synthesis Example 2 | 0.5 |
| fragrance | 0.1 |
| purified water | balance |

| (Production Example 9) Facial wash | wt.% |
|---|---|
| triethanolamine lauryl phosphate | 18.0 |
| | |
| triethanolamine laurate | 2.5 |
| triethanolamine myristate | 2.5 |
| compound in Synthesis Example 1 | 0.2 |
| glycerin | 16.0 |
| | |
| polyoxyethylene (160) sorbitan triisostearate | 2.0 |
| | |
| cationated cellulose | 0.5 |
| alkyl acrylate/methacrylate copolymer | 0.5 |
| lauryl hydroxysulfobetaine | 5.0 |
| fragrance | 0.1 |
| purified water | balance |

| (Production Example 10) Body shampoo | wt.% |
|---|---|
| sodium citrate | 2.0 |
| N-acetylglutamic acid | 1.0 |
| potassium laurate | 15.0 |
| | |
| potassium myristate | 5.0 |
| compound in Synthesis Example 2 | 0.3 |
| propylene glycol | 5.0 |
| sorbitol | 3.0 |
| methylpolysiloxane | 1.5 |
| amino-modified dimethylpolysiloxane | 2.5 |
| polyethylene powder | 0.5 |
| hydroxypropyl chitosan | 0.5 |
| fragrance | 0.1 |
| purified water | balance |

| (Production Example 11) hair dressing | wt.% |
|---|---|
| vinylpyrrolidone/N,N-dimethylaminoethylmethac rylic acid copolymer diethyl sulfate salt | 1.0 |
| polyoxyethylene hydrogenated caster oil | 0.3 |
| keratin hydrolysate | 1.0 |
| hydroxypropyl chitosan | 3.5 |
| acetylneuraminic acid | 0.1 |
| compound in Synthesis Example 1 | 0.05 |
| mannitol | 4.0 |
| 1,3-butylene glycol | 5.0 |
| ethanol | 10.0 |
| | |
| fragrance | 0.1 |
| purified water | balance |

| (Production Example 12) Shampoo | wt.% |
|---|---|
| stearyl dimethylamine | 0.1 |
| cetyl dimethylamine | 0.1 |
| cetyl alcohol | 0.5 |
| carboxyvinyl polymer | 0.4 |
| isopropyl palmitate | 1.6 |
| dimethylpolysiloxane | 0.7 |
| dimethylpolysiloxane | 0.3 |
| lauryldimethylamine oxide | 10.0 |
| | |
| lauryl carboxymethyl hydroxyethyl imidazolinium betaine | 1.0 |
| cationated cellulose | 0.5 |
| compound in Synthesis Example 1 | 0.5 |
| propylene glycol | 0.5 |
| ethylene glycol distearate | 2.0 |
| sodium citrate | 0.2 |
| fragrance | 0.1 |
| purified water | balance |

| (Production Example 13) Finishing rinse | wt.% |
|---|---|
| dialkyl dimethyl ammonium chloride | 3.0 |
| polyoxyethylene (5) lauryl ether | 5.0 |
| | |
| isostearyl glyceryl ether | 2.0 |
| compound in Synthesis Example 2 | 0.8 |
| 1,2-pentanediol | 1.5 |
| hydroxypropyl cellulose | 0.4 |
| isopropyl palmitate | 1.6 |
| dimethyl polysiloxane | 0.1 |
| citric acid | 0.5 |
| fragrance | 0.1 |
| purified water | balance |

| (Production Example 14) Hair treatment | wt.% |
|---|---|
| behenyl trimethyl ammonium chloride | 8.0 |
| behenyl alcohol | 7.0 |
| carboxymethyl chitin | 1.0 |
| isopropyl palmitate | 9.0 |
| dimethyl polysiloxane | 1.0 |
| polyoxyethylene (20) sorbitan monostearate | 0.5 |
| behenic acid | 1.0 |
| compound in Synthesis Example 1 | 1.0 |
| dipropylene glycol | 6.0 |
| glycerin | 10.0 |
| | |
| citric acid | 0.3 |
| fragrance | 0.1 |
| purified water | balance |

| (Production Example 15) Hair dye | wt.% |
|---|---|
| (Solution A) | |
| sodium citrate | 0.8 |
| aqueous ammonia solution (30%) | 5.0 |
| | |
| 2,5-diaminotoluene sulfate salt | 5.0 |
| 2-amino-4-nitrophenol | 3.0 |
| | |
| 5-aminoorthocresol | 1.0 |
| p-aminophenol | 1.0 |
| 2,6-diaminopyridine | 0.1 |
| hydroxyethyl cellulose | 2.5 |
| oleyl alcohol | 5.0 |
| compound in Synthesis Example 1 | 0.05 |
| propylene glycol | 2.0 |
| panthenyl ethyl ether | 0.2 |
| sodium sulfite | 1.0 |
| tetrasodium edetate | 0.2 |
| monoethanol amine | 1.5 |
| purified water | balance |

| (Solution B) | |
|---|---|
| aqueous hydrogen peroxide (35%) | 15.0 |
| | |
| compound in Synthesis Example 2 | 0.05 |
| 1,3-butylene glycol | 10.0 |
| | |
| stearyl trimethyl ammonium chloride | 0.5 |
| lactic acid | 0.2 |
| fragrance | 0.1 |
| purified water | balance |

| (Production Example 16) Blood flow enhancer | wt.% |
|---|---|
| polyoxypropylene diglyceryl ether | 3.0 |
| polyoxyethylene polyoxypropylene glycol | 2.0 |
| glutamic acid sodium | 2.0 |
| ethanol | 60.0 |
| | |
| compound in Synthesis Example 2 | 0.05 |
| sorbitol | 5.0 |
| 1,5-pentanediol | 4.5 |
| DL-alpha-tocopherol acetate | 1.5 |
| capsicum tincture | 0.5 |
| resorcin | 0.5 |
| dipotassium glycyrrhizate | 0.5 |
| carboxymethyl chitin | 0.5 |
| decamethylcyclopentasiloxane | 0.1 |
| purified water | balance |

| (Production Example 17) Bath agents | wt.% |
|---|---|
| sodium hydrogen carbonate | 60.0 |
| | |
| sodium borate | 5.0 |
| sodium hyaluronate | 1.0 |
| hydroxypropylmethyl cellulose | 1.0 |
| fine sorbitol powder | 5.0 |
| compound in Synthesis Example 2 | 0.5 |
| fragrance | 3.0 |
| sodium sulfate anhydrous | balance |
| | relative to 100 |

| (Production Example 18) Ointment | wt.% |
|---|---|
| 1-menthol | 3.0 |
| DL-camphor | 0.5 |
| | |
| compound in Synthesis Example 1 | 0.2 |
| propylene glycol | 15.0 |
| | |
| carboxyvinyl polymer | 1.0 |
| hydroxyethyl cellulose | 0.1 |
| triethanol amine | 0.7 |
| purified water | 25.0 |
| | |
| ethanol | balance |
| | relative to 100 |

| (Production Example 19) Patch | wt.% |
|---|---|
| 1-menthol | 3.0 |
| DL-camphor | 1.5 |
| | |
| polyacrylic acid | 4.5 |
| sodium polyacrylate | 1.5 |
| carboxymethylcellulose sodium | 4.0 |
| compound in Synthesis Example 1 | 0.1 |
| glycerin | 20.0 |
| | |
| sorbitol | 5.0 |
| polyoxyethylene nonylphenyl ether | 0.5 |
| kaolin | 5.0 |
| caster oil | 1.0 |
| purified water | balance |

The aforementioned components were dissolved, dispersed, and compounded, which was spread over a polyester non-woven fabric at 1,000 g per 1 m² to produce a patch.

### [Brief Description of the Drawings]

Fig. 1 shows an IR chart of a polyorganosiloxane-containing polylysine obtained in the Synthesis Example 8.

## Claims

1. A cosmetic composition comprising one or a combination of two or more of polyorganosiloxane-containing epsilon-polylysine compounds or a physiologically acceptable salt thereof, and polyhydric alcohol.

2. A cosmetic composition according to claim 1 comprising one or a combination of two or more of polyorganosiloxane-containing epsilon-polylysine compounds represented by the general formula (1): 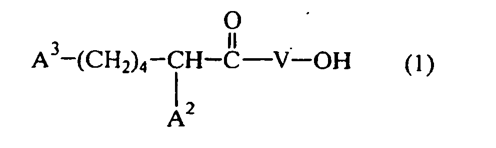 [in the general formula (1), ***V*** represents a random copolymer in which a first repeating unit represented by the general formula (5): 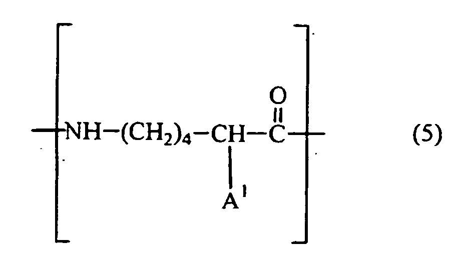 is randomly polymerized with a second repeating unit represented by the general formula (6): 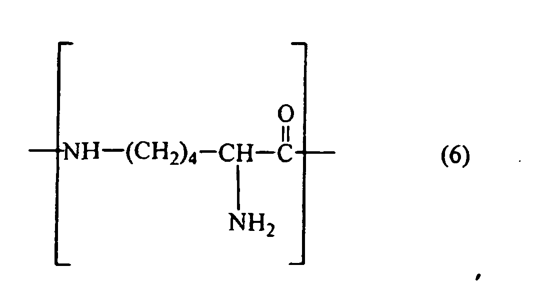 an alternating copolymer in which the first repeating unit alternates with the second repeating unit, or a block copolymer in which a block consisting only of the first repeating units is connected to another block consisting only of the second repeating units, the number of repeats ***a*** of the first repeating unit being an integer of 0 to 50, the number of repeats ***b*** of the second repeating unit being an integer of 0 to 50, ***a*** + ***b*** being equal to an integer of 1 to 50, A¹ represents a group represented by the general formula (2):
-X-Y-Z (2)
(in the general formula (2), ***X*** represents 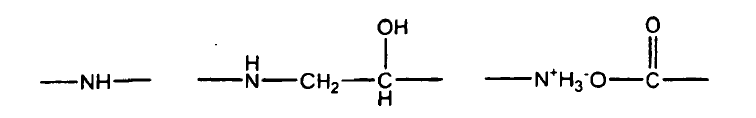 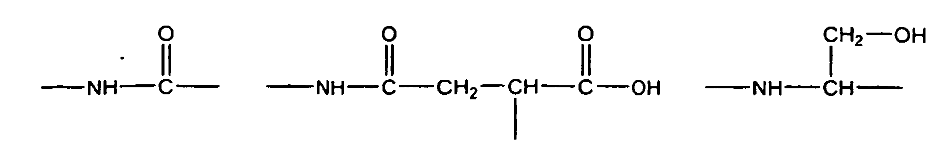 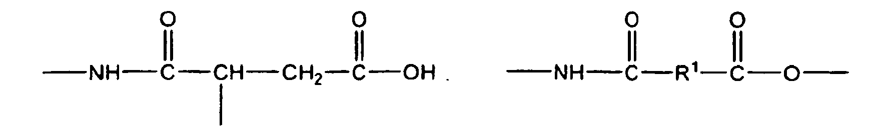 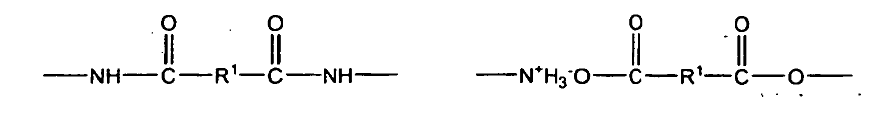 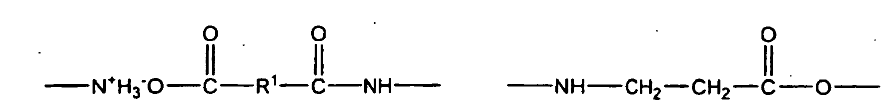 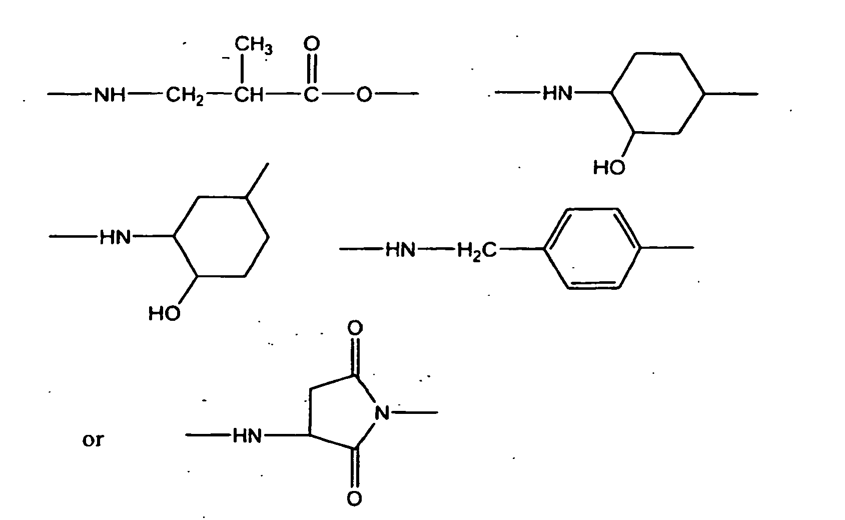 (wherein, R¹ represents a linear or branched alkylene group having 1 to 5 carbon atoms, an alkenylene group having 2 to 5 carbon atoms or an arylene group having 6 to 10 carbon atoms), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4): 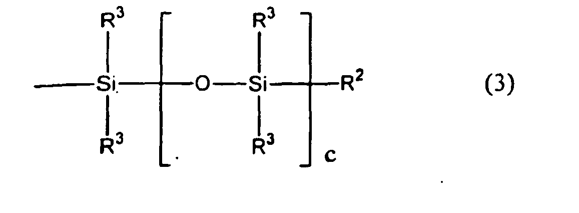 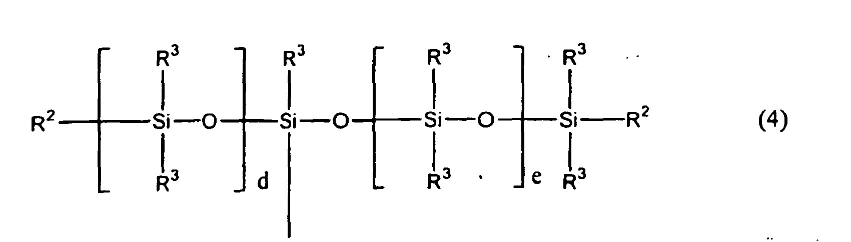 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)}, and A² and A³ are each independently an amino group or a group represented by the aforementioned general formula (2), A² and A³ are not both amino group when ***a*** is equal to 0] or a physiologically acceptable salt thereof, and polyhydric alcohol.

3. The cosmetic composition as claimed in Claim 2, comprising one or a combination of two or more of polyorganosiloxane-containing epsilon-polylysine compounds or a physiologically acceptable salt thereof, in which the number of repeats **b** of the second repeating unit is 5 or greater, the second repeating unit being represented by the general formula (6): 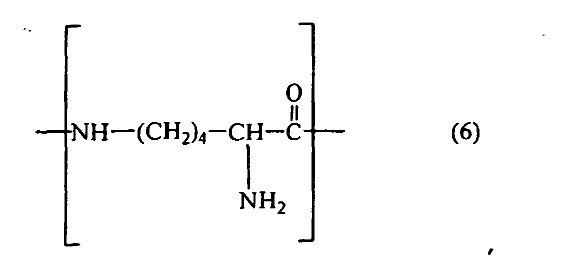 the second repeating units forming the polyorganosiloxane-containing epsilon-polylysine compound.

4. The cosmetic composition as claimed in Claim 2 or 3, wherein the number of repeats ***a*** of the first repeating unit and the number of repeats ***b*** of the second repeating unit satisfy the inequality 0.1 ≤ ***b***/ (***a*** + ***b***) ≤ 0. 98, the first repeating unit being represented by the general formula (5): 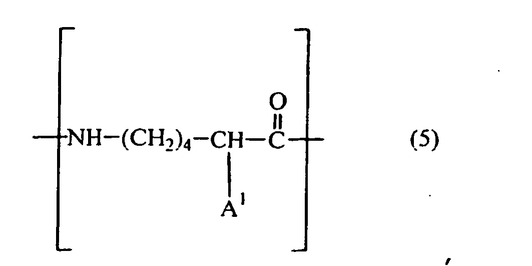 the second repeating unit being represented by the general formula (6): 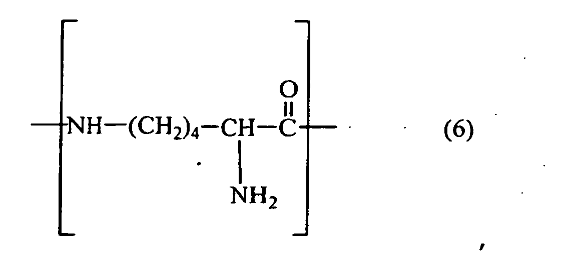 the first and second repeating units forming the polyorganosiloxane-containing epsilon-polylysine compound.

5. The cosmetic composition as claimed in any one of Claims 2-4, wherein ***X*** in the group represented by the general formula (2):
-X-Y-Z (2)
forming the polyorganosiloxane-containing epsilon-polylysine compound is a group represented by:

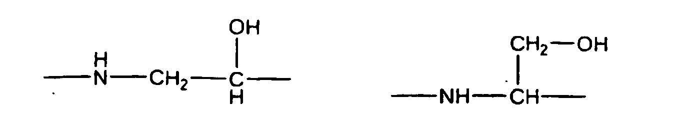

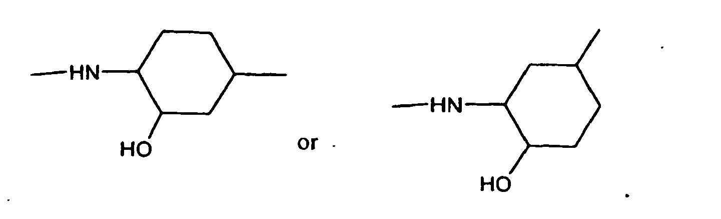

6. The cosmetic composition as claimed in any one of Claims 2-4, wherein ***X*** in the group represented by the general formula (2):
-X-Y-Z (2)
forming the polyorganosiloxane-containing epsilon-polylysine compound is a group represented by: 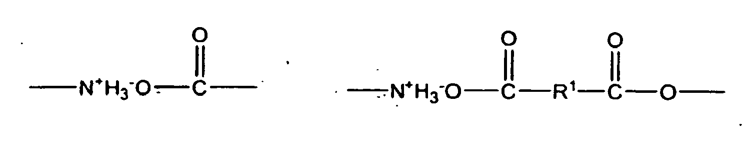 or 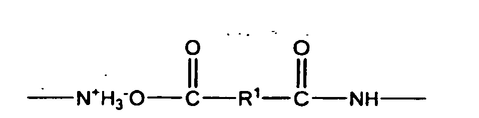 (in ***X*** in the general formula (2), R¹ represents a linear or branched alkylene group having 1 to 5 carbon atoms, an alkenylene group having 2 to 5 carbon atoms or an arylene group having 6 to 10 carbon atoms).

7. The cosmetic composition as claimed in any one of Claims 2-4, wherein ***X*** in the group represented by the general formula (2) :
-X-Y-Z (2)
forming the polyorganosiloxane-containing epsilon-polylysine compound is a group represented by: 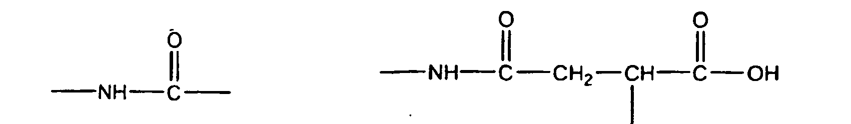 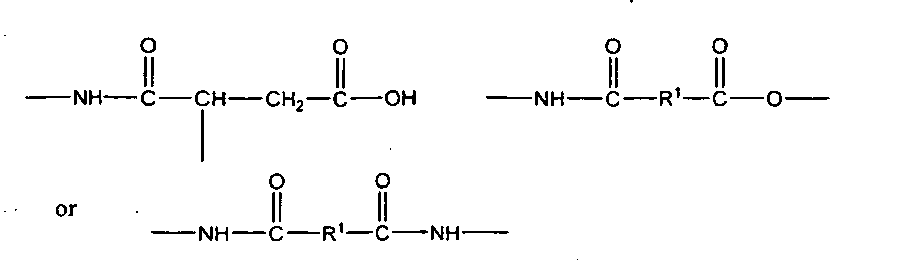 (in ***X*** in the general formula (2), R¹ represents a linear or branched alkylene group having 1 to 5 carbon atoms, an alkenylene group having 2 to 5 carbon atoms or an arylene group having 6 to 10 carbon atoms).

8. The cosmetic composition as claimed in any one of Claims 2-4, wherein ***X*** in the group represented by the general formula (2):
-X-Y-Z (2)
forming the polyorganosiloxane-containing epsilon-polylysine compound is a group represented by:

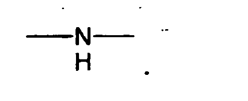

9. The cosmetic composition as claimed in any one of Claims 2-4, wherein ***X*** in the group represented by the general formula (2) :
-X-Y-Z (2)
forming the polyorganosiloxane-containing epsilon-polylysine compound is a group represented by: 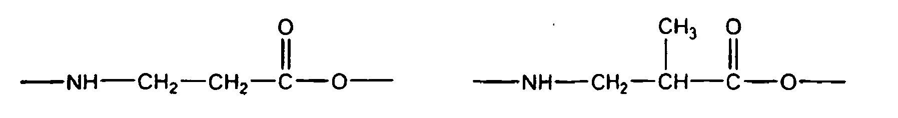 or

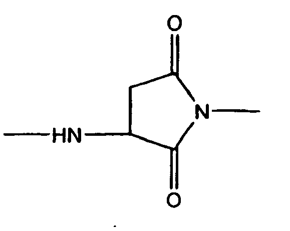

10. The cosmetic composition as claimed in any one of Claims 1-9, wherein the polyhydric alcohol is one or more selected from the group consisting of 1, 3-butylene glycol, glycerin, propylene glycol, polyethylene glycol, dipropylene glycol, 1,2-pentanediol, 1,5-pentanediol, 1,2-hexanediol, 1,6-hexanediol, mannitol and sorbitol.

11. The cosmetic composition as claimed in any one of Claims 1-9, wherein the polyhydric alcohol is one or more selected from the group consisting of ethanediol, diethylene glycol, triethylene glycol, 2-amino-2-methyl-1, 3-propanediol, 1,2-propanediol, 1,3-propanediol, polypropylene glycol, 1,2-butanediol, 1,3-butanediol, 1,4-butanediol, isopentanediol, pentylene glycol, hexylene glycol, 1,3-pentanediol, 1,4-pentanediol, 1,2,3-pentanetriol, 2,3,4-pentanetriol, 1,3,4-pentanetriol, 1,3,5-pentanetriol, 1,3-hexanediol, 1,4-hexanediol, 1,5-hexanediol, 1,2,3-hexanetriol, 1,3,9-hexanetriol, 1,3,5-hexanetriol, 1,4,6-hexanetriol, erythritol, pentaerythritol, dipentaerythritol, threitol, arabitol, xylitol, ribitol, galactitol, lactitol, maltitol, inositol, panthenol, laminitol, valienamine, validamine and validatol.

12. The cosmetic composition as claimed in any one of Claims 1-11, comprising 0.001% to 20% by weight of the polyorganosiloxane-containing epsilon-polylysine compounds or a physiologically acceptable salt thereof.

13. The cosmetic composition as claimed in any one of Claims 1 to 12, wherein the cosmetic composition is a hair treating agent.

14. The cosmetic composition as claimed in Claim 13, wherein the hair treating agent is a hair dressing, shampoo, finishing rinse, hair treatment, hair cream, hair mousse, hair setting lotion, hair color, hair dye, perm solution, blood flow enhancer, scalp lotion or anti-hair loss agent.

15. The cosmetic composition as claimed in any one of Claims 1-12, wherein the cosmetic composition is a skin care cosmetic.

16. The cosmetic composition as claimed in Claim 15, wherein the skin care cosmetic is a toner, serum, whitening toner, milky lotion, whitening milky lotion, cream, whitening cream, ointment, whitening ointment, lotion, whitening lotion, oil or facial pack.

17. The cosmetic composition as claimed in any one of Claims 1-12, wherein the cosmetic composition is a makeup cosmetic.

18. The cosmetic composition as claimed in Claim 17, wherein the makeup cosmetic is a foundation, liquid foundation, lipstick, lip gloss, eye shadow, powder, face powder, blusher, eye shadow, eye liner, mascara or eyebrow pencil.

19. The cosmetic composition as claimed in any one of Claims 1-12, wherein the cosmetic composition is a skin cleaner.

20. The cosmetic composition as claimed in Claim 19, wherein the skin cleaners is a soap, cleansing cream, cleansing lotion, cleansing milk, cosmetic composition, facial wash or body shampoo.

21. The cosmetic composition as claimed in any one of Claims 1-12, wherein the cosmetic composition is a bath agent.

22. The cosmetic composition as claimed in any one of Claims 1-12, wherein the cosmetic composition is a finishing cosmetic.

23. The cosmetic composition as claimed in any one of Claims 1-12, wherein the cosmetic composition is a patch.

24. A cosmetic composition according to claim 1 comprising one or a combination of two or more of polyorganosiloxane-containing epsilon-polylysine compounds or a physiologically acceptable salt thereof, and polyhydric alcohol, the polyorganosiloxane-containing epsilon-polylysine compound being obtained by reacting epsilon-polylysine with polyorganosiloxane.

25. The cosmetic composition as claimed in Claim 24, wherein the epsilon-polylysine is represented by the general formula (7): 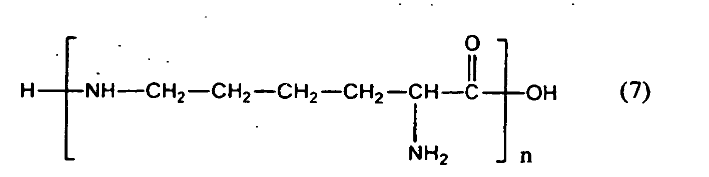 (wherein n is an integer of 2 to 51).

26. The cosmetic composition as claimed in Claim 24 or 25, wherein the polyorganosiloxane has a functional group that is reactive with an amino group in the epsilon-polylysine.

27. The cosmetic composition as claimed in any one of Claims 24-26, wherein the polyorganosiloxane is a polyorganosiloxane having an epoxy group, a polyorganosiloxane having a carboxylic acid or a carboxylic acid derivative, a polyorganosiloxane having a halogenated alkyl group or a polyorganosiloxane having an unsaturated group.

28. The cosmetic composition as claimed in any one of Claims 24-27, wherein the polyorganosiloxane is a polyorganosiloxane represented by the general formula (8): Q-Y-Z (8)
[in the general formula (8), ***Q*** represents 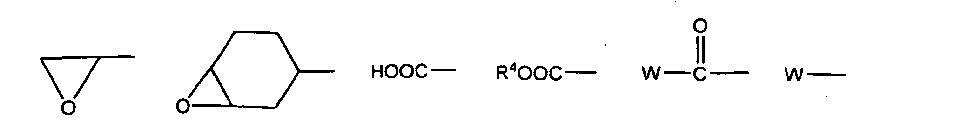 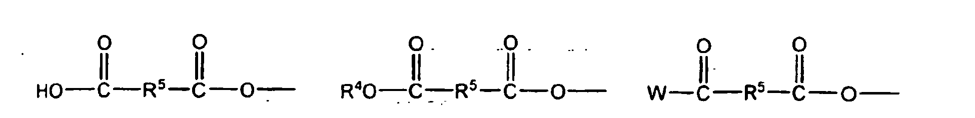 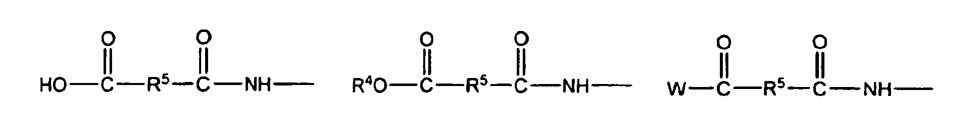 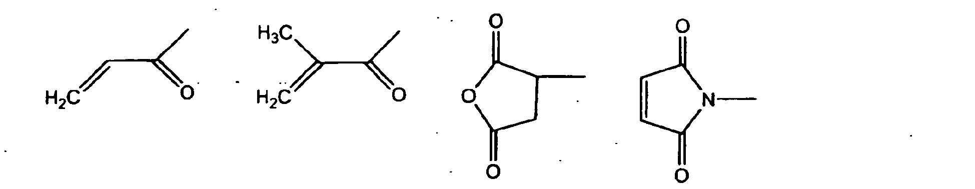 or 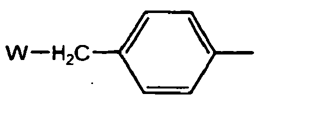 (wherein, R⁴ represents a linear or branched alkyl group having 1 to 20 carbon atoms or an aryl group having 6 to 10 carbon atoms or a trimethylsilyl group, R⁵ represents a linear or branched alkylene group having 1 to 5 carbon atoms, an alkenylene group having 2 to 5 carbon atoms or an arylene group having 6 to 10 carbon atoms, ***W*** represents chlorine, bromine or iodine), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4) : 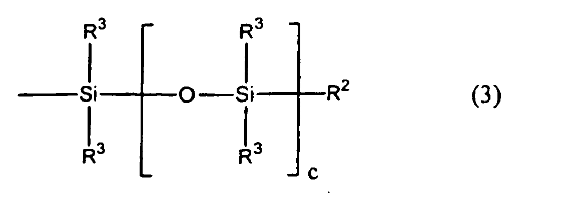 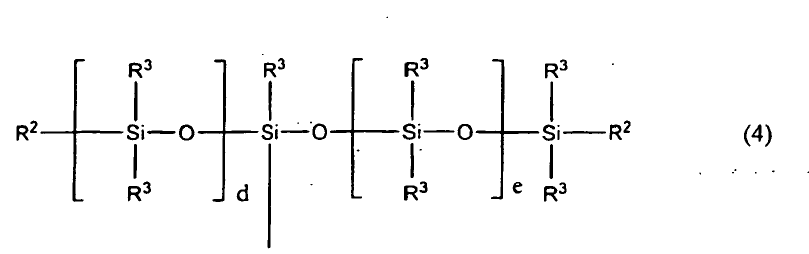 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)].

29. The cosmetic composition as claimed in any one of Claims 24-28, wherein the polyhydric alcohol is one or more selected from the group consisting of 1,3-butylene glycol, glycerin, propylene glycol, polyethylene glycol, dipropylene glycol, 1,2-pentanediol, 1,5-pentanediol, 1,2-hexanediol, 1,6-hexanediol, mannitol and sorbitol.

30. The cosmetic composition as claimed in any one of Claims 24-28, wherein the polyhydric alcohol is one or more selected from the group consisting of ethanediol, diethylene glycol, triethylene glycol, 2-amino-2-methyl-1,3-propanediol, 1,2-propanediol, 1,3-propanediol, polypropylene glycol, 1,2-butanediol, 1,3-butanediol, 1,4-butanediol, isopentanediol, pentylene glycol, hexylene glycol, 1,3-pentanediol, 1,4-pentanediol, 1,2,3-pentanetriol, 2,3,4-pentanetriol, 1,3,4-pentanetriol, 1,3,5-pentanetriol, 1,3-hexanediol, 1,4-hexanediol, 1,5-hexanediol, 1,2,3-hexanetriol, 1,3,9-hexanetriol, 1,3,5-hexanetriol, 1,9,6-hexarietriol, erythritol, pentaerythritol, dipentaerythritol, threitol, arabitol, xylitol, ribitol, galactitol, lactitol, maltitol, inositol, panthenol, laminitol, valienamine, validamine and validatol.

31. The cosmetic composition as claimed in any one of Claims 24 to 30, wherein the cosmetic composition is a hair treating agent.

32. The cosmetic composition as claimed in Claim 31, wherein the hair treating agent is a hair dressing, shampoo, finishing rinse, hair treatment, hair cream, hair mousse, hair setting lotion, hair color, hair dye, perm solution, blood flow enhancer, scalp lotion or anti-hair loss agent.

33. The cosmetic composition as claimed in any one of Claims 24-30, wherein the cosmetic composition is a skin care cosmetic.

34. The cosmetic composition as claimed in Claim 33, wherein the skin care cosmetic is a toner, serum, whitening toner, milky lotion, whitening milky lotion, cream, whitening cream, ointment, whitening ointment, lotion, whitening lotion, oil or facial pack.

35. The cosmetic composition as claimed in any one of Claims 24-30, wherein the cosmetic composition is a makeup cosmetic.

36. The cosmetic composition as claimed in Claim 35, wherein the makeup cosmetic is a foundation, liquid foundation, lipstick, lip gloss, eye shadow, powder, face powder, blusher, eye shadow, eye liner, mascara or eyebrow pencil.

37. The cosmetic composition as claimed in any one of Claims 24-30, wherein the cosmetic composition is a skin cleaner.

38. The cosmetic composition as claimed in Claim 37, wherein the skin cleaners is a soap, cleansing cream, cleansing lotion, cleansing milk, cosmetic composition, facial wash or body shampoo.

39. The cosmetic composition as claimed in any one of Claims 24-30, wherein the cosmetic composition is a bath agent.

40. The cosmetic composition as claimed in any one of Claims 24-30, wherein the cosmetic composition is a finishing cosmetic.

41. The cosmetic composition as claimed in any one of Claims 24-30, wherein the cosmetic composition is a patch.

42. A method of producing a cosmetic composition comprising:
mixing one or a combination of two or more of polyorganosiloxane-containing epsilon-polylysine compounds or a physiologically acceptable salt thereof, the polyorganosiloxane-containing epsilon-polylysine compound being obtained by reacting epsilon-polylysine with polyorganosiloxane, with polyhydric alcohol.

43. The method of producing a cosmetic composition as claimed in Claim 42, wherein the epsilon-polylysine is represented by the general formula (7): 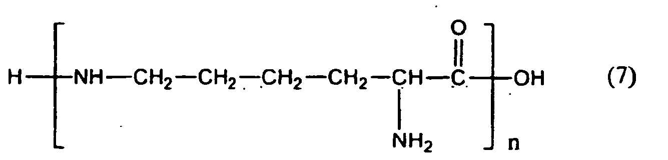 (wherein, n is an integer of 2 to 51).

44. The method of producing a cosmetic composition as claimed in Claim 42 or 43, wherein the polyorganosiloxane has a functional group that is reactive with an amino group in the epsilon-polylysine.

45. The method of producing a cosmetic composition as claimed in any one of Claims 42-44, wherein the polyorganosiloxane is a polyorganosiloxane having an epoxy group, a polyorganosiloxane having a carboxylic acid or a carboxylic acid derivative, a polyorganosiloxane having a halogenated alkyl group or a polyorganosiloxane having an unsaturated group.

46. The method of producing a cosmetic composition as claimed in any one of Claims 42-45, wherein the polyorganosiloxane is a polyorganosiloxane represented by the general formula (8): Q-Y-Z (8)
[in the general formula (8), ***Q*** represents 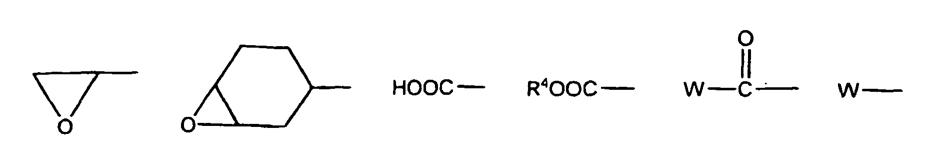 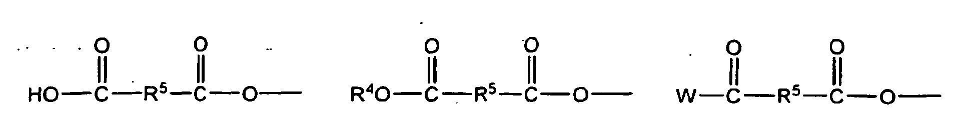 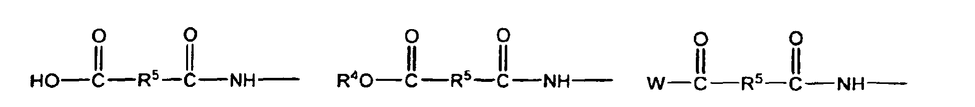 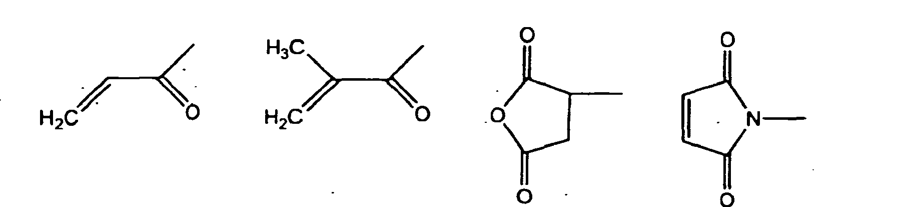 or 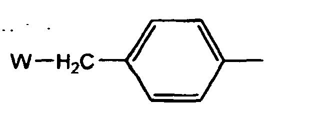 (wherein, R⁴ represents a linear or branched alkyl group having 1 to 20 carbon atoms or an aryl group having 6 to 10 carbon atoms or a trimethylsilyl group, R⁵ represents a linear or branched alkylene group having 1 to 5 carbon atoms, an alkenylene group having 2 to 5 carbon atoms or an arylene group having 6 to 10 carbon atoms, ***W*** represents chlorine, bromine or iodine), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4): 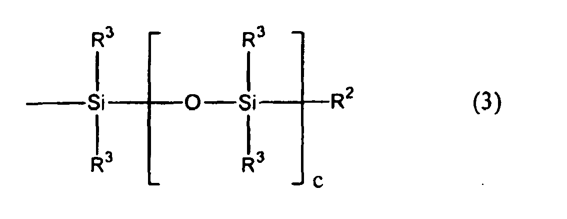 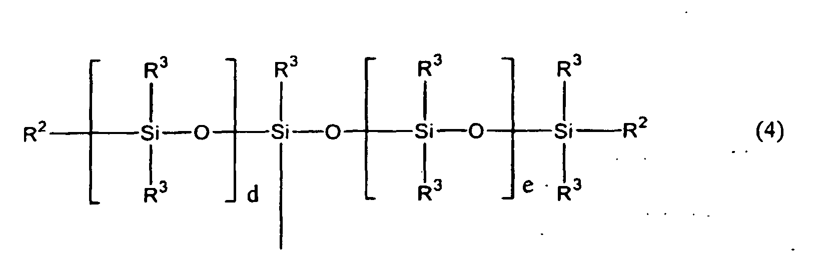 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)].

47. The method of producing a cosmetic composition as claimed in any one of Claims 42-46, wherein the polyorganosiloxane-containing epsilon-polylysine compound is represented by the general formula (1): 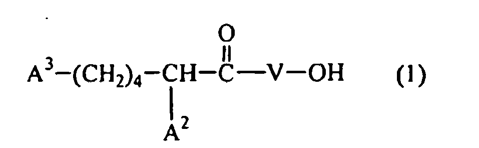 [in the general formula (1), ***V*** represents a random copolymer in which a first repeating unit represented by the general formula (5): 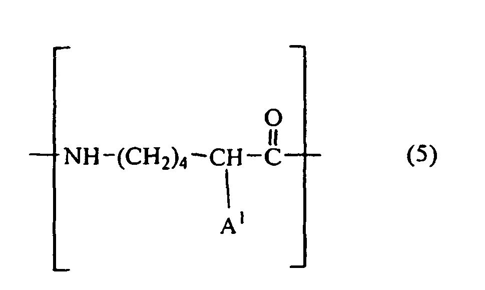 is randomly polymerized with a second repeating unit represented by the general formula (6): 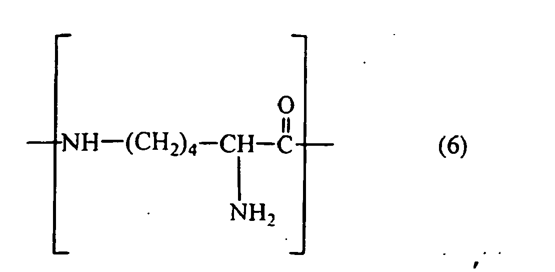 an alternating copolymer in which the first repeating unit alternates with the second repeating unit, or a block copolymer in which a block consisting only of the first repeating units is connected to another block consisting only of the second repeating units, the number of repeats ***a*** of the first repeating unit being an integer of 0 to 50, the number of repeats ***b*** of the second repeating unit being an integer of 0 to 50, ***a*** + ***b*** being equal to an integer of 1 to 50, A¹ represents a group represented by the general formula (2):
-X-Y-Z (2)
{in the general formula (2), ***X*** represents 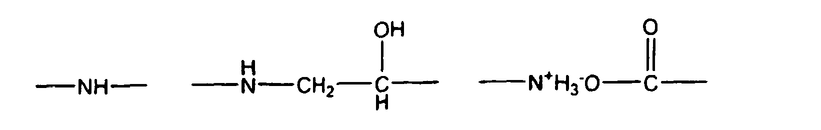 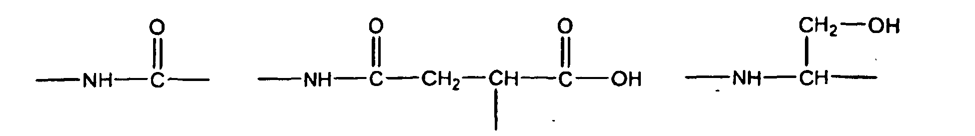 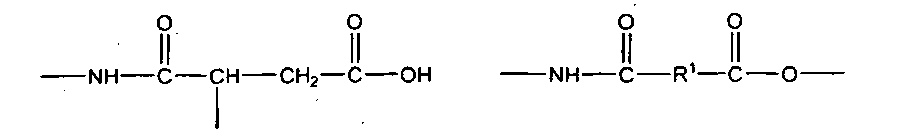 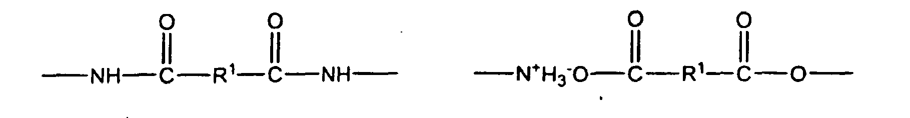 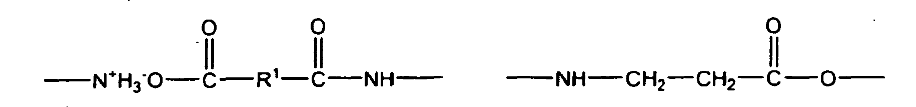 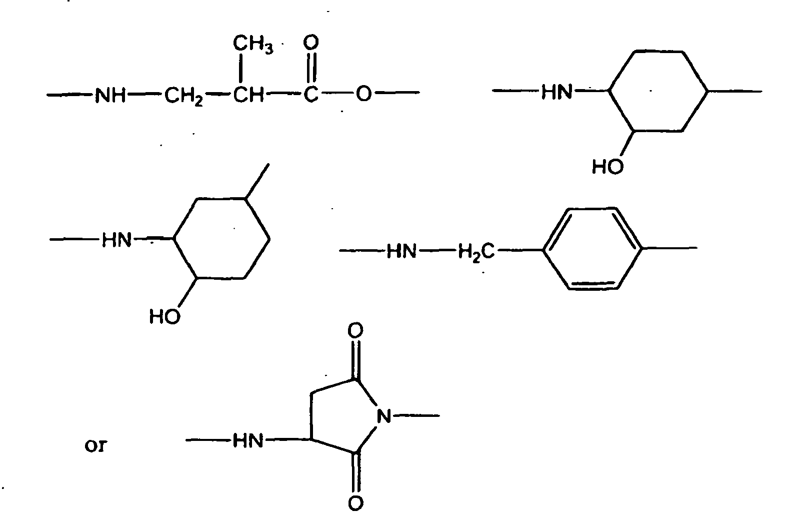 (wherein, R¹ represents a linear or branched alkylene group having 1 to 5 carbon atoms, an alkenylene group having 2 to 5 carbon atoms or an arylene group having 6 to 10 carbon atoms), ***Y*** represents a linear or branched alkylene group having 1 to 1000 carbon atoms, of which any mutually non-adjacent methylene groups may be substituted with -O-(ether), and ***Z*** represents a polyorganosiloxane group represented by the general formula (3) or (4) : 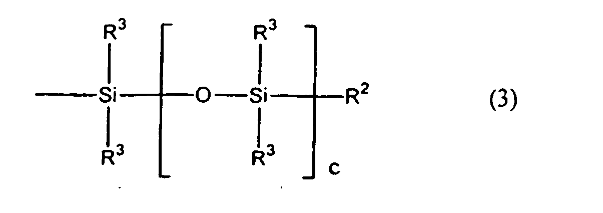 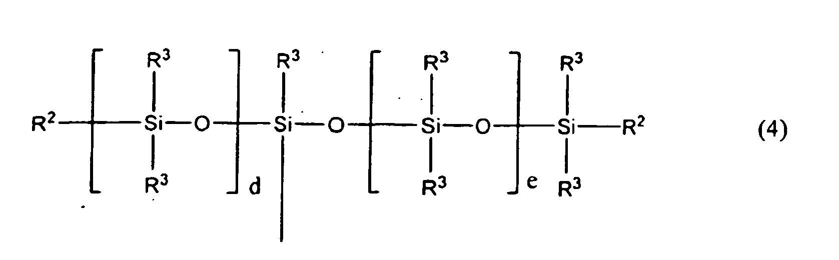 (in the general formulae (3) and (4), R² and R³ are each independently a linear or branched alkyl group having 1 to 20 carbon atoms or an aryl group having 6 to 10 carbon atoms, ***c*** is an integer of 1 to 1000, ***d*** is an integer of 1 to 1000, and ***e*** is an integer of 0 to 1000)}, and A² and A³ are each independently an amino group or a group represented by the aforementioned general formula (2), A² and A³ are not both amino group when a is equal to 0].

48. The method of producing a cosmetic composition as claimed in any one of Claims 42-47, wherein the polyhydric alcohol is one or more selected from the group consisting of 1,3-butylene glycol, glycerin, propylene glycol, polyethylene glycol, dipropylene glycol, 1,2-pentanediol, 1,5-pentanediol, 1,2-hexanediol, 1,6-hexanediol, mannitol and sorbitol.

49. The method of producing a cosmetic composition as claimed in any one of Claims 42-47, wherein the polyhydric alcohol is one or more selected from the group consisting of ethanediol, diethylene glycol, triethylene glycol, 2-amino-2-methyl-1,3-propanediol, 1,2-propanediol, 1,3-propanediol, polypropylene glycol, 1,2-butanediol, 1,3-butanediol, 1,4-butanediol, isopentanediol, pentylene glycol, hexylene glycol, 1,3-pentanediol, 1,4-pentanediol, 1,2,3-pentanetriol, 2,3,4-pentanetriol, 1,3,4-pentanetriol, 1,3,5-pentanetriol, 1,3-hexanediol, 1,4-hexanediol, 1,5-hexanediol, 1,2,3-hexanetriol, 1,3,4-hexanetriol, 1,3,5-hexanetriol, 1,4,6-hexanetriol, erythritol, pentaerythritol, dipentaerythritol, threitol, arabitol, xylitol, ribitol, galactitol, lactitol, maltitol, inositol, panthenol, laminitol, valienamine, validamine and validatol.

## Patentansprüche

1. Kosmetikzusammensetzung, umfassend ein oder eine Kombination von zwei oder mehreren Polyorganosiloxan-haltigen ε-Polylysinverbindungen oder ein physiologisch akzeptables Salz davon und einen mehrwertigen Alkohol.

2. Kosmetikzusammensetzung nach Anspruch 1, umfassend ein oder eine Kombination von zwei oder mehreren Polyorganosiloxan-haltigen ε-Polylysinverbindungen mit der allgemeinen Formel (1): 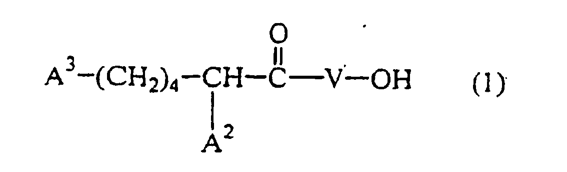 [worin in der allgemeinen Formel (1) V ein statistisches Copolymer, worin eine erste Wiederholungseinheit mit der allgemeinen Formel (5): 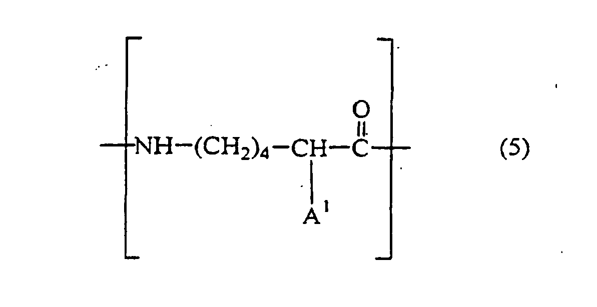 statistisch mit einer zweiten Wiederholungseinheit mit der allgemeinen Formel (6) polymerisiert: 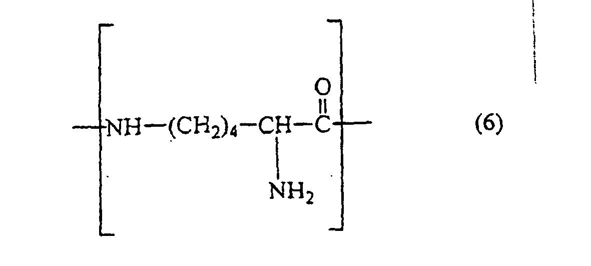 ein alternierendes Copolymer, worin die erste Wiederholungseinheit mit der zweiten Wiederholungseinheit alterniert, oder ein Blockcopolymer ist, worin ein Block, nur bestehend aus der ersten Wiederholungseinheit, mit einem anderen Block verbunden ist, der nur aus der zweiten Wiederholungseinheit besteht, wobei die Anzahl der Wiederholungen a der ersten Wiederholungseinheit eine ganze Zahl von 0 bis 50 ist, die Anzahl der Wiederholungen b der zweiten Wiederholungseinheit eine ganze Zahl vn 0 bis 50 ist, a + b eine ganze Zahl von 1 bis 50 ist, A¹ eine Gruppe mit der allgemeinen Formel (2) ist:
-X-Y-Z (2)
{wobei in der allgemeinen Formel (2) X bedeutet: 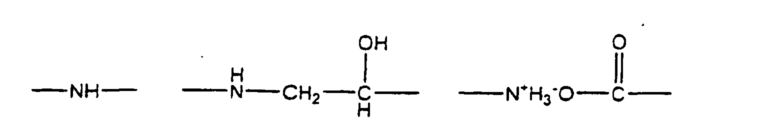 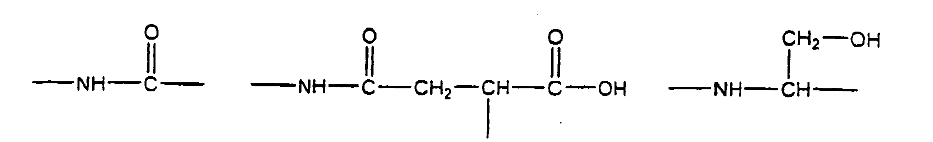 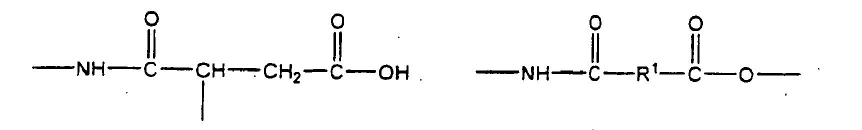 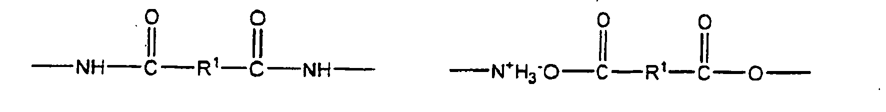 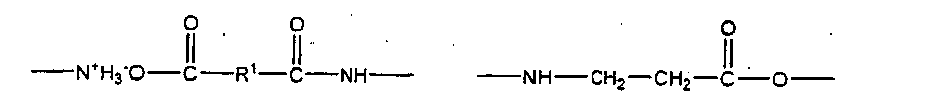 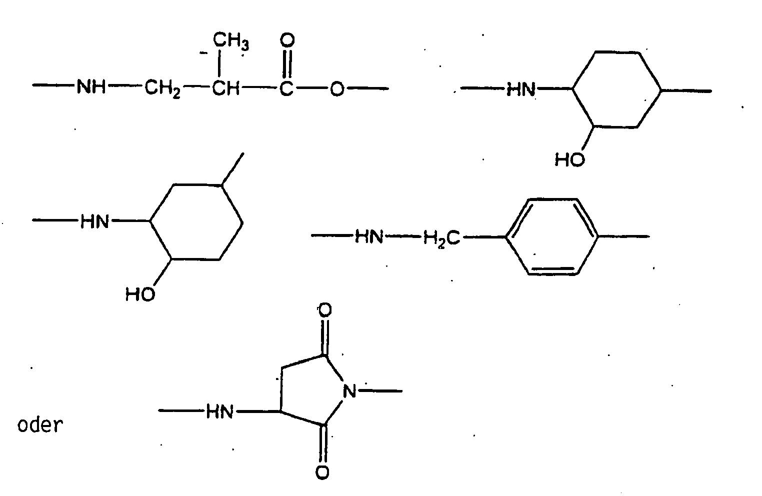 (worin R¹ eine lineare oder verzweigte Alkylengruppe mit 1 bis 5 Kohlenstoffatomen, Alkenylengruppe mit 2 bis 5 Kohlenstoffatomen oder Arylengruppe mit 6 bis 10 Kohlenstoffatomen ist), Y eine lineare oder verzweigte Alkylengruppe mit 1 bis 1.000 Kohlenstoffatomen ist, von denen irgendwelche wechselseitig nicht benachbarte Methylengruppen mit -O- (Ether) substituiert sein kann, und Z eine Polyorganosiloxangruppe mit der allgemeinen Formel (3) oder (4) ist: 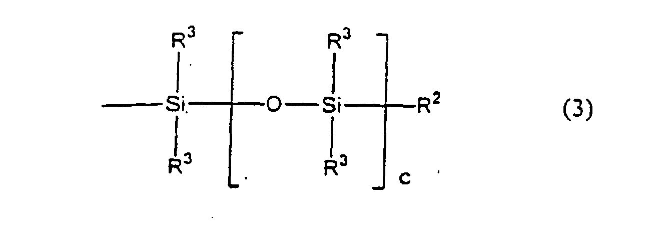 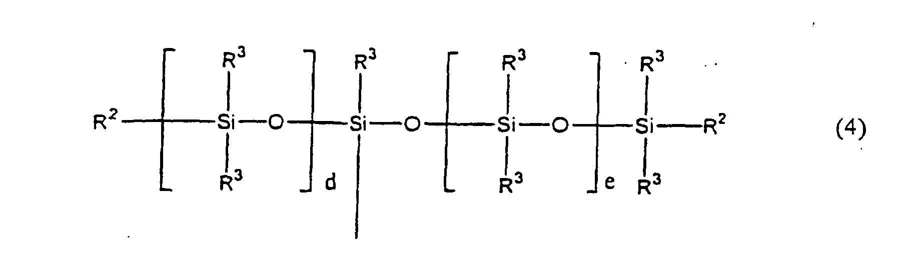 (in den allgemeinen Formeln (3) und (4) sind R² und R³ jeweils unabhängig eine lineare oder verzweigte Alkylgruppe mit 1 bis 20 Kohlenstoffatomen oder Arylgruppe mit 6 bis 10 Kohlenstoffatomen, c ist eine ganze Zahl von 1 bis 1.000, d ist eine ganze Zahl von 1 bis 1.000 und e ist eine ganze Zahl von 0 bis 1.000)}, und A² und A³ sind jeweils unabhängig eine Aminogruppe oder eine Gruppe mit der Formel (2), wobei A² und A³ nicht jeweils Aminogruppen sind, wenn a 0 ist], oder ein physiologisch akzeptables Salz davon und einen mehrwertigen Alkohol.

3. Kosmetikzusammensetzung nach Anspruch 2, umfassend eine oder eine Kombination aus zwei oder mehreren Polyorganosiloxan-haltigen ε-Polylysinverbindungen oder ein physiologisch akzeptables Salz davon, worin die Anzahl der Wiederholungen b der zweiten Wiederholungseinheit 5 oder mehr ist, wobei die zweite Wiederholungseinheit durch die allgemeine Formel (6) dargestellt ist: 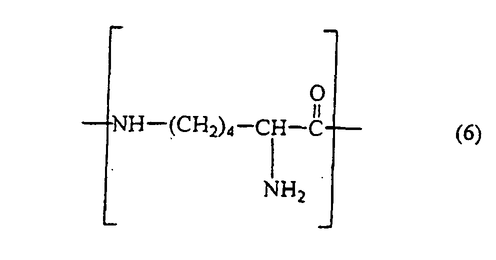 wobei die zweiten Wiederholungseinheiten die Polyorganosiloxan-haltige ε-Polylysinverbindung bilden.

4. Kosmetikzusammensetzung nach Anspruch 2 oder 3, worin die Anzahl der Wiederholungen a der ersten Wiederholungseinheit und die Anzahl der Wiederholungen b der zweiten Wiederholungseinheit die Ungleichung 0,1 s b/(a+b) ≤ 0,98 erfüllen, wobei die erste Wiederholungseinheit durch die Formel (5) dargestellt ist: 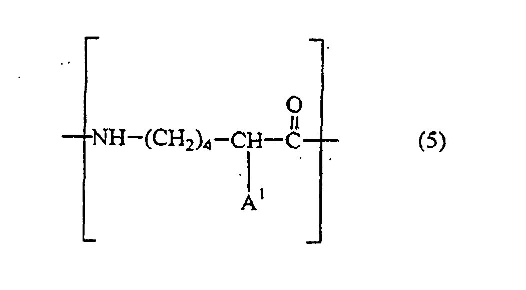 wobei die zweite Wiederholungseinheit durch die allgemeine Formel (6) dargestellt ist: 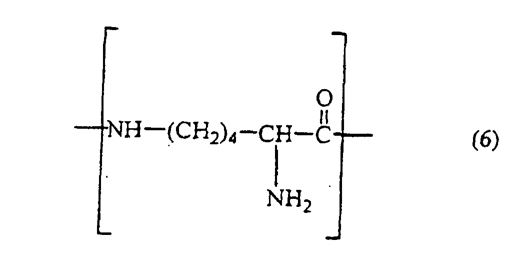 wobei die erste und die zweite Wiederholungseinheit die Polyorganosiloxan-haltige ε-Polylysinverbindung bilden.

5. Kosmetikzusammensetzung nach einem der Ansprüche 2 bis 4, worin X in der Gruppe mit der allgemeinen Formel (2):
-X-Y-Z (2),
die die Polyorganosiloxan-haltige ε-Polylysinverbindung bildet, eine Gruppe ist, dargestellt durch

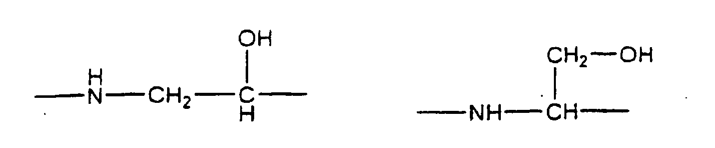

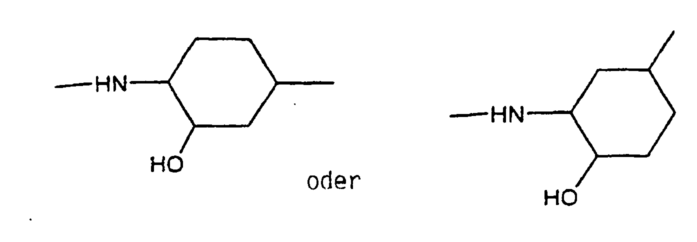

6. Kosmetikzusammensetzung nach einem der Ansprüche 2 bis 4, worin X in der Gruppe mit der allgemeinen Formel (2):
-X-X-Z (2),
die die Polyorganosiloxan-haltige ε-Polylysinverbindung bildet, eine Gruppe ist, dargestellt durch: 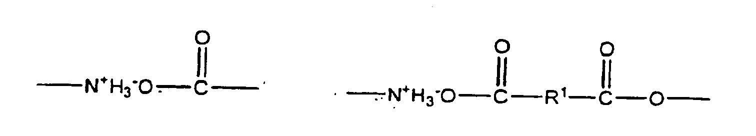 oder 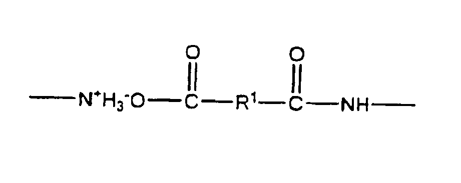 (in X in der allgemeinen Formel (2) bedeutet R¹ eine lineare oder verzweigte Alkylengruppe mit 1 bis 5 Kohlenstoffatomen, Alkylengruppe mit 2 bis 5 Kohlenstoffatomen oder Arylengruppe mit 6 bis 10 Kohlenstoffatomen).

7. Kosmetikzusammensetzung nach einem der Ansprüche 2 bis 4, worin X in der Gruppe mit der allgemeinen Formel (2):
-X-Y-Z (2),
die die Polyorganosiloxan-haltige ε-Polylysinverbindung bildet, eine Gruppe ist, dargestellt durch: 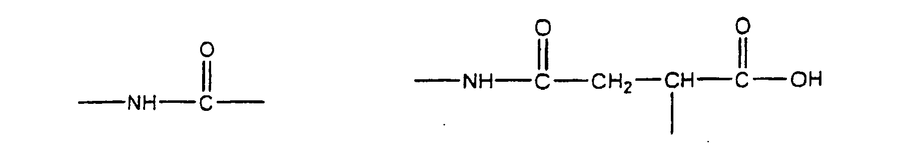 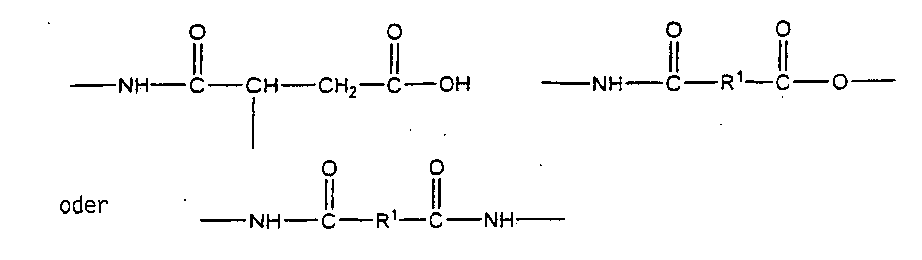 (in X in der allgemeinen Formel (2) bedeutet R¹ eine lineare oder verzweigte Alkylengruppe mit 1 bis 5 Kohlenstoffatomen, Alkenylengruppe mit 2 bis 5 Kohlenstoffatomen oder Arylengruppe mit 6 bis 10 Kohlenstoffatomen).

8. Kosmetikzusammensetzung nach einem der Ansprüche 2 bis 4, worin X in der Gruppe mit der allgemeinen Formel (2):
-X-Y-Z (2),
die die Polyorganosiloxanhaltige ε-Polylysinverbindung bildet, eine Gruppe ist, dargestellt durch:

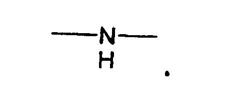

9. Kosmetikzusammensetzung nach einem der Ansprüche 2 bis 4, worin X in der Gruppe mit der allgemeinen Formel (2):
-X-Y-Z (2),
die die Polyorganosiloxan-haltige ε-Polylysinverbindung bildet, eine Gruppe ist, dargestellt durch: 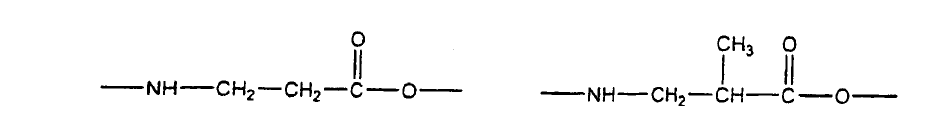 oder

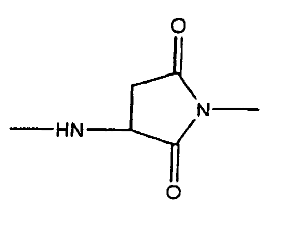

10. Kosmetikzusammensetzung nach einem der Ansprüche 1 bis 9, worin der mehrwertige Alkohol einer oder mehrere ist, ausgewählt aus der Gruppe, bestehend aus 1,3-Butylenglycol, Glycerin, Propylenglycol, Polyethylenglycol, Dipropylenglycol, 1,2-Pentandiol, 1,5-Pentandiol, 1,2-Hexandiol, 1,6-Hexandiol, Mannit und Sorbit.

11. Kosmetikzusammensetzung nach einem der Ansprüche 1 bis 9, worin der mehrwertige Alkohol einer oder mehrere ist, ausgewählt aus der Gruppe bestehend aus Ethandiol, Diethylenglycol, Triethylenglycol, 2-Amino-2-methyl-1,3-propandiol, 1,2-Propandiol, 1,3-Propandiol, Polypropylenglycol, 1,2-Butandiol, 1,3-Butandiol, 1,4-Butandiol, Isopentandiol, Pentylenglycol, Hexylenglycol, 1,3-Pentandiol, 1,4-Pentandiol, 1,2,3-Pentantriol, 2,3,4-Pentantriol, 1,3,4-Pentantriol, 1,3,5-Pentantriol, 1,3-Hexandiol, 1,4-Hexandiol, 1,5-Hexandiol, 1,2,3-Hexantriol, 1,3,4-Hexantriol, 1,3,5-Hexantriol, 1,4,6-Hexantriol, Erythrit, Pentaerythrit, Dipentaerythrit, Threit, Arabit, Xylit, Ribit, Galactit, Lactit, Maltit, Inosit, Panthenol, Laminitol, Valienamin, Validamin und Validatol.

12. Kosmetikzusammensetzung nach einem der Ansprüche 1 bis 11, umfassend 0,001 bis 20 Gew.% der Polyorganosiloxan-haltigen ε-Polylysinverbindungen oder eines physiologisch akzeptablen Salzes davon.

13. Kosmetikzusammensetzung nach einem der Ansprüche 1 bis 12, worin die Kosmetikzusammensetzung ein Haarbehandlungsmittel ist.

14. Kosmetikzusammensetzung nach Anspruch 13, worin das Haarbehandlungsmittel ein Haarkonditioniermittel, Shampoo, Spülung, Haarbehandlung, Haarcreme, Haarschaum, Haarfrisierlotion, Haarfarbe, Haarfarbstoff, Dauerwelllösung, Blutflussverstärkungsmittel, Kopfhautlotion oder Mittel gegen Haarverlust ist.

15. Kosmetikzusammensetzung nach einem der Ansprüche 1 bis 12, worin die Kosmetikzusammensetzung ein Hautpflegemittel ist.

16. Kosmetikzusammensetzung nach Anspruch 15, worin das Hautpflegekosmetikum ein Toner, Serum, Weißmachertoner, milchige Lotion, weißmachende, milchige Lotion, Creme, weißmachende Creme, Salbe, weißmachende Salbe, Lotion, weißmachende Lotion, Öl oder Gesichtspackung ist.

17. Kosmetikzusammensetzung nach einem der Ansprüche 1 bis 12, worin die Kosmetikzusammensetzung ein Makeup-Kosmetikum ist.

18. Kosmetikzusammensetzung nach Anspruch 17, worin das Makeup-Kosmetikum eine Grundierung, flüssige Grundierung, Lippenstift, Lipgloss, Lidschatten, Pulver, Gesichtspulver, Rouge, Lidschatten, Eyeliner, Mascara oder Augenbrauenstift ist.

19. Kosmetikzusammensetzung nach einem der Ansprüche 1 bis 12, worin die Kosmetikzusammensetzung ein Hautreiniger ist.

20. Kosmetikzusammensetzung nach Anspruch 19, worin der Hautreiniger eine Seife, Reinigungscreme, Reinigungslotion, Reinigungsmilch, Kosmetikzusammensetzung, Gesichtswaschmittel oder Körpershampoo ist.

21. Kosmetikzusammensetzung nach einem der Ansprüche 1 bis 12, worin die Kosmetikzusammensetzung ein Bademittel ist.

22. Kosmetikzusammensetzung nach einem der Ansprüche 1 bis 12, worin die Kosmetikzusammensetzung ein Veredelungskosmetikum ist.

23. Kosmetikzusammensetzung nach einem der Ansprüche 1 bis 12, worin die Kosmetikzusammensetzung ein Patch ist.

24. Kosmetikzusammensetzung nach Anspruch 1, umfassend eine oder eine Kombination von zwei oder mehreren Polyorganosiloxan-haltigen ε-Polylysinverbindungen oder ein physiologisch akzeptables Salz davon und einen mehrwertigen Alkohol, wobei die Polyorganosiloxanhalitge ε-Polylysinverbindung durch Reaktion von ε-Polylysin mit Polyorganosiloxan erhalten ist.

25. Kosmetikzusammensetzung nach Anspruch 24, worin das ε-Polylysin durch die allgemeine Formel (7) dargestellt ist: 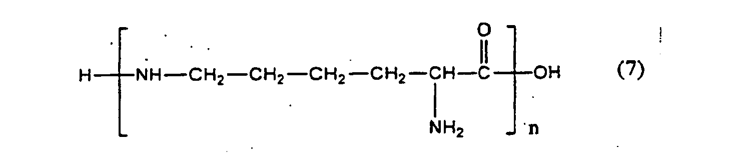 (worin n eine ganze Zahl von 2 bis 51 ist).

26. Kosmetikzusammensetzung nach Anspruch 24 oder 25, worin das Polyorganosiloxan eine funktionelle Gruppe hat, die mit einer Aminogruppe im ε-Polylysin reaktiv ist.

27. Kosmetikzusammensetzung nach einem der Ansprüche 24 bis 26, worin das Polyorganosiloxan ein Polyorganosiloxan mit einer Epoxygruppe, ein Polyorganosiloxan mit einer Carbonsäure oder einem Carbonsäurederivat, ein Polyorganosiloxan mit einer halogenierten Alkylgruppe oder ein Polyorganosiloxan mit einer ungesättigten Gruppe ist.

28. Kosmetikzusammensetzung nach einem der Ansprüche 24 bis 27, worin das Polyorganosiloxan ein Polyorganosiloxan mit der Formel (8) ist:
Q-Y-Z (8),
[wobei in der allgemeinen Formel (8) Q bedeutet: 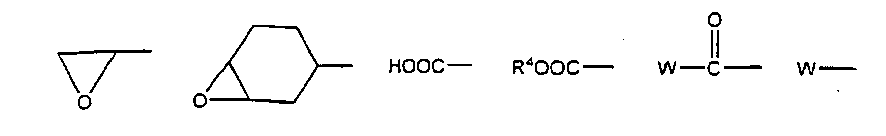 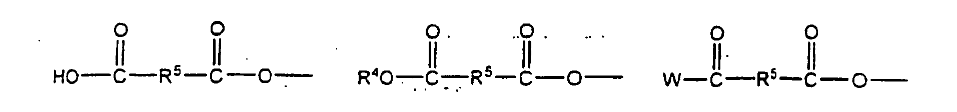 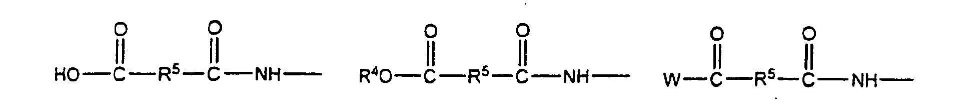 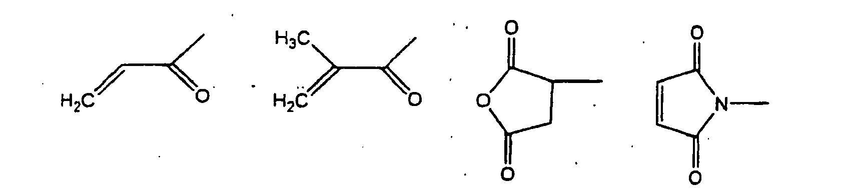 oder 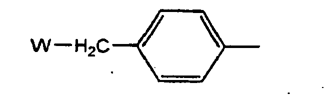 (worin R⁴ eine lineare oder verzweigte Alkylgruppe mit 1 bis 20 Kohlenstoffatomen oder eine Arylgruppe mit 6 bis 10 Kohlenstoffatomen oder eine Trimethylsilylgruppe ist, R⁵ eine lineare oder verzweigte Alkylengruppe mit 1 bis 5 Kohlenstoffatomen, Alkenylengruppe mit 2 bis 5 Kohlenstoffatomen oder Arylengruppe mit 6 bis 10 Kohlenstoffatomen ist, W Chlor, Brom oder Iod ist), Y eine lineare oder verzweigte Alkylengruppe mit 1 bis 1.000 Kohlenstoffatomen ist, wobei irgendwelche wechselseitig nicht benachbarte Methylengruppen durch -O- (Ether) substituiert sein kann, und Z eine Polyorganosiloxangruppe ist, dargestellt durch die allgemeine Formel (3) oder (4): 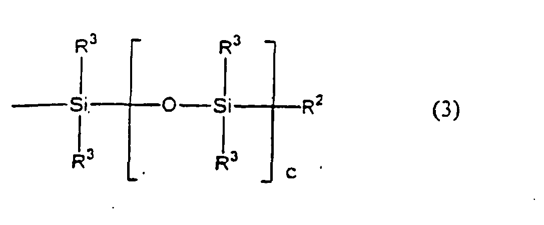 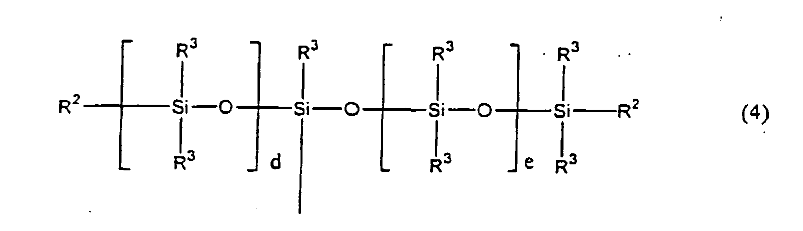 (in den allgemeinen Formeln (3) und (4) sind R² und R³ jeweils unabhängig eine lineare oder verzweigte Alkylgruppe mit 1 bis 20 Kohlenstoffatomen oder Arylgruppe mit 6 bis 10 Kohlenstoffatomen, c ist eine ganze Zahl von 1 bis 1.000, d ist eine ganze Zahl von 1 bis 1.000 und e ist eine ganze Zahl von 0 bis 1.000)].

29. Kosmetikzusammensetzung nach einem der Ansprüche 24 bis 28, worin der mehrwertige Alkohol einer oder mehrere ist, ausgewählt aus der Gruppe bestehend aus 1,3-Butylenglycol, Glycerin, Propylenglycol, Polyethylenglycol, Dipropylenglycol, 1,2-Pentandiol, 1,5-Pentandiol, 1,2-Hexandiol, 1,6-Hexandiol, Mannit und Sorbit.

30. Kosmetikzusammensetzung nach einem der Ansprüche 24 bis 28, worin der mehrwertige Alkohol einer oder mehrerer ist, ausgewählt aus der Gruppe bestehend aus Ethandiol, Diethylenglycol, Triethylenglycol, 2-Amino-2-methyl-1,3-propandiol, 1,2-Propandiol, 1,3-Propandiol, Polypropylenglycol, 1,2-Butandiol, 1,3-Butandiol, 1,4-Butandiol, Isopentandiol, Pentylenglycol, Hexylenglycol, 1,3-Pentandiol, 1,4-Pentandiol, 1,2,3-Pentantriol, 2,3,4-Pentantriol, 1,3,4-Pentantriol, 1,3,5-Pentantriol, 1,3-Hexandiol, 1,4-Hexandiol, 1,5-Hexandiol, 1,2,3-Hexantriol, 1,3,4-Hexantriol, 1,3,5-Hexantriol, 1,4,6-Hexantriol, Erythrit, Pentaerythrit, Dipentaerythrit, Threit, Arabit, Xylit, Ribit, Galactit, Lactit, Maltit, Inosit, Panthenol, Laminitol, Valienamin, Validamin und Validatol.

31. Kosmetikzusammensetzung nach einem der Ansprüche 24 bis 30, worin die Kosmetikzusammensetzung ein Haarbehandlungsmittel ist.

32. Kosmetikzusammensetzung nach Anspruch 31, worin das Haarbehandlungsmittel ein Haarkonditioniermittel, Shampoo, Spülung, Haarbehandlung, Haarcreme, Haarschaum, Haarfrisierlotion, Haarfarbe, Haarfarbstoff, Dauerwelllösung, Blutflussverstärkungsmittel, Kopfhautlotion oder Mittel gegen Haarverlust ist.

33. Kosmetikzusammensetzung nach einem der Ansprüche 24 bis 30, worin die Kosmetikzusammensetzung ein Hautpflegemittel ist.

34. Kosmetikzusammensetzung nach Anspruch 33, worin das Hautpflegekosmetikum ein Toner, Serum, Weißmachertoner, milchige Lotion, weißmachende, milchige Lotion, Creme, weißmachende Creme, Salbe, weißmachende Salbe, Lotion, weißmachende Lotion, Öl oder Gesichtspackung ist.

35. Kosmetikzusammensetzung nach einem der Ansprüche 24 bis 30, worin die Kosmetikzusammensetzung ein Make-Up-Kosmetikum ist.

36. Kosmetikzusammensetzung nach Anspruch 35, worin das Makeup-Kosmetikum eine Grundierung, flüssige Grundierung, Lippenstift, Lipgloss, Lidschatten, Pulver, Gesichtspulver, Rouge, Lidschatten, Eyeliner, Mascara oder Augenbrauenstift ist.

37. Kosmetikzusammensetzung nach einem der Ansprüche 24 bis 30, worin die Kosmetikzusammensetzung ein Hautreiniger ist.

38. Kosmetikzusammensetzung nach Anspruch 37, worin der Hautreiniger eine Seife, Reinigungscreme, Reinigungslotion, Reinigungsmilch, Kosmetikzusammensetzung, Gesichtswaschmittel oder Körpershampoo ist.

39. Kosmetikzusammensetzung nach einem der Ansprüche 24 bis 30, worin die Kosmetikzusammensetzung ein Bademittel ist.

40. Kosmetikzusammensetzung nach einem der Ansprüche 24 bis 30, worin die Kosmetikzusammensetzung ein Veredelungskosmetikum ist.

41. Kosmetikzusammensetzung nach einem der Ansprüche 24 bis 30, worin die Kosmetikzusammensetzung ein Patch ist.

42. Verfahren zur Erzeugung einer Kosmetikzusammensetzung, umfassend das Mischen von einer oder einer Kombination von zwei oder mehreren Polyorganosiloxan-haltigen ε-Polylysinverbindungen oder eines physiologisch akzeptablen Salzes davon, wobei die Polyorganosiloxanhaltige ε-Polylysinverbindung durch Reaktion von ε-Polylysin mit Polyorganosiloxan erhalten ist, mit einem mehrwertigen Alkohol.

43. Verfahren zur Erzeugung einer Kosmetikzusammensetzung nach Anspruch 42, worin das ε-Polylysin durch die allgemeine Formel (7) dargestellt ist: 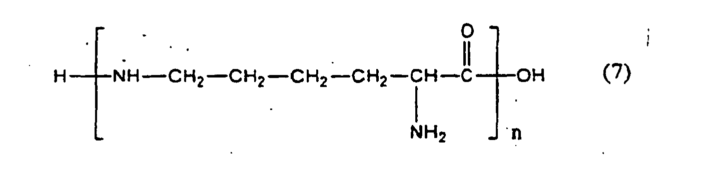 (worin n eine ganze Zahl von 2 bis 51 ist).

44. Verfahren zur Erzeugung einer Kosmetikzusammensetzung nach Anspruch 42 oder 43, worin das Polyorganosiloxan eine funktionelle Gruppe hat, die mit einer Aminogruppe im ε-Polylysin reaktiv ist.

45. Verfahren zur Erzeugung einer Kosmetikzusammensetzung nach einem der Ansprüche 42 bis 44, worin das Polyorganosiloxan ein Polyorganosiloxan mit einer Epoxygruppe, ein Polyorganosiloxan mit einer Carbonsäure oder einem Carbonsäurederivat, ein Polyorganosiloxan mit einer halogenierten Alkylgruppe oder ein Polyorganosiloxan mit einer ungesättigten Gruppe ist.

46. Verfahren zur Erzeugung einer Kosmetikzusammensetzung nach einem der Ansprüche 42 bis 45, worin das Polyorganosiloxan ein Polyorganosiloxan mit der Formel (8) ist:
Q-Y-Z (8),
[wobei in der allgemeinen Formel (8) Q bedeutet: 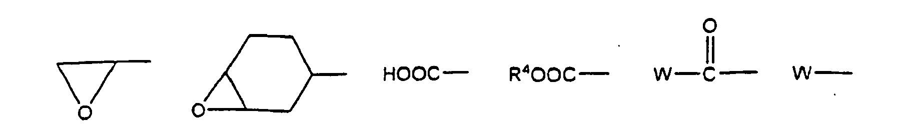 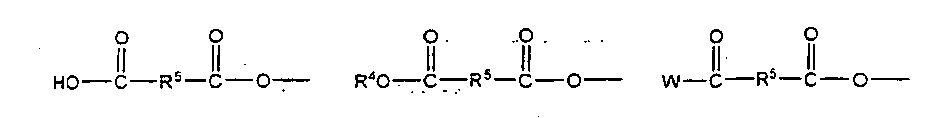 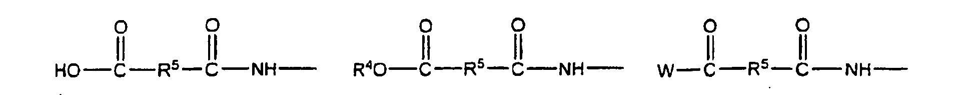 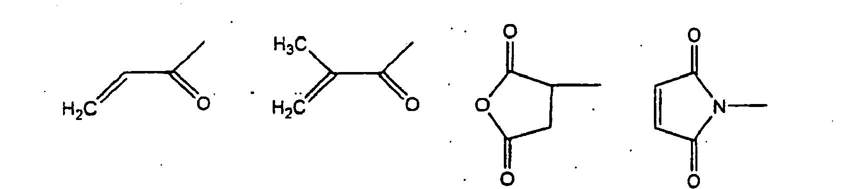 oder 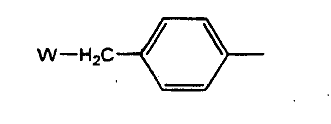 (worin R⁴ eine lineare oder verzweigte Alkylgruppe mit 1 bis 20 Kohlenstoffatomen oder eine Arylgruppe mit 6 bis 10 Kohlenstoffatomen oder eine Trimethylsilylgruppe ist, R⁵ eine lineare oder verzweigte Alkylengruppe mit 1 bis 5 Kohlenstoffatomen, Alkenylengruppe mit 2 bis 5 Kohlenstoffatomen oder Arylengruppe mit 6 bis 10 Kohlenstoffatomen ist, W Chlor, Brom oder Iod ist), Y eine lineare oder verzweigte Alkylengruppe mit 1 bis 1.000 Kohlenstoffatomen ist, wobei irgendwelche wechselseitig nicht benachbarte Methylengruppe durch -O- (Ether) substituiert sein kann, und Z eine Polyorganosiloxangruppe ist, dargestellt durch die allgemeine Formel (3) oder (4): 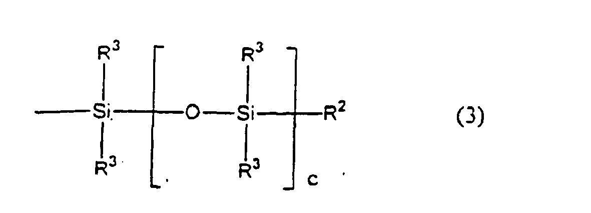 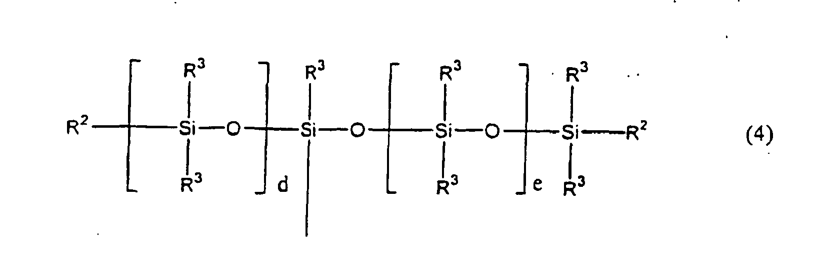 (in den allgemeinen Formeln (3) und (4) sind R² und R³ jeweils unabhängig eine lineare oder verzweigte Alkylgruppe mit 1 bis 20 Kohlenstoffatomen oder Arylgruppe mit 6 bis 10 Kohlenstoffatomen, c ist eine ganze Zahl von 1 bis 1.000, d ist eine ganze Zahl von 1 bis 1.000 und e ist eine ganze Zahl von 0 bis 1.000)].

47. Verfahren zur Erzeugung einer Kosmetikzusammensetzung nach einem der Ansprüche 42 bis 46, worin die Polyorganosiloxan-haltige ε-Polylysinverbindung durch die allgemeine Formel (1) dargestellt wird: 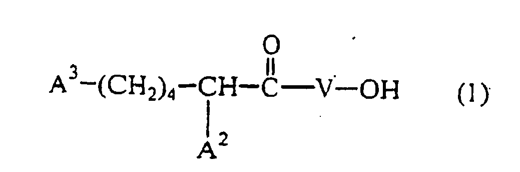 [worin in der allgemeinen Formel (1) V ein statistisches Copolymer, worin eine erste Wiederholungseinheit mit der allgemeinen Formel (5): 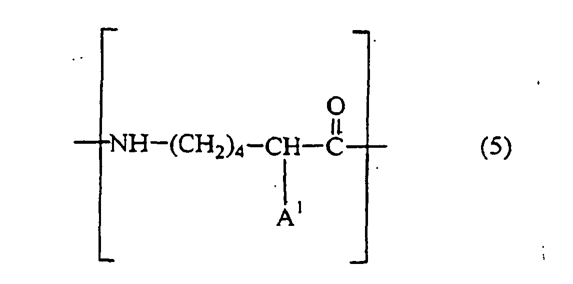 statistisch mit einer zweiten Wiederholungseinheit mit der allgemeinen Formel (6) polymerisiert wird: 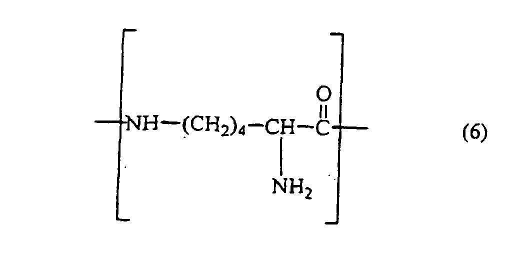 ein alternierendes Copolymer, worin die erste Wiederholungseinheit mit der zweiten Wiederholungseinheit alterniert, oder ein Blockcopolymer ist, worin ein Block, nur bestehend aus der ersten Wiederholungseinheit, mit einem anderen Block verbunden ist, der nur aus der zweiten Wiederholungseinheit besteht, wobei die Anzahl der Wiederholungen a der ersten Wiederholungseinheit eine ganze Zahl von 0 bis 50 ist, die Anzahl der Wiederholungen b der zweiten Wiederholungseinheit eine ganze Zahl von 0 bis 50 ist, a + b eine ganze Zahl von 1 bis 50 ist, A¹ eine Gruppe mit der allgemeinen Formel (2) ist:
-X-Y-Z (2)
{wobei in der allgemeinen Formel (2) X bedeutet: 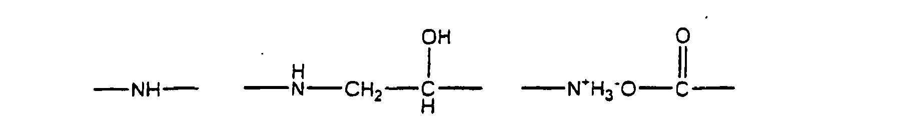 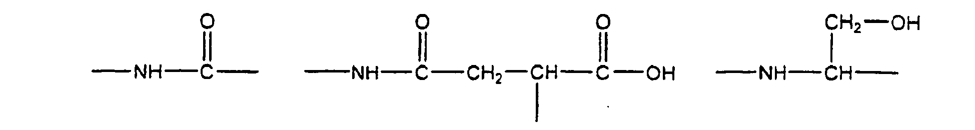 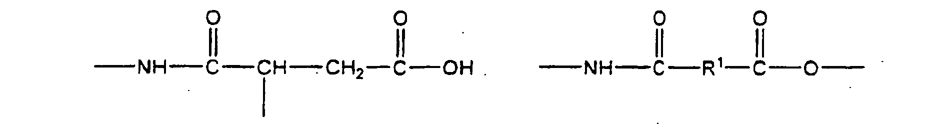 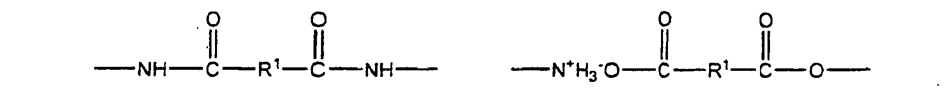 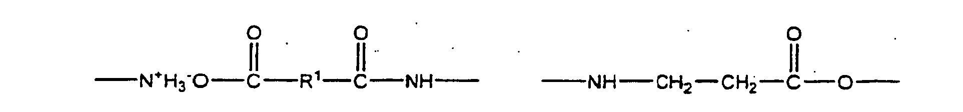 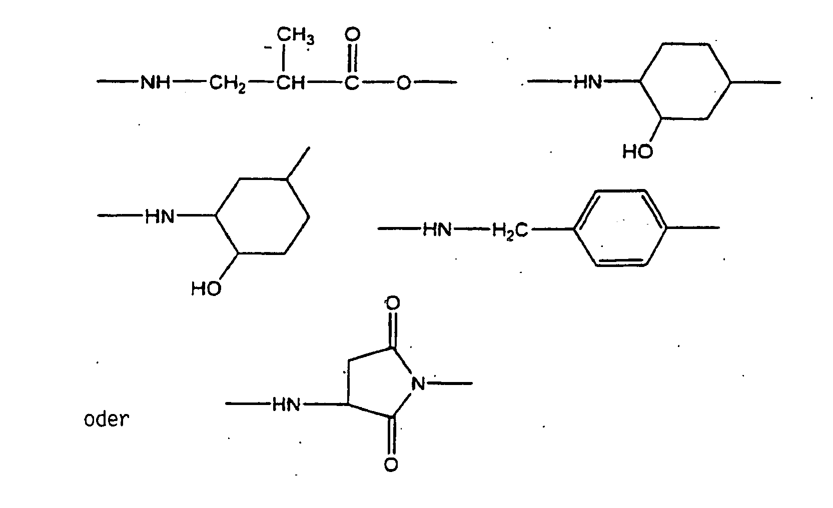 (worin R¹ eine lineare oder verzweigte Alkylengruppe mit 1 bis 5 Kohlenstoffatomen, Alkenylengruppe mit 2 bis 5 Kohlenstoffatomen oder Arylengruppe mit 6 bis 10 Kohlenstoffatomen ist), Y eine lineare oder verzweigte Alkylengruppe mit 1 bis 1.000 Kohlenstoffatomen ist, von denen irgendwelche wechselseitig nicht benachbarte Methylengruppen mit -O- (Ether) substituiert sein können, und Z eine Polyorganosiloxangruppe mit der allgemeinen Formel (3) oder (4) ist: 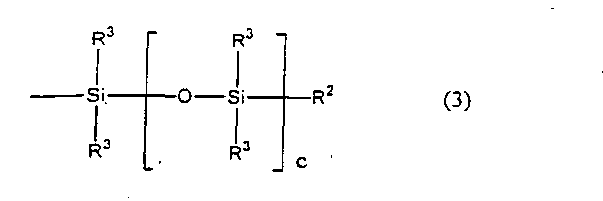 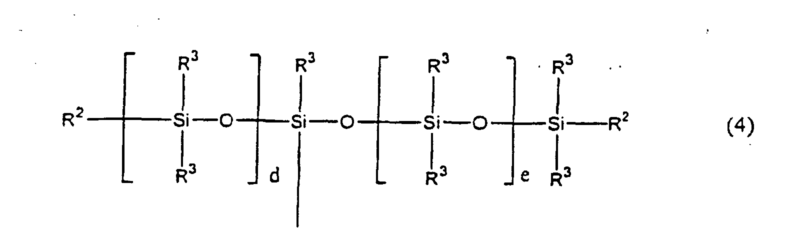 (in den allgemeinen Formeln (3) und (4) sind R² und R³ jeweils unabhängig eine lineare oder verzweigte Alkylgruppe mit 1 bis 20 Kohlenstoffatomen oder Arylgruppe mit 6 bis 10 Kohlenstoffatomen, c ist eine ganze Zahl von 1 bis 1.000, d ist eine ganze Zahl von 1 bis 1.000 und e ist eine ganze Zahl von 0 bis 1.000}, und A² und A³ jeweils unabhängig eine Aminogruppe oder eine Gruppe mit der Formel (2) sind, wobei A² und A³ nicht jeweils Aminogruppen sind, wenn a 0 ist].

48. Verfahren zur Erzeugung einer Kosmetikzusammensetzung nach einem der Ansprüche 42 bis 47, worin der mehrwertige Alkohol einer oder mehrere ist, ausgewählt aus der Gruppe, bestehend aus 1,3-Butylenglycol, Glycerin, Propylenglycol, Polyethylenglycol, Dipropylenglycol, 1,2-Pentandiol, 1,5-Pentandiol, 1,2-Hexandiol, 1,6-Hexandiol, Mannit und Sorbit.

49. Verfahren zur Erzeugung einer Kosmetikzusammensetzung nach einem der Ansprüche 42 bis 47, worin der mehrwertige Alkohol einer oder mehrere ist, ausgewählt aus der Gruppe bestehend aus Ethandiol, Diethylenglycol, Triethylenglycol, 2-Amino-2-methyl-1,3-propandiol, 1,2-Propandiol, 1,3-Propandiol, Polypropylenglycol, 1,2-Butandiol, 1,3-Butandiol, 1,4-Butandiol, Isopentandiol, Pentylenglycol, Hexylenglycol, 1,3-Pentandiol, 1,4-Pentandiol, 1,2,3-Pentantriol, 2,3,4-Pentantriol, 1,3,4-Pentantriol, 1,3,5-Pentantriol, 1,3-Hexandiol, 1,4-Hexandiol, 1,5-Hexandiol, 1,2,3-Hexantriol, 1,3,4-Hexantriol, 1,3,5-Hexantriol, 1,4,6-Hexantriol, Erythrit, Pentaerythrit, Dipentaerythrit, Threit, Arabit, Xylit, Ribit, Galactit, Lactit, Maltit, Inosit, Panthenol, Laminitol, Valienamin, Validamin und Validatol.

## Revendications

1. Composition cosmétique comprenant un ou une combinaison de deux, ou plus de deux composés d'epsilon-polylysine contenant un polyorganosiloxane ou un sel physiologiquement acceptable de ceux-ci, et un polyalcool.

2. Composition cosmétique selon la revendication 1 comprenant un ou une combinaison de deux, ou plus de deux composés d'epsilon-polylysine contenant un polyorganosiloxane représentés par la formule générale (1): 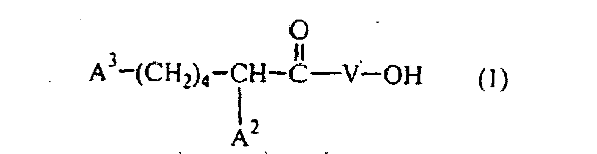 [dans la formule générale (1), V représente un copolymère statistique dans lequel un premier motif répété représenté par la formule générale (5) : 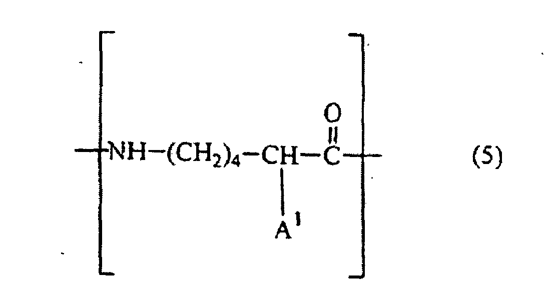 est polymérisé de manière statistique avec un second motif répété représenté par la formule générale (6): 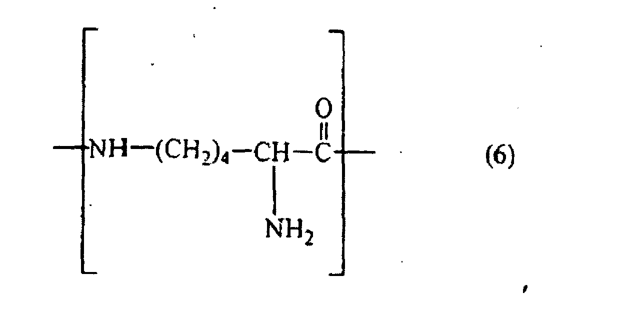 un copolymère alterné dans lequel le premier motif répété alterne avec le second motif répété, ou un copolymère séquencé dans lequel un bloc constitué uniquement des premiers motifs répétés est lié à un autre bloc constitué uniquement des seconds motifs répétés, le nombre de répétitions ***a*** du premier motif répété étant un nombre entier de 0 à 50, le nombre de répétitions ***b*** du second motif répété étant un nombre entier de 0 à 50, ***a*** + ***b*** étant égal à un nombre entier de 1 à 50, A¹ représente un groupe représenté par la formule générale (2) :
-X-Y-2 (2)
{dans la formule générale (2), ***X*** représente 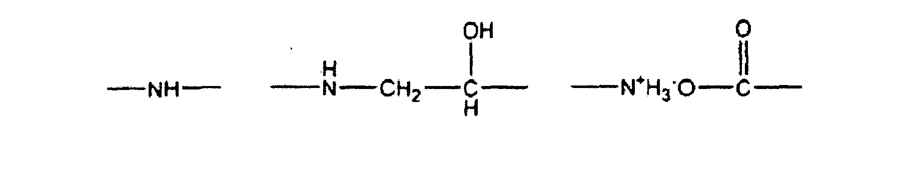 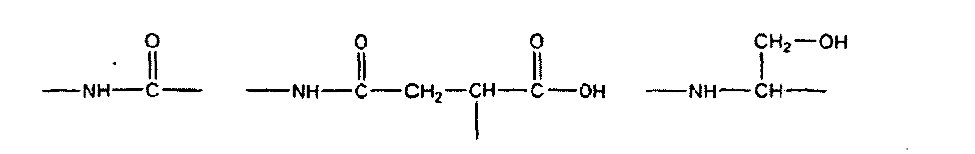 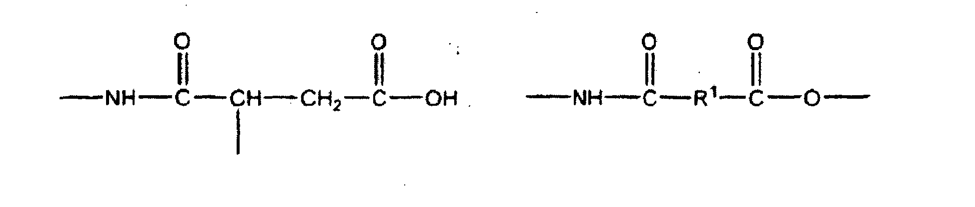 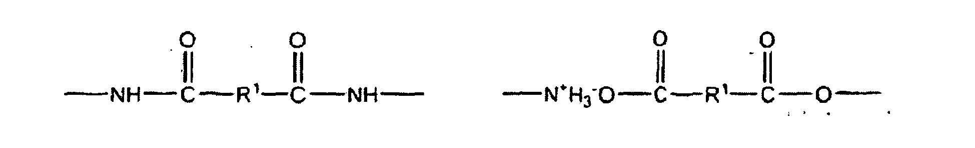 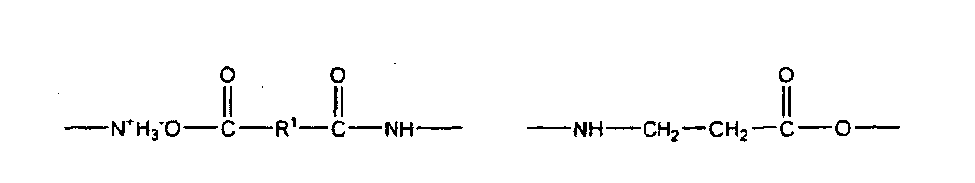 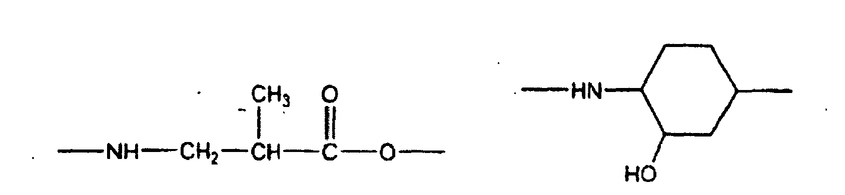 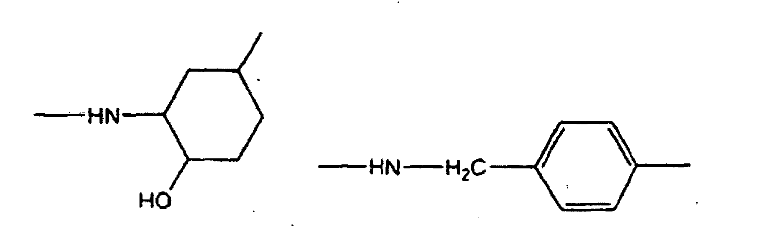 ou 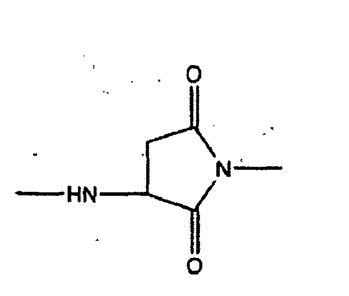 (où R¹ représente un groupe alkylène linéaire ou ramifié ayant 1 à 5 atomes de carbone, un groupe alcénylène ayant 2 à 5 atomes de carbone ou un groupe arylène ayant 6 à 10 atomes de carbone), ***Y*** représente un groupe alkylène linéaire ou ramifié ayant 1 à 1000 atomes de carbone, dont n'importe quels groupes méthylène mutuellement non adjacents peuvent être substitués avec -O- (éther), et ***Z*** représente un groupe polyorganosiloxane représenté par la formule générale (3) ou (4) : 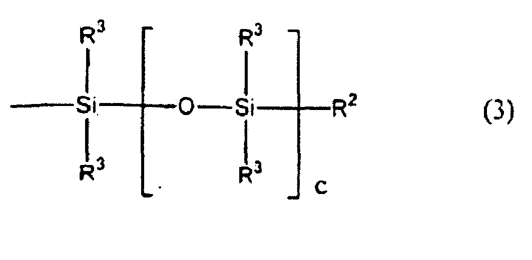 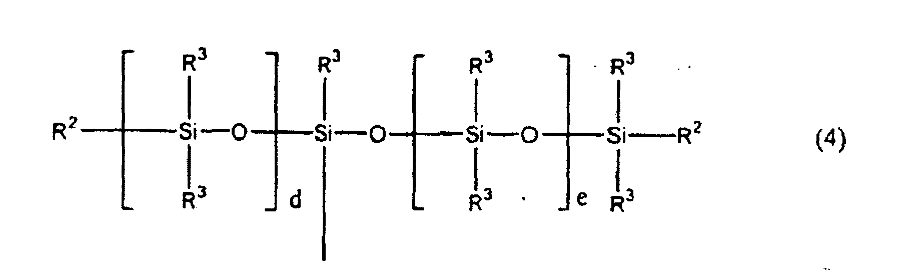 (dans les formules générales (3) et (4), R² et R³ sont chacun indépendamment un groupe alkyle linéaire ou ramifié ayant 1 à 20 atomes de carbone ou un groupe aryle ayant 6 à 10 atomes de carbone, ***c*** est un nombre entier de 1 à 1000, ***d*** est un nombre entier de 1 à 1000, et e est un nombre entier de 0 à 1000)}, et A² et A³ sont chacun indépendamment un groupe amino ou un groupe représenté par la formule générale (2) mentionnée ci-dessus, A² et A³ ne sont pas les deux à la fois un groupe amino lorsque ***a*** est égal à 0] ou un sel physiologiquement acceptable de ceux-ci, et un polyalcool.

3. Composition cosmétique telle que revendiquée dans la revendication 2, comprenant un ou une combinaison de deux, ou plus de deux composés d'epsilon-polylysine contenant un polyorganosiloxane ou un sel physiologiquement acceptable de ceux-ci, dans laquelle le nombre de répétition ***b*** du second motif répété est 5 ou plus grand, le second motif répété étant représenté par la formule générale (6) : 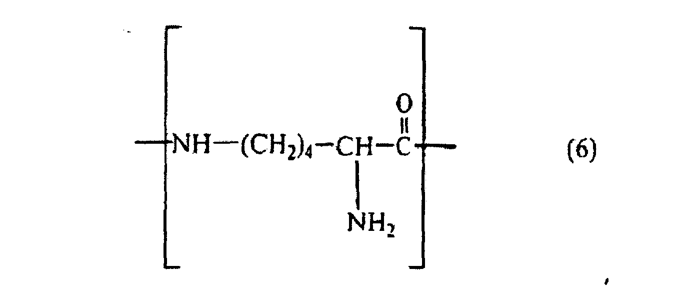 les seconds motifs répétés formant le composé d'epsilon-polylysine contenant un polyorganosiloxane.

4. Composition cosmétique telle que revendiquée dans la revendication 2 ou 3, dans laquelle le nombre de répétitions ***a*** du premier motif répété et le nombre de répétitions ***b*** du second motif répété satisfont l'inéquation 0,1 ≤ ***b***/(***a*** + ***b***) ≤ 0,98, le premier motif répété étant représenté par la formule générale (5) : 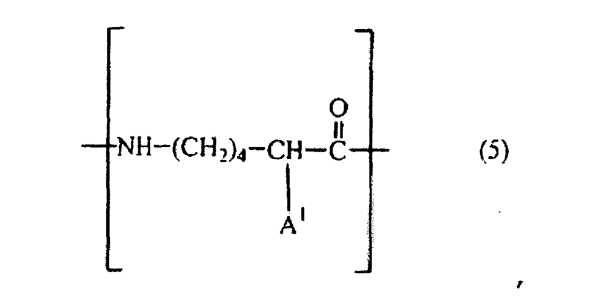 le second motif répété étant représenté par la formule générale (6) : 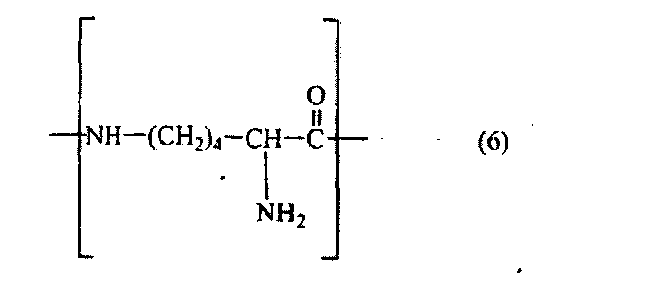 les premier et second motifs répétés formant le composé d'epsilon-polylysine contenant un polyorganosiloxane.

5. Composition cosmétique telle que revendiquée dans l'une quelconque des revendications 2 - 4, dans laquelle ***X*** dans le groupe représenté par la formule générale (2) :
-X-Y-Z (2)
formant le composé d'epsilon-polylysine contenant un polyorganosiloxane est un groupe représenté par :

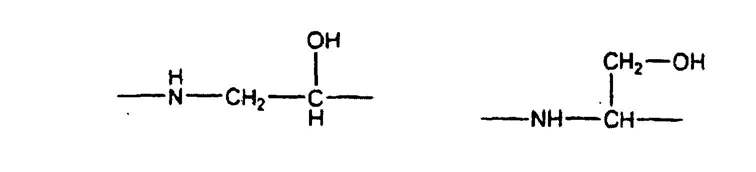

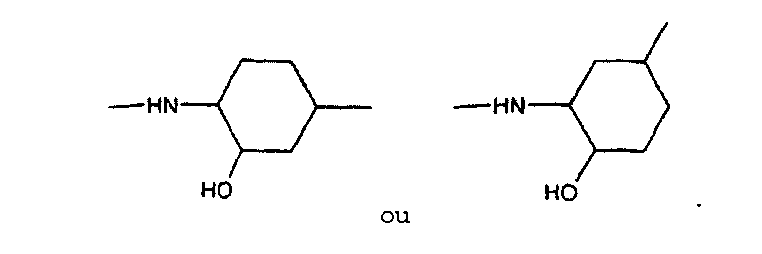

6. Composition cosmétique telle que revendiquée dans l'une quelconque des revendications 2 - 4, dans laquelle ***X*** dans le groupe représenté par la formule générale (2) :
-X-Y-Z (2)
formant le composé d'epsilon-polylysine contenant un polyorganosiloxane est un groupe représenté par : 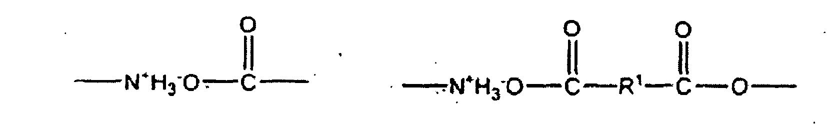 ou 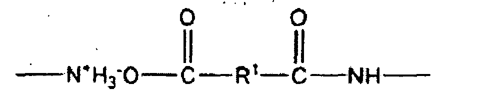 (dans ***X*** dans la formule générale (2), R¹ représente un groupe alkylène linéaire ou ramifié ayant 1 à 5 atomes de carbone, un groupe alcénylène ayant 2 à 5 atomes de carbone ou un groupe arylène ayant 6 à 10 atomes de carbone).

7. Composition cosmétique telle que revendiquée dans l'une quelconque des revendications 2 - 4, dans laquelle ***X*** dans le groupe représenté par la formule générale (2) :
-X-Y-Z (2)
formant le composé d'epsilon-polylysine contenant un polyorganosiloxane est un groupe représenté par : 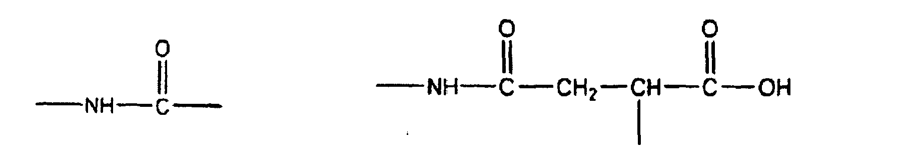 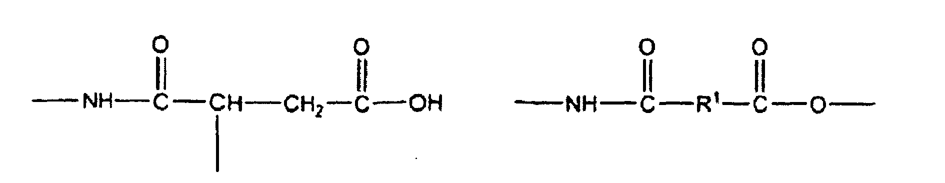 ou 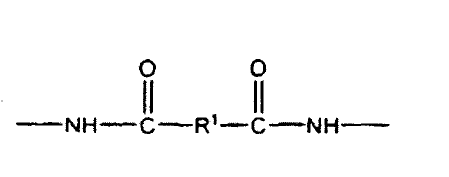 (dans ***X*** dans la formule générale (2), R¹ représente un groupe alkylène linéaire ou ramifié ayant 1 à 5 atomes de carbone, un groupe alcénylène ayant 2 à 5 atomes de carbone ou un groupe arylène ayant 6 à 10 atomes de carbone).

8. Composition cosmétique telle que revendiquée dans l'une quelconque des revendications 2 - 4, dans laquelle ***X*** dans le groupe représenté par la formule générale (2) :
-X-Y-Z (2)
formant le composé d'epsilon-polylysine contenant un polyorganosiloxane est un groupe représenté par :

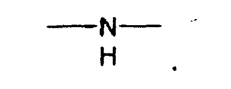

9. Composition cosmétique telle que revendiquée dans l'une quelconque des revendications 2 - 4, dans laquelle **X** dans le groupe représenté par la formule générale (2) :
-X-Y-Z (2)
formant le composé d'epsilon-polylysine contenant un polyorganosiloxane est un groupe représenté par : 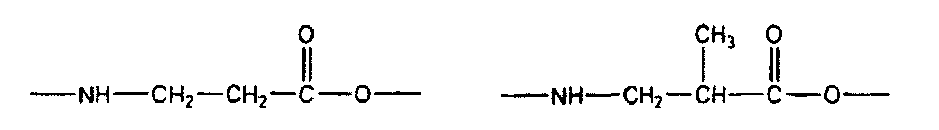 ou

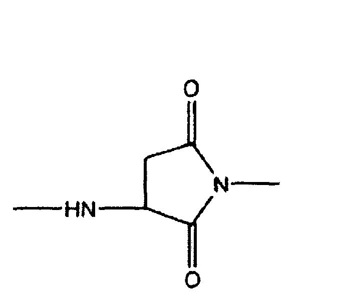

10. Composition cosmétique telle que revendiquée dans l'une quelconque des revendications 1 - 9, dans laquelle le polyalcool est un ou plusieurs polyalcools choisis dans le groupe constitué par le 1,3-butylène glycol, la glycérine, le propylène glycol, le polyéthylène glycol, le dipropylène glycol, le 1,2-pentanediol, le 1,5-pentanediol, le 1,2-hexanediol, le 1,6-hexanediol, le mannitol et le sorbitol.

11. Composition cosmétique telle que revendiquée dans l'une quelconque des revendications 1 - 9, dans laquelle le polyalcool est un ou plusieurs polyalcools choisis dans le groupe constitué par l'éthanediol, le diéthylène glycol, le triéthylène glycol, le 2-amino-2-méthyl-1,3-propanediol, le 1,2-propanediol, le 1,3-propanediol, le polypropylène glycol, le 1,2-butanediol, le 1,3-butanediol, le 1,4-butanediol, l'isopentanediol, le pentylène glycol, l'hexylène glycol, le 1,3-pentanediol, le 1,4-pentanediol, le 1,2,3-pentanetriol, le 2,3,4-pentanetriol, le 1,3,4-pentanetriol, le 1,3,5-pentanetriol, le 1,3-hexanediol, le 1,4-hexanediol, le 1,5-hexanediol, le 1,2,3-hexanetriol, le 1,3,4-hexanetriol, le 1,3,5-hexanetriol, le 1,4,6-hexanetriol, l'érythritol, le pentaérythritol, le dipentaérythritol, le thréitol, l'arabitol, le xylitol, le ribitol, le galactitol, le lactitol, le maltitol, l'inositol, le panthénol, le laminitol, la valiénamine, la validamine et le validatol.

12. Composition cosmétique telle que revendiquée dans l'une quelconque des revendications 1 - 11, comprenant 0,001 % à 20 % en poids des composés d'epsilon-polylysine contenant un polyorganosiloxane ou un sel physiologiquement acceptable de ceux-ci.

13. Composition cosmétique telle que revendiquée dans l'une quelconque des revendications 1 à 12, dans laquelle la composition cosmétique est un agent de traitement pour les cheveux.

14. Composition cosmétique telle que revendiquée dans la revendication 13, dans laquelle l'agent de traitement pour les cheveux est un produit de coiffure, un shampoing, un produit de rinçage de finition, un produit de traitement pour les cheveux, une crème pour les cheveux, une mousse pour les cheveux, une lotion de mise en plis, une teinture pour les cheveux, un colorant pour les cheveux, une solution à permanente, un améliorateur du flux sanguin, une lotion pour le cuir chevelu ou un agent contre la perte des cheveux.

15. Composition cosmétique telle que revendiquée dans l'une quelconque des revendications 1 - 12, dans laquelle la composition cosmétique est un produit cosmétique pour les soins de la peau.

16. Composition cosmétique telle que revendiquée dans la revendication 15, dans laquelle le produit cosmétique pour les soins de la peau est un tonique, un sérum, un tonique décolorant, une lotion de lait, une lotion de lait décolorante, une crème, une crème décolorante, un onguent, un onguent décolorant, une lotion, une lotion décolorante, une huile ou un nécessaire pour le visage.

17. Composition cosmétique telle que revendiquée dans l'une quelconque des revendications 1 - 12, dans laquelle la composition cosmétique est un produit cosmétique pour le maquillage.

18. Composition cosmétique telle que revendiquée dans la revendication 17, dans laquelle le produit cosmétique pour le maquillage est un fond de teint, un fond de teint liquide, un rouge à lèvres, un brillant à lèvres, une ombre à paupières, une poudre, une poudre pour le visage, un fard à joues, une ombre à paupières, un eye-liner, un mascara ou un crayon à sourcil.

19. Composition cosmétique telle que revendiquée dans l'une quelconque des revendications 1-12, dans laquelle la composition cosmétique est un produit de nettoyage pour la peau.

20. Composition cosmétique telle que revendiquée dans la revendication 19, dans laquelle le produit de nettoyage pour la peau est un savon, une crème nettoyante, une lotion nettoyante, un lait nettoyant, une composition cosmétique, un nettoyant pour le visage ou un shampoing pour le corps.

21. Composition cosmétique telle que revendiquée dans l'une quelconque des revendications 1 - 12, dans laquelle la composition cosmétique est un agent pour le bain.

22. Composition cosmétique telle que revendiquée dans l'une quelconque des revendications 1 - 12, dans laquelle la composition cosmétique est un produit cosmétique de finition.

23. Composition cosmétique telle que revendiquée dans l'une quelconque des revendications 1 - 12, dans laquelle la composition cosmétique est un patch.

24. Composition cosmétique selon la revendication 1 comprenant un ou une combinaison de deux, ou plus de deux composés d'epsilon-polylysine contenant un polyorganosiloxane
ou un sel physiologiquement acceptable de ceux-ci, et un polyalcool, le composé d'epsilon-polylysine contenant un polyorganosiloxane étant obtenu en faisant réagir une epsilon-polylysine avec un polyorganosiloxane.

25. Composition cosmétique telle que revendiquée dans la revendication 24, dans laquelle la epsilon-polylysine est représentée par la formule générale (7) : 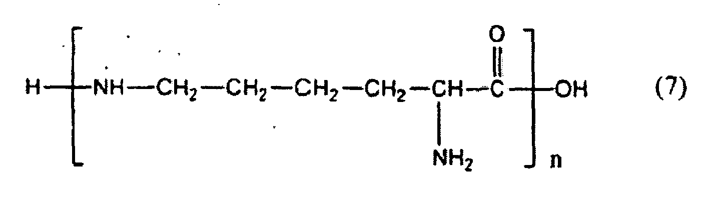 (dans laquelle n est un nombre entier de 2 à 51).

26. Composition cosmétique telle que revendiquée dans la revendication 24 ou 25, dans laquelle le polyorganosiloxane a un groupe fonctionnel qui est réactif avec un groupe amino dans la epsilon-polylysine.

27. Composition cosmétique telle que revendiquée dans l'une quelconque des revendications 24 - 26, dans laquelle le polyorganosiloxane est un polyorganosiloxane ayant un groupe époxy, un polyorganosiloxane ayant un acide carboxylique ou un dérivé d'acide carboxylique, un polyorganosiloxane ayant un groupe alkyle halogéné ou un polyorganosiloxane ayant un group insaturé.

28. Composition cosmétique telle que revendiquée dans l'une quelconque des revendications 24 - 27, dans laquelle le polyorganosiloxane est un polyorganosiloxane représenté par la formule générale (8) :
Q-Y-Z (8)
[dans la formule générale (8), Q représente 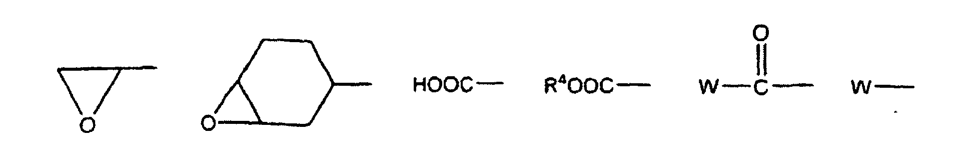 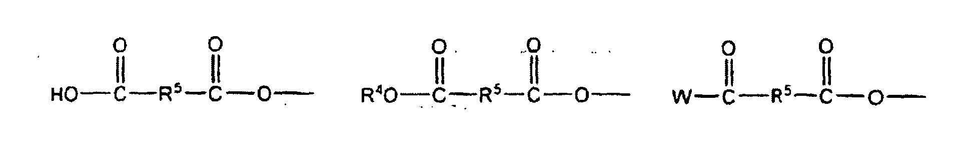 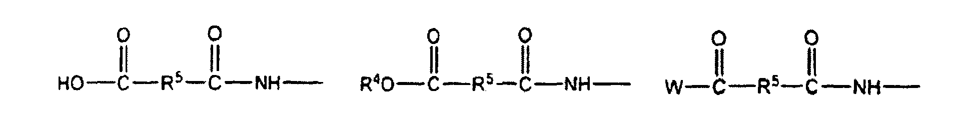 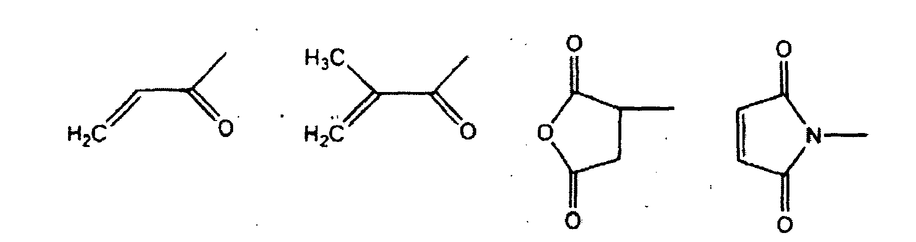 ou 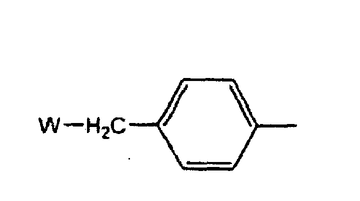 (où R⁴ représente un groupe alkyle linéaire ou ramifié ayant 1 à 20 atomes de carbone ou un groupe aryle ayant 6 à 10 atomes de carbone ou un groupe triméthylsilyle, R⁵ représente un groupe alkylène linéaire ou ramifié ayant 1 à 5 atomes de carbone, un groupe alcénylène ayant 2 à 5 atomes de carbone ou un groupe arylène ayant 6 à 10 atomes de carbone,
***W*** représente un chlore, un brome ou un iode), ***Y*** représente un groupe alkylène linéaire ou ramifié ayant 1 à 1000 atomes de carbone, dont n'importe quels groupes méthylène mutuellement non adjacents peuvent être substitués avec -O- (éther), et ***Z*** représente un groupe polyorganosiloxane représenté par la formule générale (3) ou (4) : 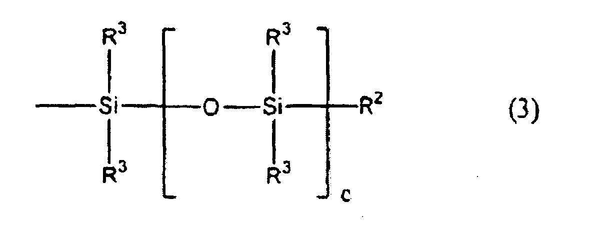 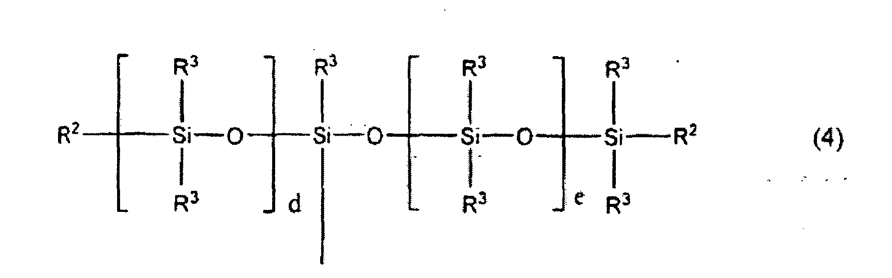 (dans les formules générales (3) et (4), R² et R³ sont chacun indépendamment un groupe alkyle linéaire ou ramifié ayant 1 à 20 atomes de carbone ou un groupe aryle ayant 6 à 10 atomes de carbone, ***c*** est un nombre entier de 1 à 1000, ***d*** est un nombre entier de 1 à 1000, et e est un nombre entier de 0 à 1000)].

29. Composition cosmétique telle que revendiquée dans l'une quelconque des revendications 24 - 28, dans laquelle le polyalcool est un ou plusieurs polyalcools choisis dans le groupe constitué par le 1,3-butylène glycol, la glycérine, le propylène glycol, le polyéthylène glycol, le dipropylène glycol, le 1,2-pentanediol, le 1,5-pentanediol, le 1,2-hexanediol, le 1,6-hexanediol, le mannitol et le sorbitol.

30. Composition cosmétique telle que revendiquée dans l'une quelconque des revendications 24 - 28, dans laquelle le polyalcool est un ou plusieurs polyalcools choisis dans le groupe constitué par l'éthanediol, le diéthylène glycol, le triéthylène glycol, le 2-amino-2-méthyl-1,3-propanediol, le 1,2-propanediol, le 1,3-propanediol, le polypropylène glycol, le 1,2-butanediol, le 1,3-butanediol, le 1,4-butanediol, l'isopentanediol, le pentylène glycol, l'hexylène glycol, le 1,3-pentanediol, le 1,4-pentanediol, le 1,2,3-pentanetriol, le 2,3,4-pentanetriol, le 1,3,4-pentanetriol, le 1,3,5-pentanetriol, le 1,3-hexanediol, le 1,4-hexanediol, le 1,5-hexanediol, le 1,2,3-hexanetriol, le 1,3,4-hexanetriol, le 1,3,5-hexanetriol, 1,4,6-hexanetriol, l'érythritol, le pentaérythritol, le dipentaérythritol, le thréitol, l'arabitol, le xylitol, le ribitol, le galactitol, le lactitol, le maltitol, l'inositol, le panthénol, le laminitol, la valiénamine, la validamine et le validatol.

31. Composition cosmétique telle que revendiquée dans l'une quelconque des revendications 24 à 30, dans laquelle la composition cosmétique est un agent de traitement pour les cheveux.

32. Composition cosmétique telle que revendiquée dans la revendication 31, dans laquelle l'agent de traitement pour les cheveux est un produit de coiffure, un shampoing, un produit de rinçage de finition, un produit de traitement pour les cheveux, une crème pour les cheveux, une mousse pour les cheveux, une lotion de mise en plis, une teinture pour les cheveux, un colorant pour les cheveux, une solution à permanente, un améliorateur du flux sanguin, une lotion pour le cuir chevelu ou un agent contre la perte des cheveux.

33. Composition cosmétique telle que revendiquée dans l'une quelconque des revendications 24 - 30, dans laquelle la composition cosmétique est un produit cosmétique pour les soins de la peau.

34. Composition cosmétique telle que revendiquée dans la revendication 33, dans laquelle le produit cosmétique pour les soins de la peau est un tonique, un sérum, un tonique décolorant, une lotion de lait, une lotion de lait décolorante, une crème, une crème décolorante, un onguent, un onguent décolorant, une lotion, une lotion décolorante, une huile ou un nécessaire pour le visage.

35. Composition cosmétique telle que revendiquée dans l'une quelconque des revendications 24 - 30, dans laquelle la composition cosmétique est un produit cosmétique pour le maquillage.

36. Composition cosmétique telle que revendiquée dans la revendication 35, dans laquelle le produit cosmétique pour le maquillage est un fond de teint, un fond de teint liquide, un rouge à lèvres, un brillant à lèvres, une ombre à paupières, une poudre, une poudre pour le visage, un fard à joues, une ombre à paupières, un eye-liner, un mascara ou un crayon à sourcil.

37. Composition cosmétique telle que revendiquée dans l'une quelconque des revendications 24 - 30, dans laquelle la composition cosmétique est un produit de nettoyage pour la peau.

38. Composition cosmétique telle que revendiquée dans la revendication 37, dans laquelle le produit de nettoyage pour la peau est un savon, une crème nettoyante, une lotion nettoyante, un lait nettoyant, une composition cosmétique, un nettoyant pour le visage ou un shampoing pour le corps.

39. Composition cosmétique telle que revendiquée dans l'une quelconque des revendications 24 - 30, dans laquelle la composition cosmétique est un agent pour le bain.

40. Composition cosmétique telle que revendiquée dans l'une quelconque des revendications 24 - 30, dans laquelle la composition cosmétique est un produit cosmétique de finition.

41. Composition cosmétique telle que revendiquée dans l'une quelconque des revendications 24 - 30, dans laquelle la composition cosmétique est un patch.

42. Procédé de production d'une composition comprenant :
de mélanger un ou une combinaison de deux, ou plus de deux composés d'epsilon-polylysine contenant un polyorganosiloxane ou un sel physiologiquement acceptable de ceux-ci, le composé d'epsilon-polylysine contenant un polyorganosiloxane étant obtenu en en faisant réagir une epsilon-polylysine avec un polyorganosiloxane, avec un polyalcool.

43. Procédé de production d'une composition cosmétique tel que revendiqué dans la revendication 42, dans lequel la epsilon-polylysine est représentée par la formule générale (7) : 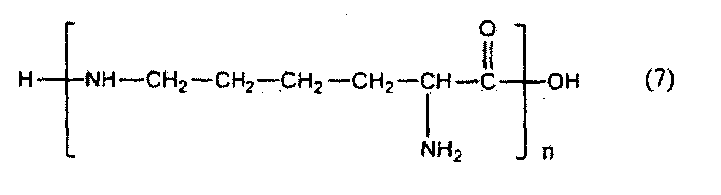 (dans laquelle n est un nombre entier de 2 à 51).

44. Procédé de production d'une composition cosmétique tel que revendiqué dans la revendication 42 ou 43, dans lequel le polyorganosiloxane a un groupe fonctionnel qui est réactif avec un groupe amino dans la epsilon-polylysine.

45. Procédé de production d'une composition cosmétique tel que revendiqué dans l'une quelconque des revendications 42 - 44, dans lequel le polyorganosiloxane est un polyorganosiloxane ayant un groupe époxy, un polyorganosiloxane ayant un acide carboxylique ou un dérivé d'acide carboxylique, un polyorganosiloxane ayant un groupe alkyle halogéné ou un polyorganosiloxane ayant un groupe insaturé.

46. Procédé de production d'une composition cosmétique tel que revendiqué dans l'une quelconque des revendications 42 - 45, dans lequel le polyorganosiloxane est un polyorganosiloxane représenté par la formule générale (8) :
Q-Y-Z (8) (8)
[dans la formule générale (8), Q représente 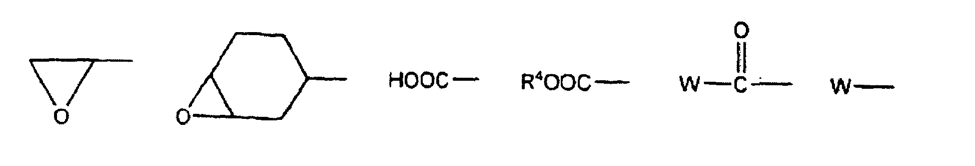 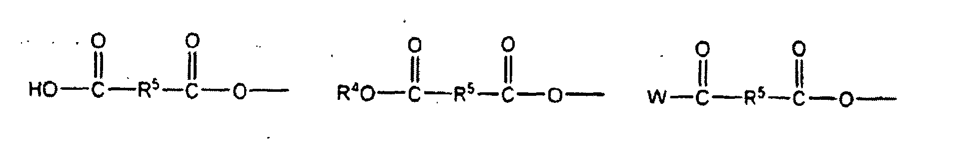 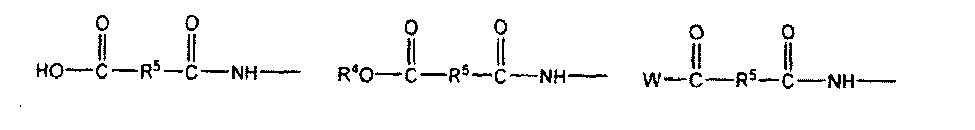 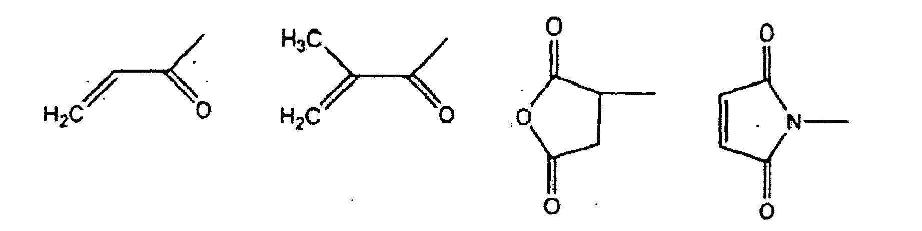 ou 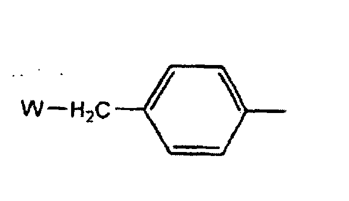 (où R⁴ représente un groupe alkyle linéaire ou ramifié ayant 1 à 20 atomes de carbone ou un groupe aryle ayant 6 à 10 atomes de carbone ou un groupe triméthylsilyle, R⁵ représente un groupe alkylène linéaire ou ramifié ayant 1 à 5 atomes de carbone, un groupe alcénylène ayant 2 à 5 atomes de carbone ou un groupe arylène ayant 6 à 10 atomes de carbone, ***W*** représente un chlore, un brome ou un iode), ***Y*** représente un groupe alkylène linéaire ou ramifié ayant 1 à 1000 atomes de carbone, dont n'importe quels groupes méthylène mutuellement non adjacents peuvent être substitué avec -O- (éther), et ***Z*** représente un polyorganosiloxane représenté par la formule générale (3) ou (4) : 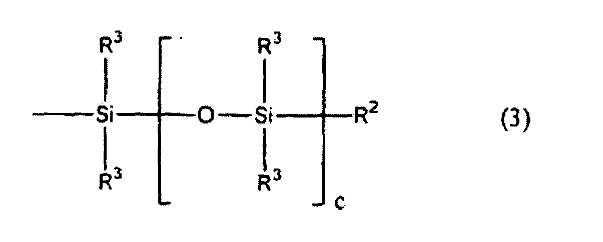 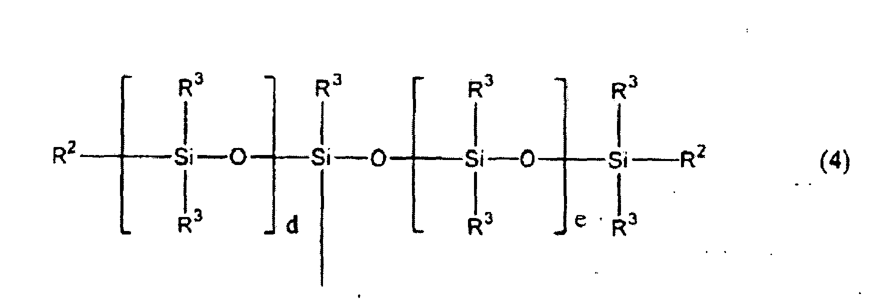 (dans les formules générales (3) et (4), R² et R³ sont chacun indépendamment un groupe alkyle linéaire ou ramifié ayant 1 à 20 atomes de carbone ou un groupe aryle ayant 6 à 10 atomes de carbone, ***c*** est un nombre entier de 1 à 1000, ***d*** est un nombre entier de 1 à 1000, et e est un nombre entier de 0 à 1000)].

47. Procédé de production d'une composition cosmétique tel que revendiqué dans l'une quelconque des revendications 42 - 46, dans lequel le composé d'epsilon-polylysine contenant un polyorganosiloxane est représenté par la formule générale (1) : 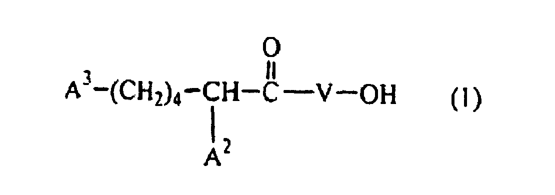 [dans la formule générale (1), ***V*** représente un copolymère statistique dans lequel un premier motif répété représenté par la formule générale (5) : 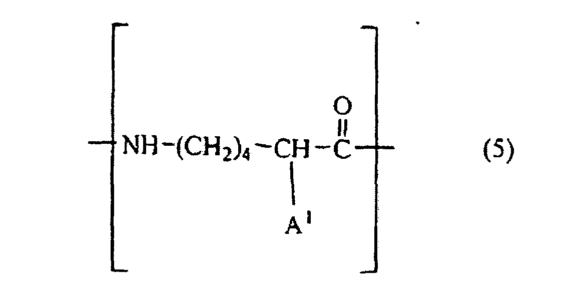 est polymérisé de manière statistique avec un second motif répété représenté par la formule générale (6) : 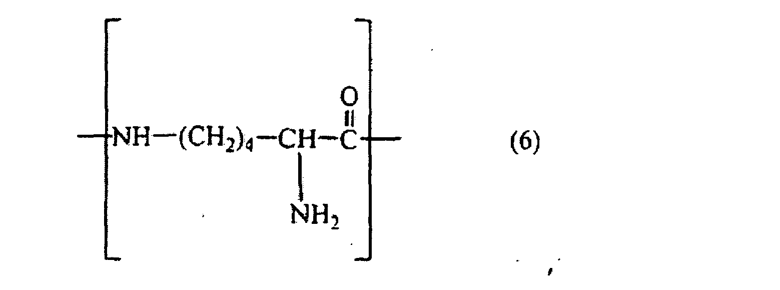 un copolymère alterné dans lequel le premier motif répété alterne avec le second motif répété, ou un copolymère séquencé dans lequel un bloc constitué uniquement des premiers motifs répétés est lié à un autre bloc constitué uniquement des second motifs répétés, le nombre de répétitions ***a*** du premier motif répété étant un nombre entier de 0 à 50, le nombre de répétitions ***b*** du second motif répété étant un nombre entier de 0 à 50, ***a*** + ***b*** étant égal à un nombre entier de 1 à 50, A¹ représente un groupe représenté par la formule générale (2) :
-X-Y-Z (2)
{dans la formule générale (2), ***X*** représente 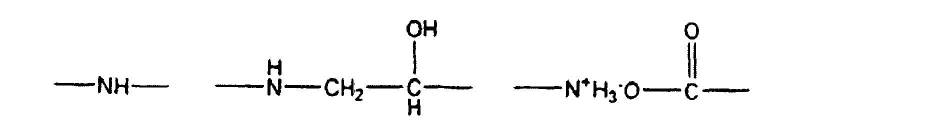 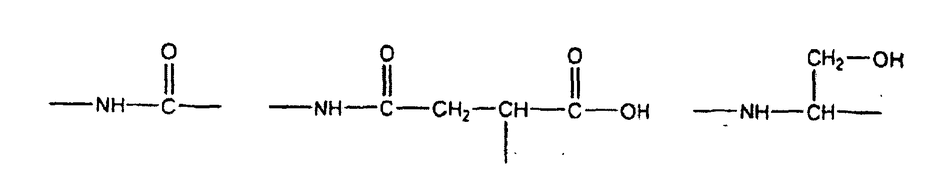 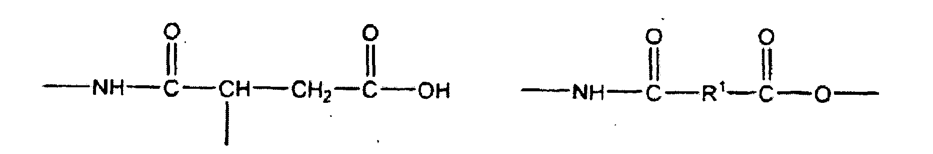 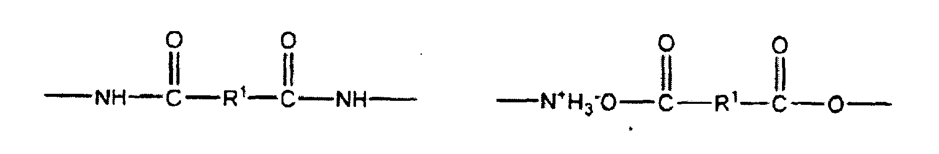 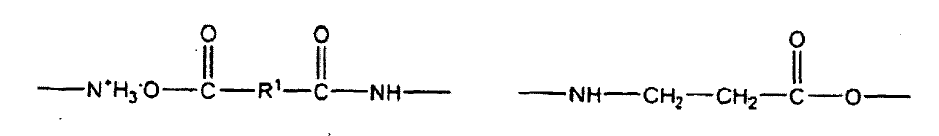 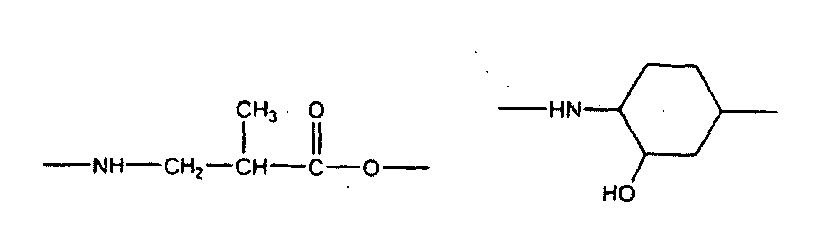 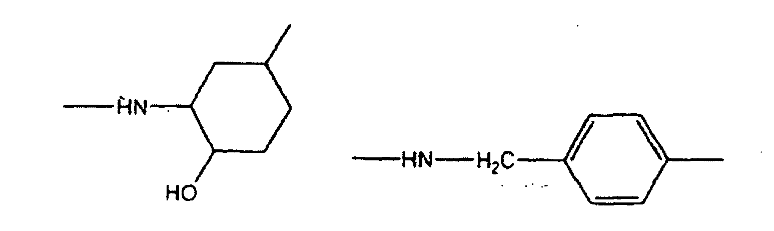 ou 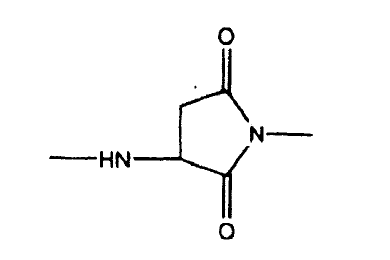 (où R¹ représente un groupe alkylène linéaire ou ramifié ayant 1 à 5 atomes de carbone, un groupe alcénylène ayant 2 à 5 atomes de carbone ou un groupe arylène ayant 6 à 10 atomes de carbone), ***Y*** représente un groupe alkylène linéaire ou ramifié ayant 1 à 1000 atomes de carbone, dont n'importe quels groupes méthylène mutuellement non adjacents peuvent être substitués avec -O- (éther), et ***Z*** représente un groupe polyorganosiloxane représenté par la formule générale (3) ou (4) : 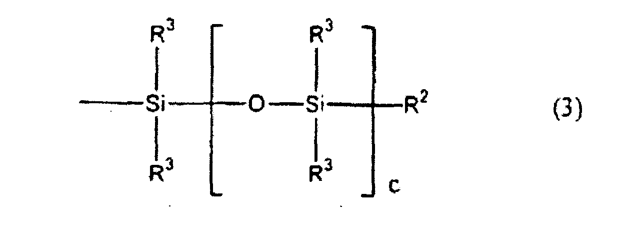 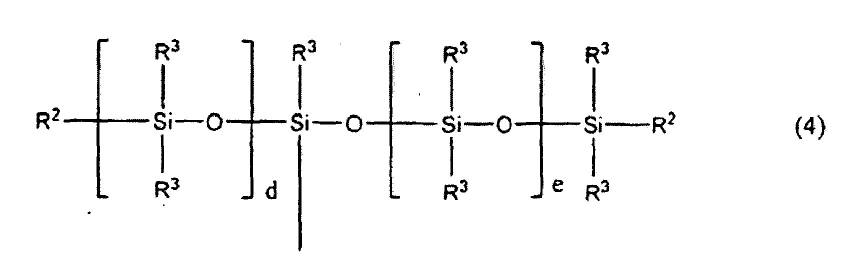 (dans les formules générales (3) et (4), R² et R³ sont chacun indépendamment un groupe alkyle linéaire ou ramifié ayant 1 à 20 atomes de carbone ou un groupe aryle ayant 6 à 10 atomes de carbone, ***c*** est un nombre entier de 1 à 1000, ***d*** est un nombre entier de 1 à 1000, et ***e*** est un nombre entier de 0 à 1000)}, et A² et A³ sont chacun indépendamment un groupe amino ou un groupe représenté par la formule générale (2) mentionnée ci-dessus, A² et A³ ne sont pas les deux à la fois un groupe amino lorsque ***a*** est égal à 0].

48. Procédé de production d'une composition cosmétique tel que revendiqué dans l'une quelconque des revendications 42 - 47; dans lequel le polyalcool est un ou plusieurs polyalcools choisis dans le groupe constitué par le 1,3-butylène glycol, la glycérine, le propylène glycol, le polyéthylène glycol, le dipropylène glycol, le 1,2-pentanediol, le 1,5-pentanediol, le 1,2-hexanediol, le 1,6-hexanediol, le mannitol et le sorbitol.

49. Procédé de production d'une composition cosmétique tel que revendiqué dans l'une quelconque des revendications 42 - 47, dans lequel le polyalcool est un ou plusieurs polyalcools choisis dans le groupe constitué par l'éthanediol, le diéthylène glycol, le triéthylène glycol, le 2-amino-2-méthyl-1,3-propanediol, le 1,2-propanediol, le 1,3-propanediol, le polypropylène glycol, le 1,2-butanediol, le 1,3-butanediol, le 1,4-butanediol, l'isopentanediol, le pentylène glycol, l'hexylène glycol, le 1,3-pentanediol, le 1,4-pentanediol, le 1,2,3-pentanetriol, le 2,3,4-pentanetriol, le 1,3,4-pentanetriol, le 1,3,5-pentanetriol, le 1,3-hexanediol, le 1,4-hexanediol, le 1,5-hexanediol, le 1,2,3-hexanetriol, le 1,3,4-hexanetriol, le 1,3,5-hexanetriol, le 1,4,6-hexanetriol, l'érythritol, le pentaérythritol, le dipentaérythritol, le thréitol, l'arabitol, le xylitol, le ribitol, le galactitol, le lactitol, le maltitol, l'inositol, le panthénol, le laminitol, la valiénamine, la validamine et le validatol.
